(19) Europäisches Patentamt
European Patent Office
Office européen des brevets

(11) EP 4 140 481 A1

(12) EUROPEAN PATENT APPLICATION

(43) Date of publication:
**01.03.2023 Bulletin 2023/09**

(21) Application number: **21193388.2**

(22) Date of filing: **26.08.2021**

(51) International Patent Classification (IPC):
***A61K 31/404*** *(2006.01)* ***A61K 31/4045*** *(2006.01)*
***A61K 31/437*** *(2006.01)* ***A61K 31/454*** *(2006.01)*
***A61K 31/496*** *(2006.01)* ***A61P 25/28*** *(2006.01)*
***A61K 31/519*** *(2006.01)* ***A61K 31/5377*** *(2006.01)*
***C07D 209/42*** *(2006.01)* ***C07D 295/13*** *(2006.01)*
***C07D 401/12*** *(2006.01)* ***C07D 403/06*** *(2006.01)*
***C07D 413/12*** *(2006.01)*

(52) Cooperative Patent Classification (CPC):
**A61K 31/404; A61K 31/4045; A61K 31/437; A61K 31/454; A61K 31/496; A61K 31/519; A61K 31/5377; A61P 25/28; C07D 209/42; C07D 295/13; C07D 401/12; C07D 403/06; C07D 413/12**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Galyan Bio, Inc.**
**Cambridge, MA 02142 (US)**

(72) Inventors:
- **GALYAN, Simon Marius**
**Cambridge, 02142 (US)**
- **JUDD, Duncan Bruce**
**Cambridge, 02142 (US)**

(74) Representative: **Kuttenkeuler, David**
**SKM-IP PartGmbB**
**Oberanger 45**
**80331 München (DE)**

Remarks:
•The complete document including Reference Table(s) and the Sequence Listing(s) can be downloaded from the EPO website
•Claims filed after the date of filing of the application (Rule 68(4) EPC).

# (54) PROTEIN-OLIGOMER BINDING AGENTS AND THERAPEUTIC USES THEREOF

(57) The present invention is based on the finding that the compounds disclosed herein bind to, preferably harmful, protein-oligomers and thereby induce a conformational change of the latter. This conformational change enhances the recognition of the protein oligomers by cellular degradation mechanisms, such as autophagy and the ubiquitin proteasome system which in turn causes an increase degradation of such, usually undesireous, protein-oligomers. Hence, in a first aspect, the invention pertains to a protein-oligomer aggregation-agent, wherein the protein-oligomer aggregation-agent comprises, or is selected from, a small molecule, an antibody, a nanoparticle, a nucleic acid, a peptide and/or a therapeutic protein. The invention provides therapeutic application of the disclosed compounds in the treatment and/or prevention of disorders associated with the presence of one or more of such oligomeric structures, preferably of protein-oligomers. The invention intends to induce aggregation of harmful oligomers and thereby induce cellular systemic mechansims that recognize and degrade structurally larger aggregates.

EP 4 140 481 A1

## Description

### FIELD OF THE INVENTION

The present invention is based on the finding that the compounds disclosed herein bind to, preferably harmful, protein-oligomers and thereby induce a conformational change of the latter. This conformational change enhances the recognition of the protein oligomers by cellular degradation mechanisms, such as autophagy and the ubiquitin proteasome system which in turn causes an increase degradation of such, usually undesireous, protein-oligomers. Hence, in a first aspect, the invention pertains to a protein-oligomer aggregation-agent, wherein the protein-oligomer aggregation-agent comprises, or is selected from, a small molecule, an antibody, a nanoparticle, a nucleic acid, a peptide and/or a therapeutic protein. The invention provides therapeutic application of the disclosed compounds in the treatment and/or prevention of disorders associated with the presence of one or more of such oligomeric structures, preferably of protein-oligomers. The invention intends to induce aggregation of harmful oligomers and thereby induce cellular systemic mechansims that recognize and degrade structurally larger aggregates.

### DESCRIPTION

Neurodegeneration is a feature of many debilitating, seemingly incurable diseases that are rapidly rising in prevalence (Gitler et al., 2017). These diseases are characterized by progressive loss of selectively vulnerable populations of neurons. Neurodegenerative diseases can be classified according to primary clinical features (e.g., dementia, Parkinsonism, or motor neuron disease), anatomic distribution of neurodegeneration (e.g., frontotemporal degenerations, extrapyramidal disorders, or spinocerebellar degenerations), or principal molecular abnormality. Among the most common neurodegenerative disorders are amyloidoses, tauopathies, α-synucleinopathies, and TDP-43 proteinopathies. The major histopathologic features in these disorders comprise abnormal protein conformation and their cellular and neuroanatomical distribution (Dugger et al.).

In Drosophila, the heat shock factor Hsp70 strongly inhibits neuronal degeneration caused by htt-Q120, a truncated form of Huntingtin containing an expanded polyglutamine domain of 120 (Chan et al., 2000). In yeast, Hsp70 and Hsp40 inhibit the assembly of mutated huntingtin into amyloid-like fibrils and induce the formation of amorphous aggregates (Muchowski et al., 2000). The ability of Hsp70 and Hsp40 to suppress polyQ-induced neurodegeneration may be based on the ability of these chaperones to ameliorate toxic forms of polyQ proteins and to direct them into nontoxic aggregates (Muchowski et al., 2000). Monomers and small soluble oligomers of mutant huntingtin disable the function of the transcription factors, such as TATA-binding protein (TBP) and CREB-binding protein (CBP), by a polyQ-mediated interaction.

Autophagy is an evolutionarily conserved process for cellular homeostasis. Autophagy is involved in the removal of misfolded proteins and damaged organelles by their encapsulation into vesicles, which combine with lysosomes to form autophagosomes. In the autophagosomes the misfolded proteins or the organelles are degraded. Autophagy enhancement can increase the life span of mammals and the insulin sensitivity. Damaged macromolecules and organelles accumulate over time, contributing to the functional deterioration seen during ageing. Autophagy-reporter studies and gene expression findings in different species indicate a deterioration of autophagy with aging. Autophagy activity slows down during aging and subsequently damaged macromolecules and organelles accumulate (Cuervo et al., 2000). Autophagy inhibition intensifies aging phenotypes in muscle, namely deterioration neuromuscular synaptic function, mitochondrial dysfunction, oxidative stress, and muscle weakness (Carnio et al., 2014). In model organisms, Atg1, beclin 1, Atg7, LC3, Atg9 and Atg12 lead to reduced lifespan by autophagy inactivation because of the premature onset of ageing (Mizushima et al., 2000). Overexpression of Atg5 in mice increases autophagy, subsequently improving motor function significantly and 17% longer lifespan compared with wildtype mice (Pyo et al., 2013). Additionally, Atg5 Tg mice have 12% less weight in comparison to wildtype littermates without significant food intake difference and improved insulin sensitivity. Known autophagy modulators have limited selectivity. For example, frequently used autophagy regulators such as 3-methyladenine, Bafilomycin-A1, and Rapamycin have off-target effects (Klionsky et al., 2016).

α-Synuclein plays a central role in the pathogenesis of several neurodegenerative diseases referred to as synucleinopathies. α-Synuclein is the main component of intracellular inclusions called Lewy bodies. α-Synuclein oligomers have been shown to be toxic to neurons via disruption of biological membranes (Fusco et al., 2017). α-Synuclein occurs under physiological conditions in neurons as a tetramer with a α-helical secondary structure which is not prone to aggregation (Bartels et al., 2011). Physiological α-Synuclein oligomers have been described which are found in cell membranes that assist NSF (Neuronal survival factor) attachment protein receptor (SNARE) complex assembly at the presynaptic terminal (Burre et al., 2014).

Dementia is a growing global epidemic, affecting nearly 50 million people worldwide. Despite clinical trials of numerous agents over a wide range of mechanisms that include neurotransmitter modulation and disease-modifying therapy targeting amyloid and tau, no treatment was approved which showed a significant reduction of clinical progression

of Alzheimer's disease (AD).

When soluble amyloid-β oligomers isolated from AD cortex are exposed to normal rat neurons, the amyloid-β oligomers trigger neurite degeneration and additionally tau hyperphosphorylation in rat neurons. But dystrophy of neurites is escaped if tau is first downregulated (Jin et al, 2011). Amyloid-β 42 soluble oligomers isolated from cortex of patients diagnosed with AD were injected in normal rodents. The dendritic spine density in the hippocampi of these animals were reduced and the memory of a learned behavior was disrupted (Shankar et al, 2008). The amyloid-β oligomer quantity was determined with selective ELISA in the cortex of subjects without dementia and with dementia of the AD type. Comparison of brains with similar plaque densities revealed that non-demented plaque-rich subjects had much lower oligomer-to-plaque ratios than patients with AD diagnosis (Esparza et al, 2013).

Retinal disease glaucoma is one of the main causes of irreversible blindness globally. It has been demonstrated that amyloid-β colocalizes with apoptotic retinal ganglion cells (RGC) in experimentally induced ocular hypertension in rats (McKinnon et al. 2002) and provokes RGC apoptosis in vivo in a dose- and time-dependent manner. Targeting amyloid-β formation and aggregation can successfully lower glaucomatous RGC apoptosis in vivo (Guo et al., 2007).

Resveratrol was described to selectively remodel amyloid-β soluble oligomers, which were recognizable with A11 antibody, and amyloid-β fibrillar intermediates, which were OC antibody positive, into non-toxic high molecular weight oligomers, which were not recognizable with A11 antibody or OC antibody. Resveratrol did not alter aggregation behaviour of monomeric amyloid-β (Ladiwala et al., 2010).

Targeting tau and truncated Tau protein species which can be formed in individuals by caspases or asparaginyl endopeptidase for the treatment of Frontotemporal dementia (FTD), Alzheimer's disease (AD), Progressive Supranuclear Palsy (PSP), Corticobasal degeneration (CBD), Pick's disease, Argyrophilic grain disease, Dementia with Lewy bodies and Parkinsonism linked to chromosome 17.

Neurofibrillary tangles (NFT) are the pathological hallmark of AD. NFT are mainly comprised of filamentous polymers of tau protein. But human tau overexpression in Drosophila led to neuronal loss without NFT (Wittmann et al., 2001). Tau oligomers were found in human brains of patients with AD diagnosis (Lasagna-Reeves et al., 2012). Not all FTDP-17 mutations raise tau filament formation (Nacharaju et al., 1999). But all FTDP-17 tau mutations discovered so far trigger tau oligomerization (Maeda and Takashima, 2019). There are two known approaches to modify tau aggregation: (1) Blocking tau oligomerization for example 1,2-dihydroxybenzene, (2) reducing tau aggregation for example DL-isoproterenol (Soeda et al., 2015).

N-terminal tau fragment (NT1) measured in plasma can differentiate normal, mildly impaired, and AD dementia populations with high specificity and sensitivity (Chen et al., 2019). A study found out that plasma NT1 levels at study entry were highly predictive of future cognitive decline, pathological tau accumulation, neurodegeneration, and transition to a diagnosis of AD (Chhatwal et al., 2020).

There are two Musashi proteins described MSI1 and MSI2. In a study, Musashi proteins showed in vitro aggregation properties by forming oligomers. An increase in Musashi proteins levels was discovered in AD brain tissues as compared with age-matched healthy subjects. Moreover, Musashi proteins were detected to form oligomers in AD brain tissues (Sengupta et al., 2018).

TAR DNA-binding protein of 43 kDa (TDP-43) is a nuclear protein that has various functions in regulating RNA metabolism, transcription, alternative splicing, miRNA processing and mRNA stabilization. TDP-43 proteinopathy is characterized by neuronal cytoplasmic inclusions containing TDP-43, which is found in a of amyotrophic lateral sclerosis (ALS) as well as frontotemporal lobar degeneration (FTLD) cases. Biochemically, pathological TDP-43 comprises the normal full-length protein, an abnormally phosphorylated form and, lower molecular weight species of 25-35 kDa that correspond to N-terminally truncated forms of TDP-43. Expression of lower molecular weight species in cell culture triggers formation of cytoplasmic aggregates and recruitment of full-length TDP-43 from the nucleus (Xiao et al., 2015).

Familial amyloid polyneuropathy (FAP) is an autosomal dominantly inherited, and progressive disease caused by amyloid fibrils consisting of the protein transthyretin (TTR). FAB and leptomeningeal amyloidosis belong to the ATTR (amyloid fibril protein derived from transthyretin) amyloidosis diseases. The TTR amyloid fibrils lead to diverse disease phenotypes like cardiomyopathy and polyneuropathy. Two main fibril morphologies have been described in TTR associated amyloid deposits. Type A fibrils are short and randomly arranged in ultrathin sections of amyloidotic tissue. They comprise of N-terminally truncated and full-length TTR. Type B fibrils are more elongated and organized into bundle-like assemblies that predominantly consist of full-length TTR protein. All tissues of a patient display either type A or type B fibrils, and the fibril type of a patient does not vary over time (Ihse et al., 2011).

CADASIL (Cerebral Autosomal Dominant Arteriopathy with Subcortical Infarcts and Leukoencephalopathy) syndrome is a rare disease affecting adults in middle-age and leading to dementia. CASADIL syndrome is characterised by a sequence of damages at the central nervous system produced by recurring ischemic strokes leading to diffuse white matter lesions. NOTCH3 gene mutations are associated with the development of the CADASIL syndrome. The predisposition of the NOTCH3 receptor to spontaneously form oligomers and higher order multimers were demonstrated in cell models. NOTCH3 mutations considerably increase receptor multimerization in higher order multimeric complexes (Opherk et al., 2009).

Hereditary cystatin C amyloid angiopathy (HCCAA) is a rare, fatal amyloid disease in young individuals caused by a mutation in cystatin C. Mutated cystatin C forms amyloid, predominantly in brain arteries and arterioles, additionally in tissues such as skin, lymph nodes, testis, and spleen. The amyloid deposition in the vessel walls causes occlusion or rupture of blood vessels, subsequently resulting in brain hemorrhage leading to dementia, personality changes and paralysis (Palsdottir et al., 2006).

Serum amyloid A (SAA) is an acute-phase plasma protein that functions in innate immunity and can trigger as a complication of chronic inflammation AA (amyloid A) amyloidosis. SAA oligomers have been demonstrated to promote damage to cellular membranes and form amyloid (Jayaraman et al., 2017).

Gelsolin mutations (D187N/Y) induce the proteolytic pathway producing amyloidogenic fragments that form aggregates that lead to gelsolin amyloidosis and lattice corneal dystrophy type 2 (LCD2). Hereditary gelsolin amyloidosis is inherited autosomal dominantly, characterized predominantly by cranial and sensory peripheral neuropathy. LCD2 is characterized by an accumulation of amyloid deposits in various tissues. The amyloid deposits often occur in blood vessel walls leading to characteristic symptoms involving the eyes, nerves, and skin.

Rhodopsin is the light receptor in photoreceptor cells of the retina. Mutations in rhodopsin can cause misfolding which leads to form aggregates that can cause retinal degeneration.

Medin amyloid is found often in the vasculature of individuals older than 50 years. Medin oligomers trigger endothelial dysfunction and vascular inflammation which was associated with aortic aneurysm, aortic dissection, and vascular dementia (Younger et al., 2020).

Glycine-alanine dipeptide repeats (GA DPRs) translated from the mutated C9orf72 gene have been associated with ALS. GA DPRs oligomers were demonstrated to disrupt nuclear membrane and nucleocytoplasmic transport in cells, subsequently triggering cytosolic retention of TDP-43 in cortical neurons.

It has been demonstrated that Atrial Natriuretic Peptide (ANP)-containing amyloid is associated with congestive heart failure (CHF) and isolated atrial amyloidosis (IAA) (Millucci et al., 2011). CHF is a common clinical disorder that results in pulmonary vascular congestion and reduced cardiac output. Despite recent advances in the management of CHF, there is a need to further improve organ damage protection.

ALS is an incapacitating disease which leads to paresis and finally to paralysis through destruction of spinal motor neurons. There are more than 180 superoxide dismutase 1 (SOD1) polymorphisms linked with amyotrophic lateral sclerosis (ALS) (Abel et al., 2012). Abnormal accumulation of mutant SOD1 proteins was detected in motor neurons of the affected spinal cords in ALS patients with SOD1 mutations (Bruijn et al., 1998). Most SOD1 mutations lead to misfolding of the SOD1 proteins triggering the formation of soluble oligomers and insoluble aggregates of SOD1 in vitro (Rodriguez et al., 2005). SOD1 oligomers were detected in spinal cords of the SOD1-ALS patients (Tokuda et al., 2017). SOD1 oligomers have been shown to be toxic (Kitamura and Kubota, 2010). Moreover, cytotoxicity assays suggest that toxicity is a property of soluble SOD1 oligomers, and not large insoluble SOD1 aggregates (Sangwan et al., 2017).

Fused-in-Sarcoma (FUS) mutations have only rarely been described in frontotemporal dementia (FTD). Inclusions immunoreactive for FUS, which are tau/TDP-43 negative, are present in a small proportion FTD cases (FTD-FUS). FUS is an RNA binding protein containing several distinct functional domains including an RNA-recognition motif and a C-terminal nuclear localization signal (NLS) (Deng et al., 2014). FUS is involved in transcriptional control, RNA processing, and DNA repair. In FTD and ALS associated with FUS, nuclear-cytoplasmic shuttling of FUS is disrupted resulting in its mislocalization into insoluble cytoplasmic inclusions (Svetoni et al., 2016).

Insulin resistance is characteristic for type 2 diabetes. Insulin resistance triggers adaptive increase of insulin production to maintain constant blood glucose concentrations. Amylin is also known as islet amyloid polypeptide (IAPP). IAPP is a 37-amino acid peptide hormone released in response to nutrients. IAPP is stored and secreted with insulin together by islet β-cells in the pancreas. IAPP has the tendency to form toxic oligomers which can damage cell membranes subsequently leading to destruction of pancreatic islet β-cells and consecutive development of type 2 diabetes (Haataja et al., 2008). Rodents do not spontaneously develop type 2 diabetes. Rodent IAPP is similar to human IAPP but rodent IAPP is not amyloidogenic due to three proline residues that improve water solubility of rodent IAPP (Westermark et al., 1990). It has been demonstrated that the toxicity to pancreatic β-cells of IAPP depends on its proclivity to form oligomers (Konarkowska et al., 2006).

Intraneuronal insulin accumulates as oligomers. This process is promoted by tau hyperphosphorylation and results in insulin resistance leading to glucose hypometabolism in AD brains (Rodriguez-Rodriguez et al. 2017). Accumulation of insulin observed in AD brains appears independently from manifestation of diabetes mellitus type 2 in these patients. This is in line with observations that peripheral and brain insulin levels are independent from each other (Talbot et al., 2012). Deficits in insulin signalling are a contributing factor to tau hyperphosphorylation and neurofibrillary tangle (NFT) formation (Calvo-Ochoa et al., 2014).

Transmissible spongiform encephalopathies are infectious diseases which can cause neurodegeneration in humans. For prion diseases the accumulation of abnormally structured prion protein is typical in the brain, and sometimes the lymphoid tissues. Soluble, low molecular weight oligomers of the prion protein are neurotoxic on primary cultures of neurons and in mice after subcortical stereotaxic injection. Monomeric prion proteins were not toxic. Also, insoluble,

fibrillar forms of prion protein displayed no neurotoxicity in vitro and were less toxic in comparison to oligomeric prion protein in mice (Simoneau et al., 2007).

Ischemic stroke is initiated by cerebral vascular occlusion leading to reduced blood flow to brain tissue. Reperfusion can be achieved either by thrombolysis using thrombolytic reagents or it can ensue spontaneously. When blood supply is restored after a period of ischemia cerebral ischemia-reperfusion injury can occur. During reperfusion autophagy can lead to a functional restoration of brain cells by clearance of abnormally folded proteins (Wang et al., 2013) and their aggregates (Liu et al., 2010) or if excessive can trigger cell death. Rapamycin is known to increase autophagy through the inhibition of mTOR (Plaza-Zabala et al., 2017). Rats treated with rapamycin after middle cerebral artery occlusion (MCAO) showed improved neurological function, reduced infarct volume with decreased apoptotic neurons due to autophagy induction (Chauhan et al., 2011).

Ischemic heart disease is the leading cause of cardiovascular disease-related disability and death worldwide, which creates huge burden on the healthcare system and economy. Ischemia is defined as inadequate blood supply to a local tissue due to obstruction of the blood vessels supplying the area. The term ischemic is used when an organ is not receiving adequate oxygen by blood. Ischemic heart disease, also called coronary heart disease (CHD) or coronary artery disease, is the term given to heart problems triggered by constricted heart (coronary) arteries that supply blood to the heart muscle. Although the narrowing can be caused by a blood clot, most often it is caused by an accumulation of plaque, called atherosclerosis. In case the blood flow to the heart muscle is completely blocked, the heart muscle cells die, which is called a heart attack or myocardial infarction.

Currently the most effective approach after a myocardial infarction is fast and effective restoration of coronary blood flow by thrombolysis, percutaneous coronary intervention, or coronary bypass operation. Various studies have noticed that reperfusion leads to significant cardiac damage, which may account for as much as 40% of the final infarction (Yellon and Hausenloy, 2007). During continued or overwhelming protein folding stress, the adaptation of unfolded protein response starts to collapse, and apoptosis follows, contributing to the progression of ischemic heart disease (Miyazaki et al., 2011).

New mutations of SARS-CoV-2 reduce vaccine efficiency demonstrating the need for additional therapeutic treatment options to fight COVID-19. Host autophagy may suppress virus infection. However, some viruses, may modify autophagy pathways and use the autophagy machinery to help their replication (Viret et al., 2018). A recent study demonstrated that SARSCoV-2-infected cells display deficiency in autophagosome maturation (Miao et al., 2021). Autophagy-inducing compounds decrease SARS-CoV-2 propagation in primary human lung cells and intestinal organoids highlighting their possible use as treatment options against COVID-19 (Gassen et al., 2021).

Exposure to the deep space radiation poses severe potential risks to central nervous system (CNS) function of astronauts. Space radiation is more accurately simulated by exposure of animals to low dose-rate neutron irradiation. Mice exposed over 6 months to low dose rate neutron irradiation developed reduced gyrus of the brain (Acharya et al., 2015). Reduced autophagy function was linked to neurotoxic effect of radiation in rodents. Thus, mTOR activation after the exposure may reduce the amount of damage on neurons via sequestering the misfolded proteins by autophagy (Poulose et al., 2011). In short-term spaceflight (13.5 days) animals exposed to radiation showed an increase of damaged proteins and lipids activating reactively in mice autophagy programs and the ubiquitin-proteasome system. Autophagy protects cells exposed to radiation by elimination of toxic misfolded proteins and damaged organelles. It has been demonstrated that after radiation cancer cells escape cell death by induction of autophagy (Paglin et al., 2005).

The above described conditions have in common that they are all associated with an protein-oligomers comprising misfolded and/or mutated protein in affected tissues. Due to their conformation, cellular degradation mechanisms fail to effectively clear the proteins and/or oligomers from the diseased areas of the body. Therefore, there is a need for new therapies that result in a conformational change of the oligomeric proteins resulting in a reduction of the amount of the harmful proteins and/or oligomers.

## BRIEF DESCRIPTION OF THE INVENTION

The present invention solves this problem, by providing protein-oligomer aggregation-agents that bind to the harmful oligomers that are associated with the above diseases. After the protein-oligomer aggregation-agents bind to the harmful oligomers, the oligomers' properties such as their conformation are altered to make the oligomers more aggregation prone, which leads to them finally aggregating. The newly formed aggregates feature reduced toxicity and improved recognition by cellular degradation pathways, mainly through autophagy. The present invention can therefore be useful to reduce unfolded proteins and thus support myocytes to survive and subsequently improve clinical outcomes in the aforementioned diseases.

Generally, and by way of brief description, the main aspects of the present invention can be described as follows:

In a **first aspect**, the invention pertains to a protein-oligomer aggregation-agent, wherein the protein-oligomer aggregation-agent comprises, or is preferably selected from, a small molecule, an antibody, a nanoparticle, a nucleic acid, a peptide and/or a therapeutic protein, wherein the protein-oligomer aggregation-agent is preferably for use in the

prevention and/or treatment of a condition associated with the undesired presence of a protein-oligomer which is capable to be bound by the protein-oligomer aggregation-agent and, thereby, induces and/or increases aggregation of the protein-oligomer.

In a **second aspect**, the invention pertains to a method for the treatment and/or prevention of a condition or a disease, the method comprises administering to a subject in need thereof a therapeutically effective amount of the protein-oligomer aggregation-agent recited in any one of the aspects and embodiments of the invention as disclosed herein below.

In a **third aspect**, the invention pertains to a method for manufacturing a medicament containing the protein-oligomer aggregation-agent of the previous aspects of the invention, comprising dissolving the aggregation-agent in a physiologically acceptable solvent and/or water yielding a stock solution of the protein-oligomer aggregation-agent. In an alternative to this aspect the invention further pertains to the use of the protein-oligomer aggregation-agent in the manufacture of a medicament for use in the prevention or treatment of a disease or condition associated with the undesired presence of a protein-oligomer which is capable to be bound by the protein-oligomer aggregation-agent and, thereby, induces and/or increases aggregation of the protein-oligomer.

In a **fourth aspect**, the invention pertains to a method for increasing the lifespan of a subject, and/or of aveliating, reducing and/or slowing down one or more physical hallmarks of aging, wherein the method comprises administering a therapeutically effective amount of the protein-oligomer aggregation-agent of the previous aspects of the invention.

In a **fifth aspect**, the invention pertains to a method for neuroprotection, wherein the method comprises administering a therapeutically effective amount of the protein-oligomer aggregation-agent of the previous aspects of the invention.

In a **sixth aspect**, the invention pertains to a method for inducing autophagy, wherein the method comprises administering a therapeutically effective amount of the previous aspects of the invention.

In a **seventh aspect**, the invention pertains to a diagnostic method for detecting a disease associated with an accumulation of oligomers in a tissue, cell, intracellular or extracellular fluid, wherein the method comprises:

(i) Providing a sample, optionally wherein the sample is provided via a biopsy of a subject;

(ii) Adding the protein-oligomer aggregation-agent of the previous aspects of the invention;

(iii)Detecting the presence or absence of aggregated polymers in the sample, wherein the presence or absence of aggregated polymers in the sample is indicative of a disease associated with an accumulation of oligomers in a tissue cell, intracellular or extracellular fluid.

In an **eighth aspect**, the invention pertains to a diagnostic method for detecting a disease associated with an accumulation of oligomers in a tissue, cell, intracellular or extracellular fluid, wherein the method comprises:

(i) Providing a sample, optionally wherein the sample is provided via a biopsy of a subject;

(ii) Adding the protein-oligomer aggregation-agent of the previous aspects of the invention;

(iii)Detecting a potential binding of the protein-oligomer aggregation-agent to the oligomers, wherein the presence or absence of binding is indicative of a disease associated with an accumulation of oligomers in a tissue cell, intracellular or extracellular fluid.

## DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a **first aspect**, the invention pertains to a protein-oligomer aggregation-agent, wherein the protein-oligomer aggregation-agent comprises, or is preferably selected from, a small molecule, an antibody, a nanoparticle, a nucleic acid, a peptide and/or a therapeutic protein, wherein the protein-oligomer aggregation-agent is preferably for use in the prevention and/or treatment of a condition associated with the undesired presence of a protein-oligomer which is capable

to be bound by the protein-oligomer aggregation-agent and, thereby, induces and/or increases aggregation of the protein-oligomer..

Preferably, the protein-oligomer aggregation-agent is a compound or composition and is selected from a small molecule, an antibody, a nanoparticle, a nucleic acid, a peptide and/or a therapeutic protein, as disclosed elsewhere herein, or a pharmaceutically acceptable salt of such compound, hydrate, solvate, prodrug, stereoisomer, or tautomer thereof. Such compound of the invention when provided as a composition is preferably provided in the composition in a therapeutically effective amount.

Generally throughout the present disclosure the term "protein-oligomer aggregation-agent" shall be understood to preferably relate to a compound or composition that when brought into direct contact with a protein-oligomer, such as binding to one or more protein-oligomer, induces aggregation of at least two protein-oligomers, or increases the tendency to form protein-oligomer aggregates, compared to the protein-oligomer(s) under comparable conditions without said compound or composition.

In general in accordance with the present the terms "monomer", "protein/peptide monomer", "monomeric protein/polypeptide", or similar terms, refer to a single, non-aggregated protein or polypeptide molecule, including any species thereof, such as phosphorylated species. As used herein, terms such as "protein oligomer" or "oligomeric protein/polypeptide" refer to individual oligomeric species comprising a (elsewhere herein defined) plurality of monomeric species, including phosphorylated species, however excludes larger aggregates in which a plurality of protein oligomers are assembled (aggregated) or bind to each other, such as during the formation of larger fibrils of the protein/peptide. Protein-oligomers in preferred embodiments and aspects of the invention are therefore composed of at least two (non-covalently) oligomerized (bound) molecules.

In context of the present invention, certain medical conditions are prevented and/or treated using the invention. In general the therapeutic success is predicated upon the fact that in each of these conditions, it is believed that oligomerized forms of polypeptides described herein are toxic to cells such as neurons in that the biomarker profiles comprising oligomeric forms and, optionally, monomeric forms of these polypeptides function in models to infer pathologic activity. In particular, increased relative amounts of oligomeric forms as compared with monomeric forms therefore indicate such pathology. Measures of these biomarkers can be used to track subject responses to therapies that are either in existence or in development as well as to predict development of disease or the state or progress of existing disease. Such measuring may target the oligomers directly, or may target the transition of oligomeric forms into larger aggregates according to the invention. Methods to detect aggregates compared to oligomeric forms, and the quantification thereof, are known to the skilled artisan as shown in the examples section and elsewhere (Zurlo E et al. 2021, PLOS ONE 16(1): e0245548).

"Compound" in the context of the invention refers to a small molecule, an antibody, or an antigen binding derivative or fragment thereof, a nanoparticle, a nucleic acid, a peptide and/or a therapeutic protein, as disclosed elsewhere herein, or a pharmaceutically acceptable salt, hydrate, solvate, prodrug, stereoisomer, or tautomer thereof.

In context of the present invention it was surprisingly identified that the certain agents have an activity to modulate, preferably induce, the aggregation of protein-oligomers. Such activity, without being bound to theory, is caused by binding of such agents to the protein oligomers and inducing thereby a conformational change which appears to have an effect on protein-oligomer aggregation and/or assembly. Since such protein-oligomers are a major hallmark and pathological causative (toxic) factor of neurodegenerative disorders, the invention predicated upon the idea to remove protein-oligomers not by inhibiting protein aggregation, but by inducing protein-oligomer aggregation. Since larger protein aggregates are more likely to be recognized and removed by cellular processes, the invention provides novel treatment options for protein aggregation associated conditions. Small molecular compounds as described herein below are a preferred aspect of protein-oligomer aggregation agents in context of the invention.

Hence, in preferred embodiments of the invention, the compound of the invention comprises the formula (I):

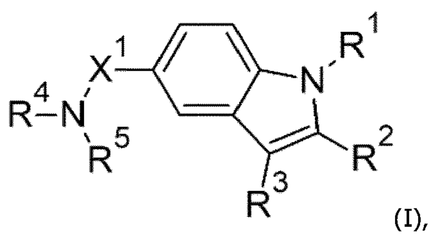
(I),

wherein

(i)

$R^1$ is independently -H, benzyl, —$C_1$-$C_6$alkyl, —$C_2$-$C_6$alkenyl, or —$C_2$-$C_6$alkynyl, wherein each alkyl, alkenyl and alkynyl is optionally substituted with one or more substituents independently selected from the group consisting of —Br and —F, or benzyl;

$R^2$ is -H, cyano, or —$C_1$-$C_6$alkyl, —$C_2$-$C_6$alkenyl, or —$C_2$-$C_6$alkynyl, wherein each alkyl, alkenyl and alkynyl is optionally substituted with one or more substituents independently selected from the group consisting of —Br and —F;

and $R^3$ is independently —H, cyano, —$C_1$-$C_6$alkyl, —$C_2$-$C_6$alkenyl, or —$C_2$-$C_6$alkynyl, wherein each alkyl, alkenyl and alkynyl is optionally substituted with one or more substituents independently selected from the group consisting of —Br and —F;

or $R^1$ and $R^2$ can optionally combine, together with the atoms to which they are attached, to form a -$C_4$-$C_8$heterocycle, wherein the heterocycle is optionally substituted with one or more substituents independently selected from the group consisting of—Br, —F, —$C_1$-$C_6$alkyl, —$C_2$-$C_6$alkenyl, and —$C_2$-$C_6$alkynyl, and wherein each alkyl, alkenyl, and alkynyl is optionally substituted with one or more-Br or -F; or $R^2$ and $R^3$ can optionally combine, together with the atoms to which they are attached, to form a -$C_4$-$C_8$carbocycle or a -$C_4$-$C_8$heterocycle, wherein the carbocycle or heterocycle is optionally substituted with one or more substituents independently selected from the group consisting of—Br, —F, —$C_1$-$C_6$alkyl, —$C_2$-$C_6$alkenyl, and —$C_2$-$C_6$alkynyl, and wherein each alkyl, alkenyl, and alkynyl is optionally substituted with one or more -Br or -F;

(ii) $X^1$ is -(C=O)- or -($CH_2$)- or -($SO_2$)-;

(iii) $R^4$ is -H or -$CH_3$;

(iv) and $R^5$ is

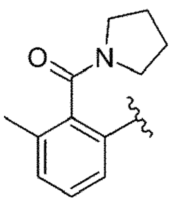, 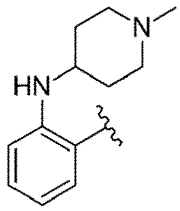, 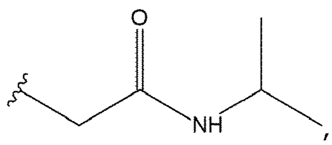, 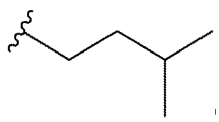,

or

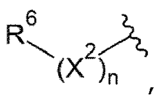,

(v) wherein

n is 1 to 4;

$X^2$ is -($CH_2$)-, -(C=O)-, -(CH(-O-Met))-, -(C($CH_3$)$_2$)-, -(CH(CH($CH_3$)$_2$))-, a -$C_6$cyclyl and/or non-substituted or mono-substituted piperidin-4-yl;

and $R^6$ is

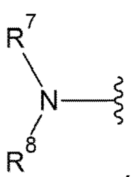, or 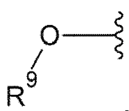,

wherein $R^7$ and $R^8$ are each independently selected from -H, cyano linear or branched —($C_1$-$C_6$) alkyl, —$C_2$-$C_6$alkenyl, or —$C_2$-$C_6$alkynyl, -O-($CH_3$), -$C_3$-$C_5$cyclyl, and/or 2-methylpropionyl, wherein each alkyl, alkenyl and alkynyl is optionally substituted with one or more substituents independently selected from the group consisting of —Br and —F; or wherein $R^7$ and $R^8$ can optionally combine, together with the nitrogen atom to which they are attached, to form a $C_3$-$C_8$heterocycle wherein the heterocycle is optionally substituted with one or more substituents independently selected from the group consisting of -Br, -F, —$C_1$-$C_6$alkyl, —$C_2$-$C_6$alkenyl, and —$C_2$-$C_6$alkynyl, and wherein each alkyl, alkenyl and alkynyl is optionally substituted with one or more -Br or -F. and $R^9$ is H, —$CH_3$ orcyclopropylmethyl;

(vi) or wherein $R^4$ and $R^5$ form together with the bridging N atom a 6-7 membered heterocyclyl containing 2 heteroatoms that is optionally mono substituted, preferably 4-(propan-2-yl)piperazin-1-yl or 4-(propan-2-yl)-1,4,diazepan-1-yl.

Further included are an isomer, a prodrug, or a derivative of the above compound, or a pharmaceutically acceptable salt or solvate of this compound.

"Alkyl" refers to a straight or branched chain saturated hydrocarbon containing 1-12 carbon atoms, preferably containing 1-10 carbon atoms, or even more preferably 1 to 6 carbon atoms. -$C_1$-$C_6$alkyl groups contain 1 to 6 carbon atoms. Examples of a -$C_1$-$C_6$alkyl group include, but are not limited to, methyl, ethyl, propyl, butyl, pentyl, isopropyl, isobutyl, sec-butyl and tert-butyl, isopentyl and neopentyl.

"Alkenyl" refers to a straight or branched chain unsaturated hydrocarbon containing 2-12 carbon atoms. The "alkenyl" group contains at least one double bond in the chain. The double bond of an alkenyl group can be unconjugated or conjugated to another unsaturated group. Examples of alkenyl groups include ethenyl, propenyl, n-butenyl, iso-butenyl, pentenyl, or hexenyl. An alkenyl group can be unsubstituted or substituted. Alkenyl, as herein defined, may be straight or branched.

"Alkynyl" refers to a straight or branched chain unsaturated hydrocarbon containing 2-12 carbon atoms. The "alkynyl" group contains at least one triple bond in the chain. Examples of alkenyl groups include ethynyl, propanyl, n-butynyl, iso-butynyl, pentynyl, or hexynyl. An alkynyl group can be unsubstituted or substituted.

The terms "heterocyclyl" or "heterocycloalkyl" or "heterocycle" refer to monocyclic or polycyclic 3 to 8-membered non-aromatic rings containing carbon and heteroatoms taken from oxygen, phosphorous, nitrogen, or sulfur and wherein there are not delocalized n electrons (aromaticity) shared among the ring carbon or heteroatoms. Heterocyclyl rings include, but are not limited to, oxetanyl, azetadinyl, tetrahydrofuranyl, pyrrolidinyl, oxazolnyl, oxazolidinyl, thiazolinyl, thiazolidinyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, and homotropanyl. A heterocyclyl or heterocycloalkyl ring can also be fused or bridged, e.g., can be a bicyclic ring.

The term "cycloalkyl" or "carbocycle" or "carbocyclyl" means monocyclic or polycyclic saturated or unsaturated carbon rings containing 3-8 carbon atoms, wherein the rings may be fused to an aromatic group. Examples of cycloalkyl groups include, without limitations, cyclopropyl, cyclobutyl, cyclopentyl, cyclohexyl, cycloheptanyl, cyclooctanyl, norboranyl, norborenyl, bicyclo[2.2.2]octanyl, or bicyclo[2.2.2]octenyl. A $C_3$-$C_8$ cycloalkyl is a cycloalkyl group containing between 3 and 8 carbon atoms. A cycloalkyl group can be fused (e.g., decalin) or bridged (e.g., norbornane).

"Aryl" refers to a radical of a monocyclic or polycyclic (*e.g.*, bicyclic or tricyclic) 4n+2 aromatic ring system (*e.g.*, having 6, 10, or 14 n electrons shared in a cyclic array) having 6-14 ring carbon atoms and zero heteroatoms provided in the aromatic ring system ("$C_{6—14}$ aryl"). In some embodiments, an aryl group has six ring carbon atoms ("$C_6$ aryl"; *e.g.*, phenyl). In some embodiments, an aryl group has ten ring carbon atoms ("$C_{10}$ aryl"; *e.g.*, naphthyl such as 1-naphthyl and 2-naphthyl). In some embodiments, an aryl group has fourteen ring carbon atoms ("$C_{14}$ aryl"; *e.g.*, anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl. Unless otherwise specified, each instance of an aryl group is independently optionally substituted, *i.e.*, unsubstituted (an "unsubstituted aryl") or substituted (a "substituted aryl") with one or more substituents. In certain embodiments, the aryl group is unsubstituted $C_{6—14}$ aryl. In certain embodiments, the aryl group is substituted $C_{6—14}$ aryl.

"Heteroaryl" refers to a radical of a 5-10 membered monocyclic or bicyclic 4n+2 aromatic ring system (*e.g.*, having 6 or 10 n electrons shared in a cyclic array) having ring carbon atoms and 1—4 ring heteroatoms provided in the aromatic ring system, wherein each heteroatom is independently selected from nitrogen, oxygen and sulfur ("5-10

membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (*e.g.*, indolyl, quinolinyl, carbazolyl, and the like) the point of attachment can be on either ring, *i.e.*, either the ring bearing a heteroatom (*e.g.*, 2—indolyl) or the ring that does not contain a heteroatom (*e.g.*, 5—indolyl).

Alkyl, alkenyl, alkynyl, carbocyclyl, heterocyclyl, aryl, and heteroaryl groups, as defined herein, are optionally substituted (*e.g.*, "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term substituted", whether preceded by the term "optionally" or not, means that at least one hydrogen present on a group (*e.g.*, a carbon or nitrogen atom) is replaced with a permissible substituent, *e.g.*, a substituent which upon substitution results in a stable compound, e.g., a compound which does not spontaneously undergo transformation such as by rearrangement, cyclization, elimination, or other reactbn. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety.

In the context of the invention, groups sometimes are depicted containing a wavy symbols, such as:

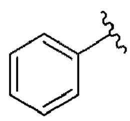

.

This wavy symbol is meant to indicate the position, at which it is connected to the compound of the invention. The wavy symbol is not meant to indicate any nature of the bond, by which it is connected to the compound of the invention.

Preferably, $R^1$ is selected from the group consisting of —H and —$C_1$-$C_6$alkyl. Preferably, $R^1$ is selected from the group consisting of -H, methyl, and benzyl. Preferably, $R^1$ is selected from the group consisting of -H and methyl. Preferably $R^1$ is -H. Preferably, $R^1$ is methyl.

Preferably, $R^2$ is selected from the group consisting of-H and —$C_1$-$C_6$alkyl. Preferably, $R^2$ is selected from the group consisting of -H, methyl, and ethyl. Preferably, $R^2$ is selected from the group consisting of -H and methyl. Preferably $R^2$ is -H. Preferably, $R^2$ is methyl. Preferably, $R^2$ is ethyl.

Preferably, $R^3$ is selected from the group consisting of —H and —$C_1$-$C_6$alkyl. Preferably, $R^3$ is selected from the group consisting of -H, methyl, and ethyl. Preferably, $R^3$ is selected from the group consisting of -H and methyl. Preferably $R^3$ is -H. Preferably, $R^3$ is methyl. Preferably, $R^3$ is ethyl.

Preferably, $R^2$ and $R^3$ can optionally combine, together with the atoms to which they are attached, to form an unsubstituted $C_4$-$C_8$carbocycle. Preferably, $R^2$ and $R^3$ can optionally combine, together with the atoms to which they are attached, to form an unsubstituted 5-6-membered carbocycle. Preferably, $R^2$ and $R^3$ can optionally combine, together with the atoms to which they are attached, to form an unsubstituted 5-membered carbocycle. Preferably, $R^2$ and $R^3$ can optionally combine, together with the atoms to which they are attached, to form an unsubstituted 6-membered carbocycle.

Preferably, $R^7$ is selected from the group consisting of-H and —$C_1$-$C_6$alkyl. Preferably, $R^7$ is selected from the group consisting of -H, methyl, ethyl, i-propyl, cyclopropyl, cyclopentyl, and t-butyl. Preferably, $R^7$ is selected from the group consisting of -H, methyl, ethyl, and i-propyl. Preferably $R^7$ is -H. Preferably, $R^7$ is methyl. Preferably, $R^7$ is ethyl. Preferably, $R^7$ is i-propyl.

Preferably, $R^8$ is selected from the group consisting of -H and —$C_1$-$C_6$alkyl. Preferably, $R^8$ is selected from the group consisting of -H, methyl, ethyl, i-propyl, cyclopropyl, cyclopentyl, and t-butyl. Preferably, $R^8$ is selected from the group consisting of -H, methyl, ethyl, and i-propyl. Preferably $R^8$ is -H. Preferably, $R^8$ is methyl. Preferably, $R^8$ is ethyl. Preferably, $R^8$ is i-propyl.

Preferably, $R^7$ and $R^8$ can optionally combine, together with the nitrogen atom to which they are attached, to form a heterocycle selected from the group consisting of azetidine, pyrrolidine, piperidine, morpholine, and piperazine, wherein the heterocycle is optionally substituted with one or more substituents independently selected from the group consisting of -Br, -F, $-C_1$-$C_6$alkyl, $-C_2$-$C_6$alkenyl, and $-C_2$-$C_6$alkynyl, and wherein each alkyl, alkenyl and alkynyl is optionally substituted with one or more -Br or -F.

Preferably, the protein-oligomer aggregation-agent comprises, preferably is a compound, having a formula selected from the group consisting of the compounds shown in Table 1. Table 1 gives specific examples for preferred protein-oligomer aggregation-agents that are part of the invention.

**Table 1:** Preferred protein-oligomer aggregation agents of the invention.

| Compound No. | Formula | Compound No. | Formula |
|---|---|---|---|
| 1 | 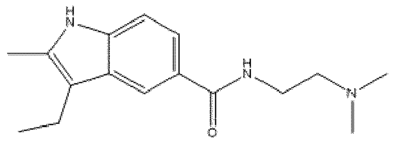 | 2 | 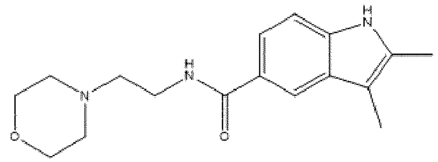 |
| 3 | 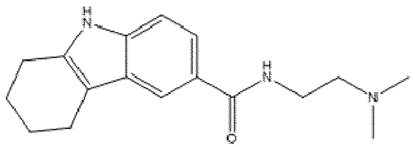 | 4 | 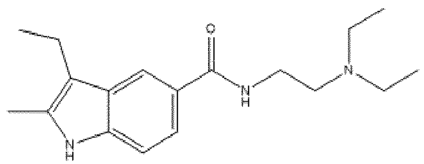 |
| 5 | 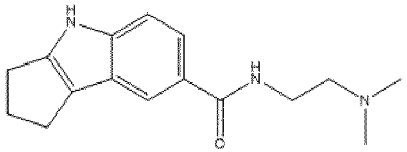 | 6 | 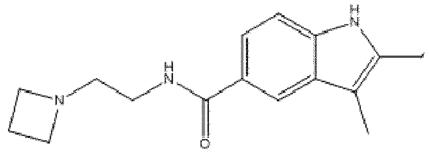 |
| 7 | 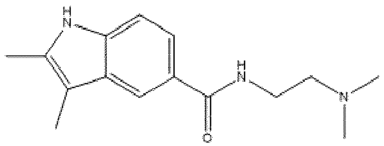 | 8 | 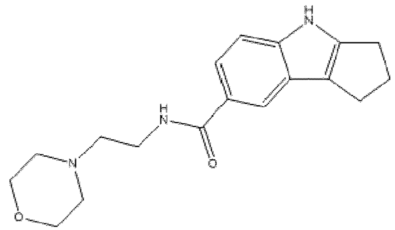 |
| 9 | 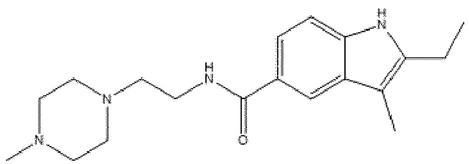 | 10 | 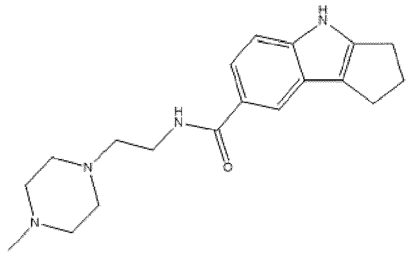 |

(continued)

| Compound No. | Formula | Compound No. | Formula |
|---|---|---|---|
| 11 | 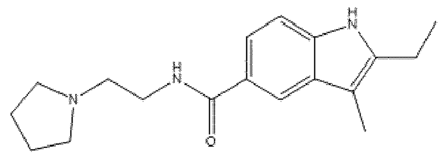 | 12 | 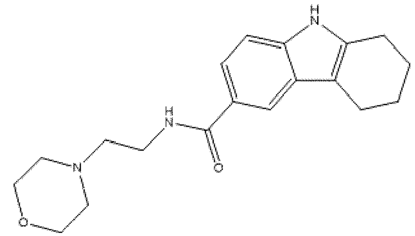 |
| 13 | 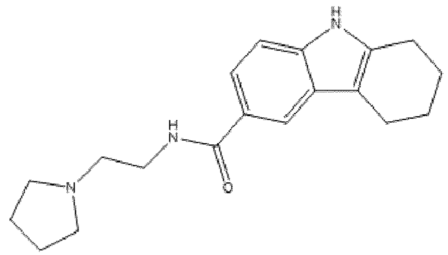 | 14 | 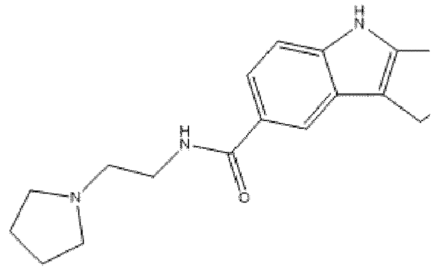 |
| 15 | 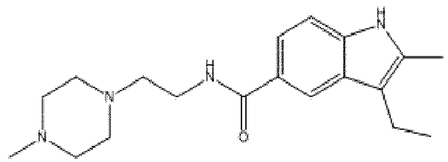 | 16 | 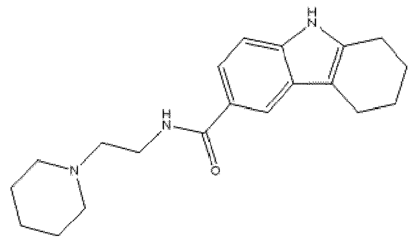 |
| 17 | 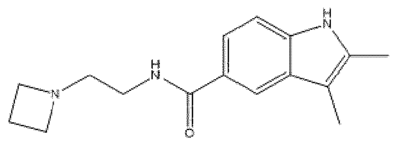 | 18 | 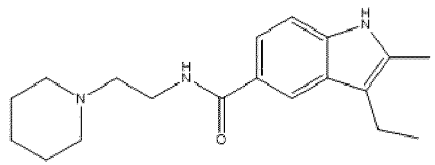 |

(continued)

| Compound No. | Formula | Compound No. | Formula |
|---|---|---|---|
| 19 | 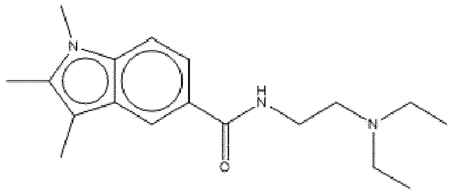 | 20 | 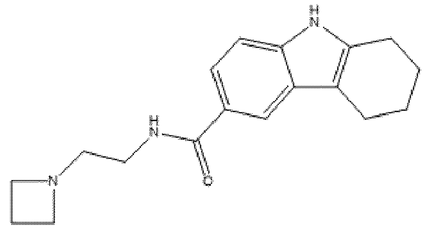 |
| 21 | 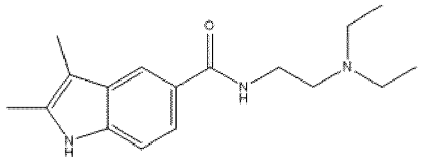 | 22 | 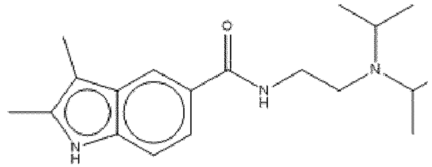 |
| 23 | 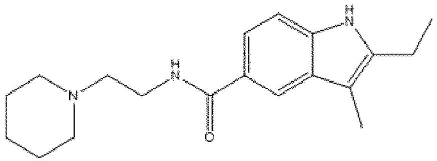 | 24 | 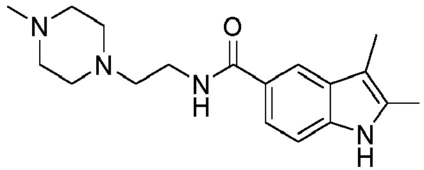 |
| 25 | 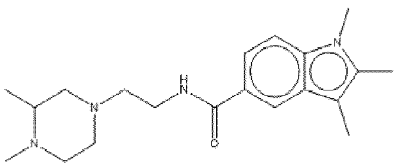 | 26 | 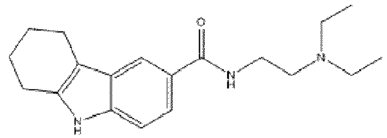 |
| 27 | 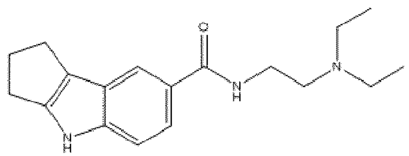 | 28 | 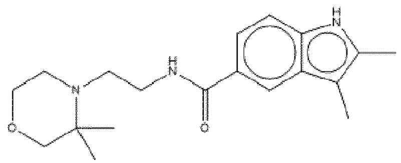 |

(continued)

| Compound No. | Formula | Compound No. | Formula |
|---|---|---|---|
| 29 | 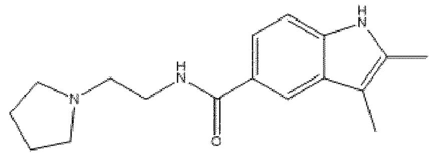 | 30 | 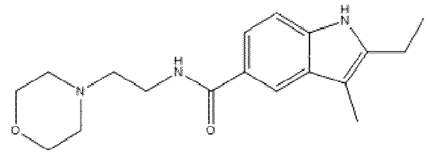 |
| 31 | 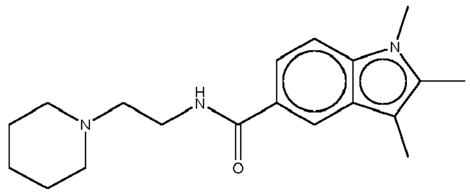 | 32 | 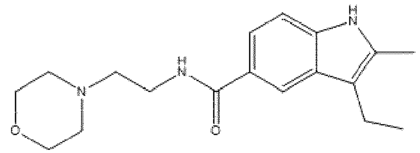 |
| 33 | 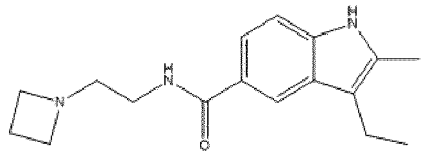 | 34 | 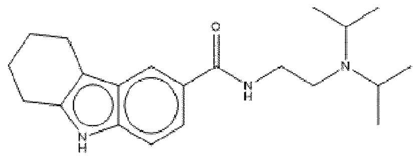 |
| 35 | 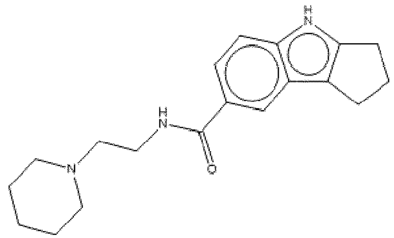 | 36 | 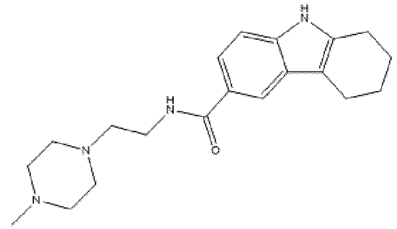 |
| 37 | 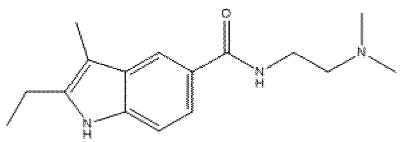 | 38 | 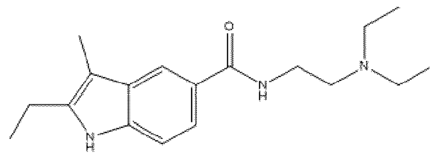 |

(continued)

| Compound No. | Formula | Compound No. | Formula |
|---|---|---|---|
| 39 | 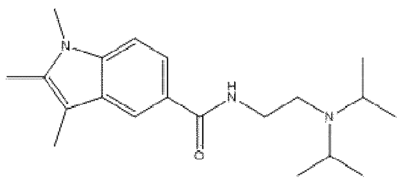 | 40 | 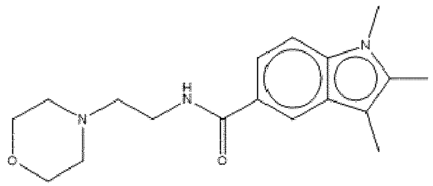 |
| 41 | 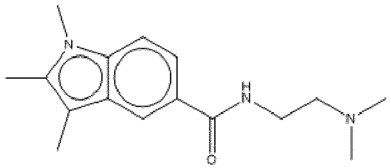 | 42 | 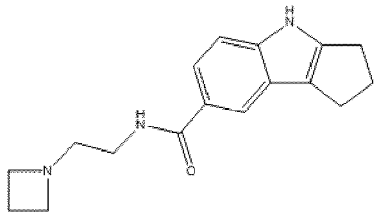 |
| 43 | 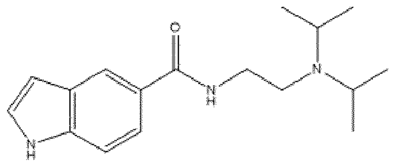 | 44 | 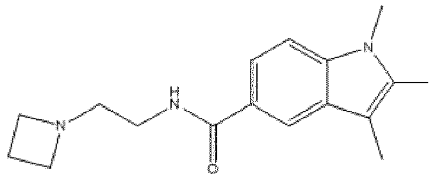 |
| 45 | 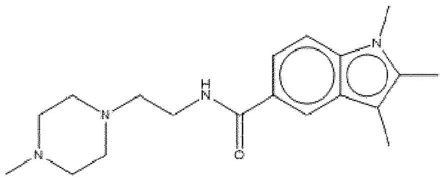 | 46 | 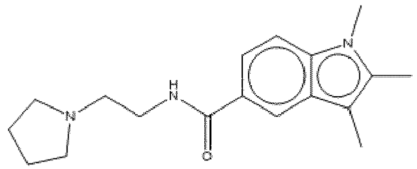 |
| 47 | 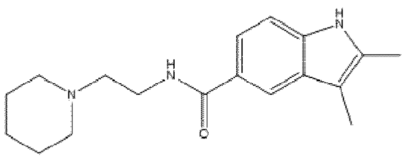 | 48 | 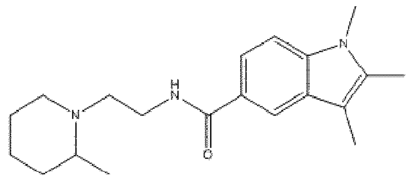 |

(continued)

| Compound No. | Formula | Compound No. | Formula |
|---|---|---|---|
| 50 | 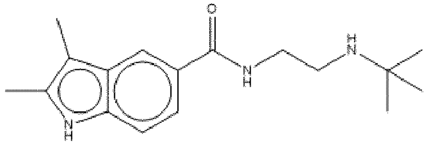 | 52 | 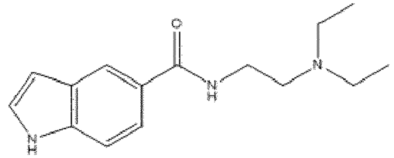 |
| 53 | 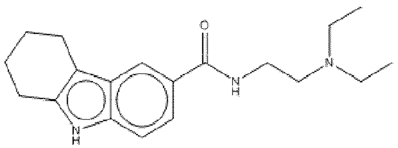 | 54 | 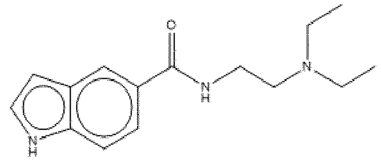 |
| 55 | 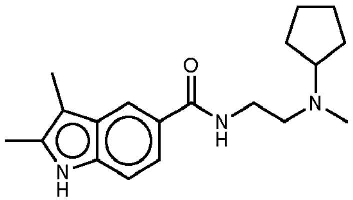 | 56 | 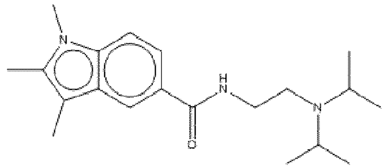 |
| 57 | 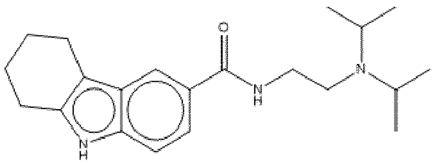 | 58 | 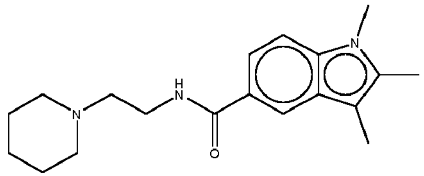 |
| 59 | 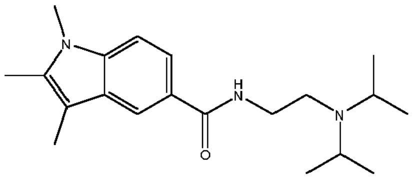 | 72 | 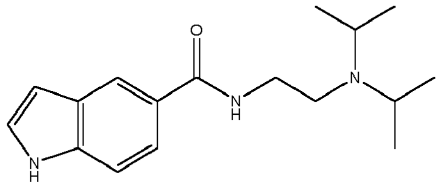 |

(continued)

| Compound No. | Formula | Compound No. | Formula |
|---|---|---|---|
| 101 | 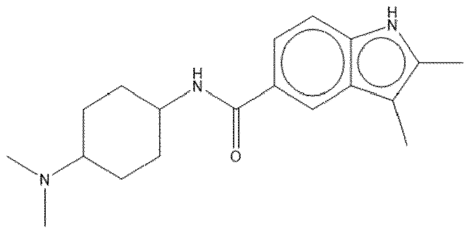 | 102 | 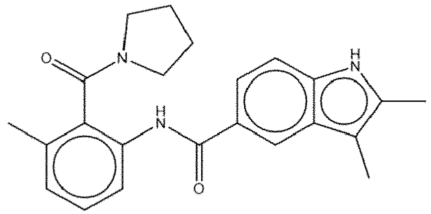 |
| 103 | 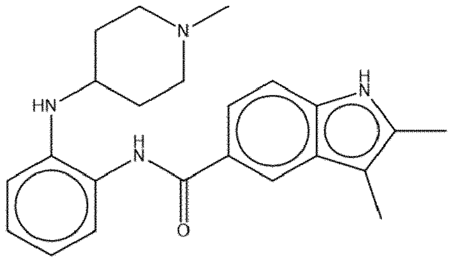 | 104 | 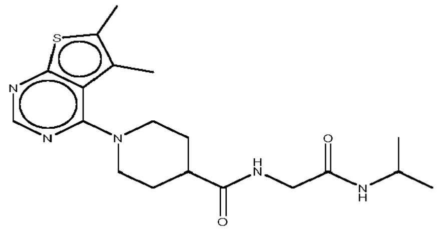 |
| 105 | 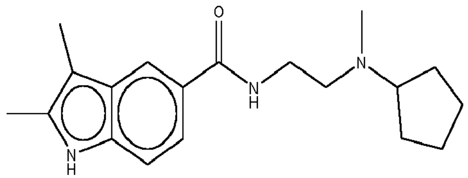 | 106 | 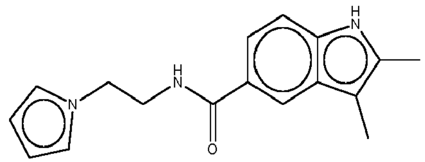 |
| 107 | 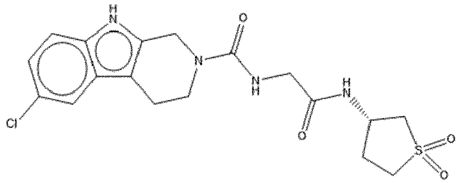 | 108 | 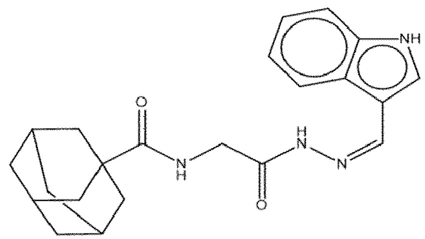 |

(continued)

| Compound No. | Formula | Compound No. | Formula |
|---|---|---|---|
| 109 | 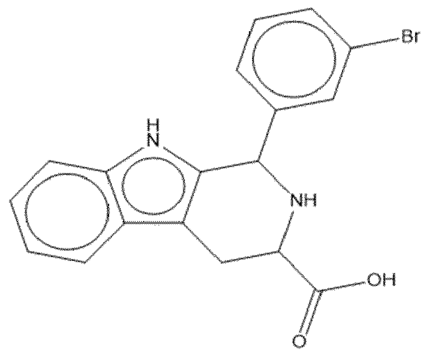 | 110 | 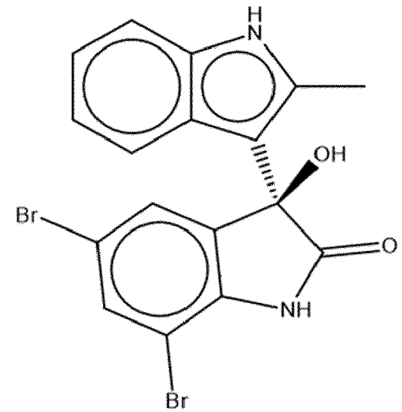 |
| 112 | 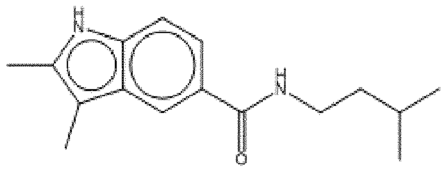 | 113 | 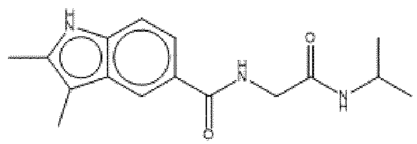 |
| 114 | 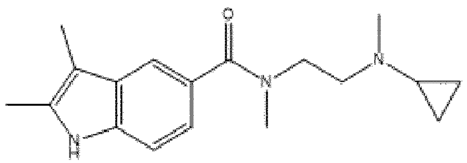 | 115 | 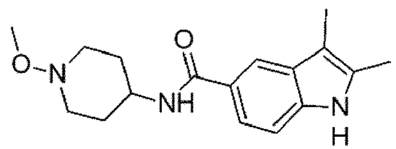 |

(continued)

| Compound No. | Formula | Compound No. | Formula |
|---|---|---|---|
| 116 | 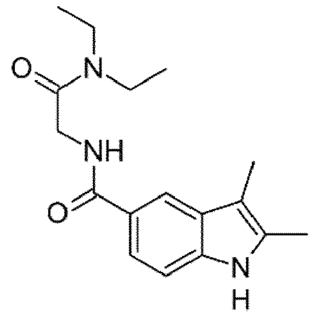 | 117 | 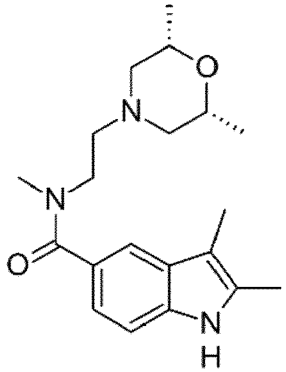 |
| 118 | 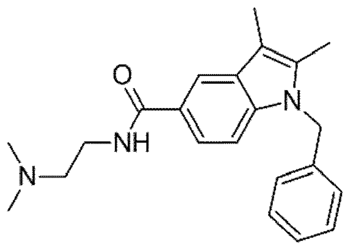 | 119 | 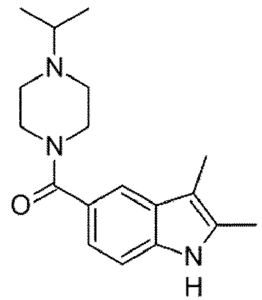 |
| 120 | 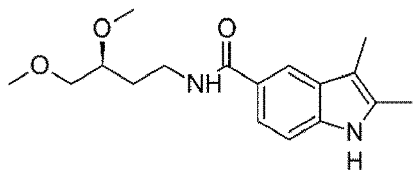 | 121 | 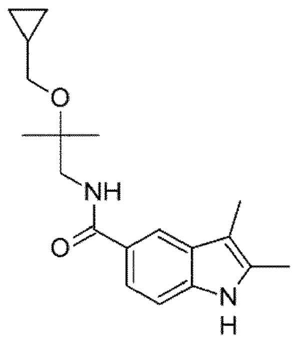 |

(continued)

| Compound No. | Formula | Compound No. | Formula |
|---|---|---|---|
| 122 | 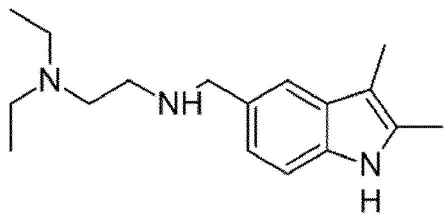 | 123 | 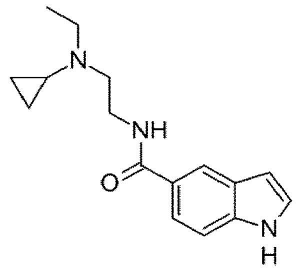 |
| 124 | 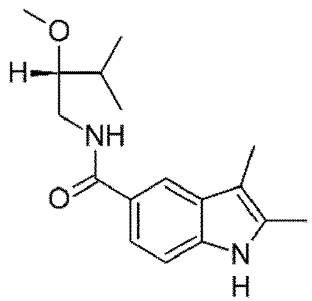 | 125 | 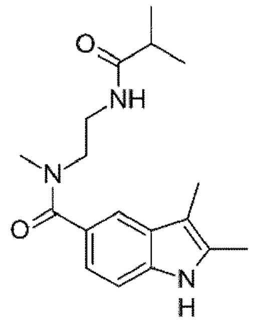 |
| 126 | 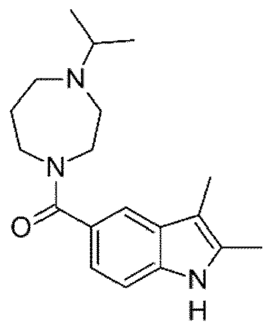 | 127 | 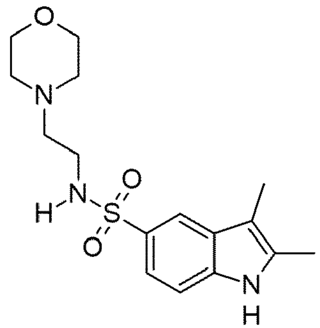 |

(continued)

| Compound No. | Formula | Compound No. | Formula |
|---|---|---|---|
| 128 | 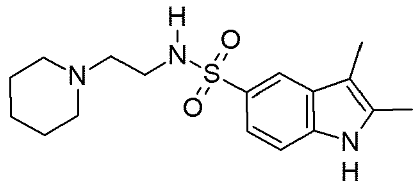 | | |

The term "tautomers" refers to a set of compounds that have the same number and type of atoms, but differ in bond connectivity and are in equilibrium with one another. A "tautomer" is a single member of this set of compounds. Typically, a single tautomer is drawn but it is understood that this single structure is meant to represent all possible tautomers that might exist. Examples include enol-ketone tautomerism. When a ketone is drawn it is understood that both the enol and ketone forms are part of the invention.

The term "isomer" refers to compounds that have the same composition and molecular weight but differ in physical and/or chemical properties. The structural difference may be in constitution (geometric isomers) or in the ability to rotate the plane of polarized light (stereoisomers). With regard to stereoisomers, the compounds may have one or more asymmetric carbon atom and may occur as racemates, racemic mixtures and as individua enantiomers or diastereomers.

The term "stereoisomers" refers to the set of compounds which have the same number and type of atoms and share the same bond connectivity between those atoms, but differ in three dimensional structure. The term "stereoisomer" refers to any member of this set of compounds. For instance, a stereoisomer may be an enantiomer or a diastereomer.

The term "enantiomers" refers to a pair of stereoisomers which are non-superimposable mirror images of one another. The term "enantiomer" refers to a single member of this pair of stereoisomers. The term "racemic" refers to a 1:1 mixture of a pair of enantiomers.

The term "diastereomers" refers to the set of stereoisomers which cannot be made superimposable by rotation around single bonds. For example, cis- and trans- double bonds, endo- and exo- substitution on bicyclic ring systems, and compounds containing multiple stereogenic centers with different relative configurations are considered to be diastereomers. The term "diastereomer" refers to any member of this set of compounds. In some examples presented, the synthetic route may produce a single diastereomer or a mixture of diastereomers. In some cases these diastereomers were separated and in other cases a wavy bond is used to indicate the structural element where configuration is variable.

The disclosure also includes pharmaceutical compositions comprising an effective amount of a disclosed compound and a pharmaceutically acceptable carrier. Representative "pharmaceutically acceptable salts" include, e.g., water-soluble and water-insoluble salts, such as the acetate, amsonate (4,4-diaminostilbene-2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fiunarate, gluceptate, gluconate, glutamate, glycollylarsanilate, hexafluorophosphate, hexylresorcinate, hydrabamine, hydrobromide, hydrochloride, hydroxynaphthoate, iodide, sethionate, lactate, lactobionate, laurate, magnesium, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts

In some embodiments of the invention, the compounds of the present disclosure are enantiomers. In some embodiments the compounds are the (*S*)-enantiomer. In other embodiments the compounds are the (*R*)-enantiomer. In yet other embodiments, the compounds of Formula I may be (+) or (-) enantiomers. It should be understood that all isomeric forms are included within the present invention, including mixtures thereof. If the compound contains a double bond, the substituent may be in the E or Z configuration. If the compound contains a disubstituted cycloalkyl, the cycloalkyl substituent may have a cis- or trans- configuration. All tautomeric forms are also intended to be included.

The term "prodrug," as used in this disclosure, means a compound which is convertible in vivo by metabolic means (e.g., by hydrolysis) to a disclosed compound. Furthermore, as used herein a prodrug is a drug which is inactive in the body, but is transformed in the body typically either during absorption or after absorption from the gastrointestinal tract into the active compound. The conversion of the prodrug into the active compound in the body may be done chemically or biologically (i.e., using an enzyme).

The term "solvate" refers to a complex of variable stoichiometry formed by a solute and solvent. Such solvents for the purpose of the invention may not interfere with the biological activity of the solute. Examples of suitable solvents include, but are not limited to, water, MeOH, EtOH, and AcOH. Solvates wherein water is the solvent molecule are typically referred to as hydrates. Hydrates include compositions containing stoichiometric amounts of water, as well as compositions containing variable amounts of water.

In preferred embodiments of the invention, the protein-oligomer aggregation-agent further comprises a pharmaceutical acceptable carrier and/or excipient. The term "carrier", as used in this disclosure, encompasses carriers, excipients, and diluents. In preferred embodiments, carriers comprise a material, composition or vehicle, such as a liquid or solid filler, diluent, excipient, solvent or encapsulating material, involved in carrying or transporting a pharmaceutical agent from one organ, or portion of the body, to another organ, or portion of the body of a subject.

A "pharmaceutically acceptable carrier" refers to an ingredient in a pharmaceutical formulation, other than the small molecule, the antibody, the nanoparticle, the nucleic acid, the peptide and/or the therapeutic protein, which is nontoxic to a subject. Pharmaceutically acceptable carriers includes any and all solvents, dispersion media, coatings,

antibacterial and antifungal agents, isotonic and absorption delaying agents, and the like that are physiologically compatible.

In certain preferred embodiments of the invention compounds of the above depicted general formula are further defined as follows:

In preferred embodiments of the invention,

(i)

$R^1$ is -H, $-CH_3$ or benzyl;
$R^2$ is -H or $-CH_3$;
and $R^3$ is -H, $-CH_3$ or ethyl;

(ii) $X^1$ is -(C=O)- or $-(CH_2)-$ or $-(SO_2)-$;
(iii) $R^4$ is -H or $-CH_3$;
(iv) and $R^5$ is

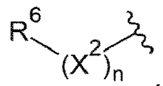
,

wherein n is 1 to 4;

$X^2$ is $-(CH_2)-$, -(C=O)-, -(CH(-O-Met))-, $-(C(CH_3)_2)-$, $-(CH(CH(CH_3)_2))-$, and/or non-substituted or mono-substituted piperidin-4-yl;
and $R^6$ is

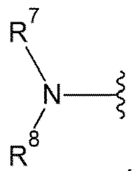
, 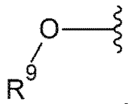
,

wherein $R^7$ and $R^8$ are each independently selected from -H, linear or branched $-(C_1-C_4)$ alkyl, $-O-(CH_3)$, cyclopropyl, and/or 2-methylpropionyl, or wherein $R^7$ and $R^8$ form together with the bridging N a 5- or 6-membered heterocyclyl that can be non-substituted, mono-substituted or bi-substituted.
and $R^9$ is H, $-CH_3$ orcyclopropylmethyl;

(v) or wherein $R^4$ and $R^5$ form together with the bridging N atom a 6-7 membered heterocyclyl containing 2 heteroatoms that is optionally mono substituted, preferably 4-(propan-2-yl)piperazin-1-yl or 4-(propan-2-yl)-1,4,diazepam-1-yl.

In the following preferred embodiments, sub-groups that are encompassed by the compound comprising the formula (I), are given.

In preferred embodiments of the invention, $R^1$ is benzyl. These embodiments refer to a group of compounds that comprise the formula

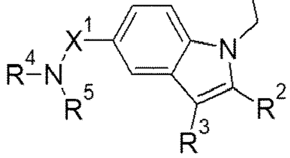
.

In specifically preferred embodiments, the protein-oligomer aggregation-agent comprises, or preferably is, compound 118 of Table 1.

In preferred embodiments of the invention, $X^1$ is —$(CH_2)$-. These embodiments refer to a group of compounds comprise the formula

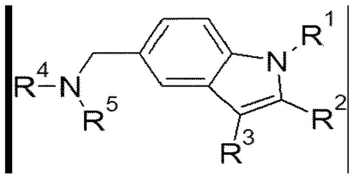

In specifically preferred embodiments, the protein-oligomer aggregation-agent is compound 122 of Table 1.

In preferred embodiments of the invention $X^1$ is —$(SO_2)$-. These embodiments refer to a group of compounds that comprise the formula

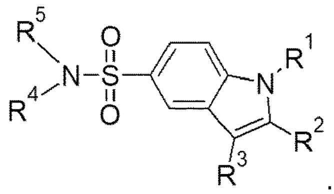

.

In specifically preferred embodiments, the protein oligomer aggregation agent is compound 127 and/or is compound 128 of Table 1.

In preferred embodiments of the invention $-(X^2)_n-$ is not $-(CH_2-CH_2)-$ and $R^5$ is not

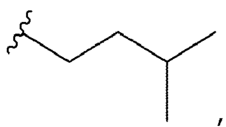

,

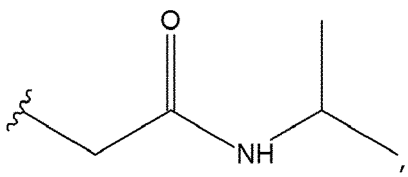

,

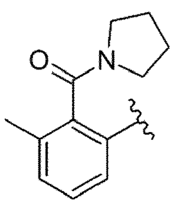

,

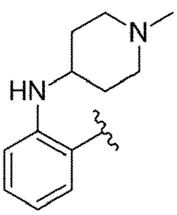

, or

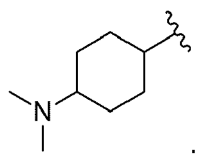

.

These embodiments refer to a group of compounds, wherein the formula is not

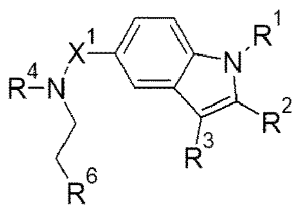

and wherein R5, when referring to fomula (I), is not

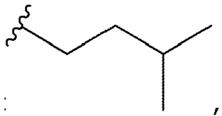, 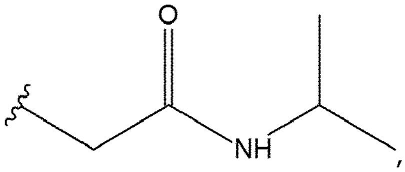, 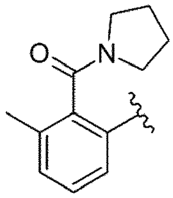, 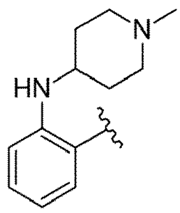,

or

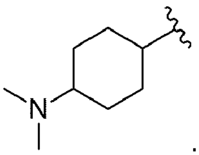.

In specifically preferred embodiments, the protein-oligomer aggregation agent of this group is compound one of compound 115, 116, 119, 120, 121, 124, 126 of Table 1.

In preferred embodiments of the invention, $R^4$ is $-CH_3$. and neither $R^7$ nor $R^8$ is cyclopropyl. These embodiments refer to a group of compounds with the formula

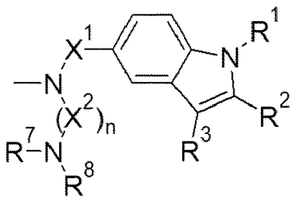,

wherein neither $R^7$ nor $R^8$ is cyclopropyl. In specifically preferred embodiments, the compound is compound 125 and/or compound 117 of Table 1.

In preferred embodiments of the invention $R^7$ and/or $R^8$ is cyclopropyl, wherein the protein oligomer aggregation-agent is not

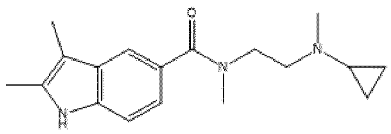.

These embodiments refer to a group of compounds with the formula

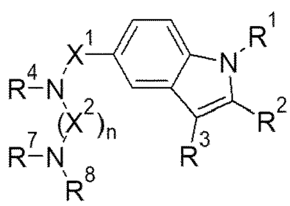,

wherein $R^7$ and/or $R^8$ is cyclopropyl. In specifically preferred embodiments, the compound is compound 123 of Table 1.

In preferred embodiments of the invention, a protein-oligomer aggregation agent comprises, preferably is, a compound shown in table 2, or an isomer, prodrug, or derivative thereof, or a pharmaceutically acceptable salt or solvate of this compound.

**Table 2:** shows formulas of even more specifically preferred protein-oligomer aggregation agents of the invention

| | | |
|---|---|---|
| 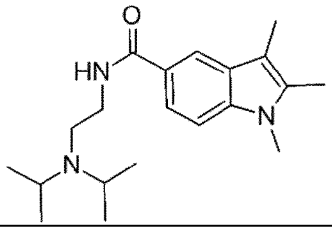 | 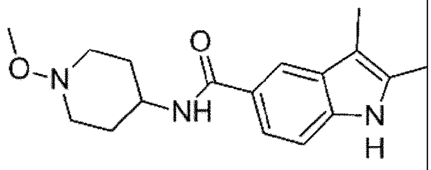 | 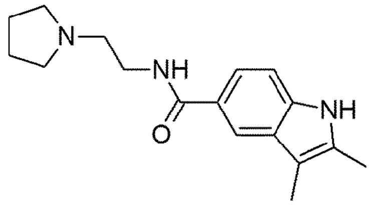 |
| 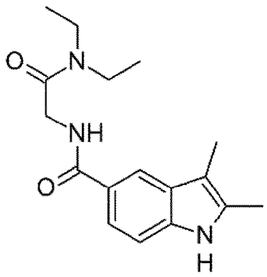 | 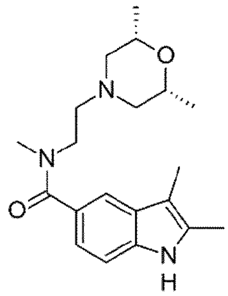 | 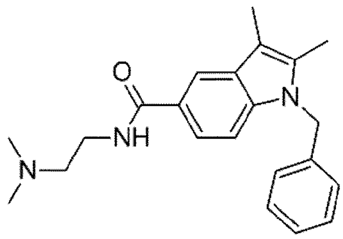 |
| 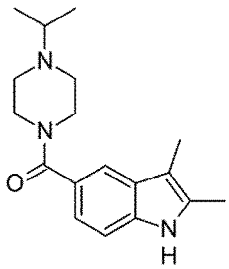 | 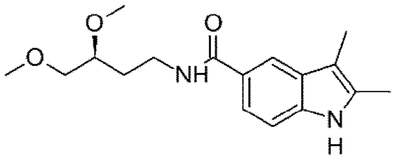 | 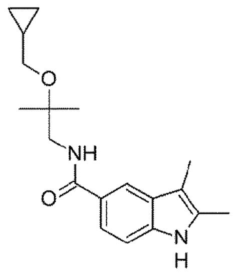 |
| 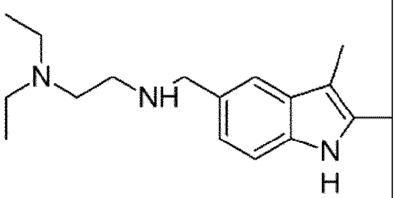 | 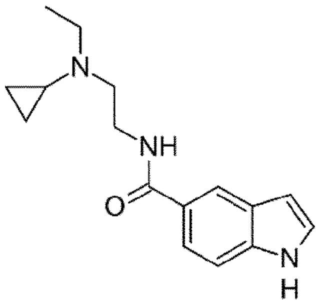 | 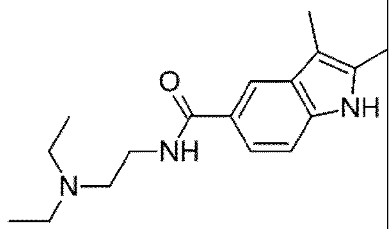 |

| | | |
|---|---|---|
| 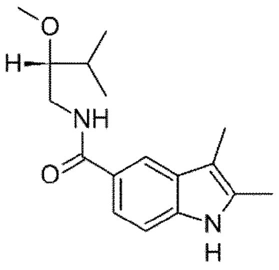 | 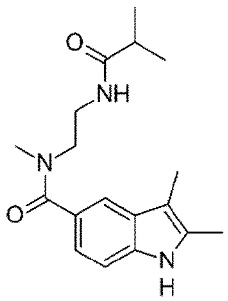 | 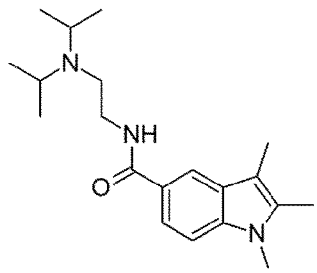 |
| 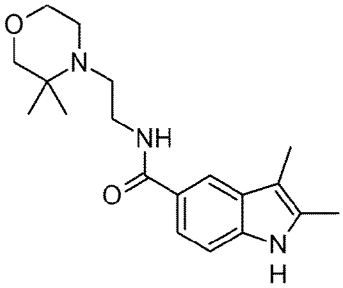 | 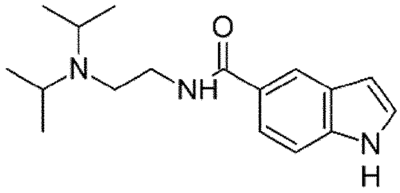 | 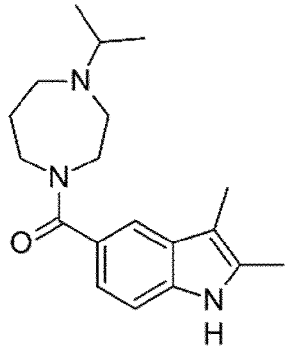 |
| 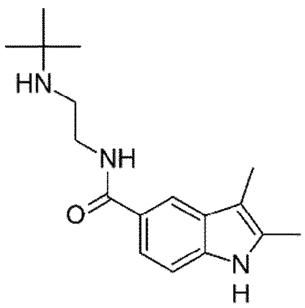 | 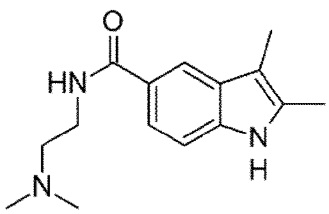 | 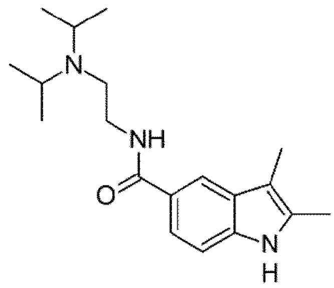 |
| 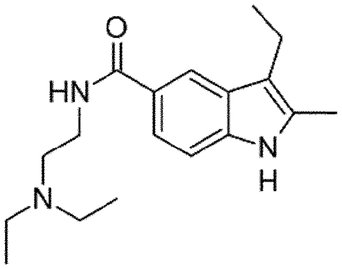 | 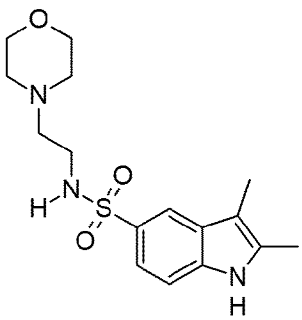 | 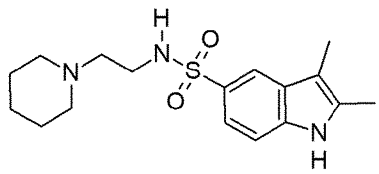 |

In preferred embodiments of the invention, a protein-oligomer aggregation agent comprises, preferably is, a compound shown in table 3, or an isomer, prodrug, or derivative thereof, or a pharmaceutically acceptable salt or solvate of this compound.

**Table 3:** shows formulas of even more specifically preferred protein-oligomer aggregation agents of the invention

| | |
|---|---|
| 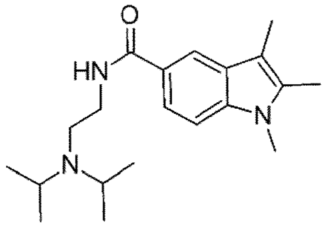 | 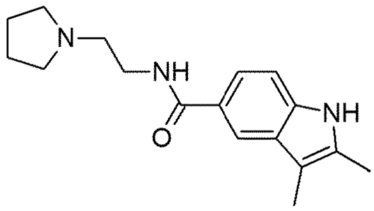 |
| 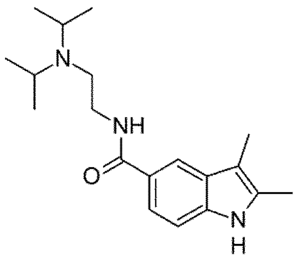 | 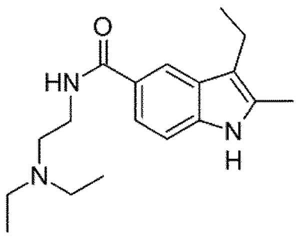 |

In preferred embodiments of the invention, a protein-oligomer aggregation agent comprises, preferably is, a compound shown in table 4, or an isomer, prodrug, or derivative thereof, or a pharmaceutically acceptable salt or solvate of this compound.

**Table 4:** shows formulas of even more specifically preferred protein-oligomer aggregation agents of the invention

| | | |
|---|---|---|
| 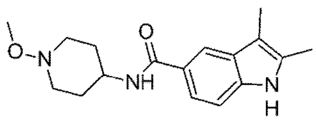 | 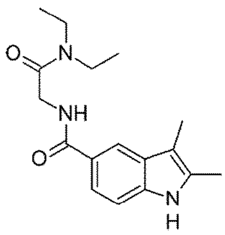 | 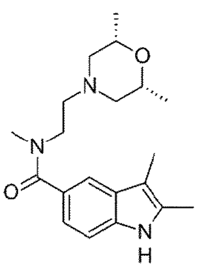 |
| 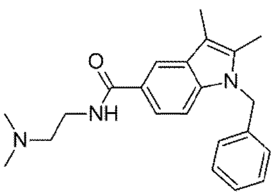 | 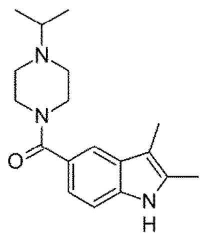 | 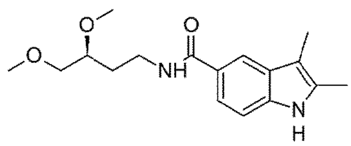 |
| 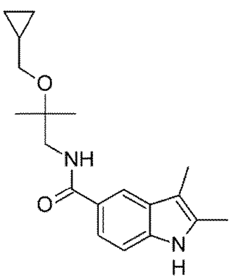 | 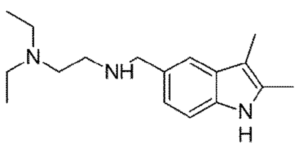 | 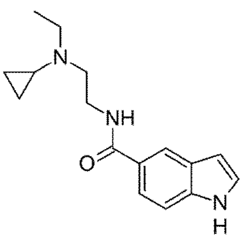 |

| | | |
|---|---|---|
| 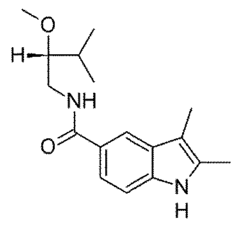 | 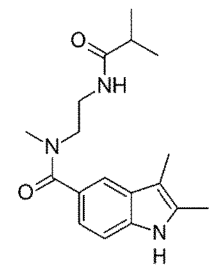 | 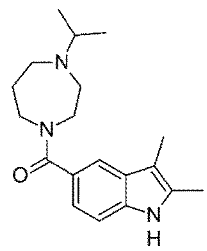 |
| 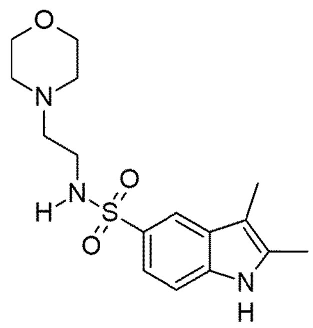 | 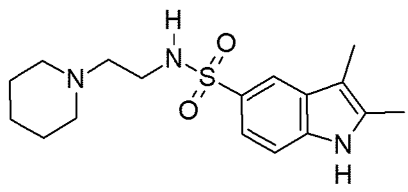 | |

In a specifically preferred embodiments of the invention compound has the formula:

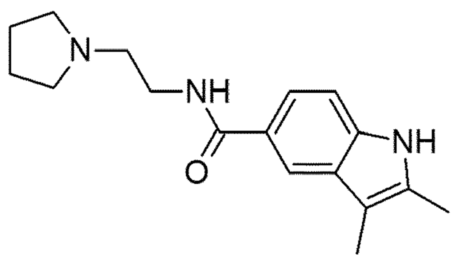

or an isomer, prodrug, or derivative thereof, or a pharmaceutically acceptable salt or solvate of this compound.

In most preferred embodiments of the invention, a protein-oligomer aggregation agent comprises, preferably is, a compound shown in table 5, or an isomer, prodrug, or derivative thereof, or a pharmaceutically acceptable salt or solvate of this compound.

**Table 5:** shows formulas of even more specifically preferred protein-oligomer aggregation agents of the invention

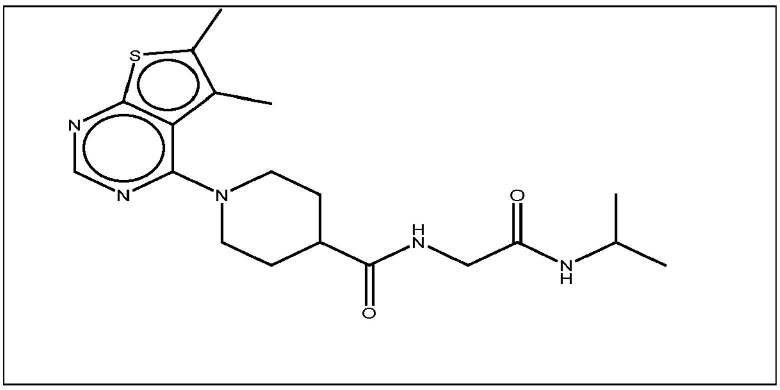

| |
|---|
| 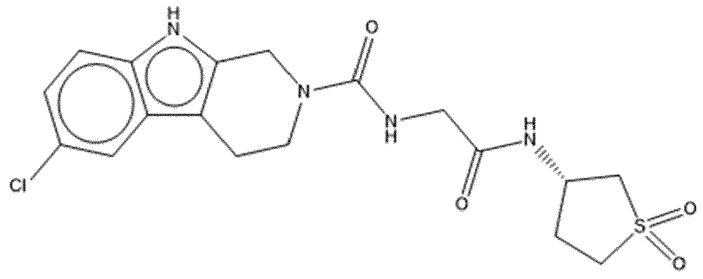 |
| 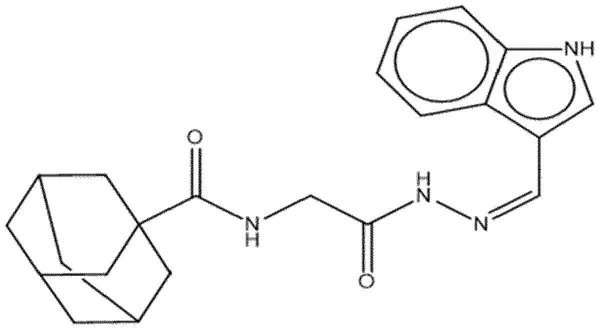 |
| 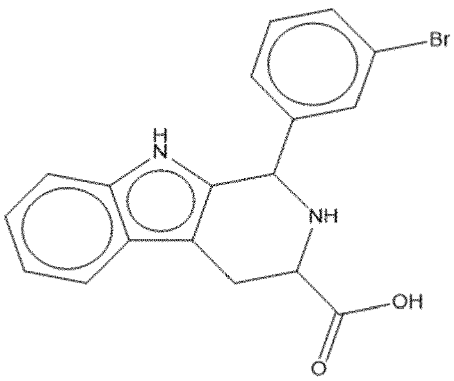 |
| 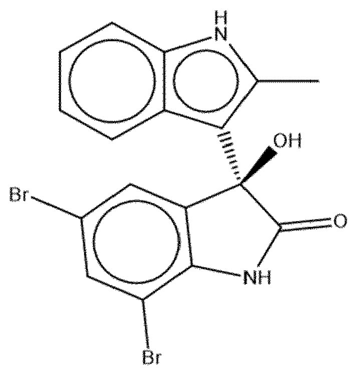 |

Preferred embodiments of the invention pertain to the protein-oligomer aggregation-agent of the invention as described herein before, for use in the treatment and/or prevention of a condition or disease. In an alternative, the invention also pertains to a use of the protein-oligomer aggregation-agent of the invention as described herein before, in the manufacture of a medicament for use in the treatment and/or prevention of a condition or disease. In such medical uses it is understood that, preferably, the invention is predicated upon the activity of the protein-oligomer aggregation-agent as disclosed to induce or enhance aggregation of a protein-oligomer which is associated with such disease or condition, and thereby induces removal and/or degradation of the protein-oligomer. Such methods for the treatment and/or prevention of diseases and conditions is described herein below.

**In a second aspect, the invention pertains to a method for the treatment and/or prevention of a condition**

**or a disease** in a subject, the method comprises administering to the subject in need thereof a therapeutically effective amount of the protein-oligomer aggregation-agent recited in any one of the previous aspects and embodiments of the invention.

As used herein, the term "prevent," "prevention," or "preventing" refers to any method to partially or completely prevent or delay the onset of one or more symptoms or features of a disease, disorder, and/or condition. Prevention treatment may be administered to a subject who does not exhibit signs of a disease, disorder, and/or condition.

The term "disorder" is used in this disclosure to mean, and is used interchangeably with, the terms disease, condition, or illness, unless otherwise indicated.

A "patient" or "subject" in context of all embodiments or aspects is a mammal, e.g., a human, mouse, rat, guinea pig, dog, cat, horse, cow, pig, or non-human primate, such as a monkey, chimpanzee, baboon or rhesus. Most preferably, a subject in accordance with the present invention in all of the herein disclosed aspects and embodiments is a human, preferably a human adult. A human adult in context of the invention is preferably a human of 14 years or older, more preferably of 18 years or older. A subject in certain preferred embodiments of all aspects of the invention is 20 years or older, more preferably is 40 years or older, and most preferably is 60 years or older, even more preferably is 70 years or older. The age range for preventive aspects and uses of the invention may be between 10 and 60 years of age of the subject, whereas the age range for therapeutic applications may be 40 to 90 years, and older individuals.

The term "treatment" or "treating" with regard to a subject, refers to improving at least one symptom of the subject’s disorder. Treating includes curing, improving, or at least partially ameliorating the disorder.

When referring to "aggregation", "aggregates", "agglomeration" and/or "agglomerates" herein, this does not indicate a mechanism by which the proteins or oligomers are joined together. "Aggregation" and "agglomeration" are used interchangeably. Preferably, aggregates are joined together by weak or strong chemical forces. Preferably, these weak and/or strong chemical forces comprise ionic bonds, covalent bonds (including coordinative covalent bonds and/or pi-stacking), dipole-dipole interactions, van der Waals forces and/or hydrogen bonding. Preferably, aggregates in the context of the invention comprise aggregated protein-oligomers.

In preferred embodiments, the condition or disease is caused by a pathological aberration in cellular protein homeostasis, preferably cellular protein metabolism, for example in the central nervous system (CNS). In preferred embodiments, the condition or disease is associated with, or caused by, an aggregation of protein-oligomers in a tissue, cell intracellular or extracellular fluid, preferably within the CNS. In preferred embodiments, the tissue, cell, intracellular or extracellular fluid is from the central nervous system (CNS), preferably wherein the tissue, cell, intracellular or extracellular fluid is from areas of the brain. CNS in the context of the invention may refer to areas of the brain, spinal cord cranial nerves, the retina, the optic nerve, olfactory nerves, and/or the olfactory epithelium and every other tissue, cell or fluid that connects directly with brain tissue without intermediate nerve fibres. The areas from the brain may comprise the prosencephalon, preferably the telencephalon and/or diencephalon, preferably the rhinencephalon, amygdala, hippocampus, cerebral cortex, (more preferably the neocortex), the basal ganglia (or basal nuclei, more preferably the striatum, the globus pallidus and/or the substantia nigra), the lateral ventricles, the epithalamus, the thalamus, the hypothalamus, the subthalamus, the pituitary gland, the pineal gland and/or the third ventricle. Preferably, the areas from the brain comprise the brain stem, preferably the mesencephalon and/or the thombencephalon, most preferably the tectum, the cerebral peduncle, the pretectum, the mesencephalic duct, the metencephalon, the pons, the cerebellum, the myelencephalon and/or the medulla oblongata. In some embodiments of the invention the CNS comprises white and/or gray matter. Preferably, the CNS comprises intracellular or extracellular fluid. Preferably, the extracellular fluid comprises blood, interstitial fluid and/or transcellular fluid including cerebrospinal fluid (CSF). CSF in the context of the invention refers to a fluid that is produced by specialized ependymal cells that comprise the choroid plexus located in the ventricles of the brain. CSF produced within the brain then circulates and surrounds the central nervous system including the brain and spinal cord. CSF continually circulates around the central nervous system, including the ventricles of the brain and subarachnoid space that surrounds both the brain and spinal cord, while maintaining a homeostatic balance of production and reabsorption into the vascular system. In preferred embodiments the tissue, cell, intracellular or extracellular fluid is a striatal tissue, cell, intracellular or extracellular fluid. Preferably, the cell is a cell derived primarily from the ectoderm. More preferably, the cell is a cell from the nervous system. Even more preferably, the cell is a cell central nervous system neuron and/or glial cell. Preferably, the cell is a neuron such as an interneuron (such as a basket cell, a cartwheel cell, a stellate cell, a golgi cell, a granule cell, a lugaro cell, a unipolar brush cell, a martinotti cell, a chandelier cell, a cajal-retzius cell, a double-bouquet cell, a neurogliaform cell, a retina horizontal cell, a amacrine cell, and/or a spinal interneuron), a principal cell (such as a spindle neuron, a fork neuron, a pyramidal cell, a stellate cell, a bushy cell, a purkinje cell and/or a medium spiny neuron), a astrocyte, a oligodendrocyte, a ependymal cell (such as a tanycyte), and/or a pituicyte. In preferred embodiments the cell is a neuron, preferably wherein the cell is a motor neuron.

In preferred embodiments, the protein-oligomers comprise at least one constituent selected from lipids, damaged organelles and/or proteins. In the context of the invention, the "protein-oligomers", also referred to as "oligomers" are preferably soluble. By "soluble" in the context of the invention is meant that the oligomer will not precipitate out of the intracellular and/or extracellular fluid. Preferably, "soluble" means that the oligomer will dissolve in bodily fluid, such

as the intracellular or extracellular fluid.

In preferred embodiments, the proteins are mutated, misfolded and/or denatured proteins, preferably wherein the proteins are denatured proteins.

Preferably, the "protein" or "proteins" is referring to any variant, mutant, isoform, polymorphism, fragmented version, truncated version, splice variant, misfolded variant, denatured variant and/or fusion variant of the protein.

In the context of the invention, when referring to "sequence identity" or "identical sequence", or "sequence identical to", preferably this refers to a sequence identity of 60%, more preferably 70%, more preferably 80%, more preferably 85%, more preferably 90%, more preferably 95%, more preferably 97%, more preferably 98%, more preferably 99%, most preferably 100 %.

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site). In a particular embodiment, for example when comparing the protein or nucleic acid sequence of an antigen recognizing construct of the invention to another protein/gene, the percentage identity can be determined by the Blast searches; in particular for amino acid identity, those using BLASTP 2.2.28+ with the following parameters: Matrix: BLOSUM62; Gap Penalties: Existence: 11, Extension: 1; Neighboring words threshold: 11; Window for multiple hits: 40.

Preferably, the protein oligomers of the invention comprise 2-10000 monomers, preferably 2 to 8000 monomers, preferably 2-6000 monomers, preferably 2-4000 monomers, preferably 2-2000 monomers, preferably 2-1000 monomers, preferably, 2-800 monomers, preferably 2-600 monomers, preferably, 2-400 monomers, preferably 2-200 monomers, preferably 20-100 monomers, preferably, 2-80 monomers, preferably, 2-60 monomers, preferably 2-50 monomers, preferably 2-40 monomers, preferably 2-30 monomers, preferably 2-20 monomers, most preferably 2-10 monomers of the mutated, misfolded and/or denatured proteins. In preferred embodiments, the oligomers comprise 2 to 50 monomers of the proteins, preferably the mutated, misfolded and/or denatured proteins.

In preferred embodiments of the invention, the proteins are selected from one or more of the group of α-synuclein, amyloid β, mutated huntingtin, tau protein, prion proteins, misfolded superoxide dismutase 1 (SOD1) polymorphisms, isled amyloid polypeptide (IAPP), Musashi protein, TAR DNA-binding protein 43 (TDP-43), misfolded transthyretin protein (TTR), NOTCH3 receptor, mutated cystatin C, serum amyloid A (SAA), mutated gelsolin, misfolded rhodopsin, medin, dipeptide repeat protein, atrial natriuretic peptide, fused-in-sarcoma, insulin oligomers, or fragments truncated, and/or alternatively spliced variants and/or fusion proteins thereof, preferably wherein the proteins are selected from one or more of the group α-synuclein, amyloid β, prion proteins, misfolded superoxide dismutase 1 (SOD1) polymorphisms, isled amyloid polypeptide (IAPP), Musashi protein, TAR DNA-binding protein 43 (TDP-43), misfolded transthyretin protein (TTR), NOTCH3 receptor, mutated cystatin C, serum amyloid A (SAA), mutated gelsolin, misfolded rhodopsin, medin, glycine-alanine dipeptide repeats, atrial natriuretic peptide, fused-in-sarcoma, insulin oligomers, or fragments, truncated, and/or alternatively spliced variants and/or fusion proteins thereof.

In preferred embodiments of the invention, the α-synuclein is mutated α-synuclein. Preferably, "a-synuclein" or "alpha-synuclein" refers to the protein that is currently described by the UNIPROT entry P37840 (17.08.2021). Preferably, the α-synuclein comprises any one of the 3 isomers that are described therein. α -synuclein is also known as Non-A beta component of AD amyloid, NON-A4 component of amyloid precursor and NACP. Preferably, the α-synuclein comprises an amino acid sequence that is identical to any one of the amino acid sequences of **SEQ ID NO. 24-26.** Preferably, the α -synuclein comprises an amino acid sequence that, when compared to the amino acid sequences **SEQ ID NO. 24-26** comprises at least one substituted amino acid and/or comprises at least one missing amino acid(s). When referring to the sequence **SEQ ID NO 24**, the at least one substituted amino acid preferably comprises at least one of the substitutions selected from the group of: A30P, E46K, H50Q, A53T, E46K, D2A, E35K, Y39F, H50A, E57K, Y125F, Y133F and/or Y136F. When referring to the sequence of **SEQ ID NO 24**, the at least one missing amino acid(s) preferably is missing at a positions selected from the group of: 67-77, 71-82, 76-77, 76, 77, 78, and/or 85-94. The term "mutated", when used in the context of mutated α -synuclein, does not indicate any mechanism by which the at least one substituted amino acid and/or at least one missing amino acid(s) is generated. Instead, it preferably refers to any α -synuclein variant that comprises the abovementioned differences to **SEQ ID NO 24**. Preferred embodiments of the invention refer to an "A30P" α -synuclein. This "A30P" mutant of α-synuclein that comprises the amino acid sequence of **SEQ ID NO 24** with an A30P mutation.

Preferably, amyloid beta, or amyloid-β, refers to a peptide that derives from the amyloid precursor protein (APP, UNIPROT entry P05607, **SEQ ID NO. 27**). Preferably, the amyloid-β comprises 36—43 amino acids. Preferably, the amyloid-β is generated from APP via imprecise cleavage by beta secretase and gamma secretase, thus resulting in

varying C-terminuses. Preferably, when referring to **SEQ ID NO. 27** this cleavage is at position 671D. Preferably, in the context of the invention, amyloid-β refers to any one of the AB36-43 variants of amyloid-β that comprises an amino acid sequence that is identical to **SEQ ID NOs. 28-35**. Preferably, the amyloid- β refers to at least one of Ab40 (**SEQ ID NO. 32**) and Ab42 (**SEQ ID NO. 34**). Preferably, the amyloid-β refers to a fragment of the amino acid sequences of **SEQ ID NOs. 28-35**. Preferably, the amyloid-β refers to a peptide that, when referring to the amino acid sequences of **SEQ ID NOs. 28-35**, comprises at least one amino acid substitution. When referring to the amino acid sequence of the AB43 variant of Amyloid beta (**SEQ ID NO. 35)**, the at least one amino acid substitution preferably is selected from the group of: D7N, A21G, E22G, A42T, E22K, E22Q, D23N, L34V, R5G, Y10F, H13R, V24C, G33V, M35L, and/or M35V.

Preferably, the protein is mutated huntingtin. Preferably, "Huntingtin" also referred to as "HTT" herein refers to the protein that is currently described by the UNIPROT entry P42858 (18.08.2021), or a variant thereof. Preferably, the huntingtin comprises an amino acid sequence that is identical to the amino acid sequence of **SEQ ID NO. 45**. Huntingtin is also known as Huntington disease protein and HD protein. Preferably, the amino acid sequence of the huntingtin comprises at least one amino acid substitution, when compared to **SEQ ID NO. 45**. **Preferably t**he at least one amino acid substitution selected from the group of: Q18QQQ, G551E, P703L, G893R, V1064I, I1091M, T1173A, T1260M, E1382A, N1385H, T1720N, D2113Y, Y2309H, F2717L, V2786I, C823S, I495A, QP(498-499)AA, Q498A, P499A, D511A, D528A, D550E, G551A, G551S, D584A. Preferably, the poly-Gln region (also referred to as poly-Q region) of the huntingtin comprises 10 to 35 repeats (when referring to SEQ ID NO 45 starting at amino acid position 18), more preferably the poly-Gln region of HTT is expanded, wherein expanded refers to 36-120 repeats. Huntingtin containing fewer than 36 glutamine residues in a row is referred to as "wild type" huntingtin. In preferred embodiments, the mutated huntingtin comprises an expanded polyQ repeat. In preferred embodiments of the invention the mutated huntingtin is truncated mutated huntingtin. In preferred embodiments, the mutated huntingtin is Exon 1 truncated mutated huntingtin.

Preferably, "Tau protein" refers to the protein that is currently described by the UNIPROT entry P10636 (11.08.2021). Preferably, the Tau protein comprises any one of the 9 isomers of Tau protein described therein (**SEQ ID NOs: 15-23**). Tau protein is also known as known as microtubule-associated protein tau, neurofibrillary tangle protein, paired helical filament-tau or PHF-tau. Preferably, the Tau protein preferably to a protein that comprises an amino acid sequence identical to the amino acid sequence of PNS-tau (**SEQ ID NO 15**), Fetal-tau (**SEQ ID NO 16**), Tau-A (**SEQ ID NO 17**), Tau-B (**SEQ ID NO 18**), Tau-C (**SEQ ID NO 19**), Tau-D (**SEQ ID NO 20**), Tau-E (**SEQ ID NO 21**), Tau-F (**SEQ ID NO 22**) or Tau-G (**SEQ ID NO 23**). Preferably, the Tau protein comprises an amino acid sequence that, when compared to the amino acid sequences of **SEQ ID NO15-23**, comprise at least one amino acid substitution. Preferably, with reference to **SEQ ID NO 15** the at least one amino acid substitution is selected from the group of: S515E, S516E, S519E, S351A, T548A, T548E, S552A, S552E, S579A, S713E, S721E, S726E, S730E, S739E, R5H, L583V, G589V, G590R, N596K, N613H, P618L, P618S, S622N, K634M, V654M, E659V, K574T, S637F, K686I, G706R, R5L. Preferably, when referring to the amino acid sequences of **SEQ ID NO15-23**, the amino acid sequence of the tau protein comprises at least one missing amino acid(s). Preferably, when referring to **SEQ ID NO 15**, the at least one missing amino acid(s) is missing at the amino acid position 597. In preferred embodiments, the tau protein is mutated tau, truncated tau and/or hyperphosphorylated tau protein. Hyperphosphorylated tau is also known as PHF-TAU or AD P-TAU. Preferably, the hyperphosphorylated Tau protein is phosphorylated at multiple threonine and/or serine amino acid positions. When referring to the amino acid sequence of Tau-F (**SEQ ID NO. 22**), the multiple phosphorylated amino acid positions preferably are chosen from the group of: S396, S404, S202, T205, T217, S262, S422, T212 and S214. However, this is not to be understood to limit hyperphosphorlyation only to the Tau-F isomer, or to these specific amino acid positions.

Preferably, the dipeptide repeat proteins are generated from expression of an expanded GGGGCC hexanucleotide in the chromosome 9 open reading frame 72 (C90orf72) locus. Preferably, the expression is conducted by repeat associated non-ATG (RAN) translation. Preferably, the dipeptide repeat protein comprises glycine-alanine (GA), glycine-arginine (GR), proline-alanine (PA), proline-arginine (PR) and/or glycine-proline (GP). In preferred embodiments of the invention, the dipeptide repeat protein is a glycine-alanine repeat protein. Preferably, the dipeptide repeat proteins comprise 2 to 10000 dipeptides, more preferably 5 to 8000 dipeptides, more preferably 10 to 6000 dipeptides, more preferably 10 to 4000 dipeptides, more preferably 10 to 2000 dipeptides, more preferably 10 to 1000 dipetpides, most preferably 10 to 500 dipeptides.

Preferably, the atrial natriuretic peptide refers to a peptide that is described by the UNIPROT entry P01160 (18.08.2021). Preferably, the atrial natriuretic peptide refers to a protein coded by the gene comprising an amino acid sequence identical to the amino acid sequence of **SEQ ID NO: 58**. Preferably, the atrial natriuretic peptide comprises an amino acid sequence identical to the amino acid sequence of **SEQ ID NO. 59**. It is also known as NPA, alpha-atrial natriuretic peptide, alpha-hANP, Atrial natriuretic factor (ANF), CDD-ANF, CDD-ANP and Carionatrin. Preferably, the atrial natriuretic peptide refers to a protein with an amino acid sequence that comprises at least one amino acid substitution when compared to the amino acid sequence of **SEQ ID NO. 59**. When referring to **SEQ ID NO. 59**, the at least one amino acid substitution is selected from the group of: R27Q and/or Y28YRR. Preferably, the atrial natriuretic peptide refers to a protein with an amino acid sequence that comprises at least one missing amino acid(s), when compared to the amino acid sequence of **SEQ ID NO. 59**. Preferably, the at least one missing amino acid(s) is missing at a position

selected from the group of: 1-6, 1-3 and/or 24-28.

Preferably, the Isled amyloid polypeptide refers to any variant of the protein described by the UNIPROT entry P10997 (18.08.2021). Isled amyloid polypeptide is the peptide subunit of amyloid found in pancreatic islets of type 2 diabetic patients and in insulinomas. Preferably, the isled amyloid polypeptide comprises an amino acid sequence identical to the amino acid sequence of **SEQ ID NO. 61**. Isled amyloid polypeptide is also known as Amylin, Diabetes-associated peptide and insulinoma amyloid peptide. Preferably, the isled amyloid polypeptide comprises an amino acid sequence that is identical to a fragment of **SEQ ID NO. 61**. Preferably, this fragment comprises the amino acids at positions 36-70, when referring to **SEQ ID NO. 61**. Preferably, the fragment comprises an amino acid sequence that is identical to the amino acid sequence of **SEQ ID NO 62**. Preferably, the isled amyloid polypeptide comprises an amino acid sequence with at least one amino acid substitution when compared to the amino acid sequence of **SEQ ID NO. 61.** Preferably, when referring to SEQ ID NO. 61 this at least one amino acid substitution is selected from the group of: F48A, F48D, F48S, S53C and/or S53G.

Preferably, the Superoxide dismutase 1 (SOD1) refers to any variant of the protein described by the UNIPROT entry P00441 (17.08.2021). It is also known as Superoxide dismutase [Cu-Zn] or hSod1. Preferably, the SOD1 comprises an amino acid sequence that is identical to the amino acid sequence of **SEQ ID NO. 55**. Preferably, the SOD1 refers to a protein with an amino acid sequence that comprises at least one amino acid substitution from the amino acid sequence of **SEQ ID NO. 55**. Preferably, when referring to the amino acid sequence of **SEQ ID NO. 55**, the at least one substitution is selected from the group consisting of: A5S, A5T, A5V, C7F, V8E, L9Q, L9V, G13R, V15G, V15M, G17S, F21C, E22G, E22K, Q23L, G38R, L39R, L39V, G42D, G42S, H44R, F46C, H47R, H49Q, H49R, E50K, T55R, N66S, L68P, L68R, G73S, D77Y, H81A, L85F, L85V, G86R, N87S, V88A, A90T, A90V, D91A, D91V, G94A, G94C, G94D, G94R, G94V, A96G, V98M, E101G, E101K, D102G, D102N, I105F, S106L, L107V, G109V, C112Y, I113M, I113T, I114T, G115A, R116G, V119L, V119VFLQ, D125G, D125V, D126H, L127S, S135N, N140K, L145F, L145S, A146T, C147R, G148R, V149G, V149I, I150T, I152T, C7S, FG(51-52)EE, C58A, C58S, H81A, H81S, D84A, D84S, C112S, K123A, K123E, E134Q, C147A, C147S, I18S and/or S99V. In preferred embodiments, the SOD1 refers to a protein with an amino acid sequence that comprises at least one missing amino acid, when referring to the amino acid sequence of **SEQ ID NO. 55.** Preferably, when referring to **SEQ ID NO. 55** the at least one missing amino acid comprises the amino acid at the position: 134.

Preferably, the Fused-in-Sarcoma in the context of the present invention refers to any variant of the protein described by the UNIPROT entry P35637 (18.08.2021). Fused-in-Sarcoma is also known as RNA-binding protein FUS, 75 kDa DNA-pairing protein, Oncogene FUS, Oncogene TLS, POMp75 and translocated in liposarcoma protein. Preferably, the Fused-in-Sarcoma comprises a sequence with identity to the amino acid sequences of **SEQ ID NO. 56** and/or **SEQ ID NO. 57**. Preferably, the Fused-in-Sarcoma refers to a protein with an amino acid sequence that comprises at least one amino acid substitution from the amino acid sequences of **SEQ ID NO. 56** and/or **SEQ ID NO. 57**. Preferably, when referring to the amino acid sequence of **SEQ ID NO. 56**, the at least one substitution is selected from the group consisting of: G191S, R216C, G225V, G230C, R234C, R244C, G507D, R514G, R514S, G515C, R518K, R521C, R521G, R521H, R522G, R524S, R524T, P525L, Y526YY, P431L, M254V and/or K312Q.

Preferably, the TAR DNA-binding protein of 43 kDa refers to any variant of the protein described by the UNIPROT entry Q13148(17.08.2021). Preferably, the TAR DNA-binding protein of 43 kDa comprises a sequence with identity to the amino acid sequences of **SEQ ID NO. 39** and/or **SEQ ID NO.40**. The TAR DNA-binding protein of 43 kDa is also known as TAR DNA-binding protein 43, TDP-43, TARDBP, ALS10. Preferably, the TDP-43 refers to a protein with an amino acid sequence that features at least one amino acid substitution compared to the amino acid sequences of **SEQ ID NO. 39** and/or **SEQ ID NO.40**. Preferably, when referring to the amino acid sequence of **SEQ ID NO. 39,** this at least one substitution is selected from the group of: A90V, D169G, N267S, G287S, G290A, G294A, G294V, G295R, G295S, G298S, A315T, A321V, Q331K, S332N, G335D, M337V, Q343R, G348C, G348V, G357R, R361S, R361T, S379C, S379P, A382T, N390D, N390S, S393L, E200G, G278V, S48E, LIVLGL(106-111)DIDLGD, and/or FGF(147-149)LGL. In preferred embodiments, the TDP-43 refers to a protein with an amino acid sequence that comprises at least one missing amino acid(s), when referring to the amino acid sequence of **SEQ ID NO. 39**. Preferably, when referring to **SEQ ID NO. 39** the at least one missing amino acid(s) comprises the amino acid(s) at an amino acid position selected from the group of: 103-183, 106-175, 106-11 and/or 193-257. In preferred embodiments of the invention, the TDP-43 refers to a lower molecular weight species of TDP-43 with a molecular weight of 25-35 kDA that corresponds to N truncated forms of TDP-43. Preferably, the TAR binding protein is a hyperphosphorylated variant of any of the the TDP-43 variants herein. In preferred embodiments of the invention, the TDP-43 is wildtype, truncated TDP-43, hyperphosphorylated TDP-43 and/or splice variants thereof.

Preferably, the NOTCH3 receptor refers to any variant of the protein described by the UNIPROT entry Q9UM47 (18.08.2021). NOTCH3 receptor is also known as neurogenic locus notch homolog protein 3. Preferably, the NOTCH3 receptor comprises a sequence with identity to the amino acid sequence of **SEQ ID NO. 60**. Preferably, the NOTCH3 refers to a protein with an amino acid sequence that comprises at least one amino acid substitution compared to the amino acid sequence of **SEQ ID NO. 60**. Preferably, when referring to the amino acid sequence of **SEQ ID NO. 60**, this

at least one substitution is selected from the group consisting of: C43G, C49F, C49Y, R54C, S60C, C65S, C67Y, W71C, C76R, C76W, C87R, C87Y, R90C, C93F, C93Y, C106W, C108W, C108Y, R110C, C117F, S118C, C123F, C123Y, C128Y, R133C, C134W, R141C, F142C, C144F, C144S, C144Y, S145C, C146R, G149C, Y150C, R153C, C155S,C162S, R169C, H170R, G171C, C174F, C174R, C174Y, S180C, R182C, C183F, C183R, C183S, C185G, C185R, Y189C, C194F, C194R, C194S, C194Y, C201Y, C206Y, R207C, C212S, R213K, C222Y, C224Y, C233S, C233Y, C240S, C245R, C251R, Y258C, C260Y, A319C, R332C, S335C, Y337C, C379S, C395R, G420C, R421C, C428S, C428Y, C440G, C440R, C446S, R449C, C455R, C484F, C484Y, C495Y, P496L, C511R, C542Y, R544C, C549Y, R558C, R578C, R607C, Y710C, R728C, C775S, G953, F984C, R985C, R1006C, C1015R, Y1021C, R1031C, D1063C, H1133Q, V1183M, R1231C, C1261R, C1261Y, L1519P, L1519P, and/or A2223V. Preferably, the NOTCH3 refers to a protein with an amino acid sequence that comprises at least one missing amino acid(s) compared to the amino acid sequence of **SEQ ID NO. 60**. Preferably, when referring to the amino acid sequence of **SEQ ID NO. 60**, this at least one missing amino acid(s) is missing at a positions selected from the group of: 77-82, 80-84, 114-120, 153-155 and/or 239-253.

Preferably, Medin in the context of the invention comprises an internal fragment of the protein lactadherin that is described by isoform 1 of the UNIPROT entry Q08431 (**SEQ ID NO. 36**) (17.08.2021). Preferably, the Medin comprises a sequence with identity to the amino acid sequence of **SEQ ID NO. 37**. Preferably, the amino acid sequence of the medin comprises at least one substitution from the amino acid sequence of **SEQ ID NO. 37**. When referring to **SEQ ID NO. 37** preferably, the at least one amino acid substitution comprises: R1W.

Rhodopsin is the light receptor in photoreceptor cells of the retina. Mutations in rhodopsin can cause misfolding which leads to form aggregates that can cause retinal degeneration. Preferably, the rhodopsin in the context of the present invention refers to any variant of the protein described by the UNIPROT entry P08100 (17.08.2021). Preferably, the rhodopsin comprises a sequence with identity to **SEQ ID NO. 38**. Preferably, the rhodopsin refers to a protein with an amino acid sequence that features at least one substitution from the amino acid sequence of **SEQ ID NO 38**. Preferably, the at least one amino acid substitution comprises: T4K, N15S, T17M, P23H, P23L, Q28H, L40R, M44T, F45L, L46R, G51A, G51R, G51V, P53R, T58R, V87D, G89D, G90D, T94I, V104I, G106R, G106W, G109R, C110F, C110Y, G114D, L125R, S127F, L131P, R135G, R135L, R135W, C140S, E150K, A164E, A164V, C167R, P171L, P171Q, P171S, Y178C, Y178N, P180S, E181K, G182S, S186P, G188E, G188R, D190G, D190N, D190Y, M207R, V209M, H211P, H211R, I214N, M216K, F220C, C222R, P267L, P267R, A292E, K296E, S297R, T342M, V345L, V345M, P347A, P347L, P347Q, P347R, P347S, E113Q, M257Y, TVSKT(336-340)AVAKA, TVS336-338AVA, and/or S343A. Preferably, the rhodopsin refers to a protein with an amino acid sequence that comprises at least one missing amino acid, when referring to the amino acid sequence of **SEQ ID NO 38**. Preferably, these at least one missing amino acids comprises the amino acids at the amino acid positions selected from the group of: 68-71, 255, and/or 264.

Preferably, the Musashi protein is a group of RNA-binding proteins called "Musashi proteins, also known as Musashi binding proteins. The group comprises MSI1 and MSI2. Preferably, the MSI1 refers in the context of the present invention to any variant of the protein that is currently described by the UNIPROT entry 043347 (17.08.2021). MSI1 is also known as RNA-binding protein Musashi homolog 1, or Musashi-1. Preferably, the MSI1 comprises an amino acid sequence that is identical to the sequence described by **SEQ ID NO. 41**. Preferably, the MSI1 refers to a protein with an amino acid sequence that comprises at least one substitution from the amino acid sequence of **SEQ ID NO. 41.** Preferably, this substitution is: R197W, T225S, Y232C, A236P, E245K and/or E160Q. Preferably, the MSI2 refers to any variants of the proteins that are currently described by any of the three isoforms of the UNIPROT entry Q96DH6 (17.08.2021). MSI2 is also known as RNA-binding protein Musashi homolog 2, or Musashi-2. Preferably, the MSI2 comprises an amino acid sequence that is identical to any one of the amino acid sequences of **SEQ ID NO. 42-44**. Preferably, the MSI2 refers to a protein with an amino acid sequence that features at least one amino acid substitution from the amino acid sequences of **SEQ ID NO. 42-44**. In preferred embodiments of the invention, the Musashi protein is Musashi protein 1 and/or Musashi protein 2.

Preferably, the cystatin C refers to any variant or fragment of the protein described by the UNIPROT entry P01034 (18.08.2021). Cystatin C is also known as Cystatin-3, Gamma-trace, Neuroendocrine basic polypeptide and Post-gamma-globulin. Preferably, the Cystatin C comprises an amino acid sequence with identity to the amino acid sequence of **SEQ ID NO. 49**. Preferably, the amino acid sequence of the cystatin C comprises a fragment of **SEQ ID NO. 49,** wherein the fragment comprises the amino acid positions 27-146. Preferably, the cystatin C refers to a protein with an amino acid sequence that features at least one amino acid substitution from the amino acid sequence **SEQ ID NO. 49.** Preferably this substitution is selected from the group consisting of: L94Q, A25T and/or A25S.

Preferably, the gelsolin refers to any variant of the protein described by the UNIPROT entry P06396 (18.08.2021). Gelsolin is also known as AGEL, Actin-depolymerizing factor, ADF and Brevin. Preferably, the gelsolin comprises an amino acid sequence with identity to any one of the amino acid sequences of **SEQ ID NO. 50-53**. Preferably, the gelsolin refers to a protein with an amino acid sequence that features at least one amino acid substitution when compared to any one of the amino acid sequence **SEQ ID NO. 50-53**. Preferably, when referring to **SEQ ID NO: 50** the amino acid substitution is selected from the group consisting of: N294D, R419W, Y603H, S22L, A129T, T201I, D214N, D214Y,

N231D, S611N and/or R668L.

Preferably, the insulin refers to any variant of the protein described by the UNIPROT entry P01308 (18.08.2021) Preferably, the insulin refers to a protein coded by an amino acid sequence that is identical to the amino acid sequence of **SEQ ID NO. 63**. Preferably the inulin refers to a protein comprising an amino acid sequence that is identical to the amino acid sequences **SEQ ID NO 64** and/or **SEQ ID NO.65.** Preferably, when referring to the amino acid sequence of **SEQ ID NO. 63**, the amino acid sequence of the insulin refers to a protein with an amino acid sequence that comprises at least one amino acid substitution. When referring to **SEQ ID NO. 63**, preferably, the amino acid substitution is selected from the group consisting of: R6C, R6H, A24D, H29D, G32R, G32S, H34D, L35P, C43G, R46Q, G47V, F48C, F48S, F49L, R55C, L68M, G84R, R89C, R89H, R89L, G90C, V92L, C96S, C96Y, S101C, Y103C and/or Y108C.

Preferably, the transthyretin protein refers to any variant of the protein described by the UNIPROT entry P02766 (18.08.2021). Transthyretin protein is also known as transthyretin, ATTR, Prealbumin, and TBPA. Preferably, the Transthyretin comprises an amino acid sequence with identity to the amino acid sequence of **SEQ ID NO. 54**, or a fragment thereof. Preferably, the Transthyretin refers to a protein with an amino acid sequence that features at least one amino acid substitution from the amino acid sequence **SEQ ID NO. 54**. Preferably the at least one amino acid substitution is selected from the group consisting of C30R, L32P, D38E, D38G, V40I, S43N, P44S, V48M, V50A, V50G, V50L, V50M, F53I, F53L, F53V, R54T, K55N, A56P, D58A, D58V, W61L, E62D, E62G, F64S, A65D, A65S, A65T, G67A, G67E, G67R, G67V, T69A, T69I, S70I, S70R, S72P, G73E, E74G, E74K, L75P, L75Q, L78H, L78R, T79K, T80A, E81G, E81K, F84L, I88L, Y89H, K90N, V91A, I93V, S97Y, Y98F, I104N, I104S, I104T, E109K, E109Q, A111S, A117G, A117S, T126N, I127M, I127V, L131M, Y134C, Y136S, A140S, V142A, V142I, N144S, A129T, Y134H, F107M, L130M, G26S, M33I, D39G, F53C, D94H H110N, V114A, G21S, P122R, R124C, R124H, Y134H, Y136V and/or T139M. In preferred embodiments of the invention, the misfolded transthyretin protein is truncated TTR.

Preferably, the serum amyloid A refers to acute-phase serum amyloid A. Preferably, the serum amyloid A refers to Serum amyloid A1 and/or Serum amyloid A2. The Serum amyloid A1 preferably refers to the proteins described by the UNIPROT entry PODJI8 (18.08.2021). Serum amyloid A1 is also known as Serum amyloid A1 protein or SAA1. Preferably, the Serum amyloid A1 comprises a sequence with identity to the amino acid sequence of **SEQ ID NO 46**: Preferably, the SAA1 refers to a protein with an amino acid sequence that comprises at least one substitution when compared to the amino acid sequence of **SEQ ID NO 46.** When referring to **SEQ ID NO 46**: preferably, the at least one amino acid substitution is selected from the group consisting of: R19A, R33A, R37A, R65A, R80A, H89A, W71R, S77T, ADQAAN(96-101)SEATVK, N101D and/or P119S. Preferably, the SAA1 refers to any one of the 5 polymorphisms SAA1.1 (also known as SAA1alpha, **SEQ ID NO. 46)**, SAA1.2 (also known as SAA1beta), SAA1.3 (also known as SAA1gamma), SAA1.4 (also known as SAA1delta) and/or SAA1.5. Preferably, the SAA1, when referring to **SEQ ID NO: 46**, comprises at least one amino acid substitution selected from the group of G15S, V70A, A75V, D78N, F86L, G90D. The Serum amyloid A2 preferably refers to the proteins described by the UNIPROT entry P0DJI9 (17.08.2021). Serum amyloid A2 is also known as Serum amyloid A2 protein or SAA2. Preferably, the SAA2 comprises a sequence with identity to **SEQ ID NO 47** - **SEQ ID NO. 48**. Preferably, the SAA2 refers to a protein with an amino acid sequence that comprises at least one substitution when compared to the amino acid sequences of **SEQ ID NO 47** - **SEQ ID NO. 48**. When referring to **SEQ ID NO 47,** preferably, the at least one amino acid substitution is selected from the group consisting of: S15G, R89H. Preferably, the SAA2 refers to any one of the 2 polymorphisms SAA2.1 (also known as SAA2alpha) and SAA2.2(also known as SAA2beta, **SEQ ID NO 47**)

Prion in the context of the invention refers to a misfolded protein that is capable to transmit its misfolded shape onto normal variants of the same protein. Preferably, the prion protein of the invention is the protein described UNIPROT entry P04156 (19.08.2021) and/or F7VJQ1 (19.08.2021). The prion protein is also known as Major prion protein, PrP, ASCR, PrP27-30, PRP33-35C. Preferably, the prion protein comprises an amino acid sequence with identity to the amino acid sequence of **SEQ ID NO. 66 and/or SEQ ID NO 67,** or a fragment thereof. Preferably, the prion protein comprises an amino acid sequence that, when compared to the amino acid sequences **SEQ ID NO. 66 or SEQ ID NO 67** comprises at least one substituted amino acid. When referring to the sequence **SEQ ID NO 66**, this at least one substituted amino acid preferably comprises at least one of the substitutions selected from the group of: D178N, V180I, E196K, E200K, V203I, R208H, V210I, E211Q, M232R, P102L, P105L, G131V, H187R, F198S, D202N, Q212P, Q217R, T183A, A117V, G127V, M129V, N171S, T183A, H187R, T188K, T188R, E196K, E200K, V203I, E211Q, E219K, P238S. Preferably, when referring to the sequence of SEQ ID NO. 66, the amino acid sequence of the prion protein comprises at least on missing amino acid(s). When referring to the sequence of **SEQ ID NO 66**, the at least one missing amino acid(s) preferably is missing at a positions selected from the group of 56-63. In preferred embodiments of the invention, the prion protein is the scrapie isoform of the prion protein (PrPSc).

When, in the context of the present invention, the at least one amino acid substitution and the at least one missing amino acid(s) of a protein is given in reference to a specific isoform of the protein, the corresponding amino acid sites of other isoforms of the protein are also meant to be included

When referring to "amino-acid substitutions" the nomenclature "AXB" indicates that the amino acid(s) A at the amino acid position X is exchanged for the amino acid(s) B. When referring to "missing amino acids" the numbers, for

example "7-13", indicate that, at the amino acid positions 7, 8, 9, 10, 11, 12 and 13, the amino acid(s) are missing.

In preferred embodiments, the condition or disease is selected from a condition or disease associated with (cellular) aging, infectious diseases, cancer and neurodegenerative conditions or diseases.

In preferred embodiments of the invention, the condition or disease is a PolyQ disease. Preferably, a PolyQ disease is a disease that is associated with an expansion of the poly-glutamine portion of huntingtin. Preferably, the PolyQ disease is Huntington's Disease, a spinocerebellar ataxia such as spinocerebellar ataxia 1, spinocerebellar ataxia 2, spinocerebellar ataxia 3 (i.e., MJD), spinocerebellar ataxia 6 (CACNL1A4), and/or spinocerebellar ataxia 7, spinal bulbar muscular atrophy, Kennedy's Disease, and/or dentatorubral-pallidoluysian atrophy. Huntington's Disease is associated with an expansion of the poly-glutamine ("polyQ") portion of HTT, above 36 glutamine ("Q") residues. Above the threshold of 36 glutamine residues, the huntingtin protein is considered to be mutated (mHTT). The consequences of the poly-glutamine expansion above 36 residues include the misfolding, loss of conformational flexibility, and/or aggregation of mHTT (see Fodale V et al. (2014). PLoS One 9(12):e112262; Cui X et al. (2014). Sci Rep 4:5601). Without wishing to be bound by theory, the conformational rigidity (i.e., reduced flexibility) is caused by an increase of alpha-helical content and mHTT and may be responsible for the pathological interactions of mHTT with different proteins in cells. This pathological protein-protein interactions can lead to the symptoms of Huntington's Disease (HD).

In preferred embodiments, the disease is a Lewy body associated disease. In the context of the invention, the term "Lewy body" refers to an aggregation of protein, preferably the α -synuclein, wherein the α -synuclein is optionally associated and/or aggregated with other proteins such as ubiquitin, neurofilament protein, and α-B crystallin. Preferably, the oligomers are comprise at least one protein type that is associated with a Lewy-body disease and/or the formation of Lewy bodies. Preferably, the Lewy bodies comprise Tau protein and/or are surrounded by neurofibrillary tangles.

In preferred embodiments of the invention, the condition or disease is a tauopathy. "Tauopathy" in the context of the invention refers to diseases that are associated with aggregation of the Tau protein and/or variants thereof. In preferred embodiments, the condition or disease is associated with neurofibrillary tangles, preferably wherein the neurofibrillary tangles comprise hyperphosphorylated tau protein. Preferably, the tauopathy comprises the formation of neurofibrillary tangles. Preferably, the tauopathy is Alzheimer's disease, primary age-related tauopathy dementia, chronic traumatic encephalopathy, progressive supranuclear palsy, corticobasal degeneration, frontotemporal dementia and parkinsonism linked to chromosome 17, lytico-bodig disease, ganglioglioma and gangliocytoma, meningioangiomatosis, postencephalitic parkinsonism, subacute sclerosing panencephalitis, lead encephalopathy, tuberous sclerosis, pantothenate kinase-associated neurodegeneration, lipofuscinosis and/or Pick's disease.

In preferred embodiments, the condition or disease is a synucleinopathy. "Synucleinopathy" in the context of the invention refers to diseases that are associated with aggregation and/or oligomerization of the protein α -synuclein and/or variants thereof. Preferably, the synucleinopathy is Parkinson's disease, dementia with Lewy Bodies, and/or multiple system atrophy.

In preferred embodiments of the invention, the condition or disease is Huntington's disease, spinocerebellar ataxia, Kennedy's disease, dentatorubral-pallidoluysian atrophy, multiple system atrophy, frontotemporal dementia, frontotemporal lobar degeneration (FTLD), Alzheimer's disease, Parkinson's disease, cerebral amyloid angiopathy (CAA), retinal disease glaucoma, progressive supranuclear palsy (PSP), corticobasal degeneration (CBD), Pick's disease, Argyrophilic grain disease, Parkinsonism linked to chromosome 17, familial amyloid polyneuropathy, leptomeningeal amyloidosis, CADASIL (Cerebral Autosomal Dominant Arteriopathy with Subcortical Infarcts and Leukoencephalopathy) syndrome, hereditary cystatin C amyloid angiopathy, chronic inflammation AA (amyloid A) amyloidosis, gelsolin amyloidosis, lattice corneal dystrophy type 2 (LCD2), retinal degradation, aortic aneurysm, aortic dissection, vascular dementia, isolated atrial amyloidosis (IAA), congestive heart failure, FUS-associated amyotrophic lateral sclerosis, FUS-associated dementia, Creutzfeldt-Jakob disease (CJD), Fatal Familial Insomnia (FFI), Gerstmann-Straussler-Scheinker disease (GSS), Huntington disease-like type 1 (HDL1), Kuru and Spongiform encephalopathy, an ischemic stroke, a ischemic heart disease, a viral infection, radiation caused brain function impairment amyotrophic lateral sclerosis (ALS), Lewy Body Dementia, Down's syndrome, type-2 diabetes mellitus and/or a prion disease, preferably wherein the disease is Lewy Body Dementia, Down's syndrome, type-2 diabetes mellitus, cerebral amyloid angiopathy (CAA), retinal disease glaucoma, progressive supranuclear palsy (PSP), corticobasal degeneration (CBD), Pick's disease, Argyrophilic grain disease, Parkinsonism linked to chromosome 17, familial amyloid polyneuropathy, leptomeningeal amyloidosis, CADASIL syndrome, hereditary cystatin C amyloid angiopathy, chronic inflammation AA (amyloid A) amyloidosis, gelsolin amyloidosis, lattice corneal dystrophy type 2 (LCD2), retinal degradation, aortic aneurysm, aortic dissection, vascular dementia, isolated atrial amyloidosis (IAA), congestive heart failure, FUS-associated amyotrophic lateral sclerosis, FUS-associated dementia, Creutzfeldt-Jakob disease (CJD), Fatal Familial Insomnia (FFI), Gerstmann-Straussler-Scheinker disease (GSS), Huntington disease-like type 1 (HDL1), Kuru and Spongiform encephalopathy, an ischemic stroke, an ischemic heart disease, a viral infection, radiation caused brain function impairment and/or a prion disease. In preferred embodiments of the invention the FTLD is FTLD-U, preferably, wherein FTLD-U is associated with cytoplasmic inclusions that stain positive for ubiquitin and negative for tau protein and alpha-synuclein. In preferred embodiments of the invention, the ALS is C9orf72-associated amyotrophic lateral sclerosis. In preferred embodiments of the invention, the viral disease

is COVID-19.

In preferred embodiments of the invention, the mutated, misfolded and/or denatured proteins are caused by radiation or due to hypoxic conditions and/or after reperfusion.

In preferred embodiments of the invention the reperfusion takes place after an ischemic event. Preferably, the ischemic event is an ischemic heart disease, a bowel ischemia, a brain ischemia (also known as ischemic stroke), an acute limb ischemia, a cutaneous ischemia, and/or a kidney ischemia. In preferred embodiments, the condition or disease is initiated by cerebral vascular occlusion such as an ischemic stroke.

In further preferred embodiments, the condition or disease is a secondary disease or side effect of, or caused by, radiation damage, such as from unintended radiation exposure, radiation therapy or radiation based diagnostics, and/or heat shock induced damages, chronic sleep deprivation, preferably wherein the condition is associated with radiation damage and/or chronic sleep deprivation. Preferably, the chronic sleep deprivation is associated with an accumulation of the oligomers, preferably wherein the oligomers comprise tau protein, amyloid-$\beta$, and/or $\alpha$-synuclein.

In further preferred embodiments, wherein the condition or disease associated with (cellular) aging is caused by a deterioration of autophagy activity.

In preferred embodiments, the use comprises bringing into contact a cell or tissue associated with the condition or disease of a subject to be treated with the protein-oligomer aggregation-agent. In preferred embodiments, the use involves an administration of a therapeutically effective amount of the protein-oligomer aggregation-agent to a subject suffering from the condition or disease. In preferred embodiments, the therapeutically effective amount is an amount that when in contact with a protein-oligomer associated with the condition or disease induces aggregation of two or more protein oligomers.

In preferred embodiments, administering to a subject in need thereof the therapeutic amount of the protein-oligomer aggregation-agent comprises local, enteral and/or parenteral administration.

Parental and/or injectable administration is generally used for subcutaneous, intramuscular or intravenous injections and infusions. Preferably, injectables are prepared in conventional forms, either as liquid solutions or suspensions or solid forms suitable for dissolving in liquid prior to injection. In preferred embodiments, the administration comprises using a patch, suppository, syringe, injector pen, spray bottle, mask, pastille and/or implant inhaler, nebulizer and/or vaporizer. Preferably, administration comprises intravenous, both bolus injection and infusion, intraperitoneal, subcutaneous or intramuscular administration, all using forms well known to those skilled in the pharmaceutical arts. Preferably, enteral administration comprises oral administration, gastric administration and rectal administration. In preferred embodiments of the invention, the local administration comprises administration of the protein-oligomer aggregation-agent to the CNS, preferably to the brain, preferably the tissue, cell, intracellular or extracellular fluid of any of the embodiments herein. Preferably, delivery of the protein-oligomer aggregation-agent to the central nervous system (e.g., parenchyma) comprises prolonged infusion into cerebrospinal fluid (CSF).

In preferred embodiments, the protein-oligomer aggregation-agent is released over time. Preferably, the protein-oligomer aggregation-agent is release over the course of 5 h, 10 h, 18 h, 24 h, 36 h, 48 h, 72 h, 1 week, 2 weeks, 1 month, 6 months, 12 months and/or 2 years. "Over the course" herein refers to a release, wherein a medicament comprising the protein-oligomer aggregation-agent continuously, periodically or non-periodically releases the protein-oligomer aggregation-agent to the body of the subject in need thereof. Preferably, the protein-oligomer aggregation-agent is released from an implant. Preferably, the implant comprises the protein-oligomer aggregation-agent of any embodiment of the invention.

In preferred embodiments, the treatment and/or prevention of a condition or disease is a first line, second line and/or third line therapy. In preferred embodiments, the treatment and/or prevention is a monotherapy or a combinational therapy. In preferred embodiments, the combinational therapy is a pharmaceutical combinational therapy, preferably a two-drug therapy. Preferably, the treatment and/or prevention is combined with at least one additional therapy selected from reperfusion therapy, gene therapy, a therapy using an additional drug. Preferably, the treatment or prevention is accompanied by psychotherapy, speech therapy and/or physical therapy. and/or occupational therapy

In a preferred embodiment of the invention, the agent further comprises at least one additional active compound.

Preferably, the additional drug and/or the additional active component is selected from at least one element of the group consisting of a benzodiazepine, tetrabenazine, deutetrabenazine, Haldol, risperidone, olanzapine, quetiapine, amantadine, levetiracetam, clonazepam. Preferably, the second/third drug and/or the additional active component is selected from at least one element of the group consisting of insulin, a amylinomimetic drug, alpha-glucosidase inhibitors, biguanides, dopamine agonsist, dipeptidyl peptidase-4 inhibitors, glucagon-like peptide-1 receptor agonsits, meglitinides, sodium-glucose transporter 2 inhibitors, sulfonylureas, pentosane polysulphate, astemizole, and/or thiazolidinediones.

In preferred embodiments, the treatment and/or prevention is a reperfusion therapy.

In preferred embodiments, administering to a subject in need thereof the therapeutic amount of the protein-oligomer aggregation-agent comprises administering the small molecule and/or peptide in a concentration of 1 to 500 mg/kg, preferably 1 to 400 mg/kg, preferably 1 to 300 mg/kg, preferably 1 to 200 mg/kg, preferably 5 to 100 mg/kg, preferably 10 to 50 mg/kg body weight of the subject in need thereof. In preferred embodiments, administering to a

subject in need thereof the therapeutic amount of the protein-oligomer aggregation-agent comprises administering the antibody and/or nanoparticle in a concentration of 0.1 to 50 mg/kg, preferably 0.2 to 40 mg/kg, preferably 0.5 to 30 mg/kg, preferably 0.5 to 20 mg/kg, preferably 1 to 20 mg/kg body weight of the subject in need thereof. In preferred embodiments, administering to a subject in need thereof the therapeutic amount of the protein-oligomer aggregation-agent comprises administering the nucleic acid and/or therapeutic protein in a concentration of 0.01 mg/kg to 500 mg/kg, preferably 0.1 to 500 mg/kg, preferably 1 to 500 mg/kg body weight of the subject in need thereof.

Preferably, effective dosage amounts of the disclosed compounds, when used for the indicated effects, range from about 0.5 mg to about 5000 mg of the disclosed compound as needed to treat the condition. Compositions for in vivo or in vitro use can contain about 0.5, 5, 20, 50, 75, 100, 150, 250, 500, 750, 1000, 1250, 2500, 3500, or 5000 mg of the disclosed compound, or, in a range of from one amount to another amount in the list of doses.

In further preferred embodiments of the present invention, a therapeutically effective amount of a protein-oligomer aggregation agent as disclosed herein before, for administration to a subject, is an amount sufficient to induce and/or enhance an aggregation of protein-oligomers at the body area where the pathology to be treated occurs, such as the central nervous system (CNS) (brain), or where an injury occurred. Accordingly, in order to administer such a therapeutically effective amount, the route of administration may be adjusted accordingly.

In preferred embodiments, the subject in need thereof has previously suffered traumatic brain injury. In preferred embodiments, the subject in need thereof is affected with Down syndrome. In preferred embodiments, the subject in need thereof suffers chronic insomnia for at least 20 years, preferably at least 15 years, preferably at least 10 years, preferably at least 5 years, preferably at least 2 years, preferably at least 1 year, preferably at least 6 months, most preferably at least 3 months. In preferred embodiments, the subject in need thereof is at least 90 years old, preferably at least 85 years old, preferably at least 80 years old, preferably at least 70 years old, preferably at least 60 years old, preferably at least 50 years old, preferably at least 30 years old, preferably at least 10 years old. In preferred embodiments of the invention, the subject in need thereof has suffered an ischemic event, preferably wherein the ischemic event is an ischemic stroke. The dosage regimen utilizing the disclosed compound is selected in accordance with a variety of factors including type, species, age, weight, sex and medical condition of the patient; the severity of the condition to be treated; the route of administration; the renal or hepatic function of the patient; and the particular disclosed compound employed. A physician or veterinarian of ordinary skill in the art can readily determine and prescribe the effective amount of the drug required to prevent, counter or arrest the progress of the condition.

In preferred embodiments of the invention, the treatment and/or prevention comprises a reduction or inhibition of the oligomers. In preferred embodiments of the invention, the reduction or inhibition of the oligomers comprises a change of the conformation of the proteins of the oligomers, a change of the surface properties of the oligomers and/or an aggregation of the oligomers, preferably an aggregation of the oligomers. In preferred embodiments of the invention, the change of the conformation of the proteins of the oligomers, the change of the surface properties of the oligomers and/or the aggregation of the oligomers is triggered by the binding of the protein-oligomer aggregation-agent of the oligomers. Preferably, the protein-oligomer aggregation-agent binds to an epitope found on the oligomers.

In preferred embodiments of the invention, the change of the conformation of the proteins of the oligomers, the change of the surface properties of the oligomers and/or the aggregation of the oligomers enhances the recognition of the oligomers by cellular degradation pathways. In preferred embodiments of the invention the enhanced recognition of the oligomers by cellular degradation pathways comprises an increased attachment of ubiquitin to the oligomers. Preferably, the binding of the protein-oligomer aggregation-agent results in the oligomers to be better recognized by ubiquitin ligases. In preferred embodiments of the invention, the degradation pathways comprise the ubiquitin proteasome system and/or autophagy. In preferred embodiments, the treatment and/or prevention comprises an induction and/or upregulation of autophagy. In preferred embodiments, the upregulation of autophagy is associated with an increased autophagic flux. In preferred embodiments, the reduction or inhibition partially or completely relies on the ubiquitin proteasome system. In preferred embodiments, the protein-oligomer aggregation-agent binds to the oligomers, preferably wherein the small molecule, the antibody, the nanoparticle, the nucleic acid, the peptide and/or the therapeutic protein binds to exposed hydrophobic domains of the oligomers. When referring to the binding of the protein-oligomer aggregation-agent to the oligomers, binding does not indicate any mechanism by which the protein-oligomer agent binds to the oligomer. Preferably, the protein-binding oligomer binds via ionic and/or covalent bond, including coordinative covalent bonds and/or pi-stacking. Preferably, the protein-binding agent binds to the protein-binding agent by "weak bonds", preferably by dipole-dipole interactions, van der Waals force and/or hydrogen bonding.

**In a third aspect, the invention pertains to a method for manufacturing a medicament** containing the protein-oligomer aggregation-agent of the previous aspects of the invention, comprising dissolving the protein-oligomer aggregation-agent in a physiologically acceptable solvent and/or water, yielding a stock solution of the protein-oligomer aggregation-agent. In a further aspect, the invention also pertains to a method for manufacturing a ready to use formulation containing the protein-oligomer aggregation-agent, the method comprising a step of admixing the protein-oligomer aggregation-agent or a stock solution of the protein-oligomer aggregation-agent with a pharmaceutically acceptable composition comprising a solvent and / or one or more pharmaceutically acceptable carriers/excipients to yield the ready to

use formulation.

Preferably, the medicament is prepared according to conventional mixing, granulating or coating methods, respectively, and the present medicament can contain from about 0.1% to about 99%, from about 5% to about 90%, or from about 1% to about 20% of the disclosed compound by weight or volume.

Preferably, the medicament is in the form of a tablet or capsule, preferably a gelatin capsule, comprising the compound of the invention and a pharmaceutically acceptable carrier. Preferably, the pharmaceutically acceptable carrier selected from at least one component of the group consisting of: a) a diluent, e.g., purified water, triglyceride oils, such as hydrogenated or partially hydrogenated vegetable oil, or mixtures thereof, corn oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride and/or polyethylene glycol; for tablets also; c) a binder, e.g., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes and/or polyvinylpyrrolidone, if desired; d) a disintegrant, e.g., starches, agar, methyl cellulose, bentonite, xanthan gum, algiic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the compound such as cyclodextrin, hydroxypropyl-cyclodextrin, PEG400, PEG200.

Preferably, medicaments in a liquid form, preferably injectable medicaments, can be prepared by dissolution, dispersion, etc. Preferably, the disclosed compound is dissolved in or mixed with a pharmaceutically acceptable solvent such as, for example, water, saline, aqueous dextrose, glycerol, ethanol, and the like. Preferably, the medicament is an injectable isotonic solution or suspension. Preferably, proteins such as albumin, chylomicron particles, or serum proteins can be used to solubilize the disclosed compounds.

Preferably, the compound of the invention can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. Preferably, a film of lipid components is hydrated with an aqueous solution of drug to a form lipid layer encapsulating the drug, as described in U.S. Pat. No. 5,262,564.

In preferred embodiments, the preferred dosage form is selected from the group consisting of pill (such as tablet or capsule) pastille, powder, thin film, solution, syrup, suspension, emulsion, paste, gel, softgel, aerosol and/or smoke)

Depending on the intended mode of administration, the disclosed compositions can be in solid, semi-solid or liquid dosage form, such as, for example, injectables, tablets, suppositories, pills, time-release capsules, elixirs, tinctures, emulsions, syrups, powders, liquids, suspensions, or the like, sometimes in unit dosages and consistent with conventional pharmaceutical practices. Preferably, the medicament is a suppository, preferably prepared from fatty emulsions or suspensions using polyalkylene glycols such as propylene glycol, as a carrier.

In a preferred embodiment of the invention, the method of manufacturing a medicament further comprises a manufacturing step to yield a preferred dosage form.

The term "administer", "administering", or "administration" as used in this disclosure refers to either directly administering a disclosed compound or pharmaceutically acceptable salt of the disclosed compound or a composition to a subject, or administering a prodrug derivative or analog of the compound or pharmaceutically acceptable salt of the compound or composition to the subject, which can form an equivalent amount of active compound within the subject's body.

**In a fourth aspect, the invention pertains to a method for increasing the lifespan of a subject**, wherein the method comprises administering a therapeutically effective amount of the protein-oligomer aggregation-agent of the previous aspects of the invention.

Without being bound to theory, the impact of cellular aging which includes the accumulation of harmful protein fragments that form protein-oligomers of the invention can be prevented, reduced of removed by applying the teaching of the present invention. Hence, administration of the compounds and/or compositions of the invention results in a beneficial extension of life span compared to non-administration of the compound. Furthermore, the invention pertains to methods for preventing and/or reducing any signs of ageing in a subject comprising the administration of the compounds of the invention to the subject. Such method therefore reduces the toxic effect induced by protein-oligomers which may negatively influence life-span, and or negatively influence any other condition or sign associated with a subject's ageing.

**In a fifth aspect, the invention pertains to a method for neuroprotection**, wherein the method comprises administering a therapeutically effective amount of the protein-oligomer aggregation-agent of the previous aspects of the invention.

In this aspect, the present invention relates to a preventive application of the compounds and compositions disclosed herein in order to reduce the likelihood of a subject to develop an adverse neurological condition. Neuroprotection in the present disclosure therefore pertains in certain embodiments to the prevention of a further loss of neuronal cells, or loss of neuronal function as a result of exposure to a neurotoxin protein-oligomer and/or resulting from a neurodegenerative disease or disorder. In preferred embodiments of this aspect the method comprises the administration of an amount of the compound or composition to the subject which is effective in reducing neuronal cell loss, or increasing neuronal cell survival.

**In a sixth aspect, the invention pertains to a method for inducing autophagy**, wherein the method comprises administering a therapeutically effective amount of the previous aspects of the invention.

**In a seventh aspect, the invention pertains to a diagnostic method for detecting** a disease associated with an accumulation of protein-oligomers in a tissue, cell, intracellular- or extracellular fluid of a subject, wherein the method comprises:

(i) Providing a sample (of the tissue, cell, intracellular- or extracellular fluid of the subject), optionally wherein the sample is a biopsy of a tissue or bodyfluid associated with the disease in the subject;

(ii) Bringing into contact the sample and the protein-oligomer aggregation-agent of the previous aspects of the invention;

(iii)Detecting the presence or absence of aggregated protein-oligomer in the sample, wherein the presence or absence of aggregated protein-oligomer in the sample is indicative of a disease associated with an accumulation of oligomers in a tissue cell, intracellular or extracellular fluid.

Preferably, the disease is at least one of the conditions and/or diseases of the previous embodiments of the invention. Preferably, the method is an *in-vitro* assay. Preferably, the biopsy is a sample of the tissue, cell, intracellular or extracellular fluid. Optionally, the method contains a step (i-b): purifying the oligomer from the biopsy.

Preferably, the method is based on detecting an aggregation behavior of the oligomer, when subjected to the protein-oligomer binding agent. The aggregation behavior comprises measuring particle size, preferably particle size distribution and/or surface charge such as the particles' Zeta-potential of the oligomers or aggregates thereof. In this context, "particle" as in "particle size distribution" refers to the oligomers and/or oligomer-aggregates. Preferably, the method comprises comparing the aggregation behavior of the oligomers prior and after addition of the protein-oligomer aggregation-agent.

Preferably, the presence or absence of aggregated polymers in the sample is detected by a method that is capable of determining particle size. In preferred embodiments of the invention, step(iii) comprises conducting spectroscopic methods, such as dynamic light scattering, static light scattering (preferably multi-angle static light scattering) and/or nanoparticle tracking analysis. Preferably, step (iii) comprises the use microscopy based methods, preferably wherein the microscopy based methods are comprise electron microscopy such as scanning electron microscopy. Preferably, step (iii) comprises small angle X-ray scattering, centrifuging based methods (preferably differential centrifugation, density gradient centrifugation and/or analytical ultracentrifugation), sieving and/or chromatography based methods (such as size exclusion chromatography, liquid chromatography, high performance liquid chromatography and/or ultrahigh pressure liquid chromatography) and/or methods based on electrophoresis (such as capillary zone electrophoresis, electrophoretic light scattering). Preferably step (iii) comprises the use of mass correlation spectroscopy. In preferred embodiments, step (iii) comprises combinations of any of the methods mentioned herein.

**In an eight aspect, the invention pertains to a diagnostic method for detecting** a disease associated with an accumulation of oligomers in a tissue, in a cell, or in an intracellular- or extracellular fluid, wherein the method comprises:

(i) Providing a sample, optionally wherein the sample is a biopsy sample of a subject;

(ii) Bringing into contact the sample and the protein-oligomer aggregation-agent of the previous aspects of the invention;

(iii)Detecting a binding of the protein-oligomer aggregation-agent to the protein-oligomer, wherein the presence or absence of binding is indicative of a disease associated with an accumulation of oligomers in a tissue cell, intracellular or extracellular fluid.

Preferably, the disease is at least one of the conditions and/or diseases of the previous embodiments of the invention. Preferably, the method is an *in-vitro* assay. Preferably, the biopsy is a sample of the tissue, cell, intracellular or extracellular fluid. Optionally, the method contains a step (i-b): purifying the oligomer from the biopsy.

Preferably, the detection of the potential binding of the protein-oligomer aggregation-agent to the oligomer comprises fixating the protein-oligomer aggregation-agent to a surface. Preferably, the surface is a particle, such as a nanoparticle or microparticle, or a well. Preferably, the binding is detected by a microscopy or spectroscopy based technique or immunological assay, preferably UV/Vis or fluorescence spectroscopy. Preferably the method comprises the use of a dye-tagged protein-oligomer aggregation agent, preferably wherein the dye is a fluorescent dye. Preferably, the detection of the potential binding of the protein-oligomer aggregation-agent comprises the use of a dye or dye-tagged compound that is capable of binding to the protein and/or oligomer. Preferably, the dye-tagged compound is a dye-tagged antibody.

In a **further aspect** of the present invention there is provided a method for removing protein-oligomers in a subject, the method comprising a step of contacting the protein-oligomers with a protein-oligomer aggregation agent of the present invention, and thereby induce protein-oligomer aggregation. In context of the present invention it was surprisingly found that compounds disclosed herein which have an effect to enhance protein-oligomer aggregation. The aforementioned aspects and embodiments with respect to treatments or uses in treatments of certain diseases associated with pathological protein-oligomers shall apply similarly in this aspect.

In yet **another aspect** the invention pertains to a method for the identification of a compound useful in the treatment of a condition associated with the presence of (pathological and/or toxic) protein oligomers, the method comprising the steps of:

(i) Contacting a candidate compound with at least two of the protein-oligomers associated with the condition;

(ii) Determining the formation of an oligomer-aggregate composed of a plurality of protein oligomers of the at least two protein-oligomers associated with the condition in the presence of the candidate compound compared to the formation of an oligomer-aggregate composed of a plurality of protein oligomers of the at least two protein-oligomers associated with the condition in the absence of the candidate compound;

Wherein an increased formation of an oligomer aggregate in the presence of the compound compared to the absence of the compound indicates that the compound is useful in the treatment of a condition associated with the presence of the pathological/toxic protein oligomer.

Preferably, the method is in-vitro assay. Preferably, the disease is at least one of the conditions and/or diseases of the previous embodiments of the invention. In preferred embodiments of the invention, contacting the candidate compound with at least two of the protein-oligomers associated with the condition is preceded by isolating the at least two protein-oligomers associated with the condition from a subject. Preferably, isolating the at least two protein-oligomers associated with the condition from a subject comprises taking a sample of the subjects tissue, cell, intracellular or extracellular fluid. Preferably, isolating the at least two protein-oligomers associated with the condition from a subject further comprises purifying the at least two protein-oligomers.

In preferred embodiments, step (ii) comprises measuring the particle size distribution, overall particle number, particle weight and/or optical density in a sample comprising the oligomers and/or oligomer aggregates prior and after exposure to the protein-oligomer aggregation agent. In preferred embodiments, a decreasing overall particle number indicates an increased formation of oligomer-aggregates. In preferred embodiments, a particle size distribution that is shifted to larger particle sizes indicates an increased formation of oligomer-aggregates. In preferred embodiments, increase in the optical density of a solution is indicative for an increased formation of oligomer-aggregates. Aggregation of the oligomers increases the average weight of the particles, such as protein-oligomers or aggregates thereof, in a solution. Preferably, an increase of the average particle weight of the particles in a solution is indicative for an increased formation of an oligomer aggregate.

In preferred embodiments of the invention, step(ii) comprises conducting spectroscopic methods, such as dynamic light scattering, static light scattering (preferably multi-angle static light scattering) and/or nanoparticle tracking analysis. Preferably, step (ii) comprises the use microscopy based methods, preferably wherein the microscopy based methods are comprise electron microscopy such as scanning electron microscopy. Preferably, step (ii) comprises small angle X-ray scattering, centrifuging based methods(preferably differential centrifugation, density gradient centrifugation and/or analytical ultracentrifugation), sieving and/or chromatography based methods (such as size exclusion chromatography, liquid chromatography, high performance liquid chromatography and/or ultrahigh pressure liquid chromatography) and/or methods based on electrophoresis (such as capillary zone electrophoresis, electrophoretic light scattering, preferably gel electrophoresis. Preferably step(ii) comprises the use of mass correlation spectroscopy. In preferred embodiments, step (ii) comprises combinations of any of the methods mentioned herein.

In a preferred embodiment of the invention, the condition is a neurodegenerative disorder as defined elsewhere herein, and/or is a condition associated with aging, which may include any pathology associated with aging and/or signs of aging in the appearance of a subject, such as in the skin or hair.

In certain additional preferred embodiments the present invention further pertains to the following itemized

embodiments:

Item 1: A protein-oligomer aggregation-agent, wherein the protein-oligomer aggregation agent comprises a small molecule, an antibody, a nanoparticle, a nucleic acid, a peptide and/or a therapeutic protein, and preferably is a compound as disclosed in the above paragraphs 50-92, in particular a compound of table 1. Such compounds of item 1 are preferably characterized in that they induce aggregation of a protein-oligomer as described herein elsewhere.

Item 2: The protein-oligomer aggregation-agent of item 1 for use in the treatment and/or prevention of a condition or disease. IN this item it is preferred that the protein-oligomer is toxic and/or harmful to a biological cell, such as a protein-oligomer that is neurotoxic, such as toxic to a neuronal cell.

Item 3: The protein-oligomer aggregation-agent for use of item 2, wherein the condition or disease is caused by a pathological aberration in cellular protein homeostasis, preferably cellular protein metabolism, for example in the central nervous system (CNS). More preferably, the pathological aberration is caused by the presence of a protein-oligomer.

Item 4: The protein-oligomer aggregation-agent for use of item 2 or 3, wherein the condition or disease is associated with, or caused by, an aggregation of protein-oligomers in a tissue, cell, intracellular or extracellular fluid, preferably within the CNS. The therapeutic effect is therefore achieved in context of the invention by inducing aggregation of the protein-oligomer into protein-oligomer-aggregate(s) such as a protein fibril.

Item 5: The protein-oligomer aggregation-agent for use of any one of items 2 to 4, wherein the condition or disease is selected from a condition or disease associated with (cellular) aging, infectious diseases, cancer and neurodegenerative conditions or diseases. Preferably, the protein-oligomer aggregation-agent is administered to the subject in an amount and route suitable for inducing or enhancing protein-oligomer aggregation of the protein oligomers associated with the disease to be treated in the subject.

Item 6: The protein-oligomer aggregation-agent for use of any one of items 2 to 5, wherein the condition or disease is a secondary disease or side effect of, or caused by, radiation damage, such as from unintended radiation exposure, radiation therapy or radiation based diagnostics, and/or heat shock induced damages, chronic sleep deprivation, preferably wherein the condition is associated with radiation damage and/or chronic sleep deprivation.

Item 7: The protein-oligomer aggregation-agent for use of item 5 or 6, wherein the condition or disease associated with (cellular) aging is caused by a deterioration of autophagy activity.

Item 8: The protein-oligomer aggregation-agent for use of any one of items 2 to 7, wherein the condition or disease is Huntington's disease, spinocerebellar ataxia, Kennedy's disease, dentatorubral-pallidoluysian atrophy, multiple system atrophy, frontotemporal dementia, frontotemporal lobar degeneration (FTLD), Alzheimer's disease, Parkinson's disease, cerebral amyloid angiopathy (CAA), retinal disease glaucoma, progressive supranuclear palsy (PSP), corticobasal degeneration (CBD), Pick's disease, Argyrophilic grain disease, Parkinsonism linked to chromosome 17, familial amyloid polyneuropathy, leptomeningeal amyloidosis, CADASIL (Cerebral Autosomal Dominant Arteriopathy with Subcortical Infarcts and Leukoencephalopathy) syndrome, hereditary cystatin C amyloid angiopathy, chronic inflammation AA (amyloid A) amyloidosis, gelsolin amyloidosis, lattice corneal dystrophy type 2 (LCD2), retinal degradation, aortic aneurysm, aortic dissection, vascular dementia, isolated atrial amyloidosis (IAA), congestive heart failure, FUS-associated amyotrophic lateral sclerosis, FUS-associated dementia, Creutzfeldt-Jakob disease (CJD), Fatal Familial Insomnia (FFI), Gerstmann-Straussler-Scheinker disease (GSS), Huntington disease-like type 1 (HDL1), Kuru and Spongiform encephalopathy, an ischemic stroke, a ischemic heart disease, a viral infection, radiation caused brain function impairment amyotrophic lateral sclerosis (ALS), Lewy Body Dementia, Down's syndrome, type-2 diabetes mellitus and/or a prion disease, preferably wherein the disease is Lewy Body Dementia, Down's syndrome, type-2 diabetes mellitus, cerebral amyloid angiopathy (CAA), retinal disease glaucoma, progressive supranuclear palsy (PSP), corticobasal degeneration (CBD), Pick's disease, Argyrophilic grain disease, Parkinsonism linked to chromosome 17, familial amyloid polyneuropathy, leptomeningeal amyloidosis, CADASIL syndrome, hereditary cystatin C amyloid angiopathy, chronic inflammation AA (amyloid A) amyloidosis, gelsolin amyloidosis, lattice corneal dystrophy type 2 (LCD2), retinal degradation, aortic aneurysm, aortic dissection, vascular dementia, isolated atrial amyloidosis (IAA), congestive heart failure, FUS-associated amyotrophic lateral sclerosis, FUS-associated dementia, Creutzfeldt-Jakob disease (CJD), Fatal Familial Insomnia (FFI), Gerstmann-Straussler-Scheinker disease (GSS), Huntington disease-like type 1 (HDL1), Kuru and Spongiform encephalopathy, an ischemic stroke, an ischemic heart disease, a viral infection, radiation caused brain function impairment and/or a prion disease.

Item 9: The protein-oligomer aggregation agent for use of item 8, wherein the FTLD is FTLD-U, preferably wherein the FTLD-U is associated with cytoplasmic inclusions that stain positive for ubiquitin and negative for tau protein and alpha-synuclein.

Item 10:The protein-oligomer aggregation agent for use of item 8 or 9, wherein the ALS is C9orf72-associated amyotrophic lateral sclerosis.

Item 11:The protein-oligomer aggregation agent for use of any one of items 8 to 10, wherein the viral disease is

COVID-19.

Item 12:The protein-oligomer aggregation-agent for use of any one of items 2 to 11, wherein the condition or disease is a PolyQ disease.

Item 13:The protein-oligomer aggregation-agent for use of any one of items 2 to 12, wherein the condition or disease is a tauopathy.

Item 14:The protein-oligomer aggregation-agent for use of any one of items 2 to 13, wherein the condition or disease is a synucleinopathie.

Item 15:The protein-oligomer aggregation-agent for use of any one of items 2 to 14, wherein the condition or disease is initiated by cerebral vascular occlusion such as an ischemic stroke.

Item 16:The protein-oligomer aggregation-agent for use of any one of items 4 to 15, wherein the tissue, cell, intracellular or extracellular fluid is from the central nervous system (CNS), preferably wherein the tissue, cell, intracellular or extracellular fluid is from areas of the brain.

Item 17:The protein-oligomer aggregation-agent for use of any one of items 4 to 16, wherein the tissue, cell, intracellular or extracellular fluid is a striatal tissue, cell, intracellular or extracellular fluid.

Item 18:The protein-oligomer aggregation-agent for use of any one of items 4 to 17, wherein the cell is a neuron, preferably wherein the cell is a motor neuron.

Item 19:The protein-oligomer aggregation-agent for use of any one of items 4 to 18, wherein the protein-oligomers comprise at least one constituent selected from lipids, damaged organelles and/or proteins.

Item 20:The protein-oligomer aggregation-agent for use of item 19, wherein the proteins are mutated, misfolded and/or denatured proteins, preferably wherein the proteins are denatured proteins.

Item 21:The protein-oligomer aggregation-agent for use of any one of items 4 to 20, wherein the oligomer is a soluble oligomer, an aggregate or a fibre.

Item 22:The protein-oligomer aggregation-agent for use of any one of items 20 or 21, wherein the oligomers comprise 2 to 50 monomers of the proteins, preferably the mutated, misfolded and/or denatured proteins.

Item 23:The protein-oligomer aggregation-agent for use of any one of items 20 or 21, wherein the mutated, misfolded and/or denatured proteins are caused by radiation, due to hypoxic conditions and/or after reperfusion.

Item 24:The protein oligomer-aggregation agent for use of item 23, wherein the reperfusion takes place after an ischemic event.

Item 25:The protein-oligomer aggregation-agent for use of any one of items 19 to 24, wherein the proteins are selected from one or more of the group of α synuclein, amyloid β, mutated huntingtin, tau protein, prion proteins, misfolded superoxide dismutase 1 (SOD1) polymorphisms, isled amyloid polypeptide (IAPP), Musashi protein, TAR DNA-binding protein 43 (TDP-43), misfolded transthyretin protein (TTR), NOTCH3 receptor, mutated cystatin C, serum amyloid A (SAA), mutated gelsolin, misfolded rhodopsin, medin, dipeptide repeat protein, atrial natriuretic peptide, fused-in-sarcoma, insulin oligomers, or fragments truncated, and/or alternatively spliced variants and/or fusion proteins thereof, preferably wherein the proteins are selected from one or more of the group αsynuclein, amyloid β prion proteins, misfolded superoxide dismutase 1 (SOD1) polymorphisms, isled amyloid polypeptide (IAPP), Musashi protein, TAR DNA-binding protein 43 (TDP-43), misfolded transthyretin protein (TTR), NOTCH3 receptor, mutated cystatin C, serum amyloid A (SAA), mutated gelsolin, misfolded rhodopsin, medin, glycine-alanine dipeptide repeats, atrial natriuretic peptide, fused-in-sarcoma, insulin oligomers, or fragments, truncated, and/or alternatively spliced variants and/or fusion proteins thereof.

Item 26:The protein-oligomer aggregation agent for use of item 25, wherein the dipeptide repeat protein is a glycine-alanine dipeptide repeat protein.

Item 27:The protein-oligomer aggregation-agent for use of item 25, wherein the mutated huntingtin comprises an expanded polyQ repeat.

Item 28:The protein-oligomer aggregation agent for use of item 25 to 27, wherein the mutated huntingtin is truncated mutated huntingtin.

Item 29:The protein-oligomer aggregation-agent for use of item 25 to 28, wherein the mutated huntingtin is Exon 1 truncated mutated huntingtin.

Item 30:The protein-oligomer aggregation-agent for use of any one of items 4 to 29, wherein the oligomers are Lewy bodies.

Item 31:The protein-oligomer aggregation-agent for use of any one of items 25 to 30, wherein the α synuclein is mutated α synuclein.

Item 32:The protein-oligomer aggregation-agent for use of any one of items 25 to 31, wherein the tau protein is mutated tau, truncated tau and/or hyperphosphorylated tau protein.

Item 33:The protein-oligomer aggregation-agent for use of any one of items 2 to 32, wherein the condition or disease is associated with neurofibrillary tangles, preferably wherein the neurofibrillary tangles comprise hyperphosphorylated tau protein.

Item 34:The protein-oligomer aggregation-agent for use of items 25 to 33, wherein the prion protein is the scrapie

isoform of the prion protein (PrPSc).

Item 35:The protein-oligomer aggregation-agent for use of items 25 to 34, wherein the Musashi protein is Musashi protein 1 and/or Musashi protein 2.

Item 36:The protein-oligomer aggregation agent for use of items 25 to 35, wherein the TDP-43 is wildtype, truncated TDP-43, hyperphosphorylated TDP-43 and/or splice variants thereof.

Item 37:The protein aggregation agent for use of items 35 to 36, wherein the misfolded transthyretin protein is truncated TTR.

Item 38:The protein-oligomer aggregation-agent for use of any one of items 2 to 37, wherein the use comprises bringing into contact a cell or tissue associated with the condition or disease of a subject to be treated with the protein-oligomer aggregation-agent.

Item 39:The protein-oligomer aggregation-agent for use of item 38, wherein the use involves an administration of a therapeutically effective amount of the protein-oligomer aggregation agent to a subject suffering from the condition or disease,

Item 40:The protein-oligomer aggregation-agent for use of item 39, wherein the therapeutically effective amount is an amount that when in contact with a protein-oligomer associated with the condition or disease induces aggregation of two or more protein oligomers.

Item 41:A method for the treatment and/or prevention of a condition or a disease, wherein the method comprises administering to a subject in need thereof a therapeutically effective amount of the protein-oligomer aggregation-agent recited in any one of items 1 to 40.

Item 42:The method of item 41, wherein administering to a subject in need thereof the therapeutic amount of the aggregation agent comprises local, enteral and/or parenteral administration.

Item 43:The method of item 42, wherein the local administration comprises administration of the aggregation agent to the CNS, preferably to the brain, preferably the tissue, cell, intracellular or extracellular fluid of any of the previous items.

Item 44:The method of item 42 or 44, wherein the administration comprises using a patch, suppository, syringe, injector pen, spray bottle, mask, pastille and/or implant inhaler, nebulizer and/or vaporizer.

Item 45:The method of any one of items 42 to 44, wherein the aggregation agent is released over time.

Item 46:The method of any one of items 41 to 45, wherein the treatment and/or prevention of a condition or disease is a first line, second line and/or third line therapy.

Item 47:The method of any one of items 41 to 46, wherein the treatment and/or prevention is a monotherapy or a combinational therapy.

Item 48:The method of item 47, wherein the combinational therapy is a pharmaceutical combinational therapy, preferably a two-drug therapy.

Item 49:The method of any one of items 41 to 48, wherein the treatment and/or prevention is a reperfusion therapy.

Item 50:The method of any one of items 41 to 49, wherein administering to a subject in need thereof the therapeutic amount of the protein-oligomer aggregation-agent comprises administering the small molecule and/or peptide in a concentration of 1 to 500 mg/kg, preferably 1 to 400 mg/kg, preferably 1 to 300 mg/kg, preferably 1 to 200 mg/kg, preferably 5 to 100 mg/kg, preferably 10 to 50 mg/kg body weight of the subject in need thereof.

Item 51:The method of any one of items 41 to 50, wherein administering to a subject in need thereof the therapeutic amount of the protein-oligomer aggregation-agent comprises administering the antibody and/or nanoparticle in a concentration of 0.1 to 50 mg/kg, preferably 0.2 to 40 mg/kg, preferably 0.5 to 30 mg/kg, preferably 0.5 to 20 mg/kg, preferably 1 to 20 mg/kg body weight of the subject in need thereof.

Item 52:The method of any one of items 41 to 51, wherein administering to a subject in need thereof the therapeutic amount of the protein-oligomer aggregation-agent comprises administering the nucleic acid and/or therapeutic protein in a concentration of 0.01 mg/kg to 500 mg/kg, preferably 0.1 to 500 mg/kg, preferably 1 to 500 mg/kg body weight of the subject in need thereof.

Item 53:The method of any one of items 41 to 52, wherein the treatment and/or prevention comprises a reduction or inhibition of the oligomers.

Item 54:The method of item 53, wherein the reduction or inhibition of the oligomers comprises a change of the conformation of the proteins, a change of the surface properties of the oligomers and/or an aggregation of the oligomers, preferably an aggregation of the oligomers.

Item 55:The method of any one of items 41 to 54, wherein the treatment and/or prevention comprises an induction and/or upregulation of autophagy.

Item 56:The method of any one of items 55, wherein the upregulation of autophagy is associated with an increased autophagic flux.

Item 57:The method of any one of items 53 to 56, wherein the reduction or inhibition partially or completely relies on the ubiquitin proteasome system.

Item 58:The method of any one of items 41 to 57, wherein the protein-oligomer aggregation-agent binds to the

proteins and/or oligomers, preferably wherein the small molecule, the antibody, the nanoparticle, the nucleic acid, the peptide and/or the therapeutic protein binds to exposed hydrophobic domains of the oligomers.

Item 59:The method of any one of items 53 to 58, wherein the change of the conformation of the proteins of the oligomers, change of the surface properties of the oligomers and/or the aggregation of the oligomers enhances the recognition of the oligomers by cellular degradation pathways.

Item 60:The method of any one of items 53 to 59, wherein the change of the conformation of the proteins of the oligomers, change of the surface properties of the oligomers and/or the aggregation of the oligomers is triggered by the binding of the protein-oligomer aggregation agent of the oligomers

Item 61:The method of any one of items 59 or 60, wherein the enhanced recognition of the oligomers by cellular degradation pathways comprises an increased attachment of ubiquitin to the oligomers.

Item 62:The method of any one of items 56 to 61, wherein the degradation pathways comprise the ubiquitin proteasome system and/or autophagy.

Item 63:The method of any one of items 41 to 62, wherein the subject in need thereof has previously suffered traumatic brain injury.

Item 64:The method of any one of items 41 to 63, wherein the subject in need thereof is affected with Down syndrome.

Item 65:The method of any one of items 41 to 64, wherein the subject in need thereof suffers chronic insomnia for at least 20 years, preferably at least 15 years, preferably at least 10 years, preferably at least 5 years, preferably at least 2 years, preferably at least 1 year, preferably at least 6 months, most preferably at least 3 months.

Item 66:The subject of any one of items 41 to 65, wherein the subject in need thereof is at least 90 years old, preferably at least 85 years old, preferably at least 80 years old, preferably at least 70 years old, preferably at least 60 years old, preferably at least 50 years old, preferably at least 30 years old, preferably at least 10 years old.

Item 67:The subject of any one of items 41 to 66, wherein the subject in need thereof has suffered an ischemic event, preferably wherein the ischemic event is an ischemic stroke.

Item 68:A method for manufacturing a medicament containing the aggregation agent of item 1 and/or the aggregation agent for use of any one of items 2 to 40, comprising dissolving the aggregation agent in a physiologically acceptable solvent and/or water yielding a stock solution of the aggregation agent.

Item 69:The method of manufacturing a medicament of item 68, further comprising a manufacturing step to yield a preferred dosage form.

Item 70:The method for manufacturing a medicament of item 69, wherein the preferred dosage form is selected from the group consisting of pill (such as tablet or capsule) pastille, powder, thin film, solution, syrup, suspension, emulsion, paste, gel, softgel, aerosol and/or smoke)

Item 71:A method for increasing the lifespan of a subject, wherein the method comprises administering a therapeutically effective amount of the aggregation agent.

Item 72:A method for neuroprotection, wherein the method comprises administering a therapeutically effective amount of the aggregation agent of any one of the previous items.

Item 73:A method for inducing autophagy, wherein the method comprises administering a therapeutically effective amount of the aggregation agent of any one of the previous items.

Item 74:A diagnostic method for detecting a disease associated with an accumulation of oligomers in a tissue, cell, intracellular or extracellular fluid, wherein the method comprises:

(i) Providing a sample, optionally wherein the sample is provided via a biopsy of a subject;
(ii) Adding the protein-oligomer aggregation-agent of any one of the previous items;
(iii) Detecting the presence or absence of aggregated polymers in the sample,

wherein the presence or absence of aggregated polymers in the sample is indicative of a disease associated with an accumulation of oligomers in a tissue cell, intracellular or extracellular fluid.

Item 75:A diagnostic method for detecting a disease associated with an accumulation of oligomers in a tissue, cell, intracellular or extracellular fluid, wherein the method comprises:

(i) Providing a sample, optionally wherein the sample is provided via a biopsy of a subject;
(ii) Adding the protein-oligomer aggregation-agent of any one of the previous items;
(iii) Detecting a potential binding of the protein-oligomer aggregation-agent to the oligomers,

wherein the presence or absence of binding is indicative of a disease associated with an accumulation of oligomers in a tissue cell, intracellular or extracellular fluid.

Item 76:The protein-oligomer aggregation-agent of item 1, wherein the agent further comprises at least one additional active compound.

Item 77:The protein-oligomer aggregation-agent of item 1, wherein the protein-oligomer aggregation agent further

comprises a pharmaceutical acceptable carrier and/or excipient.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by $\pm$20%, $\pm$15%, $\pm$10%, and for example $\pm$5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

BRIEF DESCRIPTION OF THE FIGURES AND SEQUENCES

The figures show:

**Figure 1** is a graph showing the saturation kinetics of 3B5H10 MAb on Mur46 and Mur16 coupled flow cells. 3B5H10 MAb (Sigma Aldrich, Saint Louis, USA) was diluted 1:600 and flowed over all four flow cells to the point of saturation using injections. The y-axis shows the Response Units (RU). The x-axis shows time (seconds).

**Figure 2** is a graph showing the concentration of Compound 22 that penetrated the blood brain barrier, in vivo. Male CD1 mice were treated with Compound 22 at a dose of 33.3 mg/kg body weight. Following isolation of proteins precipitated from homogenized mouse brain tissue, the concentration of Compound 22 was determined using Ultra-High Performance Liquid Chromatography combined with Time-of-Flight Mass Spectrometry (UHPLC-TOF). Compound 22 penetrates the blood brain area.

**Figure 3** is a bar graph showing the nuclear diffuse mHTT levels of STHdh 111/111 cells and StHdh 7/7 cells treated with Compound 22. Nuclear diffuse mHTT was significantly reduced by 32% (p = 0.02) in STHdh 111/111 cells treated with Compound 22 in comparison to untreated STHdh 111/111 cells. But wildtype huntingtin was not reduced in STHdh 7/7 cells treated with Compound 22 in comparison to non-treated STHdh 7/7.

**Figure 4** is a line graph showing the cell viability of STHdh7/7 and STHdh 111/111 cells treated with Compound 22. Heat shock triggers accumulation of misfolded proteins which are degraded. Cell viability was in Compound 22 treated striatal neurons with mHTT almost at wildtype level in the first 24h ctr control.

**Figure 5** is a bar graph showing the cell viability of STHdh 7/7 striatal cells following treatment with heat shock and small molecule compounds at 10$\mu$M.

**Figure 6** is a schematic diagram of the study of Compound 22 on motor behavior in R6/2 mice of Huntington's disease. Motor function testing using rotarod were commenced at 4 weeks (pre-treatment baseline) and continued at 6, 8 and 10 weeks of age, accompanied with grip strength at 4 weeks (pre-treatment baseline), 10 and 12 weeks of age. At the end point of 12 weeks of age, the mice were subjected to tissue collection.

**Figure 7** (**A**) is a line graph showing the effects of chronic administration of Compound 22 (33 mg/kg) on body weight of pooled genders of R6/2 mice aged 3 to 12 weeks. Data are presented as mean ± SEM (WT Vehicle, n = 10; R6/2 Vehicle, n = 10; R6/2 Compound 22 33mg/kg, n = 10). # $p < 0.05$, R6/2 Vehicle vs. WT Vehicle. **Figure 7** (**B**) is a line graph showing the effects of chronic administration of Compound 22 (33 mg/kg) on body weight of female R6/2 mice aged 3 to 12 weeks. Data are presented as mean ± SEM (WT Vehicle, n = 5; R6/2 Vehicle, n = 5; R6/2 Compound 22 33mg/kg, n = 5). # $p < 0.05$, R6/2 Vehicle vs. WT Vehicle. **Figure 7** (**C**) is a line graph showing the effects of chronic administration of Compound 22 (33 mg/kg) on body weight of male R6/2 mice aged 3 to 12 weeks. Data are presented as mean ± SEM (WT Vehicle, n = 5; R6/2 Vehicle, n = 5; R6/2 Compound 22 33mg/kg, n = 5). # $p < 0.05$, R6/2 Vehicle vs. WT Vehicle.

**Figure 8** is a graph showing the effects of chronic administration of Compound 22 (33 mg/kg) on rotarod latency of pooled genders of R6/2 mice at 4 to 10 weeks of age. Data are presented as mean ± SEM (WT Vehicle, n = 10; R6/2 Vehicle, n = 10; R6/2 Compound 22 33 mg/kg, n = 10). # $p < 0.05$, R6/2 Vehicle vs. WT Vehicle.

**Figure 9** is a bar graph showing the effects of chronic administration of Compound 22 (33 mg/kg) on rotarod latency of pooled genders of R6/2 mice at 4 to 10 weeks of age. Data are presented as percentage from 4-week pre-treatment baseline (mean + SEM) (WT Vehicle, n = 10; R6/2 Vehicle, n = 10; R6/2 Compound 22 33 mg/kg, n = 10). # $p < 0.05$, R6/2 Vehicle vs. WT Vehicle.

**Figure 10** is a graph showing the effects of chronic administration of Compound 22 (33 mg/kg) on rotarod latency of female R6/2 mice at 4 to 10 weeks of age. Data are presented as mean ± SEM (WT Vehicle, n = 10; R6/2 Vehicle, n = 10; R6/2 Compound 22 33 mg/kg, n = 10). # $p < 0.05$, R6/2 Vehicle vs. WT Vehicle.

**Figure 11** is a bar graph showing the effects of chronic administration of Compound 22 (33 mg/kg) on rotarod latency of female R6/2 mice at 4 to 10 weeks of age. Data are presented as percentage from 4-week pre-treatment baseline (mean + SEM) (WT Vehicle, n = 10; R6/2 Vehicle, n = 10; R6/2 Compound 22 33 mg/kg, n = 10). # $p < 0.05$, R6/2 Vehicle vs. WT Vehicle; * $p < 0.05$, R6/2 Compound 22 33 mg/kg vs. R6/2 Vehicle.

**Figure 12** is a graph showing the effects of chronic administration of Compound 22 (33 mg/kg) on rotarod latency of male R6/2 mice at 4 to 10 weeks of age. Data are presented as mean ± SEM (WT Vehicle, n = 10; R6/2 Vehicle, n = 10; R6/2 Compound 22 33 mg/kg, n = 10). # $p < 0.05$, R6/2 Vehicle vs. WT Vehicle.

**Figure 13** is a bar graph showing the effects of chronic administration of Compound 22 (33 mg/kg) on rotarod latency of male R6/2 mice at 4 to 10 weeks of age. Data are presented as percentage from 4-week pre-treatment baseline (mean + SEM) (WT Vehicle, n = 10; R6/2 Vehicle, n = 10; R6/2 Compound 22 33 mg/kg, n = 10). # $p < 0.05$, R6/2 Vehicle vs. WT Vehicle.

**Figure 14** (**A**) is a bar graph showing the effects of chronic administration of Compound 22 (33 mg/kg) on grip strength of pooled genders of R6/2 mice from 4-12 weeks of age. Data are presented as mean ± SEM (WT Vehicle, n = 10; R6/2 Vehicle, n = 10; R6/2 Compound 22 33 mg/kg, n = 10). # $p < 0.05$, R6/2 Vehicle vs. WT Vehicle. **Figure 20** (**B**) is a bar graph showing the effects of chronic administration of Compound 22 (33 mg/kg) on grip strength of female R6/2 mice from 4-12 weeks of age. Data are presented as mean ± SEM (WT Vehicle, n = 10; R6/2 Vehicle, n = 10; R6/2 Compound 22 33 mg/kg, n = 10). # $p < 0.05$, R6/2 Vehicle vs. WT Vehicle. **Figure 20** (**C**) is a bar graph showing the effects of chronic administration of Compound 22 (33 mg/kg) on grip strength of male R6/2 mice from 4-12 weeks of age. Data are presented as mean ± SEM (WT Vehicle, n = 10; R6/2 Vehicle, n = 10; R6/2 Compound 22 33 mg/kg, n = 10). # $p < 0.05$, R6/2 Vehicle vs. WT Vehicle

**Figures 15A-F** are a series of confocal fluorescence images of striatum region from W from WT vehicle, TG vehicle and TG Compound 22 samples labelled with EM48 mHTT antibody and detected with fluorophore in the 488 nm channel. Grayscale images were pseudocolored with LUT "green" (top row) and "Green Fire Blue" (bottom row) in Fiji to visualize gradient in signal intensities. For the latter LUT, increasing signal intensities are represented from blue to green to white. Overall, the strongest mHTT signal is seen in TG vehicle while both WT vehicle and TG Compound 22 displayed a decreased signal. Scale bars = 10 $\mu$m.

**Figures 15A and D** - WT vehicle sample showing basal binding of the antibody to endogenous mouse HTT. Single bright foci of homogenous size were seen, which were thought to represent spontaneous self-aggregation of the antibody resulting from the absence of specific antigen.

**Figures 15B and 15E** - TG vehicle sample displaying a substantial raise in total signal over the WT. Bright, large and more heterogeneous foci were described as IBs, with surrounding diffuse, oligomeric mHTT (see especially green-colored areas in lower depiction).

**Figures 15C and 15F** - TG Compound 22 sample with an apparent slump in mHTT signal compared to the TG vehicle control. Both intensities of IBs and diffuse mHTT seemed to be substantially lowered in the treated sample. In general, the intensity level appeared to resemble the intensity of the WT control.

**Figure 16 (A)** is a bar graph of the nuclear diffuse mHTT levels in the cortex of R6/2 mice treated with Compound 22. Nuclear diffuse mHTT was reduced by 40% in treated animals (n= 9) in comparison to vehicle treated TG mice (p = 0.01). TG transgenic R6/2 mice, wt wildtype, mHTT mutated huntingtin. **Figure 16 (B)** is a graph of the rotarod correlation with diffuse mHTT in R6/2 mice treated with Compound 22. Pearson r: -0.8, p= 0.01, n=9 (all transgenic female mice), confidence interval of r= -0.9561 to -0.2896 for nuclear diffuse mHTT. TG, transgenic R6/2 mice; mHTT mutated huntingtin.

**Figure 17** is a bar graph showing the rotarod latency until mice fall in R6/2 mice treated with Compound 22. R6/2 mice treated with Compound 22 showed significantly improved rotarod performance at 10 weeks of age compared to vehicle treated R6/2 mice. The mean latency to fall was 108.3 s $\pm$ 32.9 s in treated transgenic R6/2 mice and 55.7 s $\pm$ 19.1 s ($p < 0.05$). Compound 22 improved the latency to fall 94% in comparison to untreated model mice.

**Figure 18** is a bar graph showing CREB-binding protein (CBP) colocalization with mHTT. 4-fold reduction of colocalization in treated animals showed an improvement in motor symptoms in comparison to untreated TG mice (p = 0.02)

**Figure 19** is a graph showing the circular dichroism spectra for long Q length (Q46) Exon1 and Compound 22. Increasing doses of Compound 22 decreases the proportion of a predominantly $\alpha$-helical state of Exon1 Q46.

**Figure 20** is a graph showing the circular dichroism spectra for short Q length(Q16) Exon 1, Exon1 Q46 and Exon 1Q46 bound to Compound 4.

**Figure 21** is a graph showing the circular dichroism spectra of TRX exposed to different doses of Compound 4.

**Figure 22** is a graph showing the circular dichroism spectra between 206 nm - 210 nm of Exon1 Q16, Exon 1 Q46 and Exon1 Q46 bound to Compound 9.

**Figure 23 (A)** is a bar graph showing the autophagic flux of STHdh 111/111 neurons treated with 5 $\mu$M Compound 22 compared with untreated STHdh 111/111 neurons and with STHdh 7/7 cells expressing wildtype huntingtin. **Figure 23 (B)** is a bar graph showing that the area stained with LC3 antibody is reduced in treated STHdh 111/111 cells compared to untreated cells. LC3 area is area stained with LC3 antibody. **p < 0.001

**Figure 24 (A)** is a graph showing that STHdh neurons exposed to autophagy inhibitor NH4CL did not show reduction of mHTT upon treatment of Compound 22. FI - fluorescence intensity of mHTT stained with 3B5H10 primary antibody against mHTT. **Figure 24 (B)** is a graph showing that STHdh neurons exposed to 125 nM of Ubiquitin Proteasome System (UPS) inhibitor MG132, had a reduction of mHTT upon treatment of Compound 22. FI - fluorescence intensity of mHTT stained with 3B5H10 primary antibody against mHTT. But the reduction of mHTT with inhibitor was less in comparison to mHTT reduction without UPS inhibitor MG132.

**Figure 25 (A)** is a graph showing that Slc32a1 is downregulated in STHdh 111/111 (Q111-0) neurons in comparison to STHdh 7/7 (Q7-0). Treatment with Compound 22 improves SLC32a1 expression in STHdh 111/111 cells (Q111-10). **Figure 25 (B)** is a graph showing that Rasgrp1 is upregulated in STHdh 111/111 (Q111-0) neurons in comparison to STHdh 7/7 (Q7-0). Treatment with Compound 22 improves Rasgrp1 expression in STHdh 111/111 cells (Q111-10). **Figure 25 (C)** is a graph showing that Olig2 is downregulated in STHdh 111/111 (Q111-0) neurons in comparison to STHdh 7/7 (Q7-0). Treatment with Compound 22 improves Olig2 expression in STHdh 111/111 cells (Q111-10). **Figure 25 (D)** is a graph showing that NGFR is downregulated in STHdh 111/111 (Q111-0) neurons in comparison to STHdh 7/7 (Q7-0). Treatment with Compound 22 improves NGFR expression in STHdh 111/111 cells (Q111-10). **Figure 25** (E) is a graph showing that Kcna is downregulated in STHdh 111/111 (Q111-0) neurons in comparison to STHdh 7/7 (Q7-0). Treatment with Compound 22 improves Kcna expression in STHdh 111/111 cells (Q111-10).

**Figure 26** is a bar graph showing wtHTT levels of STHdh 7/7 cells treated with Compound 4. Compound 4 does not reduce wtHTT in striatal neurons (STHdh 7/7). Immunofluorescence for HTT shows no statistical difference between non—treated (NT) and Compound 4 treated neurons.

**Figure 27** shows the results from experiments, in which striatal neurons from mice with mHTT expression (STHdh 111/111) were treated with different concentrations of Compound 4. MW1 was used to detect mHTT. mHTT was quantified with fluorescence intensity.

**Figure 28** is a bar graph that is exhibiting cell viability levels of STHdh 111/111 cells 48 hours after 3 hours heat shock (HS) cells. Cell viability values cells treated with Compound 4 is compared to untreated cells, treated with Compound 29, Compound 39 or Compound 22.

**Figure 29** is a graph showing the concentration of Compound 4 that penetrated the blood brain barrier, in vivo. Male ICR (Beijing Vital River Laboratory Animal Technology Co., Ltd, China) mice were intravenously injected with Compound 4 at a dose of 1 mg/kg body weight. Following isolation of proteins precipitated from homogenized mouse brain tissue, the concentration of Compound 4 was determined using (Liquid Chromatography with tandem mass spectrometry (LC-MS/MS). Compound 4 penetrates the blood brain area.

**Figure 30** is a graph shows the results from experiments, in which striatal neurons from mice with mHTT expression (STHdh 111/111) were treated with different concentrations of Compound 22. 3B5H10 was used to detect mHTT. mHTT was quantified with fluorescence intensity and data were normalized. The ubiquitin-proteasome system (UPS) inhibitor MG-132 (Selleckchem, Houston Texas, USA) inhibited partially Compound 22 effect on mHtt reduction. But MG-132 could not completely abolish Compound 22 ability to reduce mHtt, indicating that the UPS system also plays a role in Compound 22 effect on mHtt quantity.

**Figure 31** is a bar graph showing phosphorylated Tau species levels in STHdh 7/7 cells treated with Compound 4. Anti-phospho-Tau (pSer396) antibody (Sigma Aldrich, Saint Louis, USA), was used to detect Tau species (). The antiserum pSer396 was produced against synthesized peptide derived from human Tau around the phosphorylation site of Ser396. 26.3% reduction of phosphorylated tau (Ser396) in wt STHdh 7/7 striatal neurons treated with 10 $\mu$M in comparison to untreated cells (** p=0.01, Welch's t-test). 20% reduction of phosphorylated tau (Ser396) in wt STHdh 7/7 striatal neurons treated with 5 $\mu$M in comparison to untreated cell (* p=0.03, Welch's t-test). Cells were heat shocked for 3 hours at baseline. Furthermore, the graph is showing that 10 $\mu$M Compound 4 treatment could reduce in STHdh 7/7 exposed to ammonium chloride 15,2% phospho-Tau (Ser396) in comparison to non-treated STHdh 7/7. The autophagy inhibitor ammonium chloride blocked the phospho-Tau (Ser396) reduction in Compound 4 treated STHdh 7/7 neurons.

**Figure 32** is a bar graph showing phosphorylated Tau species levels in STHdh 7/7 cells treated with Compound 4. Anti-phospho-Tau (pSer404) antibody (Sigma Aldrich, Saint Louis, USA), was used to detect Tau species. The antiserum pSer404 was produced against synthesized peptide derived from human Tau around the phosphorylation site of Ser404. 10 $\mu$M Compound 4 reduces phospho-Tau (Ser404) 40% in striatal neurons (STHdh 7/7) in comparison to untreated STHdh 7/7 (p=0.003, Welch's t-test). Immunofluorescence for HTT shows no statistical difference between non—treated (NT) and Compound 4 treated neurons. The autophagy inhibitor ammonium chloride blocked the phospho-Tau (Ser404) reduction in Compound 4 treated STHdh 7/7 neurons. Cells were **not** heat shocked at baseline.

**Figure 33** shows STHdh 111/111 cells that are striatal cells expressing mutated huntingtin (Q111). In the **left image** are neurons which were not treated with Compound 4. These cells show impaired morphology and high rate of cell death. In the **right image** are neurons which were treated with Compound 4. The neurons depict improved cell viability, dendrite outgrowth and increased cell size.

**Figure 34** graph demonstrating that genes associated with the autophagy pathway were upregulated in (A) STHdh 7/7 neurons and (B) treated STHdh 111/111 neurons each with 10 $\mu$M or 20 $\mu$M Compound 22 in comparison to untreated cells.

**Figure 35** is a timeline showing that zebrafish embryos were treated with Compound 4 prior to MPP+ exposure at 0 hpf, and as co-incubation with MPP+ at 24 hpf onwards.

**Figure 36** is a graph showing that larvae exposed to MPP+ alone moved significantly less frequently with 13.3

movement bouts in 30 minutes than naïve larvae with 40.8 movement bouts in 30 minutes ($p < 0.001$), and larvae exposed to 1 μM Compound 4 moved significantly less frequently with 21.0 movement bouts in 30 minutes than naïve larvae ($p < 0.01$), whereas no significant difference was observed for the drug treatment groups compared to MPP+

**Figure 37** is a graph showing effect of Compound 4 on sleep parameters following co-incubation with MPP+. **(A)** shows the effect on Sleep fragmentation. **(B)** shows the effect on Sleep ratio. **(C)** shows the effect on Sleep velocity. **(D)** shows the effect on Wak bout duration.

**Figure 38** is a graph showing effect of Compound 4 on sleep bout duration of larvae exposed to MPP+. Bar graph showing mean sleep bout duration during nighttime from 10 p.m. to 8 a.m.

**Figure 39** is a graph showing movement plots representing the entire recording sequence assessing neuroprotection. Distance moved per 30 seconds for naïve versus MPP+ (top) and MPP+ versus 1 μM Compound 4 (bottom).

**Figure 40** is a graph showing the movement plots representing the entire recording sequence assessing neuroprotection. Distance moved per 30 seconds for MPP+ versus 10 μM Compound 4 (top) and MPP+ versus 20 μM Compound 4 (bottom).

**Figure 41** is a proton NMR spectrum of Compound 4 (Example 28).

**Figure 42** is a graph showing DLS measurement of the volume of (A) wild type α-Synuclein (asyn-WT) and (B) wild type α-Synuclein (asyn-WT) with equimolar Compound 4 solved in Methanol. In (A), 99,3% of proteins had the volume size 6.150 nm. 0.2% of proteins had a volume of 102.3 nm and 0.4% of the rest of proteins had a volume of 586.3 nm. The asyn-WT concentration was 70 μM. In **(B),** 99,4% of proteins had the volume size 6.139 nm. 0.3% of proteins had a volume of 80.87 nm and 0.3% of the rest of proteins had a volume of 338.7 nm. The Compound 4 concentration and asyn-WT concentration were 70 μM. **(C)** Overlay of the data of **(A)** and **(B).** The volumes of asyn-WT with and without Compound 4 solved in methanol are almost identical.

**Figure 43** is a graph showing DLS measurement of the volume of **(A)** wild type α-Synuclein (asyn-WT) and **(B)** wild type α-Synuclein (asyn-WT) with equimolar Compound 4 solved in DMSO. In **(A),** the average size of 99.3% of the volume of wt α-Synuclein was 6.493 nm. The size of the second peak was 33.27 nm but this peak was only 0.2% of asyn-WT. The third peak of asyn-WT 0.2% was comprising of proteins with the size of 94,75 nm. In **(B),** the asyn-WT was exposed to 1% DMSO. The average size of 96.2% of the volume of wt α-Synuclein was 144.0 nm. The size of the second peak was 4794 nm but this peak was 3.8% of asyn-WT. **(C)** Overlay of the data of **(A)** and **(B).**

**Figure 44** is a graph showing DLS measurement of the volume of wild type α-Synuclein (asyn-WT) without DMSO and with 1% DMSO. The volumes of asyn-WT with and without 1% DMSO are almost identical. Thus, DLS cannot detect the small size difference of monomeric wild type α-Synuclein and the small oligomeric wild type α-Synuclein triggered with exposure to 1% DMSO.

**Figure 45** is a graph showing DLS measurement of the size distribution by volume of **(A)** A30P mutant α-Synuclein (Asyn-A30P) without Compound 4 and **(B)** A30P mutant α-Synuclein (Asyn-A30P) with exposure to 100 μM equimolar Compound 4. **(C)** Overlay of the data of **(A)** and **(B).**

**Figure 46** is a graph showing DLS measurement of the size distribution by number of **(A)** A30P mutant α-Synuclein (Asyn-A30P) without Compound 4 and **(B)** A30P mutant α-Synuclein (Asyn-A30P) with exposure to 100 μM equimolar Compound 4 solved. **(C)** Overlay of the data of **(A)** and **(B).**

**Figure 47** is a graph showing DLS measurement of the size distribution by volume of **(A)** Q16 without Compound 4 and **(B)** Q16 with exposure to equimolar Compound 4. **(C)** Overlay of the data of **(A)** and **(B).**

**Figure 48** is a graph showing DLS measurement of the size distribution by number of **(A)** Q16 without Compound 4 and **(B)** Q16 with exposure to equimolar Compound 4. **(C)** Overlay of the data of **(A)** and **(B).**

**Figure 49** is a graph showing DLS measurement of the size distribution by intensity of **(A)** Q46 without Compound 4 and **(B)** Q46 with exposure to equimolar Compound 4.

**Figure 50** is a graph showing DLS measurement of (**A**) the size distribution by number of amyloid β 1-40 4 (154 μM). **(B)** The size distribution by number of amyloid β 1-40 4 in Buffer 1 after adding equimolar Compound 4. The concentration of amyloid β 1-40 was 154 μM. **(C)** The size distribution by volume of amyloid β 1-40 4 (154 μM). (**D**) The size distribution by volume of amyloid β 1-40 in Buffer 1 after adding equimolar Compound 4. The concentration of amyloid β 1-40 was 154 μM.

**Figure 51** is a graph showing DLS measurement of the size distribution by intensity of **(A)** amyloid β 1-40 in Buffer 2. The concentration of amyloid β 1-40 was 155 μM. **(B)** The size distribution by intensity of amyloid β 1-40 in Buffer 2 after adding equimolar Compound 4. The concentration of amyloid β 1-40 was 155 μM.

**Figure 52** shows toxicity studys of Compound 4 on *C. elegans* worms. **(A)** is a study on the growth an toxicity of Compound 4 when exposing worms of C. elegans to the compound. (A) Growth and toxicity assay. Representative images used for measuring worm size and growth with Compound 4 exposure. **(B)** shows data of an acute toxicity assay prior to a lifespan experiment. Large L4 stage worms were plated on solid media identical to that of the actual lifespan assay. Worms were examined for immediate toxic effects and then scored intermittently for survival until the start of the lifespan experiment.

**Figure 53** shows plots of Growth and toxicity assays of worm size data obtained from high resolution imaging and WormLab. Line = mean length or area, box = $25^{th}$ to 75th percentile, whiskers = min and max

**Figure 54** shows survival curves. **(A)** Kaplan-Meier estimate of the survival function. **(B).** Nelson-Aalen estimate of hazard rates from lifespan assay. The hazard function describes the probability at a given time that a typical individual who is currently alive will soon die.

**Figure 55** shows a Worm Activity (Aggregate Motility) analysis over duration of the worms' lifespan. Centroid Distance. The "normalized minimum centroid distance" measures the changes in location of active worms as a group between time points

**Figure 56** shows a Worm Activity (Aggregate Motility) analysis over duration of the worms: lifespan. Spatial Distribution. "(1-normalized mutual information)"measures changes in the spatial distribution of active worms between time points.

In the context of the invention, the amino acid sequences that are listed in the attached sequence listing file are also part of the description.

EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

**Example 1 — Preparation of 2,3-dimethyl-1H-indole-5-carboxylic acid (Intermediate 1)**

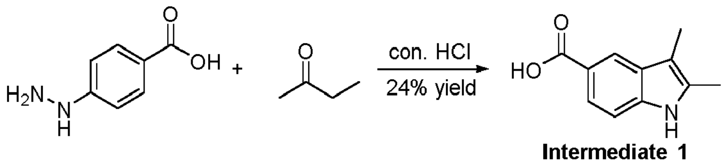


**Intermediate 1**

To a stirred solution of 4-hydrazinobenzoic acid (10.0 g, 65.7 mmol) in 1,4-dioxane was added butan-2-one (5.21 g, 72.3 mmol) and 2.00 mL con. HCl The reaction mixture was stirred at 120°C for 96 h. LC-MS check showed 87% area of Intermediate 1 and 5% area of hydrazone intermediate at 254 nm. The reaction was diluted with 300 mL $H_2O$, extracted with DCM (100 mL * 3). The organic phases were combined and dried over $Na_2SO_4$, concentrated. The

residue was washed with 20.0 mL EtOAc to give pure Intermediate 1 (DCM/MeOH = 10/1, $R_f$ = 0.60). Yield: 3.00 g (24%).

**Example 2 - Preparation of 2,3-dimethyl-1H-indol-5-amine (Intermediate 2)**

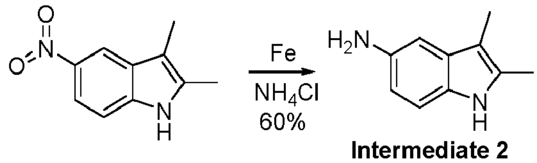


**Intermediate 2**

To a stirred solution of 2,3-dimethyl-5-nitro-1H-indole (1.90 g, 10.0 mmol) in 40.0 mL MeOH and 10.0 mL $H_2O$ was added Fe powder (2.23 g, 40.0 mmol) and $NH_4Cl$ (2.14 g, 40.0 mmol). The reaction mixture was stirred at 65°C for 1 h. TLC check (EtOAc/petroleum = 1/1) showed the reaction was completed, a new spot was observed ($R_f$ = 0.30). The reaction was filtered through a thick pad of celite. The filter cake was washed with MeOH (3* 40.0 mL). The filtrate was concentrated to remove the solvent of MeOH. The residue was diluted with 20.0 mL brine, extracted with DCM (3* 50.0 mL). The organic phase was combined, dried over $Na_2SO_4$, filtered and concentrated to give desired product. Yield: 1.00 g (60%).

**Example 3 - Preparation of 1,2,3-Trimethyl-1H-indole-5-carboxylic acid (Intermediate 3)**

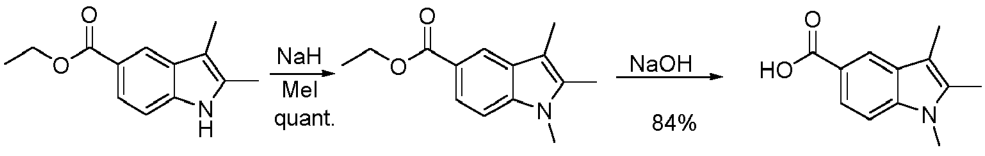


**Intermediate 3**

To a stirred solution of ethyl 2,3-Dimethyl-1*H*-indole-5-carboxylate (1.40 g, 6.44 mmol) in 5.00 mL DMF was added NaH (387 mg, 9.67 mmol, 60% in mineral oil) and MeI (2.74 g, 19.3 mmol, 3.0 eq.) at 0°C. The reaction mixture was stirred at 25°C for 2 h. TLC (petroleum Ether/ EtOAc = 5/1) check showed all starting material ($R_f$ = 0.40) was consumed, a major new spot ($R_f$= 0.50) was observed. The reaction was diluted by 25.0 mL cold $H_2O$, extracted with DCM (10.0 mL *3). The combined organic layers were dried over $Na_2SO_4$, concentrated to give desired product. Yield: 1.60 g (quant.).

To a stirred solution of 2,3-Dimethyl-1*H*-indole-5-carboxylate (1.49 g, 6.44 mmol) in 20.0 mL EtOH and 5.00 mL $H_2O$ was added NaOH (773 mg, 19.3 mmol). The reaction mixture was stirred at 25°C for 16 h. TLC check (petroleum Ether/ EtOAc = 5/1) showed half of 2,3-Dimethyl-1*H*-indole-5-carboxylate still remained. The reaction was heated to 60°C for 7 h till full consumption of 2,3-Dimethyl-1*H*-indole-5-carboxylate by TLC check. The reaction was concentrated to remove most of the EtOH. The residue was diluted with 25.0 mL brine, acidified to pH = 3-4 and extracted with DCM (10.0 mL * 3). The organic phase was combined and dried over $Na_2SO_4$, concentrated to give desired product. Yield: 1.10 g (84%).

**Example 4 - Preparation of 2,3-Dimethyl-5-nitro-1-(phenylsulfonyl)-1*H*-indole (Intermediate 4A)**

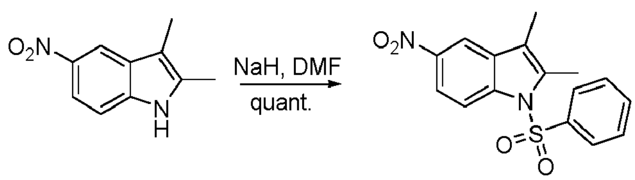


**Intermediate 4A**

To a stirred solution of NaH (2.27 g, 56.8 mmol) in 20.0 mL DMF was added 2,3-Dimethyl-5-nitro-1*H*-indole (5.40 g, 28.4 mmol) in portions at 0°C. The reaction mixture was stirred for 30 minutes, benzenesulfonyl chloride (6.02 g, 34.1 mmol) was added and the mixture was allowed to stir for 2 h. The reaction was diluted by 60.0 mL $H_2O$, extracted with EtOAc (30.0 mL * 3). The combined organic layers were dried over $Na_2SO_4$, concentrated to give desired product. Yield: 9.58 g (quant.).

**Example 5** - **Preparation of 2,3-Dimethyl-1-(phenylsulfonyl)-1*H*-indol-5-amine (Intermediate 4B)**

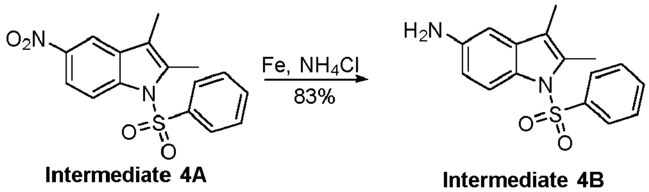


**Intermediate 4A** **Intermediate 4B**

To a stirred solution of Intermediate 4A (9.58 g, 29.0 mmol) in 20.0 mL EtOH and 5.00 mL $H_2O$ was added $NH_4Cl$ (4.65 g, 87.0 mmol). The reaction mixture was heated to 85 °C, then Fe powder (9.72 g, 174 mmol) was added in portions. After the addition, the reaction was heated to 85 °C for 2 h. the reaction mixture was cooled to 40°C and filtered through a pad of celite, washed with a large amount of EtOH. The filtrate was concentrated. The residue was diluted with water and EtOAc, separated. The aqueous phase was extracted with EtOAc (100 mL * 3). The combined organic phases were washed with brine, dried over $Na_2SO_4$, concentrated. The residue was purified by silica gel column (EtOAc/petroleum ether = 1/10 to 1/8) to give pure desired product. Yield: 7.46 g (83%).

**Example 6** - **Preparation of *N*-(2-(Diethylamino)ethyl)-2,3-dimethyl-1-(phenylsulfonyl)-1*H*-indole-5-sulfonamide (Intermediate 4C)**

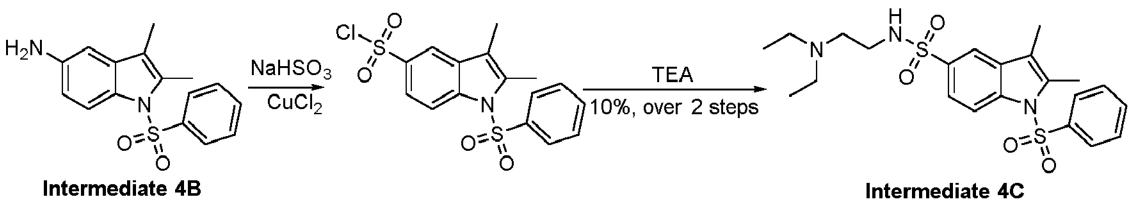


**Intermediate 4B** **Intermediate 4C**

To conc. HCl (36%, 0.30 mL) at -5°C was added Intermediate 4B (200 mg, 0.67 mmol). The mixture was stirred under -5°C for 30 min. Then a solution of $NaNO_2$ (50.5 mg, 0.73 mmol) in $H_2O$ (4.00 mL) was added to the mixture under -5°C. The resulting mixture (A) was further stirred at -5°C for 30 min. In parallel, to a solution of $CuCl_2 \cdot 2H_2O$ (25.2 mg, 0.17 mmol) in conc. HCl (36%, 0.30 mL) at -5°C was added $NaHSO_3$ (277 mg, 2.66 mmol) to form mixture B. The resulting mixture (B) was stirred under -5°C for 30 min. After that, the mixture (A) was added into mixture (B) and the reaction was stirred at - 5°C for 45 min. The reaction was quenched with ice (1.00 g) and after 10 minutes, water (10.0 mL) was added. The resulting mixture was extracted with EtOAc (5.00 mL * 3). The organic solvents were concentrated and dried over vacuo to afford crude desired product, which was used directly in next step without any purification.

The crude product was dissolved in 5.00 mL DCM was added $N^1$,$N^1$-diethylethane-1,2-diamine (11.7 mg, 0.10 mmol), TEA (13.9 mg, 0.14 mmol). The reaction mixture was stirred at 25°C for 2 h. TLC check (DCM/MeOH = 10/1) showed a major new spot ($R_f$ = 0.60) formed. The reaction was diluted with 5.00 mL DCM and 10.0 mL $H_2O$. The organic phase was separated, the aqueous layer was extracted with DCM (5.00 mL * 2). The organic phase was combined, dried over $Na_2SO_4$, filtered and concentrated. The residue was purified by preparative TLC (DCM/MeOH = 10/1) to give desired product. Yield: 24.0 mg (10%, over 2 steps).

**Example 7** - **Preparation of Ethyl 2,3-dimethyl-1-(phenylsulfonyl)-1*H*-indole-5-carboxylate (Intermediate 4D)**

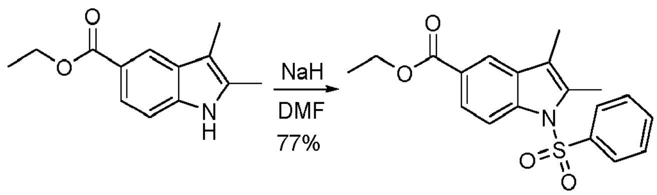


**Intermediate 4D**

To a stirred solution of NaH (2.76 g, 69.0 mmol) in 80.0 mL DMF was added ethyl 2,3-dimethyl-1H-indole-5-carboxylate (10.0 g, 46.0 mmol) at 0°C. The reaction mixture was stirred 30 minutes at 0°C, benzenesulfonyl chloride (10.6 g, 59.8 mmol) was added drop wise and the mixture was allowed to stir for 16 h at 0-28°C. TLC check (EtOAc/ petroleum ether = 1/5) showed roughly 10% of the starting material ($R_f$ = 0.30) remained, one new spot ($R_f$ = 0.40) formed. The reaction was quenched with 150 mL $H_2O$ at 0°C, extracted with EtOAc (50.0 mL * 3). The combined organic phases were washed with brine, dried over $Na_2SO_4$, concentrated. The residue was purified by silica gel column (EtOAc/petroleum ether = 1/8 to 1/4) to give pure desired product. Yield: 12.6 g (77%).

**Example 8** - **Preparation of (2,3-Dimethyl-1-(phenylsulfonyl)-1*H*-indol-5-yl)methanol (Intermediate 4E)**

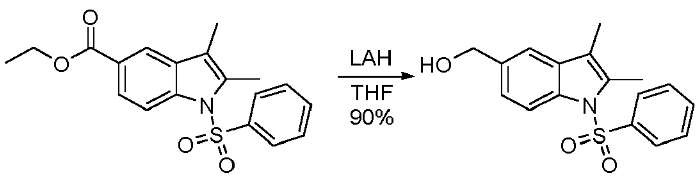


**Intermediate 4D** **Intermediate 4E**

To Intermediate 4D (12.6 g, 35.3 mmol) in 100 mL dry THF was added LAH (1.61 g, 42.4 mmol) in portions at 0°C. The reaction mixture was stirred 45 minutes at 0-25°C. TLC check (EtOAc/ petroleum ether = 1/2) showed complete conversion of the starting material ($R_f$ = 0.60), one new spot ($R_f$ = 0.30) could be observed. The reaction was quenched and diluted with 200 mL $H_2O$ at 0°C, extracted with EtOAc (70.0 mL * 3). The combined organic phases were washed with brine, dried over $Na_2SO_4$, concentrated. The residue was purified by silica gel column (EtOAc/petroleum ether = 1/2) to give pure desired product. Yield: 9.97 g (90%).

**Example 9** - **Preparation of 2,3-Dimethyl-1-(phenylsulfonyl)-1*H*-indole-5-carbaldehyde (Intermediate 4F)**

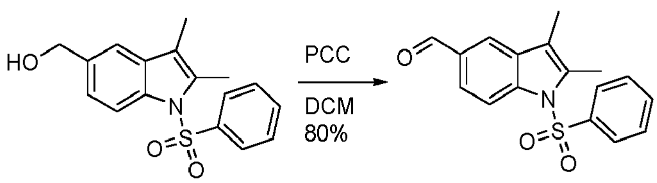


**Intermediate 4E** **Intermediate 4F**

To Intermediate 4E (5.00 g, 15.9 mmol) in 30.0 mL DCM was added silica gel (20.0 g) and PCC (5.13 g, 23.8 mmol). The reaction mixture was stirred at 28°C for 10 min. TLC check (EtOAc/ petroleum ether = 1/2) showed complete conversion of the starting material ($R_f$ = 0.30), one new spot ($R_f$ = 0.50) could be observed. The reaction was filtered through a pad of celite, and the filtrate was diluted with 100 mL $H_2O$, extracted with DCM (20.0 mL * 3). The combined organic phases were washed with brine, dried over $Na_2SO_4$, concentrated. The residue was purified by silica gel column (EtOAc/petroleum ether = 1/6) to give pure desired product. Yield: 4.00 g (80%).

**Example 10 - Preparation of *N*-((2,3-Dimethyl-1-(phenylsulfonyl)-1*H*-indol-5-yl)methyl)-2-(pyrrolidin-1-yl)ethan-1-amine (Intermediate 4G)**

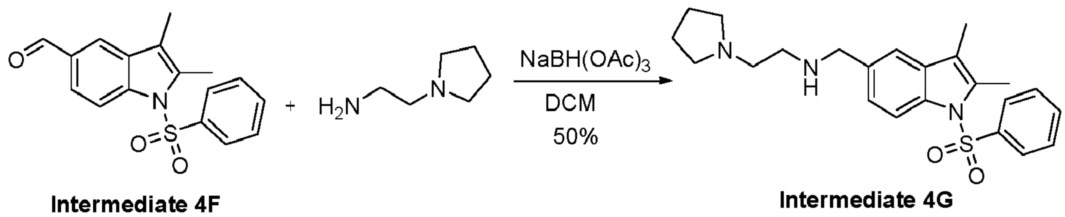


Intermediate 4F

Intermediate 4G

To a solution of Intermediate 4F (300 mg, 0.96 mmol) and 2-(pyrrolidin-1-yl)ethan-1-amine (114 mg, 1.00 mmol) in 15.0 mL DCM was added $NaBH(OAc)_3$ (305 mg, 1.44 mmol) and AcOH (57.6 mg, 0.96 mmol) at 0°C. The reaction was stirred at 0-28°C for 16 h. TLC check (MeOH/ DCM = 1/20) showed complete conversion of the starting material ($R_f$ = 0.95), one new spot ($R_f$ = 0.50) could be observed. The reaction was diluted with 50.0 mL $H_2O$, extracted with DCM (15.0 mL * 3). The combined organic phases were washed with brine, dried over $Na_2SO_4$, concentrated. The residue was purified by Chromatotron (MeOH/DCM = 1/100 to 1/30) to give pure desired product. Yield: 192 mg (50%).

**Example 11 - Preparation of *N*[1]-((2,3-Dimethyl-1-(phenylsulfonyl)-1*H*-indol-5-yl)methyl)-*N*[2],*N*[2]-diethylethane-1,2-diamine (Intermediate 4H)**

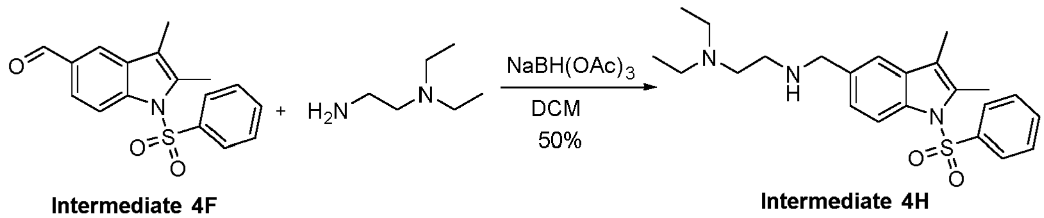


Intermediate 4F

Intermediate 4H

To a solution of Intermediate 4F (300 mg, 0.96 mmol) and $N^1$,$N^1$-diethylethane-1,2-diamine (116 mg, 1.00 mmol) in 15.0 mL DCM was added $NaBH(OAc)_3$ (305 mg, 1.44 mmol) and AcOH (57.6 mg, 0.96 mmol) at 0°C. The reaction was stirred at 0-28°C for 16 h. TLC check (MeOH/ DCM = 1/20) showed complete conversion of the starting material ($R_f$= 0.95), one new spot ($R_f$= 0.50) formed. The reaction was diluted with 50.0 mL $H_2O$, extracted with DCM (15.0 mL * 3). The combined organic phases were washed with brine, dried over $Na_2SO_4$, concentrated. The residue was purified by Chromatotron (MeOH/DCM = 1/100 to 1/30) to give pure desired product. Yield: 195 mg (50%).

**Example 12 - Preparation of 2,3-dimethyl-N-(2-morpholinoethyl)-1H-indole-5-carboxamide (Compound 2)**

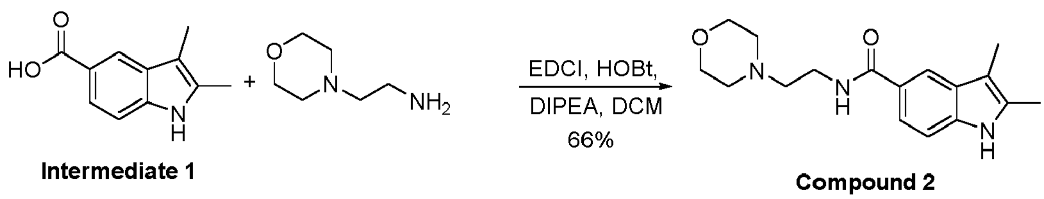


Intermediate 1

Compound 2

To a stirred solution of Intermediate 1 (50.0 mg, 0.26 mmol) in 5.00 mL DCM was added HOBt (42.8 mg, 0.32 mmol), EDC (49.2 mg, 0.32 mmol), 2-morpholinoethan-1-amine (41.3 mg, 0.32 mmol) and DIEA (68.3 mg, 0.53 mmol). The reaction mixture was stirred at 25°C for 16 h. The reaction mixture was stirred at 25°C for 16 h. TLC check (DCM/MeOH = 10/1, UV) indicated complete consumption of the starting material ($R_f$ = 0.50), one main new spot ($R_f$ = 0.30) could be observed. The reaction was diluted with brine and extracted with DCM (3* 20.0 mL). The organic phase was combined and dried over $Na_2SO_4$, filtered and concentrated. The residue was purified by preparative TLC (DCM/MeOH = 10/1)

to give pure target compound. Yield: 53.0 mg (66%).

**Example 13 - Preparation of N-(4-(dimethylamino)cyclohexyl)-2,3-dimethyl-1H-indole-5-carboxamide (Compound 101)**

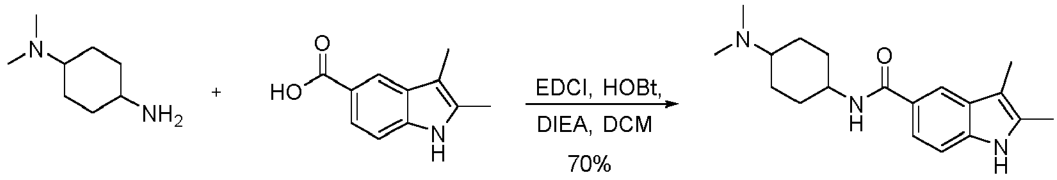


Intermediate 1 Compound 101

To a solution of Intermediate 1 (50.0 mg, 0.25 mmol) in 8.00 mL DCM at 0°C was added *N¹*,*N¹*-dimethylcyclohexane-1,4-diamine (27.0 mg, 0.27 mmol), DIPEA (64.0 mg, 0.49 mmol), HOBt (49.0 mg, 0.37 mmol) and EDCI (70.0 mg, 0.37 mmol). Then the reaction was stirred at 20°C for 16 h. TLC check (DCM/MeOH = 10/1, UV) indicated complete consumption of the starting material ($R_f$ = 0.50), one main new spot ($R_f$ = 0.20) could be observed. The mixture was cooled and partitioned between DCM and water, separated. The aqueous phase was extracted with DCM. The combined organic layers were washed with brine, dried over $Na_2SO_4$, filtered and concentrated. The residue was purified by PTLC (DCM/MeOH = 10/1) to give 55.0 mg pure target compound. Yield: 55.0 mg (70%).

**Example 14 - Preparation of N-(2,3-Dimethyl-1H-indol-5-yl)-3-(dimethylamino)propanamide**

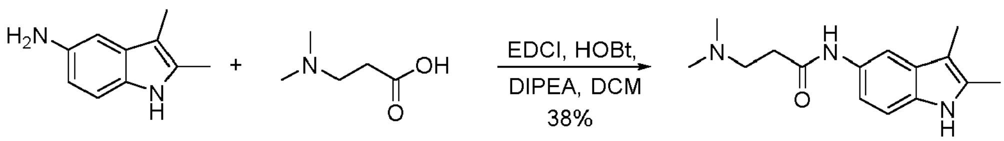


Intermediate 2 Compound 7

**(Compound 7)**

To a stirred solution of Intermediate 2 (50.0 mg, 0.31 mmol) in 5.00 mL DCM was added HOBt (46.0 mg, 0.34 mmol), EDCI (52.9 mg, 0.34 mmol), 3-(dimethylamino)propanoic acid (33.2 mg, 0.28 mmol) and DIPEA (73.3 mg, 0.57 mmol). The reaction mixture was stirred at 25°C for 16 h. TLC check (DCM/MeOH = 1/10, UV) indicated complete consumption of the starting material ($R_f$ = 0.50), one main new spot ($R_f$ = 0.30) could be observed. The reaction was diluted with brine and extracted with DCM (3* 20.0 mL). The organic phase was combined and dried over $Na_2SO_4$, filtered and concentrated. The residue was purified by preparative TLC (DCM/MeOH = 1/10) to give pure title compound. Yield: 28.0 mg (38%).

**Example 15 - Preparation of N-(2-(Azetidin-1-yl)ethyl)-2,3-dimethyl-1H-indole-5-carboxamide (Compound 17)**

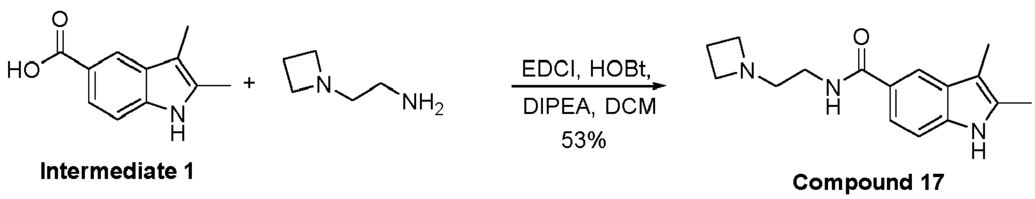


Intermediate 1 Compound 17

To a stirred solution of Intermediate 1 (50.0 mg, 0.26 mmol) in 5.00 mL DCM was added HOBt (42.8 mg, 0.32 mmol), EDC (49.2 mg, 0.32 mmol), 2-(azetidin-1-yl)ethan-1-amine (31.8 mg, 0.32 mmol) and DIEA (68.3 mg, 0.53 mmol).

The reaction mixture was stirred at 25°C for 16 h. The reaction mixture was stirred at 25°C for 16 h under Ar. TLC check (DCM/MeOH = 10 /1) showed full consumption of Intermediate 1 ($R_f$ = 0.60), a major new spot ($R_f$ = 0.30) was observed. The reaction was diluted with 15.00 mL DCM and 15.0 mL $H_2O$. The organic phase was separated, the aqueous layer was extracted with DCM (10.0 mL * 2). The organic phase was combined, dried over $N_2SO_4$, filtered and concentrated. The residue was purified by preparative TLC (DCM/MeOH = 10/1) to give pure target compound. Yield: 38.0 mg (53%).

**Example 16 - Preparation of *N*-(2-(Diethylamino)ethyl)-1,2,3-dimethyl-1*H*-indole-5-carboxamide (Compound 19)**

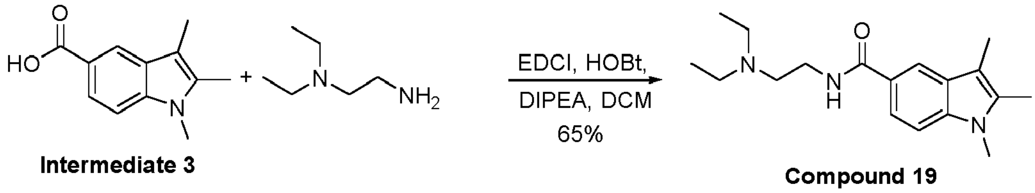


**Intermediate 3**

**Compound 19**

To a stirred solution of Intermediate 3 (50.0 mg, 0.25 mmol) in 5.00 mL DCM was added HOBt (39.9 mg, 0.30 mmol), EDCI (45.8 mg, 0.30 mmol), $N^1,N^1$-diethylethane-1,2-diamine (34.3 mg, 0.30 mmol) and DIEA (63.6 mg, 0.49 mmol). The reaction mixture was stirred at 25°C for 16 h. The reaction mixture was stirred at 25°C for 16 h. TLC check (DCM/MeOH = 10/1) showed full consumption of Intermediate 3 ($R_f$ = 0.50), a major new spot ($R_f$ = 0.30) was observed. The reaction was diluted with 5.00 mL DCM and 10.0 mL $H_2O$. The organic phase was separated, the aqueous layer was extracted with DCM (5.00 mL * 2). The organic phase was combined, dried over $Na_2SO_4$, filtered and concentrated. The residue was purified by preparative TLC (DCM/MeOH = 10/1) to give target compound. Yield: 49.0 mg (65%).

**Example 17 - Preparation of *N*-(2-(Diethylamino)ethyl)-2,3-dimethyl-1H-indole-5-carboxamide (Compound 21)**

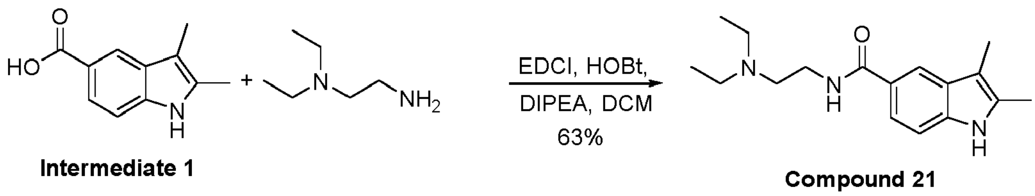


**Intermediate 1**

**Compound 21**

To a stirred solution of Intermediate 1 (50.0 mg, 0.26 mmol) in 5.00 mL DCM was added HOBt (42.8 mg, 0.32 mmol), EDCI (49.2 mg, 0.32 mmol), *N*1,*N*1-diethylethane-1,2-diamine (36.8 mg, 0.32 mmol) and DIEA (68.3 mg, 0.53mmol). The reaction mixture was stirred at 25°C for 16 h. The reaction mixture was stirred at 25°C for 16 h under Ar. TLC check (DCM/MeOH = 10 /1) showed full consumption of Intermediate 1 ($R_f$ = 0.60), a major new spot ($R_f$ = 0.30) was observed. The reaction was diluted with 10.0 mL DCM and 10.0 mL $H_2O$. The organic phase was separated, the aqueous layer was extracted with DCM (5.00 mL * 2). The organic phase was combined, dried over $Na_2SO_4$, filtered and concentrated. The residue was purified by preparative TLC (DCM/MeOH = 10/1) 2 times to give pure target compound. Yield: 48.0 mg (63%).

**Example 18 - Preparation of 3-Ethyl-2-methyl-*N*-(2-(piperidin-1-yl)ethyl)-1*H*-indole-5-carboxamide (Compound 24)**

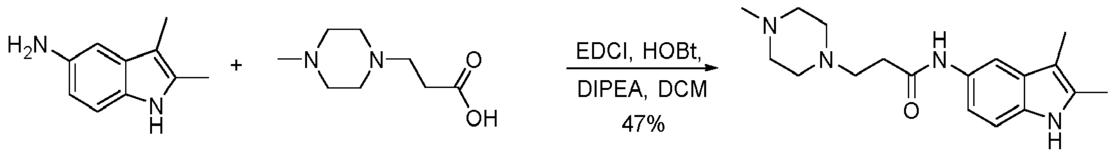


Intermediate 2

Compound 24

To a stirred solution of Intermediate 2 (50.0 mg, 0.31 mmol) in 5.00 mL DCM was added HOBt (46.0 mg, 0.34 mmol), EDCI (52.9 mg, 0.34 mmol), 3-(4-methylpiperazin-1-yl)propanoic acid (48.9 mg, 0.28 mmol) and DIPEA (73.3 mg, 0.57 mmol). The reaction mixture was stirred at 25°C for 16 h. TLC check (DCM/MeOH = 1/10, UV) indicated complete consumption of the starting material ($R_f$ = 0.50), one main new spot ($R_f$ = 0.30) could be observed. The reaction was diluted with brine and extracted with DCM (3* 20.0 mL). The organic phase was combined and dried over $Na_2SO_4$, filtered and concentrated. The residue was purified by preparative TLC (DCM/MeOH = 1/10) to give pure target compound. Yield: 42.0 mg (47%).

**Example 19 - Preparation of *N*-(2-(3,4-Dimethylpiperazin-1-yl)ethyl)-1,2,3-trimethyl-1*H*-indole-5-carboxamide (Compound 25)**

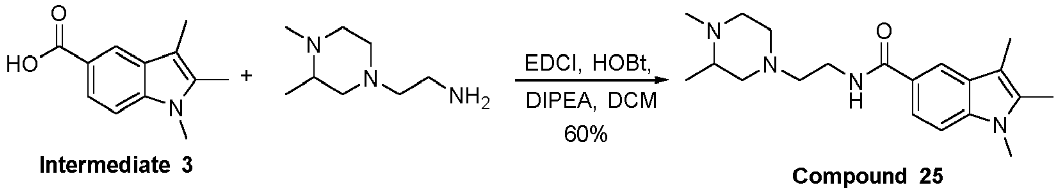


Intermediate 3

Compound 25

To a stirred solution of Intermediate 3 (50.0 mg, 0.25 mmol) in 5.00 mL DCM was added HOBt (39.9 mg, 0.30 mmol), EDCI (45.8 mg, 0.30 mmol), 2-(3,4-dimethylpiperazin-1-yl)ethan-1-amine (47.2 mg, 0.30 mmol) and DIEA (63.6 mg, 0.49 mmol). The reaction mixture was stirred at 25°C for 16 h. The reaction mixture was stirred at 25°C for 16 h. TLC check (DCM/MeOH = 10 /1) showed full consumption of Intermediate 3 ($R_f$ = 0.50), a major new spot ($R_f$ = 0.30) was observed. The reaction was diluted with 5.00 mL DCM and 10.0 mL $H_2O$. The organic phase was separated, the aqueous layer was extracted with DCM (5.00 mL * 2). The organic phase was combined, dried over $Na_2SO_4$, filtered and concentrated. The residue was purified by preparative TLC (DCM/MeOH = 8/1) to give target compound. Yield: 50.0 mg (60%).

**Example 20 - Preparation of 2,3-Dimethyl-*N*-(2-(pyrrolidin-1-yl)ethyl)-1*H*-indole-5-carboxamide (Compound 29)**

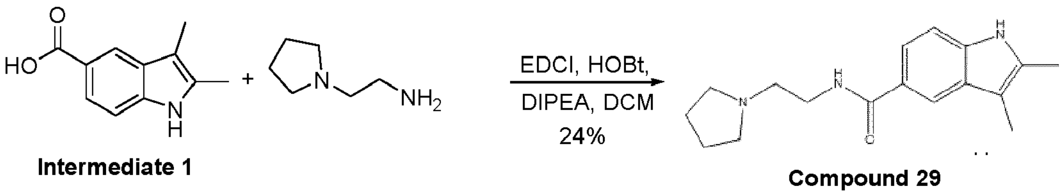


Intermediate 1

Compound 29

To a stirred solution of Intermediate 1 (50.0 mg, 0.26 mmol) in 5.00 mL DCM was added HOBt (42.8 mg, 0.32 mmol), EDCI (49.2 mg, 0.32 mmol), 2-(pyrrolidin-1-yl)ethan-1-amine (36.2 mg, 0.32 mmol) and DIEA (68.3 mg, 0.53 mmol). The reaction mixture was stirred at 25°C for 16 h. The reaction mixture was stirred at 25°C for 16 h. TLC check (DCM/MeOH = 10/1, UV) indicated complete consumption of the starting material ($R_f$ = 0.50), one main new spot ($R_f$ = 0.30) could be observed. The reaction was diluted with brine and extracted with DCM (3* 20.0 mL). The organic phase was combined and dried over $Na_2SO_4$, filtered and concentrated. The residue was purified by preparative TLC (DCM/MeOH = 10/1) 2 times to give pure target compound. Yield: 18.0 mg (24%).

**Example 21 - Preparation of *N*-(2-(Dimethylamino)ethyl)-1,2,3-trimethyl-1*H*-indole-5-carboxamide (Compound 41)**

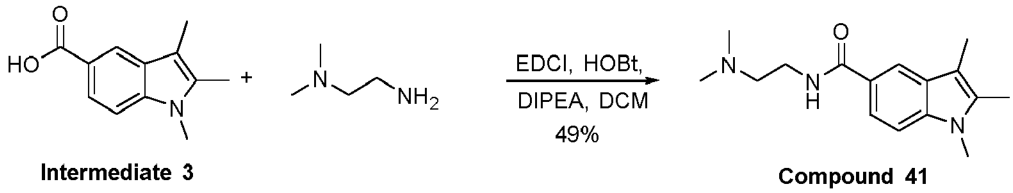


Intermediate 3

Compound 41

To a stirred solution of Intermediate 3 (50.0 mg, 0.25 mmol) in 5.00 mL DCM was added HOBt (39.9 mg, 0.30 mmol), EDC (49.2 mg, 0.30 mmol), *N*1,*N*1-dimethylethane-1,2-diamine (26.0 mg, 0.30 mmol) and DIEA (63.6 mg, 0.49 mmol). The reaction mixture was stirred at 25°C for 16 h. TLC check (DCM/MeOH = 10/1) showed full consumption of Intermediate 3 ($R_f$ = 0.50), a major new spot ($R_f$ = 0.30) was observed. The reaction was diluted with 5.00 mL DCM and 10.0 mL $H_2O$. The organic phase was separated, the aqueous layer was extracted with DCM (5.00 mL * 2). The organic phase was combined, dried over $Na_2SO_4$, filtered and concentrated. The residue was purified by preparative TLC (DCM/MeOH = 10/1) to give pure target compound. Yield: 33.0 mg (49%).

**Example 22 - Preparation of *N*-(2-(Azetidin-1-yl)ethyl)-1,2,3-dimethyl-1*H*-indole-5-carboxamide (Compound 44)**

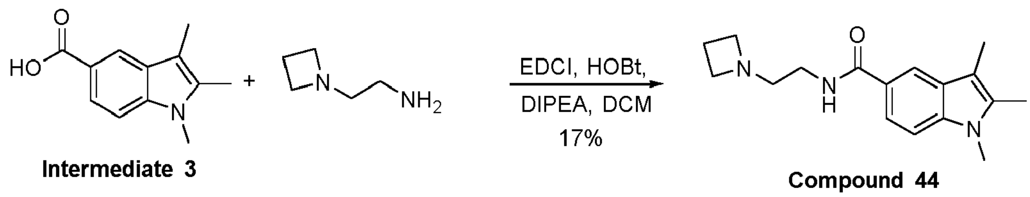


Intermediate 3

Compound 44

To a stirred solution of Intermediate 3(50.0 mg, 0.25 mmol) in 5.00 mL DCM was added HOBt (39.9 mg, 0.30 mmol), EDCI (45.8 mg, 0.30 mmol), 2-(azetidin-1-yl)ethan-1-amine (29.6 mg, 0.30 mmol) and DIEA (63.6 mg, 0.49 mmol). The reaction mixture was stirred at 25°C for 16 h. The reaction mixture was stirred at 25°C for 16 h. TLC check (DCM/MeOH = 10/1) showed full consumption of Intermediate 3 ($R_f$ = 0.50), a major new spot ($R_f$ = 0.30) was observed. The reaction was diluted with 5.00 mL DCM and 10.0 mL $H_2O$. The organic phase was separated, the aqueous layer was extracted with DCM (5.00 mL * 2). The organic phase was combined, dried over $Na_2SO_4$, filtered and concentrated. The residue was purified by preparative TLC (DCM/MeOH = 10/1) 3 times to give pure target compound. Yield: 12.0 mg (17%).

**Example 23 - Preparation of 1,2,3-Trimethyl-*N*-(2-morpholinoethyl)-1*H*-indole-5-carboxamide (Compound 45)**

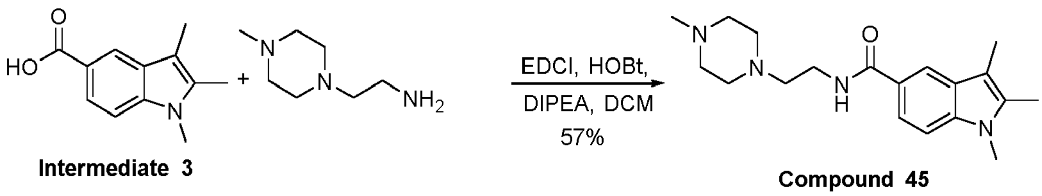


Intermediate 3

Compound 45

To a stirred solution of Intermediate 3(50.0 mg, 0.25 mmol) in 5.00 mL DCM was added HOBt (39.9 mg, 0.30 mmol), EDCI (45.8 mg, 0.30 mmol), 2-(4-methylpiperazin-1-yl)ethan-1-amine (42.3 mg, 0.30 mmol) and DIEA (63.6 mg, 0.49 mmol). The reaction mixture was stirred at 25°C for 16 h. The reaction mixture was stirred at 25°C for 16 h. TLC check (DCM/MeOH = 10 /1) showed full consumption of Intermediate 3 ($R_f$ = 0.50), a major new spot ($R_f$ = 0.30) was

observed. The reaction was diluted with 5.00 mL DCM and 10.0 mL $H_2O$. The organic phase was separated, the aqueous layer was extracted with DCM (5.00 mL * 2). The organic phase was combined, dried over $Na_2SO_4$, filtered and concentrated. The residue was purified by preparative TLC (DCM/MeOH = 10/1) to give target compound. Yield: 46.0 mg (57%).

**Example 24 - Preparation of 1,2,3-Trimethyl-*N*-(2-(pyrrolidin-1-yl)ethyl)-1*H*-indole-5-carboxamide (Compound 46)**

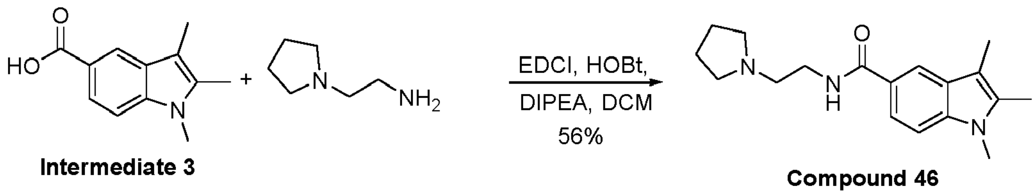


**Intermediate 3**

**Compound 46**

To a stirred solution of Intermediate 3 (50.0 mg, 0.25 mmol) in 5.00 mL DCM was added HOBt (39.9 mg, 0.30 mmol), EDCI (45.8 mg, 0.30 mmol), 2-(pyrrolidin-1-yl)ethan-1-amine (33.7mg, 0.30 mmol) and DIEA (63.6 mg, 0.49 mmol). The reaction mixture was stirred at 25°C for 16 h. The reaction mixture was stirred at 25°C for 16 h. TLC check (DCM/MeOH = 10/1) showed full consumption of Intermediate 3 ($R_f$ = 0.50), a major new spot ($R_f$ = 0.30) was observed. The reaction was diluted with 5.00 mL DCM and 10.0 mL $H_2O$. The organic phase was separated, the aqueous layer was extracted with DCM (5.00 mL * 2). The organic phase was combined, dried over $Na_2SO_4$, filtered and concentrated. The residue was purified by preparative TLC (DCM/MeOH = 10/1) to give target compound. Yield: 41.0 mg (56%).

**Example 25 - Preparation of 2,3-Dimethyl-*N*-(2-(piperidin-1-yl)ethyl)-1*H*-indole-5-carboxamide (Compound 47)**

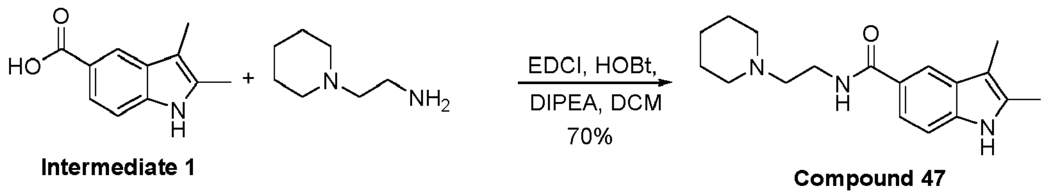


**Intermediate 1**

**Compound 47**

To a stirred solution of Intermediate 1 (50.0 mg, 0.26 mmol) in 5.00 mL DCM was added HOBt (42.8 mg, 0.32 mmol), EDC (49.2 mg, 0.32 mmol), 2-(piperidin-1-yl)ethan-1-amine (40.7 mg, 0.32 mmol) and DIEA (68.3 mg, 0.53 mmol). The reaction mixture was stirred at 25°C for 16 h. The reaction mixture was stirred at 25°C for 16 h. The reaction mixture was stirred at 25°C for 16 h. TLC check (DCM/MeOH = 10/1, UV) indicated complete consumption of the starting material ($R_f$ = 0.50), one main new spot ($R_f$ = 0.30) could be observed. The reaction was diluted with brine and extracted with DCM (3* 20.0 mL). The organic phase was combined and dried over $Na_2SO_4$, filtered and concentrated. The residue was purified by preparative TLC (DCM/MeOH = 10/1) to give pure target compound. Yield: 56.0 mg (70%).

**Example 26 - Preparation of 2,3-Dimethyl-*N*-(2-morpholinoethyl)-1*H*-indole-5-carboxamide (Compound 24)**

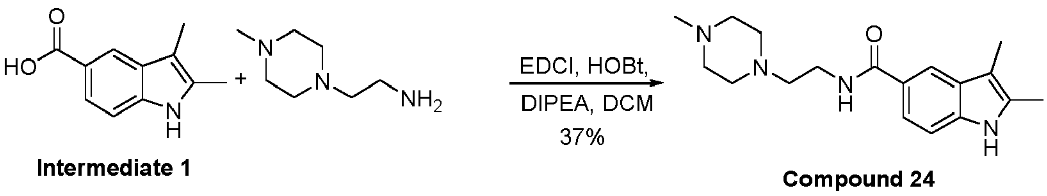


**Intermediate 1**

**Compound 24**

To a stirred solution of Intermediate 1 (50.0 mg, 0.26 mmol) in 5.00 mL DCM was added HOBt (42.8 mg, 0.32

mmol), EDC (49.2 mg, 0.32 mmol), 2-(4-methylpiperazin-1-yl)ethan-1-amine (45.4 mg, 0.32 mmol) and DIEA (68.3 mg, 0.53 mmol). The reaction mixture was stirred at 25°C for 16 h. The reaction mixture was stirred at 25°C for 16 h. The reaction mixture was stirred at 25°C for 16 h. TLC check (DCM/MeOH = 10/1, UV) indicated complete consumption of the starting material ($R_f$ = 0.50), one main new spot ($R_f$ = 0.30) could be observed. The reaction was diluted with brine and extracted with DCM (3* 20.0 mL). The organic phase was combined and dried over $Na_2SO_4$, filtered and concentrated. The residue was purified by preparative TLC (DCM/MeOH = 10/1) to give pure target compound. Yield: 31.0 mg (37%).

**Example 27 - Preparation of 1,2,3-Trimethyl-*N*-(2-(piperidin-1-yl)ethyl)-1*H*-indole-5-carboxamide (Compound 31)**

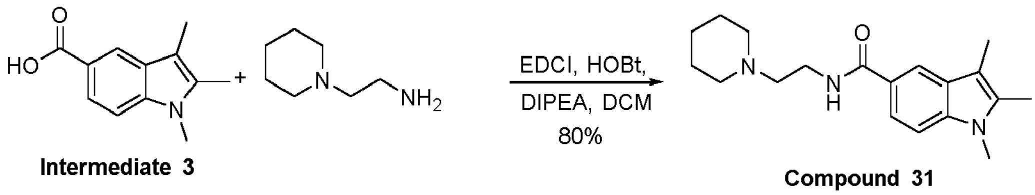


**Intermediate 3**

**Compound 31**

To a stirred solution of Intermediate 3 (50.0 mg, 0.25 mmol) in 5.00 mL DCM was added HOBt (39.9 mg, 0.30 mmol), EDCI (45.8 mg, 0.30 mmol), 2-(piperidin-1-yl)ethan-1-amine (37.9 mg, 0.30 mmol) and DIEA (63.6 mg, 0.49 mmol). The reaction mixture was stirred at 25°C for 16 h. The reaction mixture was stirred at 25°C for 16 h. TLC check (DCM/MeOH = 10/1) showed full consumption of Intermediate 3 ($R_f$ = 0.50), a major new spot ($R_f$ = 0.30) was observed. The reaction was diluted with 5.00 mL DCM and 10.0 mL $H_2O$. The organic phase was separated, the aqueous layer was extracted with DCM (5.00 mL * 2). The organic phase was combined, dried over $Na_2SO_4$, filtered and concentrated. The residue was purified by preparative TLC (DCM/MeOH = 10/1) to give target compound. Yield: 62.0 mg (80%).

**Example 28 - Preparation of *N*-(2-(diethylamino)ethyl)-3-ethyl-2-methyl-1H-indole-5-carboxamide (Compound 4)**

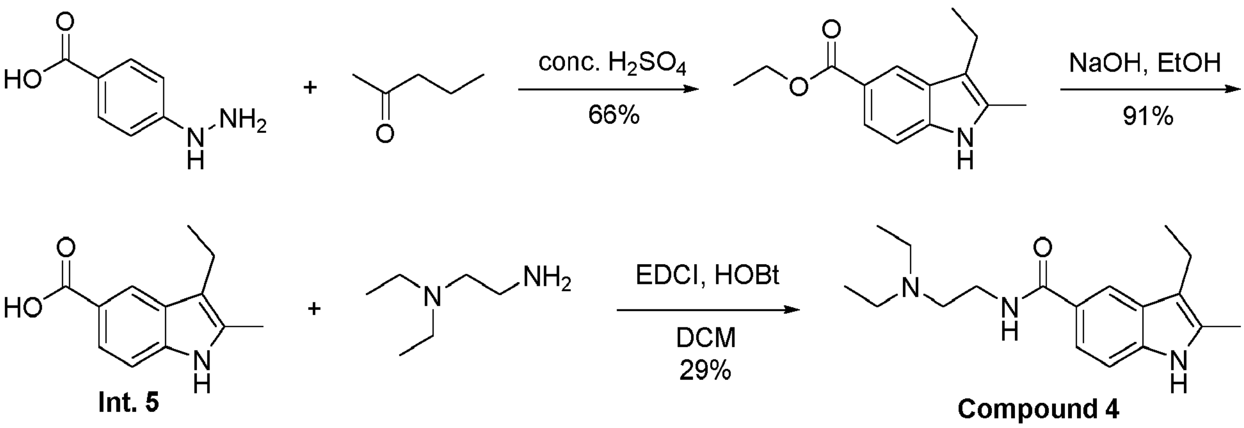


**Int. 5**

**Compound 4**

To a stirred solution of Intermediate 5 (100 mg) was added *N*,*N*-dimethylethane-1,2-diamine, EDCI, HOBt, and IDEA. The reaction mixture was stirred at 26°C for 16 h to afford 43.0 mg (29% yield) after purification by PTLC.

**Example 29 - In-Silico Screen for Compounds that Interact with Mutant Huntingtin (mHTT)**

Using a mutant Huntingtin protein (mHTT), a structure-based virtual screening was performed to determine the structures of small molecules that can interact with the surface of the polyQ peptide of the mHTT. Interaction of small molecules with this surface is predicted to reverse the conformational changes of mHTT to wildtype HTT. A final set of 39 compounds were selected for further testing.

**Example 30 - Mutant Huntingtin (mHTT) Protein Expression**

Exon 1 of the HTT huntingtin (NCBI Gene ID 3064) was expressed as a fusion protein with (i) TRX only (MurTRX); (ii) 16Q (Mur16); and (iii) 46Q (Mur46). MurTRX, Mur16, and Mur46 were produced and purified by HIStag and size exclusion chromatography (SEC). The protocol for MurTRX, Mur16 and Mur46 expression was adopted from Bennett, M.J. et al. Proc. Natl. Acad. Sci. USA 99, 11634-11639. 2002.

*Generation of MurTRX, Murl6 and Mur46 Expression Vectors* - The TRX-linker-histag (MurTRX), TRX-linker-16Q-histag (Mur16), TRX-linker-46Q-histag (Mur46) were synthesized by IDT DNA (Integrated DNA Technologies, Coralville, Iowa USA). These fragments were cloned into the *Nco*I and BamHI sites of a pET28a+ expression vector (Novagen part of Merck-Millipore) and overexpressed in BL21(DE3) plysE cells (Bioline Ltd, London, UK). The linker segment had the sequence GSGSGERQHMDSPDLGTDDDDK (SEQ ID NO: 1). Sequence alignments for selected colonies are shown below.

MurTRX Sequence Alignment:

```
70HG41      MIAPILDEIADEYQGKLTVAKLNIDQNPGTAPKYGIRGIPTLLLFKNGEVAATKVGALSK

Designed    MIAPILDEIADEYQGKLTVAKLNIDQNPGTAPKYGIRGIPTLLLFKNGEVAATKVGALSK

            ************************************************************


70HG41      GQLKEFLDANLAGSGSGERQHMDSPDLGTDDDDKGSGHHHHHH (SEQ ID NO: 2)

Designed    GQLKEFLDANLAGSGSGERQHMDSPDLGTDDDDKGSGHHHHHH (SEQ ID NO: 3)

            *******************************************
```

Mur16 Sequence Alignment:

```
sequenced    MSDKIIHLTDDSFDTDVLKADGAILVDFWAEWCGPCKMIAPILDEIADEYQGKLTVAKLN
designed     MSDKIIHLTDDSFDTDVLKADGAILVDFWAEWCGPCKMIAPILDEIADEYQGKLTVAKLN
             ************************************************************

sequenced    IDQNPGTAPKYGIRGIPTLLLFKNGEVAATKVGALSKGQLKEFLDANLAGSGSGERQHMD
designed     IDQNPGTAPKYGIRGIPTLLLFKNGEVAATKVGALSKGQLKEFLDANLAGSGSGERQHMD
             ************************************************************

sequenced    SPDLGTDDDDKMATLEKLMKAFESLKSFQQQQQQQQQQQQQQQQPPPPPPPPPPPQLPQP
designed     SPDLGTDDDDKMATLEKLMKAFESLKSFQQQQQQQQQQQQQQQQPPPPPPPPPPPQLPQP
             ************************************************************

sequenced    PPQAQPLLPQPQPPPPPPPPPPGPAVAEEPLHRGSGHHHHHH (SEQ ID NO: 4)
designed     PPQAQPLLPQPQPPPPPPPPPPGPAVAEEPLHRGSGHHHHHH (SEQ ID NO: 5)
             ******************************************
```

Mur46 Sequence Alignment:

```
sequenced      XPSXNILFTLRRRYTMSDKIIHLTDDSFDTDVLKADGAILVDFWAEWCGPCKMIAPILDE
designed       ---K-FCLTLRRRYTMSDKIIHLTDDSFDTDVLKADGAILVDFWAEWCGPCKMIAPILDE
                    : :****************************************************

sequenced      IADEYQGKLTVAKLNIDQNPGTAPKYGIRGIPTLLLFKNGEVAATKVGALSKGQLKEFLD
designed       IADEYQGKLTVAKLNIDQNPGTAPKYGIRGIPTLLLFKNGEVAATKVGALSKGQLKEFLD
               ************************************************************

sequenced      ANLAGSGSGERQHMDSPDLGTDDDDKMATLEKLMKAFESLKSFQQQQQQQQQQQQQQQQQ
designed       ANLAGSGSGERQHMDSPDLGTDDDDKMATLEKLMKAFESLKSFQQQQQQQQQQQQQQQQQ
               ************************************************************

sequenced      QQQQQQQQQQQQQQQQQQQQQQQQQQQQQPPPPPPPPPPPQLPQPPPQAQPLLPQPQPPP
designed       QQQQQQQQQQQQQQQQQQQQQQQQQQQQQPPPPPPPPPPPQLPQPPPQAQPLLPQPQPPP
               ************************************************************

sequenced      PPPPPPPGPAVAEEPLHRGSGHHHHHH--AAALEHHHHHH-DPAANKARKEAELAAATAE
designed       PPPPPPPGPAVAEEPLHRGSGHHHHHHAA-------------------------------
               ***************************             *

sequenced      Q-LA-PLGASKRVLRGFLLKGGTISGLANGTRPVAAH-ARRVWWLRAA-PLHLPAP-RPL
designed       ------------------------------------------------------------
                *  *                                *          *       *

sequenced      LSLSSLPXXXXSPAFPVKL-IGGSL-XS  (SEQ ID NO: 6)
designed       ----------------------------  (SEQ ID NO: 7)
                  *  *
```

*mHTT Protein Expression* - Colonies were picked and 5 mL cultures were grown. Cells were induced at Optical Density (OD) 0.7-0.8 for four hours at 37°C. 1 mL of the post-induction culture was spun down and the pellet resuspended in lysis buffer. This was spun down at 10,000 RPM for 10 minutes and the soluble fraction loaded on a 4-20% polyacrylamide gel (NuSep). An anti-His western blot was performed to detect protein expression. Mur16 protein was expressed. Mur46 protein was expressed. Weaker protein expression signal was observed for MurTRX. An ELISA assay was performed to confirm the expression and detection of the proteins. MurTRX, Mur16 and Mur46 are recognized by anti-His antibodies. Mur16 and Mur46 are recognized by anti-polyQ antibody. The anti-polyQ antibody, 3B5H10 MAb (Sigma Aldrich, Saint Louis, USA), was used in the ELISA assay.

**Example 32** - **Determination of mHTT and Compound Interactions by Surface Plasmon Resonance (SPR)**

A Biacore Surface Plasmon Resonance (SPR) assay was used as a label-free method to determine the interaction of small molecule compounds with the mHTT target.

*MurTRX, Murl6 and Mur46 Protein Purification* - Proteins were loaded onto IMAC resin (ThermoFisher Scientific HisPur) and eluted in 200 mM imidazole, purified by gel filtration FPLC (HiLoad superdex-200, 26/60; GE Life sciences) and concentrated using 3 kDa cut off Vivaspin 20 PES centrifugal concentrators (Sartorius AG). Proteins were biotinylated using a 1:0.5 molar ratio of EZ-link™ Sulfo-NHS-LC-LC-Biotin (ThermoFisher Scientific) and thoroughly dialysed against PBS prior to Biacore coupling.

*Neutravidin CM5 amine coupling immobilisation* - It was determined that the ligand density of streptavidin found

on a standard streptavidin (SA) Chip was not high enough for the assay (~3,000RU). A customized biotin capture chip using NeutrAvidin as the capture ligand was created for this assay. To obtain high enough ligand density on the sensor chip surface, NeutrAvidin (Thermoscientific Pierce, Waltham, MA USA) was immobilised on all four sensor chip surfaces to a level of approximately 20,000 RU.

*Capture of the Mur ligands to NeutrAvidin* - Biotinylated proteins were diluted in HBS EP+ running buffer and captured on the sensor chip surface. Mur46 was coupled to the sensor chip first. After saturating FC3 with Mur46, target response levels for Mur16 and MurTRX were calculated based on their respective molecular weights. This ensured that the same molarity of each target was coupled to the sensor chip surface, ensuring that the same proportion of non-specific binding to TRX for all analytes was the same across all flow cells.

*Sensor chip validation Poly-Q 3B5H10 MAb binding* - To investigate the binding properties of the coupled chip, 3B5H10 MAb (Sigma Aldrich, Saint Louis, USA) was diluted 1:600 and flowed over all four flow cells to the point of saturation using injections **(Figure 1).**

**Table 6.** Practical RMAX

| **Ligand** | **3B5H10 practical RMAX (RU)** | |
|---|---|---|
| **Mur 16** | 9,190 | 19 |
| **Mur 46** | 4,362 | 15.5 |

The molecular weight of the MAb is ~150kDa, which is ~5x larger than the largest ligand, Mur46. Interestingly, with the same molarity of Mur 16/46 coupled to the flow cells, double the level of MAb binding was observed with Mur46 in comparison to Mur 16 **(Table 6).** Assuming that the proportion of ligand bound to both flow cells is active, this would indicate that there are double the MAb accessible epitopes on the 46Q than the 16Q.

*Primary Screen - Methods* - Briefly, compounds were diluted in 100% DMSO to a final concentration of 100mM. These were diluted wherever possible to ImM, 0.1mM and 0.01mM in 10% DMSO in PBS+ running buffer. The entire assay was run in 10% DMSO in the BIACore T200 (GE, Little Chalfont, United Kingdom) and an appropriate solvent correction window was selected for the assay. Where compounds were found to precipitate at 1 mM, compounds were prepared in 10% DMSO at 2-fold lower concentrations e.g. 0.5 mM, 0.05 mM and 0.005 mM. Samples were run by identity, from the lowest to the highest concentration with one concentration per cycle. Low-pH regeneration was performed after each sample injection and an extra-wash with 50% DMSO was performed on the machine to eliminate sample carry-over. The assay was run with a 10 HZ data collection frequency in a multi (4-1, 3-1, 2-1) configuration and compounds were in contact with the ligands for 60 seconds. Blank samples (required for double referencing) were passed over the sensor surface every 21 cycles in triplicates, as was the positive MAb control and solvent correction.

*Primary Screen - Results* - 39 compounds determined from the in-silico screen were tested. Following solvent correction and blank subtraction, double referenced binding levels for samples were plotted against binding levels on both Mur 16 and Mur 46. 4 compounds showed binding to the target structures Mur16 and Mur46 **(Table 7).** The 4 compounds demonstrated higher binding to the drug targets with increasing concentration of the compound. The 4 compounds consistently bind with a higher response on the 16 Q surface than the 46 Q repeat surface, despite the MAb positive control showing the opposite trend.

**Table 7.** Compounds that demonstrated binding to Mur16 and/or Mur46

| Compound Number | Biacore affinity ($RU^2$) | Chemical Structure |
|---|---|---|
| 109 | 3 | 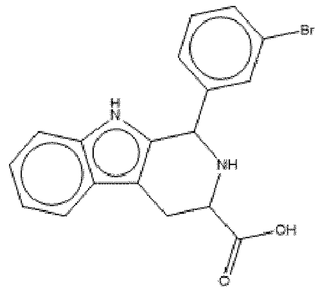 |

(continued)

| Compound Number | Biacore affinity ($RU^2$) | Chemical Structure |
|---|---|---|
| 107 | 92 | 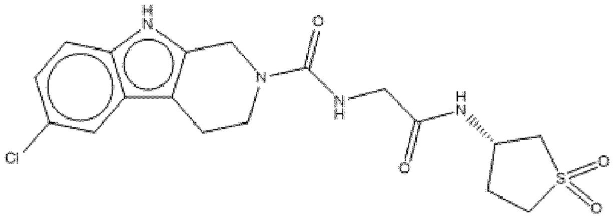 |
| 113 | 160 | 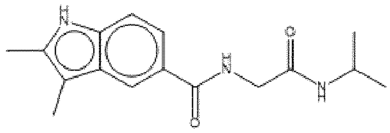 |
| 110 | 80 | 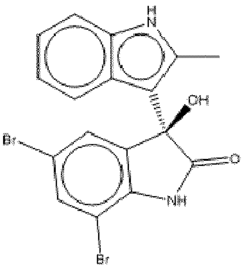 |

*Analog Screening* - *Methods* - Analog or derivative compounds from the secondary screen were determined and were used in an analog Biacore SPR screen. Compounds were stored at 100% DMSO with a concentration of 100 mM. For the screening the compounds were diluted to 1mM in 5% DMSO in PBS+ running buffer. This was serially diluted to 0.1 and 0.01 mM (retaining 5% DMSO). Compounds which did not dissolve at 1mM in 5% DMSO were diluted 10 fold to 0.1mM in 5% PBS+ and run in the assay at 0.1, 0.01 and 0.001mM concentrations MAb 3B5H10 (1:5000) was used as a positive control whilst running buffer was used as a negative. Solvent correction was carried out every 20 cycles as recommended by BIACore. Samples were flowed over the sensor chip surface for 60 seconds and regeneration was carried out with 0.1M glycine, pH 3.0 for 30 seconds. A system wash with 50% DMSO was carried out after each compound injection to reduce the chance of compounds cross contamination across the length of the run.

*First Analog Screening* - *Results* - The results of the first analog screen are shown in **Table 9.**

*Second Analog Screening* - *Results* - The results of the second analog screen are shown in **Table 9. Table 9** lists the compound identification numbers and the binding of the compounds to the target structure.

*Third Analog Screening* - *Results* - The results of the third analog screen are shown in **Table 9. Table 8** lists the compound identification numbers and the binding of the compounds to the target structure in comparison to the parent compound.

**Table 8:** Binding and Solubility of Analog Compounds

| **Compound** | **Binding properties** | **Solubility** |
|---|---|---|
| **57** | ++ | +/- |
| **102** | + **(Mur16)**<br>**+ + (Mur46)** | +/- |
| **106** | + **(Mur16)**<br>**+ + (Mur46)** | +/- |
| **105** | ++ | +/- |
| **103** | ++ | +/- |

(continued)

| Compound | Binding properties | Solubility |
|---|---|---|
| **114** | +/- | +/- |
| ++ very improved in comparison to parent compound<br>+ improved in comparison to parent compound<br>- reduced in comparison to parent compound<br>+/- in par with parent compound | | |

**Table 9** shows a summary of the compounds and analog compounds that were tested using the Biacore screening method.

**Table 9:** Affinity of Compounds to Mur46 Determined by Biacore Screening. Biacore affinity was measured at 0.1 mM compound concentration; * means 1 mM concentration of the compound was used for Biacore measurement in case there was no binding detected at 0.1 mM; ** means 0.01 mM concentration for measurement was used in case the compound was not soluble at 0.1 mM; *** means 0.001 mM concentration for measurement was used in case the compound was not soluble at 0.1 mM and at 0.01 mM

**Table 9.** Biacore Affinity Values for Binding of Compounds to Mur46

| Compound Number | Biacore affinity ($RU^2$) | Chemical Structure |
|---|---|---|
| 102 | 28 | 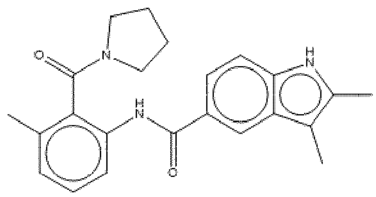 |
| 103 | 124 | 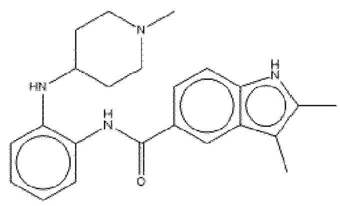 |
| 104 | 116 | 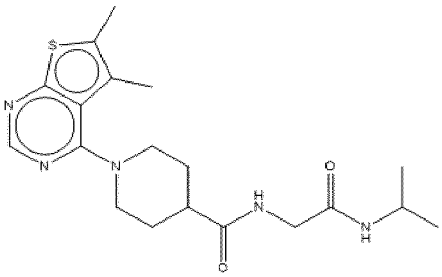 |
| 57 | 214 | 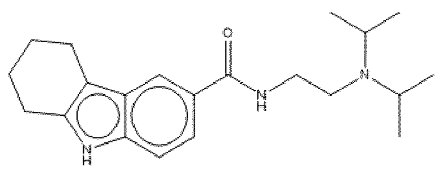 |

(continued)

| Compound Number | Biacore affinity (RU2) | Chemical Structure |
|---|---|---|
| 105 | 118 | 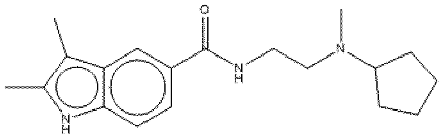 |
| 106 | 93 | 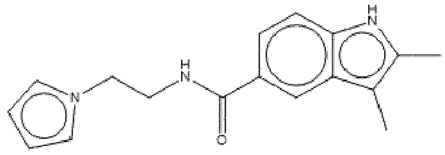 |
| 108 | 6** | 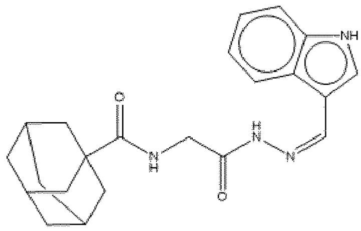 |
| 112 | 130 | 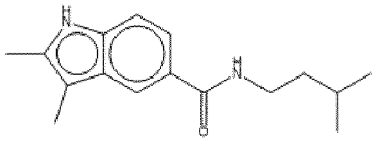 |
| 7 | 122 | 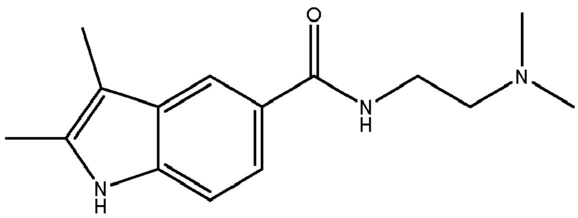 |
| 114 | 20 | 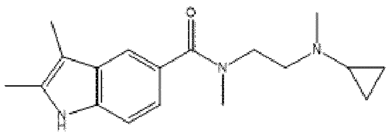 |

Compounds from the Biacore screening were analyzed in a modified Parallel Artificial Permeation Assay (PAMPA) assay for their ability to permeate the blood brain barrier. The filter material of a 96-well microplate is impregnated with brain lipids (Di, L. et al., Eur. J. Med. Chem. 2003 Mar;38(3):223-232). The test compounds were added to the donor compartment. After diffusion, the concentration of the compounds in the acceptor compartment were determined by Liquid Chromatography with tandem mass spectrometry (LC-MS-MS) to calculate the flux rate. Based on these permeation data, the drugs were classified as blood brain barrier permeating (BBB+) with a flux rate >50% or non-permeating (BBB-) with a flux rate <50%. **Table 10** shows the results of this experiment for Compound 53 and Compound 7.

**Table 10.** Assessment of Blood Brain Barrier Permeation by LC-MS-MS

| Compound number | Flux [%] | SD | SE | Mean Recovery [%] | SD | Classification |
|---|---|---|---|---|---|---|
| 53 | 76.7 | 1.1 | 0.6 | 103.5 | 1.7 | BBB+ |
| 7 | 70.8 | 4.7 | 2.7 | 88.5 | 2.6 | BBB+ |

Compound 22 pharmacokinetic properties was measured in three male CD1 mice at several timepoints. Compound 22 was dissolved in 10% DMSO, 10%, cremaphor, and 80% saline. The tested dose was 33.3 mg/kg body weight. Brain tissue was homogenized and protein precipitated with acetonitrile. The concentration of Compound 22 was measured in plasma and brain with Ultra-High Performance Liquid Chromatography combined with Time-of-Flight Mass Spectrometry (UHPLC - TOF). The results of this experiment show that Compound 22 can penetrate the blood brain area in vivo **(Figure 2).** As such, Compound 22 was selected for testing in an animal model of HD the R6/2 model. As described below, Compound 22 and its analogs were further tested for their effects on striatal cell viability and effects of mHTT levels.

**Example 33 - Effects of Compounds on Striatal Cell Viability and mHTT Levels**

Compounds were tested for their ability to reduce diffuse levels of mHTT. Diffuse mHTT can be both monomeric and or oligomeric. Diffuse mHTT is correlated to pathological parameters in Huntington's disease.

*Cell culture* - ST HDH Q111/111 (CH00095, Coriell Institute) striatal derived cell line were grown at 33°C in DMEM (Sigma-Aldrich), supplemented with 10% fetal bovine serum (FBS), 1% Penicillin-Streptomycin (ThermoFisher Scientific), and 0.4 mg/ml G418 (Geneticin; Invitrogen). ST HDH Q111/111 are mouse striatal cells with polyQ length of 111.

*Pre-Treatment of striatal cell line* - First the medium was removed and new DMEM (DMEM, high glucose, HEPES, no phenol red) medium, supplemented with 10% fetal bovine serum (FBS), 1% Penicillin-Streptomycin, containing 10 μM compound was added. The cells were incubated at 33 °C for 24 hours.

*Heat shock* - Cells were heat shocked for 3 hours at 41 °C.

*Treatment* - Medium was removed and new DMEM (DMEM, high glucose, HEPES, no phenol red) medium, supplemented with 1% fetal bovine serum (FBS), 1% Penicillin-Streptomycin, containing 10 μM compound was added. The cells were incubated at 33 °C for 48 hours.

*Cell Viability* - Cell viability was measured according to manufacturer recommendations with CellTiter Glo (Promega). 96-well plates for used either for cell viability determination or cell staining.

*Staining* - Cells were permeabilized with 0.3% Triton X-100 in 4 °C PBS (pH 7.4 from ThermoFisher) for 10 minutes. Then the cells were washed two times with 4 °C PBS. Subsequently, cells are blocked with 3% NGS (normal goat serum from Thermo Fisher) in 4 ° C PBS for 1 hour. Cells were incubated with primary antibodies in blocking buffer (PBS, 3% NGS, 0.02% NaN3) over night at 4 °C. Primary antibodies were mouse antipolyglutamine monoclonal antibody 3B5H10 (Sigma-Aldrich) at 1:250 dilution and Guinea Pig anti-MAP2 polyclonal antibody (Synaptic Systems) at 1:500 dilution. 3B5H10 binds to diffuse mHTT. Diffuse mHTT is a monomeric and small oligomeric mHTT. Diffuse mHTT is correlated to pathological parameters in Huntington's disease. Compounds that bind the mHTT will reduce the levels of diffuse mHTT detected.

Cells were washed 2 times with 4 °C PBS, and then incubated with secondary antibodies at 1:2000 dilution in blocking buffer for 1hour at room temperature. Secondary antibodies were Goat anti-Guinea Pig IgG (H+L) Highly Cross-Adsorbed Secondary Antibody, Alexa Fluor 647 (ThermoFisher Invitrogen) and Goat anti-Mouse IgG (H+L) Cross-Adsorbed Secondary Antibody, Alexa Fluor 488 (ThermoFisher Invitrogen). Cells were washed 2 times with 4 °C PBS, and then the nuclei were counterstain with DAPI (ThermoFisher) for 10 min at room temperature in the dark. Cells were washed 2 times with 4 °C PBS, and then 4 °C PBS was added to cells and then images were acquired. **Table 11** shows the antibodies used in immunocytochemistry studies.

**Table 11:** Antibodies Used in Immunocytochemistry Studies

| **Antibodies** | | |
|---|---|---|
| Mouse anti-human-mHtt mab (clone mEM48) | Merck | MAB5374 |
| Guinea Pig anti-MAP2 pab | Synaptic Systems | 188 004 |
| Alexa Fluor 488 Goat anti-Mouse IgG (H+L) | ThermoFisher Invitrogen | A-11001 |
| Alexa Fluor 647 Goat anti-Rabbit IgG (H+L) | ThermoFisher Invitrogen | A-21244 |
| Alexa Fluor 647 Goat anti-Guinea Pig IgG (H+L) | ThermoFisher Invitrogen | A-21450 |

*Fluorescence Imaging* - Imaging sessions were performed on a confocal microscope system (Carl Zeiss Microscopy) equipped with a dual spinning disk unit (Yokogawa). All components of the imaging system were controlled

via the ZEN 2 software suite (Carl Zeiss Microscopy). The laser lines used were 405 nm, 488 nm and 639 nm to excite DAPI or the respective fluorophores. The fluorescence images obtained of the immunofluorescence labelled tissue sections were quantified with the help of the "Image Processing and Analysis in Java" or short ImageJ software distributed under the GNU General Public License by the NIH (Rueden, C.T. et al., BMC Bioinformatics. 2017 Nov 29;18(1):529), i.e. the edition used was the Fiji distribution (Schindelin, J. et al., Nature methods. 2012 Jun 28;9(7):676-82). Nested t test was performed to calculate the significance amongst repeated measurements using GraphPad Prism software.

*RESULTS*-**Table 12a** summarizes the effect of the compounds on the levels of mHTT in striatal cells. STHdh 111/111 were heat shocked for 3 hours and treated for 48 hours with small molecule compounds. Table 7 lists the percentage of mHTT and the cell viability in striatal cells in comparison to untreated STHdh 111/111 striatal cells after 48 hours. The compounds were ranked by efficacy.

**Table 12a.** Effect of Compounds on mHTT levels and cell viability of STHdh 111/111 striatal cells

| Compound Number | mHTT after treatment | Cell Viability striatal cells 3h heat shocked | Chemical Structure |
|---|---|---|---|
| 1 | 30 | 111 | 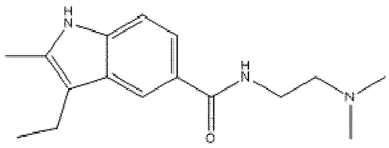 |
| 2 | 32 | 100 | 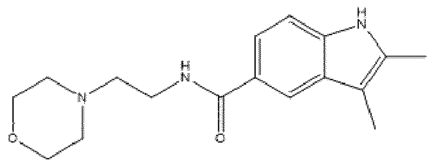 |
| 3 | 33 | 86 | 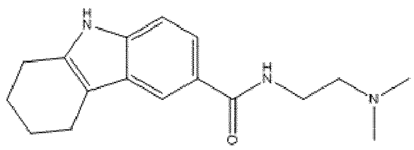 |
| 4 | 34 | 125 | 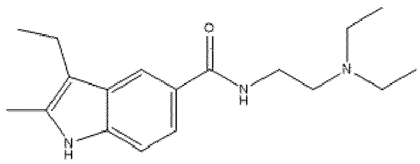 |
| 5 | 36 | 104 | 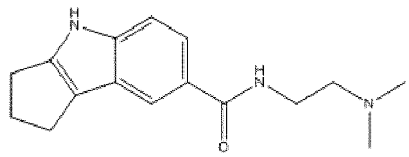 |
| 6 | 37 | 113 | 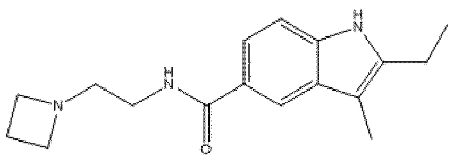 |
| 7 | 45 | 96 | 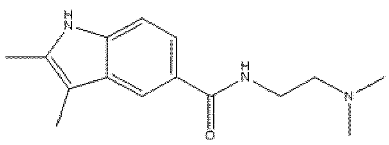 |

(continued)

| Compound Number | mHTT after treatment | Cell Viability striatal cells 3h heat shocked | Chemical Structure |
|---|---|---|---|
| 9 | 37 | 88 | 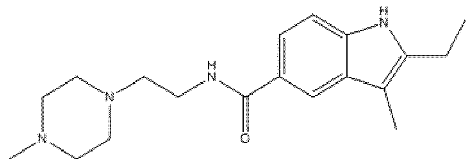 |
| 101 | 38 | 110 | 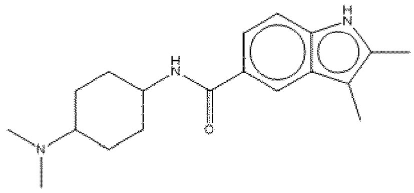 |
| 8 | 38 | 115 | 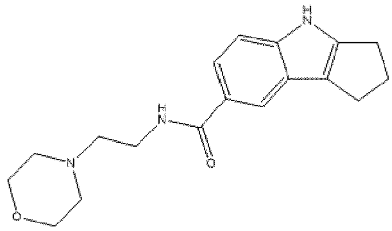 |
| 10 | 40 | 96 | 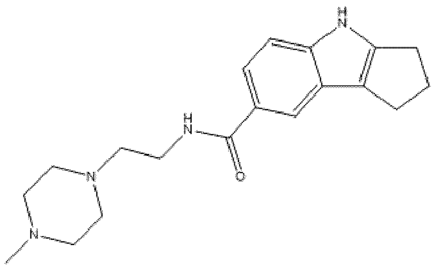 |
| 11 | 40 | 114 | 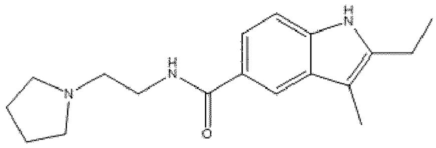 |
| 12 | 40 | 106 | 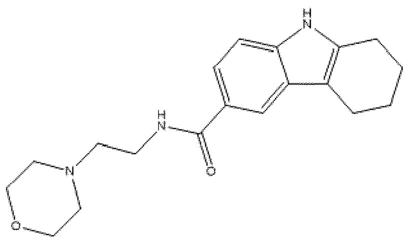 |

(continued)

| Compound Number | mHTT after treatment | Cell Viability striatal cells 3h heat shocked | Chemical Structure |
|---|---|---|---|
| 13 | 41 | 117 | 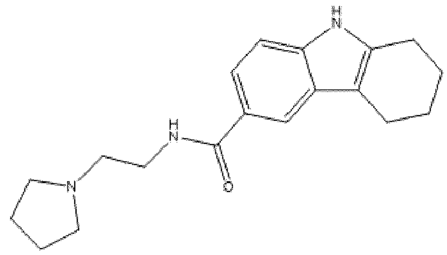 |
| 14 | 41 | 113 | 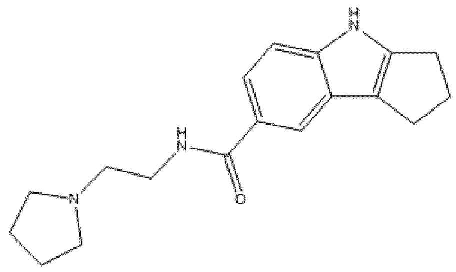 |
| 15 | 42 | 104 | 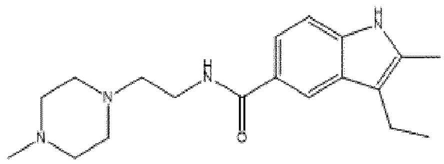 |
| 16 | 43 | 117 | 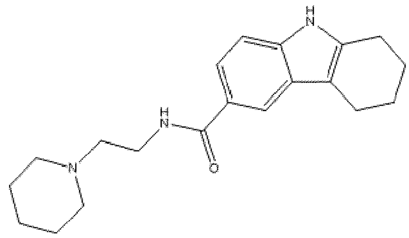 |
| 17 | 44 | 99 | 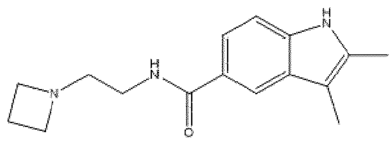 |
| 18 | 44 | 103 | 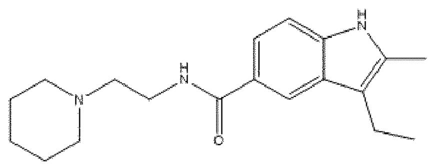 |
| 19 | 45 | 76 | 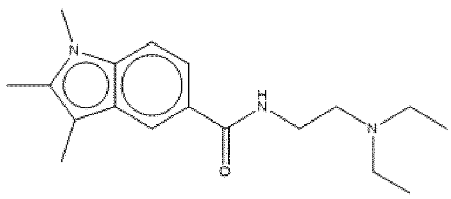 |

(continued)

| Compound Number | mHTT after treatment | Cell Viability striatal cells 3h heat shocked | Chemical Structure |
|---|---|---|---|
| 20 | 45 | 118 | 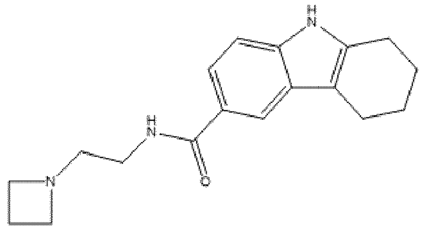 |
| 21 | 46 | 87 | 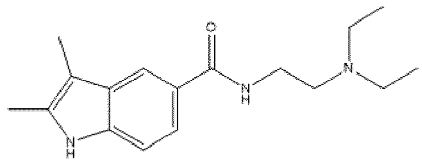 |
| 22 | 46 | 102 | 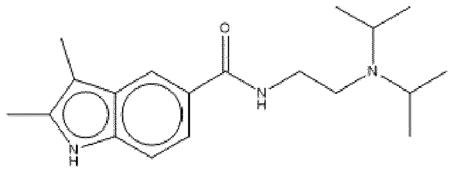 |
| 23 | 47 | 84 | 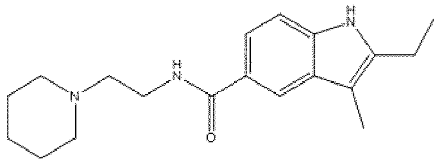 |
| 24 | 47 | 103 | 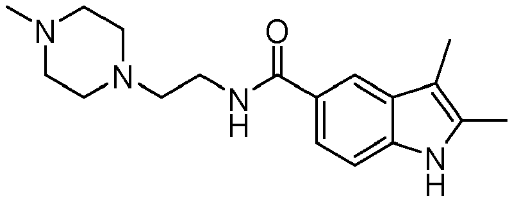 |
| 25 | 48 | 109 | 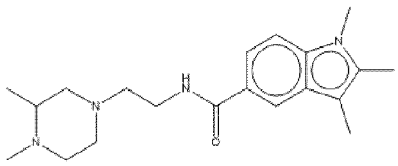 |
| 26 | 49 | 82 | 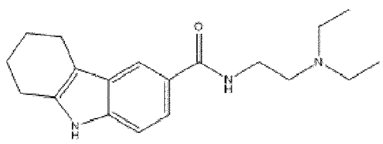 |
| 27 | 50 | 66 | 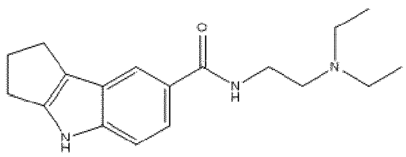 |

(continued)

| Compound Number | mHTT after treatment | Cell Viability striatal cells 3h heat shocked | Chemical Structure |
|---|---|---|---|
| 28 | 51 | 18 | 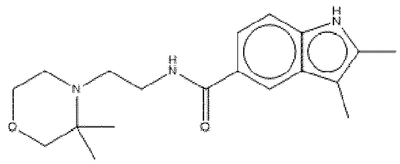 |
| 29 | 51 | 103 | 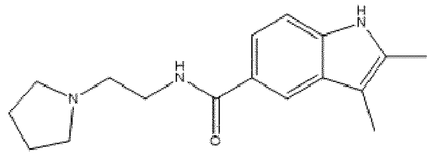 |
| 30 | 51 | 55 | 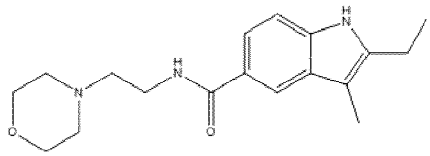 |
| 31 | 52 | 116 | 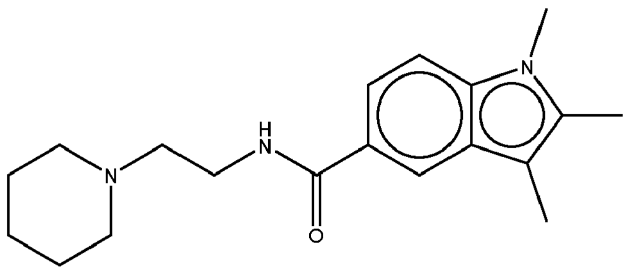 |
| 32 | 53 | 98 | 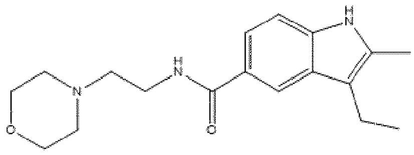 |
| 33 | 55 | 79 | 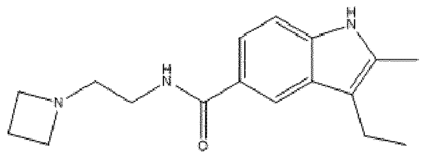 |
| 34 | 56 | 60 | 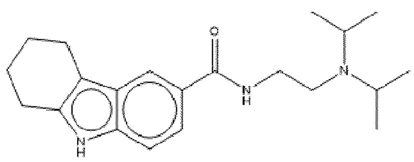 |
| 35 | 56 | 103 | 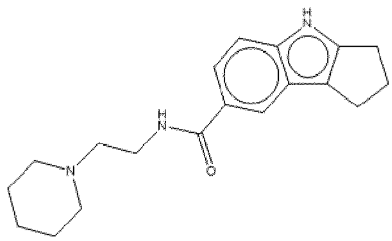 |

(continued)

| Compound Number | mHTT after treatment | Cell Viability striatal cells 3h heat shocked | Chemical Structure |
|---|---|---|---|
| 36 | 56 | 84 | 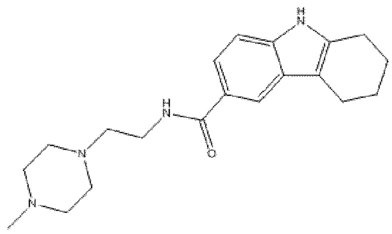 |
| 37 | 56 | 78 | 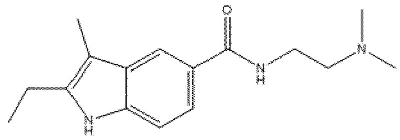 |
| 38 | 57 | 81 | 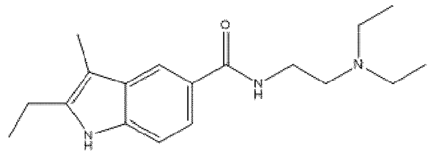 |
| 39 | 58 | 117 | 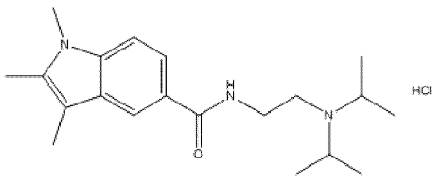 |
| 40 | 62 | 85 | 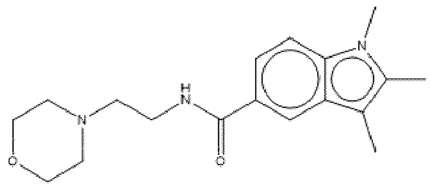 |
| 41 | 65 | 75 | 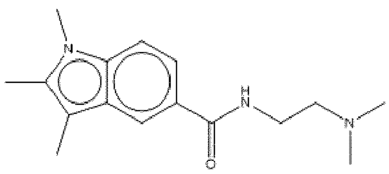 |
| 42 | 65 | 88 | 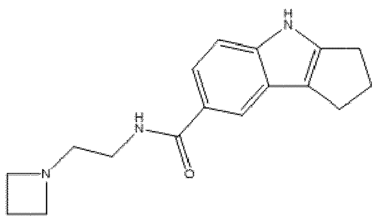 |
| 43 | 65 | 32 | 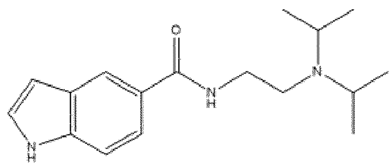 |

(continued)

| Compound Number | mHTT after treatment | Cell Viability striatal cells 3h heat shocked | Chemical Structure |
|---|---|---|---|
| 44 | 66 | 75 | 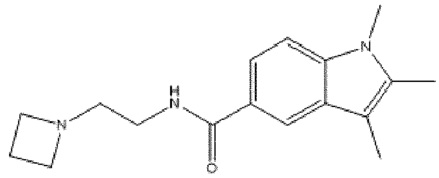 |
| 45 | 66 | 81 | 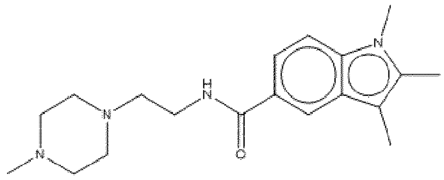 |
| 46 | 66 | 96 | 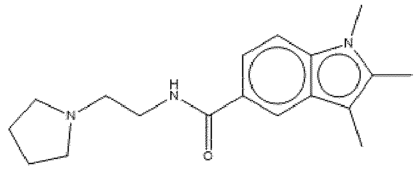 |
| 47 | 66 | 66 | 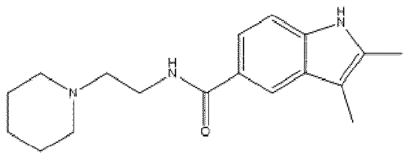 |
| 48 | 68 | 108 | 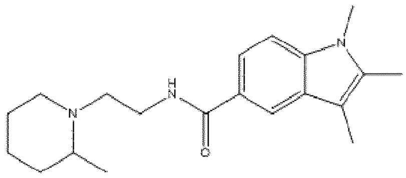 |
| 50 | 72 | 67 | 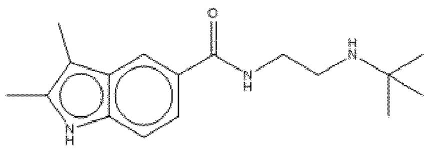 |
| 52 | 73 | 59 | 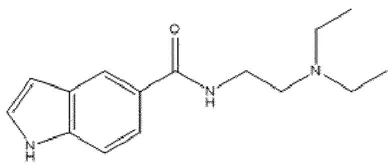 |
| 53 | 75 | 37 | 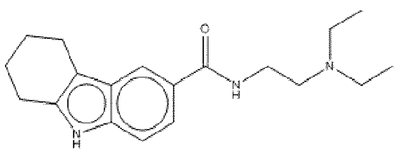 |

(continued)

| Compound Number | mHTT after treatment | Cell Viability striatal cells 3h heat shocked | Chemical Structure |
|---|---|---|---|
| 54 | 90 | 13 | 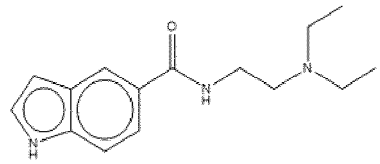 |
| 55 | 91 | 67 | 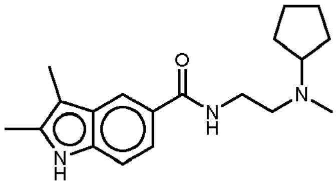 |
| 56 | 109 | 27 | 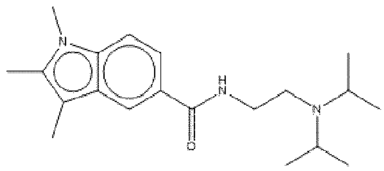 |
| | Nuclear mHTT after treatment | | |
| 127 | 23.9% | | 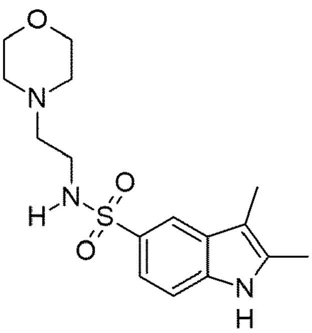 |
| 128 | 79.9% | | 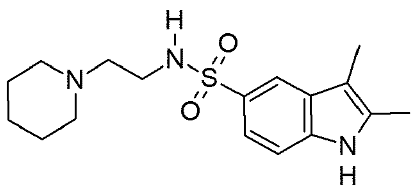 |

RESULTS-Table 12bsummarizes the effect of the compounds on cell viability. STHdh 7/7 were heat shocked for 3 hours and treated for 48 hours with small molecule compounds. Table 12b lists the cell viability in striatal cells after 48 hours.

**Table 12b.** Effect of Compounds on mHTT levels and cell viability of STHdh 7/7 striatal cells as a percentage in comparison to pre-heat shock cell viability.

| Compound Number | Cell Viability striatal cells 3h heat shocked (%) | Chemical Structure |
|---|---|---|
| | 29.2 | No treatment |

(continued)

| Compound Number | Cell Viability striatal cells 3h heat shocked (%) | Chemical Structure |
|---|---|---|
| 39 | 153.6 | 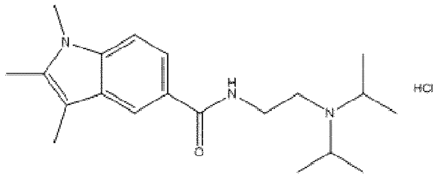 |
| 115 | 136.4 | 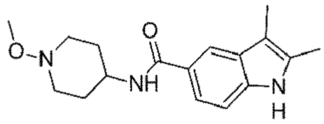 |
| 29 | 131.1 | 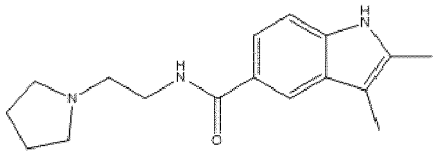 |
| 116 | 117.6 | 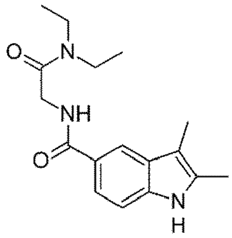 |
| 117 | 117.6 | 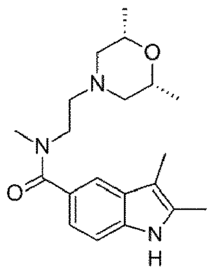 |
| 118 | 109.7 | 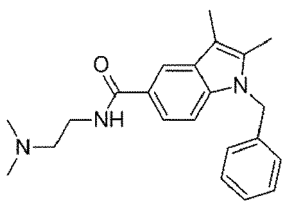 |
| 119 | 101.2 | 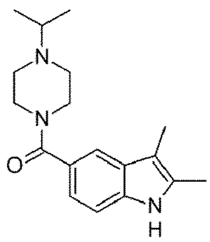 |

(continued)

| Compound Number | Cell Viability striatal cells 3h heat shocked (%) | Chemical Structure |
|---|---|---|
| 120 | 100 | 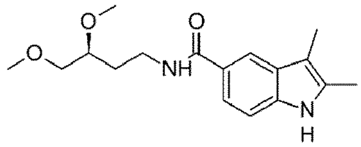 |
| 121 | 100 | 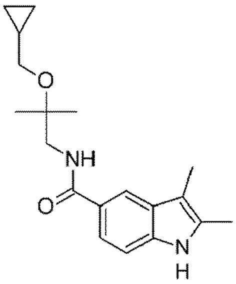 |
| 122 | 91.2 | 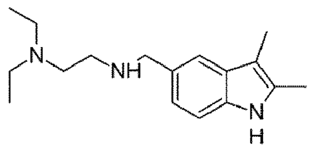 |
| 123 | 83.8 | 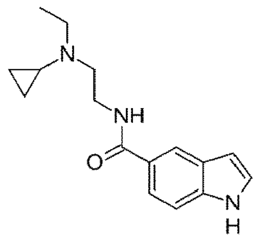 |
| 21 | 82.6 | 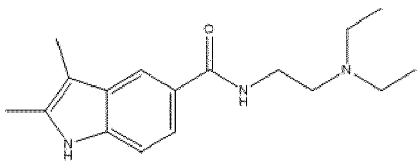 |
| 124 | 76.2 | 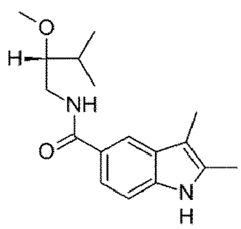 |
| 125 | 67.9 | 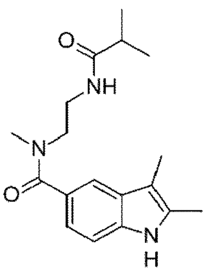 |

(continued)

| Compound Number | Cell Viability striatal cells 3h heat shocked (%) | Chemical Structure |
|---|---|---|
| 56 | 64.1 | 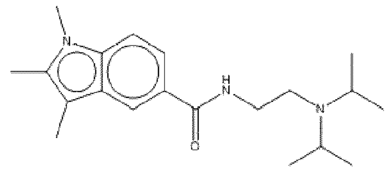 |
| 28 | 52.4 | 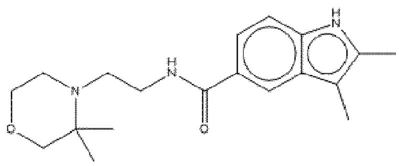 |
| 43 | 45.7 | 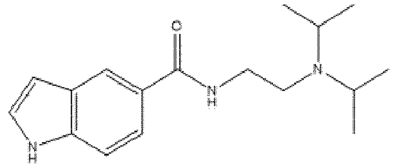 |
| 126 | 42.5 | 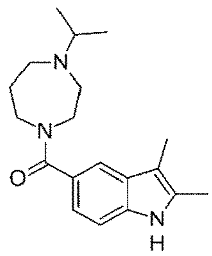 |
| 50 | 40.9 | 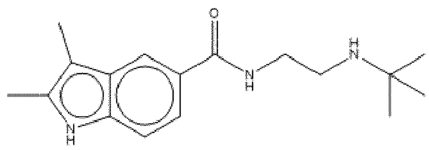 |
| 7 | 40.7 | 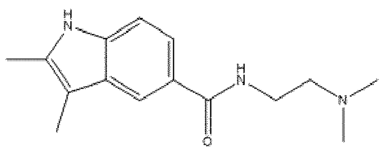 |
| 22 | 34.9 | 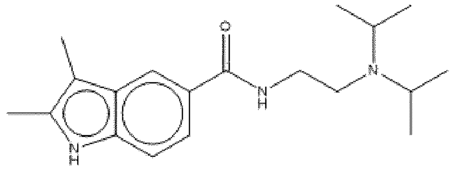 |

*Effect of Compound 22 on Diffuse mHTT levels* - ST HDH Q111/111 (CH00095, Coriell Institute) striatal derived cell line from a knock in transgenic mouse containing homozygous HTT loci with a humanized Exon 1 with 111 polyglutamine repeats. ST HDH Q7/7 (CH00097, Coriell Institute) striatal derived cell line from a knock in transgenic mouse containing HTT loci with a humanized Exon 1 containing 7 polyglutamine repeats. Nuclear diffuse mHTT was significantly reduced by 32% (p = 0.02) in STHdh 111/111 cells treated with Compound 22 in comparison to untreated STHdh 111/111 cells (**Figure** 3). But wildtype huntingtin was not reduced in STHdh 7/7 cells treated with Compound 22, in comparison to non-treated STHdh 7/7.

*Effect of Compound 22 on Cell Viability* - Heat shock triggers accumulation of misfolded proteins, which are

degraded. At 24 hours after treatment, the cell viability of Compound 22 treated STHdh 111/111 cells was almost equivalent to the cell viability of STHdh 7/7 control cells with wildtype huntingtin (**Figure** 4). The cell viability of the control STHdh 111/111 after 24 hours was lower than the cell viability of STHdh 111/111 cells treated with Compound 22.

*Physicochemical properties of Compound* 22 — Compound 22 is both soluble and it can cross the blood brain barrier. Significant concentrations can accumulate in the brain as indicated by comparison to the $EC_{50}$ value. The oral bioavailability in mice is 10%. This is due to the short microsomal stability in mice. In humans, the microsomal stability is 20 times higher in comparison to mice. **Table 13** describes physicochemical properties of Compound 22.

**Table 13**: Brain Exposure and Physicochemical Properties of Compound 22

| | Compound 22 |
|---|---|
| Thermodynamic Solubility | 2.1 mM |
| Kinetic solubility | 188 μM |
| Log D | 1.21 (pH 7,4) |
| Brain exposure | $C_{max}$ = 5.56 μM with i.p. administration and formulation |
| Blood Brain Barrier Penetrability | $AUC_{brain}/AUC_{plasma}$ = 2.24 |

*Effect of Compound 22 on Cell Viability of Wildtype Striatal Neurons* - STHdh 7/7 cells are striatal cells that express wildtype huntingtin. These cells were cultured in the same conditions as described above for STHdh 111/111. The neurons were first heat shocked for 3 hours at 41 °C as described above. After 48 hours of treatment with the small molecule Compounds, cell viability was determined using the method described above. The "buffer" control is represents cells treated with a buffer only, without any compounds added. Some of the compounds show higher cell viability in comparison to control heat shocked STHdh 7/7 neurons with buffer and no treatment (**Figure** 5). These compounds possess neuroprotective properties and protect striatal neurons from heat shock.

**Example 34 — Effects of Compound 22 on Motor Behavior in R6/2 Mice of Huntington's Disease**

*SUMMARY*

*Purpose* - The objective of this study was to investigate the effects of Compound 22 on body weight and motor deficits in transgenic R6/2 mice of Huntington's disease.

*Methods* — Total of 20 female and male R6/2 mice and 10 female and male wild-type littermate control mice (WT) were used in the study. The mice were genotyped and the R6/2 mice were divided into different treatment groups based on their litter and baseline body weight. The treatment with Vehicle or Compound 22 (33 mg/kg; 5 ml/kg, i.p. QD) was started at 4 weeks of age after the baseline behavioral tests (**Figure 6**). Body weights were measured at 3 weeks of age and twice a week until the end of the study. Motor function testing using rotarod were commenced at 4 weeks (pre-treatment baseline) and continued at 6, 8 and 10 weeks of age, accompanied with grip strength at 4 (pre-treatment baseline), 10 and 12 weeks of age. At the end point of 12 weeks of age the mice were subjected to tissue collection.

*Results* - *Body Weight:* There were no significant differences between the Compound 22 and vehicle treated R6/2 mice. *Rotarod:* Within females R6/2 mice treated with Compound 22 showed significantly improved rotarod performance at 10 weeks of age compared to vehicle treated R6/2 mice. This effect was seen namely when the data was analyzed as normalizing the data of subsequent age points to that of the 4-week baseline data of each group. *Grip Strength:* There were no significant differences between the Compound 22 and vehicle treated R6/2 mice.

*Conclusions* -The current findings are well in line with previously reported results in studies produced at CRL Finland in terms of the phenotype progression of the R6/2 mice. Reviewing the data of the pooled genders in this study the genotype difference in body weight was significant starting from 10 weeks whereas in a previous study the body weight difference was significant from 9 weeks although the weekly averages were close to the same level (Beaumont, V. et al. Neuron. 2016 Dec 21;92(6):1220-1237). The rotarod latency of the R6/2 mice was significantly decreased from 6 weeks onwards both in the current study and in the previously reported one (Beaumont V et al., 2016). Also the grip strength data were close to similar in both studies at 12 weeks of age.

Regarding the efficacy of the Compound 22 treatment, the most notable finding in the current study was that the chronic treatment with Compound 22 (33 mg/kg, i.p., QD) significantly improved the rotarod performance of female R6/2 mice at 10 weeks of age, however showing no similar enhancement within males when comparing to vehicle treated R6/2 mice. Body weight loss was significantly improved in female R6/2 mice treated with Compound 22 at week 12 in comparison to untreated female R6/2 mice. This improvement was not observed in male R6/2 mice treated with Compound

22 at week 12 in comparison to untreated male R6/2 mice. Compound 22 treatment had no significant effects in grip.

*PURPOSE OF THE STUDY* - The objective of this study was to investigate the effects of Compound 22 on body weight and motor deficits in transgenic R6/2 mice of Huntington's disease.

Total of 20 female and male R6/2 mice and 10 female and male wild-type littermate control mice (WT) were used in the study. The mice were genotyped and the R6/2 mice were divided into different treatment groups based on their litter and baseline body weight. The treatment with Vehicle or Compound 22 (33 mg/kg; 5 ml/kg, i.p. QD) was started at 4 weeks of age after the baseline behavioral tests. Body weights were measured at 3 weeks of age and twice a week until the end of the study. Motor function testing using rotarod were commenced at 4 weeks (pre-treatment baseline) and continued at 6, 8 and 10 weeks of age, accompanied with grip strength at 4 (pre-treatment baseline), 10 and 12 weeks of age. At the end point of 12 weeks of age the mice were subjected to tissue collection.

*MATERIALS AND METHODS*

*Animals* - All animal experiments were carried out according to the National Institute of Health (NIH) guidelines for the care and use of laboratory animals, and approved by the National Animal Experiment Board, Finland. The animal facility at site is accredited by the Association for Assessment and Accreditation of Laboratory Animal Care (AAALAC), International.

20 female and male R6/2 mice and 10 female and male wild-type littermate control mice were bred at Charles River, Germany. Mice derived from two consecutive rounds of breeding were randomly entered into the treatment plan below, using an equal number of males and females, and allocating them equally to the different treatment groups.

The experimental groups were:

**Group 1)** 10 wild-type mice (mixed gender) treated with Vehicle (5 ml/kg, i.p., QD) starting at 4 weeks of age

**Group 2)** 10 R6/2 mice (mixed gender) treated with Vehicle (5 ml/kg, i.p., QD) starting at 4 weeks of age

**Group 3)** 10 R6/2 mice (mixed gender) treated with Compound 22 (33 mg/kg; 5 ml/kg, i.p. QD) starting at 4 weeks of age

*Husbandry* - All mice were housed in groups of up to 5 per cage, in a temperature (22±1°C) and humidity (30-70%) controlled environment with a normal light-dark cycle (7:00-20:00 h light). All mice were housed in cages with clean bedding covering the ground that was changed as frequently as needed, at least once a week to provide the animals with dry bedding. This basic environment was enriched with the addition of a red mouse igloo (K3327), shredded paper and a wooden chewing stick. Food and water were available ad libitum to the mice in their home cages. The water spouts were fitted with extensions to allow mice to easily access from floor level. Each cage contained mice of only one gender and treatment group. In each cage was included also a wild-type mouse to provide normal social stimulation to R6/2 mice.

*Breeding and Weaning* - 20 female and male R6/2 mice and 10 female and male wild-type littermate control mice ($F_1$ generation) were bred by Charles River Laboratories, Sulzfeld, Germany by mating ($F_0$ generation) WT males (C57BL/6J; systematically re-infused with pedigreed JAX mice, stock 000664) with ovarian transferred (OT) TG females (JAX, stock 006494). After weaning mice were sent from Germany to Charles River, Kuopio, Finland at an age of 3 weeks. Following genotyping and acclimation, the mice were enrolled in the study.

*Genotyping* - Mice were ear marked at the age of 15-21 days and tail samples were collected at the same time for genotyping with PCR. Genotyping was performed at Charles River Discovery Services, Kuopio. DNA was isolated from tail or ear samples with Phire Animal Tissue Direct PCR-kit (Thermo Scientific, ref. F140WH) according to the kit's instructions. Then 1 μl of DNA was multiplied in the PCR reaction with mouse specific (Gapdh) primers and human specific (Htt) primers.

Primers sequences and final working concentrations are listed below. After the PCR, multiplied DNA was separated by agarose gel electrophoresis. The expected products were 272bp (human specific product) and 372bp (mouse specific product). Thus wild-type (WT) mouse has one 372bp band while transgenic (TG) mouse has both the 272bp and 372bp bands.

Human specific 25 pmol/ μl, (5'-3'): TCATCAGCTTTTCCAGGGTCGCCAT (SEQ ID NO: 8)

Human specific 25 pmol/μl, (5'-3'): CGCAGGCTAGGGCTGTCAATCATGCT (SEQ ID NO: 9)

Mouse specific 5 pmol/μl, (5'-3'): ACTCCACTCACGGCAAATTCAACGGCAC (SEQ ID NO: 10)

Mouse specific 5 pmol/μl, (5'-3'): GGTCATGAGCCCTTCCACAATGCCAAAG (SEQ ID NO: 11)

Tail samples of all mice were taken at the end of the study for possible CAG repeat analysis.

*Plasma Sample Collection for Bile Acid Analysis* - At 4 weeks of age blood samples for bile acid analysis were collected from saphenous vein into pre-cooled (ice bath) Li-Hep-tubes. The tubes were kept on ice and plasma was separated by centrifugation at 2000 g (+4°C). About 50 μl of plasma from each mouse was aliquoted into pre-cooled polypropylene tubes and stored at -80°C until analyzed. Plasma samples were analyzed using Thermofisher Konelab Xti 20 according to manufactures instructions. The mice having abnormally high bile acid levels in the plasma (more than 10 μmol / l) were removed from the study.

*General Health Status and Humane End-Points* - Animals were monitored daily by laboratory personnel. The following humane end-points applied to all animals, unless otherwise mentioned in the experimental license granted by the National Ethics Committee. If the animal reached the humane end-points, it was euthanized.

The animals' welfare was assessed by observing the following signs: general appearance (dehydration, weight loss, abnormal posture, condition of skin and fur, signs of pain); ambulation (reluctance or difficulties to move); behavior (apathy, abnormal behavior); clinical signs (eating, drinking, urinating, defecating). In addition, the mouse was euthanized if the mouse was not able to right itself within 20 sec when put on one side.

If there was a deviation from normal, the animal was closely monitored and treated, when possible (e.g. hydration, analgesia, warming). As a general rule, the animal was monitored no longer than 24-48 hours, after which the animal was euthanized, if its condition had not markedly improved.

*Compound Delivery and Dosing* - Treatment with Compound 22 (33 mg/kg; 5 ml/kg, i.p. QD) or Vehicle was started at age week 4 and continued until the 12 weeks of age. The test articles were handled and stored and the dose formulations were prepared according to detailed instructions provided by the Sponsor.

*Body Weight* - Body weight was measured starting at 3 weeks of age before treatment onset and two times per week on the same day (on Monday and Friday) until the end of the study.

*Motor Function and Cognition* - The behavioral tests were conducted during the diurnal phase, between 8 a.m. - 5 p.m. The mice were transported to the behavioral test rooms from the animal housing rooms in their home cages. The mice were allowed to acclimate in the behavioral test room conditions at least for an hour before the tests. The behavioral tests were conducted under normal white light conditions.

*Rotarod* - The Rotarod test was perfomed at 4 (pre-treatment baseline), 6, 8 and 10 weeks of age. Each testing day included a training trial of 5 min at 4 RPM on the Rotarod apparatus (AccuScan Instruments, Columbus, USA). 30 minutes later, the animals were tested for 3 consecutive accelerating trials of 6 min with the speed changing from 0 to 40 RPM over 360 seconds and with an inter-trial interval of at least 30 min. The latency to fall from the rod was recorded. Mice remaining on the rod for more than 360 s were removed and their time scored as 360 sec.

*Grip Strength* - Mice were tested at 4 (pre-treatment baseline), 10 and 12 weeks of age. Mice were taken to the experimental room and, one at a time, were placed on the grip strength apparatus (San Diego Instruments, San Diego, USA) in such a way that the animal grabbed a small mesh grip with its forepaws. The entire apparatus was placed on a table top for testing. Animals were lowered to the platform and then slowly pulled away from the handle by the tail until the animal released the handle. The equipment automatically measures the strength of the animal's grip in grams. Five scores were recorded per animal in consecutive sequence, and the average of three best scores for each animal was used for the results. Mice were returned to their home cage after testing.

*End-Point and Tissue Processing* - Approximately one hour after the last dose the mice were terminally anesthetized with pentobarbital. A blood sample was collected via cardiac puncture in EDTA coated tubes on ice and plasma was separated by centrifugation (2000 g for 10 min). The plasma was aliquoted in two samples and fresh frozen on liquid nitrogen. Thereafter the mice were transcardially perfused with ice cold heparinized saline (Heparin 2.5 IU/ml) 25 ml)), followed by perfusion with ice cold 4 % PFA (80 ml). The brains were fixed by immersion in 4 % paraformaldehyde for minimum of 24 h after which brain samples were cryoprotected by 30 % sucrose solution for 72 h after which the brain samples were frozen in liquid nitrogen. Frozen brain specimens were stored at -80 °C.

*Statistical Analysis* - All values are presented as mean ± standard error of mean (SEM), and differences are considered to be statistically significant at the $P<0.05$ level. Statistical analyses were performed using GraphPad Prism statistical software. Differences among means were analyzed by using unpaired t-test.

*RESULTS*

*Body Weight* - The effects of chronic administration of Compound 22 (33 mg/kg) on body weight of R6/2 mice from 3 to 12 weeks are presented in FIGS. 11-13. There were no significant differences between the vehicle and Compound 22 treated R6/2 mice in body weight (unpaired t-test, $p > 0.05$) (FIGS. 11-13). The vehicle treated R6/2 mice had decreased body weight compared to wild-type mice at 10-12 weeks of age within pooled genders and females, and

at 9-12 weeks of age within males (unpaired t-test, # $p < 0.05$) (FIGS. 11-13).

*Rotarod* - The effects of chronic administration of Compound 22 (33 mg/kg) on rotarod performance of R6/2 mice are presented in FIGS. 14-19. Within females R6/2 mice treated with Compound 22 showed significantly improved rotarod performance at 10 weeks of age compared to vehicle treated R6/2 mice (unpaired t-test, * $p < 0.05$) (**Figure** 17). This effect was seen namely when the data was analyzed as normalizing the data of subsequent age points to that of the 4-week baseline data of each group. However, there were no significant differences between the Compound 22 and vehicle treated R6/2 mice within pooled genders or males (unpaired t-test, $p > 0.05$) (FIGS. 14-15 and 18-19). Vehicle treated R6/2 mice had decreased rotarod latency at 4-10 weeks within pooled genders and females, and at 6-10 weeks of age within males compared to wild-type mice (# $p < 0.05$, unpaired t-test) (FIGS. 14-19).

*Grip Strength* - The effects of chronic administration of Compound 22 (33 mg/kg) on grip strength of R6/2 mice are presented in FIGS. 17-19. There were no significant differences between the vehicle and Compound 22 treated R6/2 mice in grip strength (unpaired t-test, $p > 0.05$) (FIGS. 20-22). The vehicle treated R6/2 mice had lower grip strength at 12 weeks of age within pooled genders and males compared to wild-type mice (unpaired t-test, # $p < 0.05$) (FIGS. 20 and 22).

*CONCLUSIONS*

The current findings are well in line with previously reported results in studies produced at CRL Finland in terms of the phenotype progression of the R6/2 mice. Reviewing the data of the pooled genders in this study the genotype difference in body weight was significant starting from 10 weeks whereas in a previous study the body weight difference was significant from 9 weeks although the weekly averages were close to the same level (Beaumont, V. et al. Neuron. 2016 Dec 21;92(6):1220-1237). The rotarod latency of the R6/2 mice was significantly decreased from 6 weeks onwards both in the current study and in the previously reported one (Beaumont V et al., 2016). Also the grip strength data were close to similar in both studies at 12 weeks of age.

Regarding the efficacy of the Compound 22 treatment, the most notable finding in the current study was that the chronic treatment with Compound 22 (33 mg/kg, i.p., QD) significantly improved the rotarod performance of female R6/2 mice at 10 weeks of age, however showing no similar enhancement within males when comparing to vehicle treated R6/2 mice. Compound 22 treatment had no significant effects in body weight development or grip strength of the R6/2 mice.

**Example 35** - **Immunohistochemistry Study with Tissue Isolated from R6/2 Study**

*Preparation of Immunohistology slides* - Brain tissue collected within the animal study was prepared for IHC studies by Charles River staff. The brains were fixed by immersion in 4% paraformaldehyde for at least 24 h after which brain samples were cryoprotected by 30% sucrose solution for 72 h. Finally, the brain samples were flash-frozen in liquid nitrogen and stored at -80 °C. The brain samples were cut using a microtome cryostat system, producing coronal brain tissue sections of 40 μm thickness. Those were mounted on individual adhesive-coated microscope glass slides with frosted ends.

*Staining* - Goat anti-Rabbit IgG (H+L) Alexa Fluor 647 (1:500, ThermoFisher Invitrogen) was used as a secondary antibody for binding to CBP antibody. DAPI (Sigma-Aldrich) was used to identify the nuclei. Goat anti-Mouse IgG (H+L), Alexa Fluor 488 (1:500, A-11034, ThermoFisher Invitrogen) was used as a secondary antibody for binding to EM48 antibody. Mouse anti-human-mHTT (EM48, 1:500, Sigma-Aldrich), was used to stain huntingtin. Rabbit anti-CBP (1:100, Sigma-Aldrich) was used a primary antibody to stain CBP.

The blocking buffer was freshly prepared and consisted of PBS (Sigma-Aldrich) with 5% normal goat serum (NGS), 0.2% BSA, 0.2% lysine and 0.2% glycine. Samples were covered with 750 μL of blocking buffer per sealing chamber and incubated at 4 °C for 24 hours. Subsequently, on day two samples were washed three times 10 min each in PBS, before working dilutions of primary antibodies were applied in 750 μL primary antibody buffer per chamber. The primary buffer consisted of PBS with 2% BSA/0.3% Triton X-100 (Sigma-Aldrich) and 0.02% $NaN_3$ as preservative agent. The samples were incubated with primary antibodies at 4°C for 73 hours. On day five, samples were washed like described previously and then incubated at 4°C for 24 hours with secondary antibody in 750 μL secondary antibody buffer at 1:500 working dilutions per chamber. The secondary buffer consisted of PBS with 3% NGS/0.3% Triton X-100/0.02% $NaN_3$. On day six, samples were washed and then incubated with DAPI containing mounting medium Fluoroshield (Sigma-Aldrich), in order to counterstain the nuclei and preserve the fluorescence. Therefore, one drop of mounting medium (Dako) was added per tissue section and the sample carefully coverslipped avoiding introduction of air bubbles. The samples were stored for 24 hours at room temperature shielded from light before being stored at 4°C until imaging.

*Results* - **Figures 23A-23F** show the distinction of inclusion bodies (IB) and diffuse species of mHTT by confocal fluorescence imaging. **Figures 23A-23F** represent images taken of either the striatum or the cortex for the analysis of mHTT accumulation within nuclei of R6/2 mice. In the transgenic samples it can be seen that large IBs are clearly visible

in a multitude of nuclei. Moreover, most IBs appear to be surrounded by diffuse protein, visibly as a hazy signal in the nucleus. Consequently, this distinction of diffuse mHTT and IBs was implemented in the analysis macro to quantify the different species of mHTT within striatum and cortex of R6/2 mice. To this end both an upper threshold of diffuse protein fluorescence signal and an adjacent lower threshold of IB fluorescence intensity were set and the images quantified appropriately.

Diffuse mHTT was reduced in the cortex of animals treated with Compound 22. Diffuse mHTT consists of monomers and oligomers. Diffuse forms of mHTT are highly toxic in comparison to mHTT aggregates. Nuclear diffuse mHTT was reduced by 40% in treated animals (n= 9) in comparison to vehicle treated TG mice (p = 0.01) **(Figure 16 (A)).** Compound 22 lowers mHTT in the cortex of R6/2 model mice.

The reduction of diffuse mHTT correlates with motor symptoms. **Figure 16 (B)** shows that the motor symptoms strongly negatively correlate with diffuse mHTT in the nuclei of the cortex (Pearson r: -0.8, p= 0.01, n=9 (all transgenic female mice), confidence interval of r= -0.9561 to -0.2896 for nuclear diffuse mHTT). mHTT can be used as a biomarker for clinical efficacy as measured by motor symptoms. Therefore, lowering mHTT is a strategy to treat symptoms of Huntington's disease.

**Example 36 - Compound 22 Improves Motor Symptoms in R6/2 Mice**

10 female R6/2 mice and 10 female wild-type littermate control mice (F1 generation) were bred by Charles River Laboratories, Sulzfeld, Germany by mating (F0 generation) WT males (C57BL/6J; systematically re-infused with pedigreed JAX mice, stock 000664) with ovarian transferred (OT) TG females (JAX, stock 006494). After weaning mice were sent from Germany to Charles River, Kuopio, Finland at an age of 3 weeks. Following genotyping and acclimation, the mice were enrolled in the study.

The treatment with Vehicle or Compound 22 (33 mg/kg; 5 ml/kg, intraperitoneal once daily was started at 4 weeks of age after the baseline behavioral tests. Motor function testing using rotarod were commenced at 4 weeks (pre-treatment baseline) and continued at 6, 8 and 10 weeks of age. R6/2 mice treated with Compound 22 showed significantly improved rotarod performance at 10 weeks of age compared to vehicle treated R6/2 mice. The mean latency to fall was 108.3 s $\pm$ 32.9 s in treated transgenic R6/2 mice and 55.7 s $\pm$ 19.1 s ($p < 0.05$) **(Figure 17).** The R6/2 model is a very aggressive model and an improvement by 20% is regarded as a positive outcome. Compound 22 almost doubled the latency to fall in treated transgenic model mice indicating that Compound 22 improves motor symptoms in R6/2 mice.

**Example 37 - Co-localization of CREB-binding protein (CBP) with mHTT and Quantification of CBP Abundance**

CREB-binding protein (CBP) is a central coactivator of gene transcription. CBP serves as a molecular scaffold, facilitating interaction of CREB and other transcriptional regulators by enhancing the formers transcriptional activity toward cAMP-responsive genes. Additionally, it shows histone acetyltransferase (HAT) activity, thereby regulating gene transcription activity through epigenetic modifications, i.e. the modulation of histone acetylation status as well as of other transcriptional factors (Jiang, H. et al., Neurobiol Dis. 2006 Sep;23(3):543-551). In HD, the homeostatic functions of CBP are disrupted, since the mHTT shows aberrant interaction with CBP. CBP nuclear depletion analysis was performed based on scientific evidence, showing enhanced CBP degradation via the UPS pathway mediated by mHTT binding resulting in nuclear depletion of CBP (La Spada, A.R. et al., Nat Rev Genet. 2011 Apr;11(4):247-2589; Cong, S.Y. et al., Mol Cell Neurosci. 2005 Dec;30(4):560-571).

CBP colocalization with mHTT and quantitative assessment of the images were measured (Lee, J. et al., Acta Neuropathol. 2017 Nov;13(5):729-748; Steffan, J.S. et al., Nature. 2001 Oct 18;413(6857):739-743; Nucifora, F.C. et al., Science. 2001 Mar 23;291(5512):2423-2428). In brief, regions of interest (colocalized areas) in multi-channel images from two or three of the overlapping fluorescence dyes were selected using the Plugin CoLoc2 of FIJI. The colocalization score of identified areas was calculated. We used the FIJI plugin Coloc2 for measuring colocalization of CBP with diffuse mHTT. We detected reduced colocalization of CBP with mHTT in treated TG mice **(Figure 18).**

**Example 38 - Circular Dichroism Study**

*Protocol*

Protein samples were diluted with buffer to 7.5 $\mu$M prior to Circular Dichroism (CD) measurements. Measurements were conducted at room temperature.

Four spectra at each measurement were taken every 1 nm from 200 to 260 nm, scanning at 50 nm/min with an averaging time of 1 sec. Spectra were obtained from samples in 20 mM phosphate, pH 7.4. Ten scans were averaged for each sample spectrum

Single-wavelength readings at 222 nm were obtained. After subtraction of the Trx contribution, the $\alpha$-helical

content of Httex1 was estimated from its mean residue ellipticity (MREHttex1) at 222 nm. This calculation was conducted as described by Bravo-Arredondo (Bravo-Arredondo, J.M. et al., J Biol Chem. 2018 Dec 21;293(51):19613-19623).

MRE Exon-1 Q16 and Q46 (Mean Residue Ellipticity) was measured every 1 sec for 300 sec; the 301 readings for each sample were averaged and MRE Trx subtracted. The number of amino acids in each sample to experience a change in helicity was estimated using a previously developed helix-coil transition model, which gave the change in fraction of helicity (Δ*fHelix*) by the following equation:

$$f_{Helix} = \frac{MRE_{Httex1} - MRE_{Coil}}{MRE_{Helix} - MRE_{Coil}}$$

where *MRE* Exon1 Q26 or Q46 ($MRE_{Httex1}$ in the formula) describes the helix-coil transition, obtained from the difference between MRE Eon1 Q26 or Q46 at 222 nm of the fusion protein at the given temperature (in our case 37 °C) and the product of MRE Trx at 222 nm at the same temperature and the fraction of residues comprised by Trx in that particular construct, and

$$MRE_{Coil} = 2220 - 53T, MRE_{Helix} = (-44{,}000 + 250T)\left(1 - \frac{3}{N_r}\right)$$

given T as the temperature in °C and $N_r$ as the number of residues.

In order to calculate an equilibrium of the two states model, percent α-helicity was calculated using the relation of $MRE_{Httex1}$ = 0 and -34700 (deg $cm^2$ $dmol^{-1}$) for 0% and 100% helicity, respectively.

**Table 14:** Comparison between Kd, reversal of conformational changes of Exon1 Q46 and reduction of mHTT in comparison to untreated striatal neurons

| Compound ID | Kd (μM) | Reduction of the proportion of a predominantly α-helical state to wildtype levels with 2 μM (Compound 22) or 1.6 μM (Compound 4 and Compound 101) compound exposure | mHTT after 3 h heat shock and following 48 hours of compound treatment in comparison |
|---|---|---|---|
| | | | to untreated STHdh 111/111 |
| Compound 4 | 0.06 | Complete reversal | 34% |
| Compound 101 | 0.07 | 59.3% | 38% |
| Compound 22 | 0.7 | 36.6% | 46% |

*Compound 22:* In this CD measurement the predominately α-helical state of Exon1 Q46 mHTT was 23.6% and thus 6% higher than the 17.6% proportion of predominantly α-helical state of Exon1 Q16 mHTT. Treatment with 2 μM Compound 22 reduced the proportion of a predominantly α-helical state to 21.4% of Exon1 Q46 and thus reduced the proportion of a predominantly α-helical state by 36.7% in comparison to short Q length Exon1 **(Figure 19)**.

*Compound 4:* Compound 4 was used for CD measurements. Compound 4 exhibited a $K_d$ of 60 nM to Exon1 Q46 protein. Compound 22 exhibited a $K_d$ of 701 nM. Thus, Compound 4 more efficiently reversed the conformational change in mutated Exon1 Q46 than Compound 22 **(Table 14).** In this CD measurement the predominately α-helical state of Exon1 Q46 mHTT was 23.2% and thus 3.4% higher than the 19.9% proportion of predominantly α-helical state of Exon1 Q16 mHTT **(Table 15**). Treatment with 1.6 μM Compound 4 reduced in Exon1 Q46 the proportion of a predominantly α-helical state to 19.5% and thus reversed the proportion of a predominantly α-helical state completely back to short Q length Exon1 **(Figure 20).**

Thioredoxin (Trx) was used as a control to demonstrate that exposure to the compounds have no impact on the α-helix structure of proteins which are not mutated huntingtin **(Figure 21)**. Trx contains 31% α-helix.

**Table 15:** Percentage of Predominantly α-Helical Status of Exon1 Q46 Exposed to Different Doses of Compound 4.

| conc. Compound (μM) | Fraction Helix fH (%) |
|---|---|
| 0 | 23.2 |
| 0.01 | 22.4 |
| 0.059 | 20.9 |
| 0.158 | 21.1 |
| 0.647 | 20.0 |
| 1.62 | 19.5 |
| 4.04 | 19.3 |
| 13.68 | 18.3 |
| 37.77 | 18.1 |

*Compound 101*

The CD measurements protocol for Compound 101 was the same as outlined above except that from 206 nm - 210 nm and 220 nm - 224 nm, the step size was 0.2 nm, and the bandwidth was 1nm.

The long Q length (Q46) Exon1 increase the proportion of a predominantly α-helical state about 2.3% from 18.1% in short Q length Exon1 to 20.4% in long length Exon1 Q46. Treatment with 1.6 μM Compound 101 reduced the proportion of a predominantly α-helical state to 19.0% of Exon1 Q46 and thus reduced the proportion of a predominantly α-helical state by 59.3% in comparison to short Q length Exon1 **(Figure 22** and **Table 11).**

**Table 16:** Percentage of Predominately α-helical Status of Exon1 Q46 Exposed to Different Doses of Compound 101

| conc. Compound (μM) | Fraction Helix fH (%) |
|---|---|
| 0 | 20.4 |
| 0.01 | 20.5 |
| 0.059 | 20.1 |
| 0.158 | 19.9 |
| 0.647 | 19.1 |
| 1.62 | 19.0 |
| 4.04 | 18.5 |
| 13.68 | 18.7 |
| 37.77 | 18.2 |

**Example 39 -Autophagy flux increase is responsible for mHTT reduction in Compound 22 treated neurons**

*Cell culture* - ST HDH Q111/111 (CH00095, Coriell Institute) striatal derived cell line were grown at 33°C in DMEM (Sigma-Aldrich), supplemented with 10% fetal bovine serum (FBS), 1% Penicillin-Streptomycin (ThermoFisher Scientific), and 0.4 mg/ml G418 (Geneticin; Invitrogen).

*Pre-Treatment of striatal cell line* - First the medium was removed and new DMEM (DMEM, high glucose, HEPES, no phenol red) medium, supplemented with 10% fetal bovine serum (FBS), 1% Penicillin-Streptomycin, containing 10 μM compound was added. The cells were incubated at 33 °C for 24 hours.

*Heat shock* - Cells were heat shocked for 3 hours at 41 °C.

*Treatment* - Medium was removed and new DMEM (DMEM, high glucose, HEPES, no phenol red) medium, supplemented with 1% fetal bovine serum (FBS), 1% Penicillin-Streptomycin, containing no compound, or 10 μM Compound 22, or 10 mM NH4Cl (Sigma Aldrich), or 125 nM MG132 (Sigma Aldrich), or 10 μM Compound 22 with 10 mM NH4Cl, or 10 μM Compound 22 with 125 nM MG132 was added. The cells were incubated at 33 °C for 48 hours.

*Cell Viability* - Cell viability was measured according to manufacturer recommendations with CellTiter Glo (Promega). 96-well plates for used either for cell viability determination or cell staining.

*Staining* - Cells were permeabilized with 0.3% Triton X-100 in 4 °C PBS (pH 7.4 from ThermoFisher) for 10 minutes. Then the cells were washed two times with 4 °C PBS. Subsequently, cells are blocked with 3% NGS (normal goat serum from Thermo Fisher) in 4 ° C PBS for 1 hour. Cells were incubated with primary antibodies in blocking buffer (PBS, 3% NGS, 0.02% NaN3) over night at 4 °C. Primary antibodies were mouse antipolyglutamine monoclonal antibody 3B5H10 (Sigma-Aldrich) at 1:250 dilution and Guinea Pig anti-MAP2 polyclonal antibody (Synaptic Systems) at 1:500 dilution. For LC3 staining primary antibodies were mouse anti-LC3 mab (5F10) (nanoTools) at 1:500 dilution and Guinea Pig anti-MAP2 polyclonal antibody (Synaptic Systems) at 1:500 dilution.

Cells were washed 2 times with 4 °C PBS, and then incubated with secondary antibodies at 1:2000 dilution in blocking buffer for 1hour at room temperature. Secondary antibodies were Goat anti-Guinea Pig IgG (H+L) Highly Cross-Adsorbed Secondary Antibody, Alexa Fluor 647 (ThermoFisher Invitrogen) and Goat anti-Mouse IgG (H+L) Cross-Adsorbed Secondary Antibody, Alexa Fluor 488 (ThermoFisher Invitrogen). Cells were washed 2 times with 4 °C PBS, and then the nuclei were counterstain with DAPI (ThermoFisher) for 10 min at room temperature in the dark. Cells were washed 2 times with 4 °C PBS, and then 4 °C PBS was added to cells and then images were acquired.

*Fluorescence Imaging* - Imaging sessions were performed on a confocal microscope system (Carl Zeiss Microscopy) equipped with a dual spinning disk unit (Yokogawa). All components of the imaging system were controlled via the ZEN 2 software suite (Carl Zeiss Microscopy). The laser lines used were 405 nm, 488 nm and 639 nm to excite DAPI or the respective fluorophores. The fluorescence images obtained of the immunofluorescence labelled tissue sections were quantified with the help of the "Image Processing and Analysis in Java" or short ImageJ software distributed under the GNU General Public License by the NIH (Rueden, C.T. et al., BMC Bioinformatics. 2017 Nov 29;18(1):529), i.e. the edition used was the Fiji distribution (Schindelin, J. et al., Nature methods. 2012 Jun 28;9(7):676-82). Nested t test was performed to calculate the significance amongst repeated measurements using GraphPad Prism software.

*RESULTS - Compound 22 improves autophagic flux* - An increased level of LC3-II or an accumulation of GFP-LC3 puncta is not always indicative of autophagy induction and may represent a blockade in autophagosome maturation (Fass, E. et al., J Biol Chem. 2006 Nov 24;281(47):36303-16). Autophagic flux is generally defined as a measure of the autophagic system's degradation activity (Klionsky, D.J. et al., Autophagy. 2012 Nov:7(11):1273-94). If autophagic flux is occurring, the level of LC3-II will be increased in the presence of a lysosomal degradation inhibitor because the transit of LC3-II through the autophagic pathway will be blocked (Tanida, I. et al., Autophagy. 2005 Jun;273(11):2553-62).

Autophagic flux was calculated as the area stained with LC3 antibody per cell in STHdh 111/111 with autophagy inhibitor NH4Cl minus area stained with LC3 antibody per cell in STHdh 111/111 without the autophagy inhibitor NH4Cl.

The Compound 22 treated STHdh 111/111 cells have a positive autophagic flux and the untreated STHdh Q111/111 cells have a negative autophagic flux **(Figure 23 (A))**.

This explains that in striatal neurons treated with Compound 22 LC3 stained area in STHdh 111/111 neurons is decreased and in untreated neurons LC3 is increased ($p < 0.001$) **(Figure 23 (B)).** Several research groups observed that the autophagosome marker LC3 expression is increased in STHdh Q111/111 neurons expressing mutated huntingtin compared to wild type (WT) controls (Walter, C. et al., Neuropharmacology. 2016 Sep;108:24-38).

*Autophagosomal degradation is mainly responsible for mHTT reduction in neurons treated with Compound* 22 - Striatal neurons STHdh 111/111 which express mHTT were exposed to 10 mM of the autophagy inhibitor NH4Cl. The autophagy inhibitor NH4Cl blocked the mHTT lowering effects of Compound 22 completely **(Figure 24 (A)).** The ubiquitin proteasome system (UPS) is one of the main pathways for the degradation of misfolded proteins. Compound 22 could reduce mHTT in STHdh 111/111 cells which were exposed to the UPS inhibitor MG132 **(Figure 24 (B)).** But the mHTT reduction in Compound 20 treated striatal cells exposed to MG132 is less in comparison striatal cells only treated with Compound 20 without exposure to MG132. This observation indicates that the mHTT reduction in Compound 20 treated cells is in part due to the UPS **(Figure 35).**

**Example 40** - **Effects of Compound 22 on Gene Expression**

Gene expression of certain transcripts of interest were measured using Nanostring Technology. STHdh 7/7 and STHdh 111/111 cells were used. Some STHdh 7/7 and STHdh 111/111 cell samples were not heat shocked and some wells were treated with Compound 22 at the indicated concentration on for 48 hours **(Table 17).** Some STHdh 7/7 and STHdh 111/111 cell samples were heat shocked for 3 hours some wells were treated with Compound 22 at the indicated concentration on for 48 hours **(Table 18).** Cells are washed with PBS, trypsinised, and gathered in a 15 ml falcon tube. After a short spin down for 3 minutes at 300 g, the cell pellet was washed with PBS once, before it was frozen down in liquid nitrogen. RNA was extracted with a standard protocol. The nCounter® Mouse Neuropathology Panel (Nanostring, Seattle) was used for gene expression analysis according to the manufacturer's protocols.

**Table 17.** Cell samples that were not subject to heat shock treatment

| SLOT | Cell Line | Compound 22 (μM) | Treatment time |
|---|---|---|---|
| 1 | STHdh7/7 | 0 | **48 h** |
| 2 | STHdh7/7 | 10 | **48 h** |
| 3 | STHdh7/7 | 20 | **48 h** |
| 4 | STHdh111/111 | 0 | **48 h** |
| 5 | STHdh111/111 | 10 | **48 h** |
| 6 | STHdh111/111 | 20 | **48 h** |

**Table 18.** Cell samples subject to heat shock treatment

| SLOT | Cell Line | Compound 22 (μM) | Time | Heat Shock |
|---|---|---|---|---|
| 1 | STHdh7/7 | 0 | **48 h** | **42°C / 3h** |
| 2 | STHdh7/7 | 10 | **48 h** | **42°C / 3h** |
| 3 | STHdh7/7 | 20 | **48 h** | **42°C / 3h** |
| 4 | STHdh111/111 | 0 | **48 h** | **42°C / 3h** |
| 5 | STHdh111/111 | 10 | **48 h** | **42°C / 3h** |
| 6 | STHdh111/111 | 20 | **48 h** | **42°C / 3h** |

Genes associated with the autophagy pathway were upregulated in STHdh 7/7 neurons treated with 10 μM or 20 μM Compound 22 in comparison to untreated cells. Sqstm1, Atp6v0c, Ctns, Gnptg, Tcirg1 and Gnptab were equal to or greater than 2 times the standard deviation from the mean upregulated in STHdh 7/7 neurons treated with 20 μM Compound 22 **(Figure 34).**

Genes associated with the autophagy pathway were upregulated in STHdh 111/111 neurons treated with 10 μM or 20 μM Compound 22 in comparison to untreated cells. Slc1a1, Ctse, Atp6vlh, Atp6v0d1, Ap3s1, Lamp1, Cd68, Gsub, Gba, Man2b1, Ppt1, Hexb, Npc1 and Gga1 were equal to or greater than 1 time the standard deviation from the mean upregulated in STHdh 111/111 neurons treated with 20 μM Compound 22 **(Figure 34).**

SLC32A1 (member 1 of the solute carrier family 32) is involved in the filling of vesicles at GABAergic and glycinergic synapses. GABA and glycine are the main inhibitory neurotransmitters in the brainstem and spinal cord (Reinus, R. at al., Front Behav Neurosci. 2015 Mar 27;9:71). SLC32A1 expression is downregulated in STHdh Q111/111 neurons expressing mHTT by 89% in comparison to wildtype STHdh Q7/7 level **(Figure 25 (A)).** In Compound 22 treated STHdh 111/111 cells the Slc32a1 expression is upregulated 12-fold in comparison to untreated wildtype STHdh 111/111 neurons. Slc32a1 is downregulated in STHdh 111/111 (Q111-0) neurons in comparison to STHdh 7/7 (Q7-0). Treatment with Compound 22 improves SLC32a1 expression in STHdh 111/111 cells (Q111-10).

The GTPase Ras homolog-enriched in the striatum (Rhes) inhibits dopaminergic signaling in the striatum. Rhes is involved in the motor control of the stratum. Rhes is implicated in HD. It was described that the guanine nucleotide exchange factor (GEF) RasGRP1 inhibits Rhes role in striatal motor activity (Shahani, N. et al., Sci Signal. 2016 Nov 15;9(454):ra111). Rasgrp1 expression is upregulated in STHdh Q111/111 neurons expressing mHTT by 31% in comparison to wildtype STHdh Q7/7 level **(Figure 25 (B)).** In Compound 22 treated STHdh 111/111 Rasgrp1 expression is downregulated by 29% in comparison to untreated STHdh 111/111 neurons.

White matter abnormalities are prominent neuropathological features in Huntington's disease (HD). They noted that the first group, which are sharply downregulated by mHTT, includes well-known oligodendrocyte lineage transcription factors OLIG2 (Osipovitch, M. et al., Cell Stem Cell. 2019 Jan 3;24(1):107-122.e7). Olig2 expression is downregulated in STHdh Q111/111 neurons expressing mHTT by 35% in comparison to wildtype STHdh Q7/7 level **(Figure 25 (C)).** In Compound 22 treated STHdh 111/111 Olig2 expression is doubled in comparison to untreated STHdh 111/111 neurons. Olig2 is downregulated in STHdh 111/111 (Q111-0) neurons in comparison to STHdh 7/7 (Q7-0). Treatment with Compound 22 improves Olig2 expression in STHdh 111/111 cells (Q111-10).

Intranuclear mutated huntingtin decreases the expression of nerve growth factor receptor (NGFR) in HD models (Li, S.H. et al. Mol Cell Biol. 2002 Mar;22(5):1277-87). NGFR expression is downregulated in STHdh Q111/111 neurons expressing mHTT by 51% in comparison to wildtype STHdh Q7/7 level **(Figure 25 (D)).** In Compound 22 treated STHdh

111/111 NGFR expression is increased by 60% in comparison to untreated STHdh 111/111 neurons. NGFR is downregulated in STHdh 111/111 (Q111-0) neurons in comparison to STHdh 7/7 (Q7-0). Treatment with Compound 22 improves NGFR expression in STHdh 111/111 cells (Q111-10).

The KCNA1 gene encodes the alpha subunit of the potassium channel Kv1.1 which is found in brain tissue where it transports potassium ions into neurons. NGFR expression is downregulated in STHdh Q111/111 neurons expressing mHTT by 42% in comparison to wildtype STHdh Q7/7 level **(Figure 25 (E)).** In Compound 22 treated STHdh 111/111 NGFR expression is increased by 68% in comparison to untreated STHdh 111/111 neurons. Kcna is downregulated in STHdh 111/111 (Q111-0) neurons in comparison to STHdh 7/7 (Q7-0). Treatment with Compound 22 improves Kcna expression in STHdh 111/111 cells (Q111-10).

**Example 41- Effects of Compound 4 on Striatal mHTT Levels**

*Effect of Compound* 4 *on Diffuse mHTTlevels* - ST HDH Q111/111 (CH00095, Coriell Institute) striatal derived cell line from a knock in transgenic mouse containing homozygous HTT loci with a humanized Exon 1 with 111 polyglutamine repeats. ST HDH Q7/7 (CH00097, Coriell Institute) striatal derived cell line from a knock in transgenic mouse containing HTT loci with a humanized Exon 1 containing 7 polyglutamine repeats. Diffuse mHTT quantified with immunocytochemistry as described in Example 33. Diffuse mHTT was significantly reduced by 68% ($p < 0.0001$, Welch's t-test) in STHdh 111/111 cells treated with Compound 4 in comparison to untreated STHdh 111/111 cells. But wildtype huntingtin was not reduced in STHdh 7/7 cells treated with Compound 4, in comparison to non-treated STHdh 7/7 **(Figure 26).**

*EC50 value of Compound* 4 *for reducing mutated huntingtin (mHTT)*

Diffuse mHTT quantified with immunocytochemistry as described in Example 33 with the exception that instead of 3B5H10 antibody for staining mHTT, MW1 (mouse anti-polyQ specific mab, Merck, catalog number: MABN2427) was used as primary antibody to stain mutated huntingtin. The EC 50 value for mHTT reduction was 130 nM **(Figure 27)**. EC 50 value was determined with non-linear regression.

*Effect of Compound* 4 *on Cell Viability* - Heat shock triggers accumulation of misfolded proteins, which are degraded. At 48 hours after 3 hours heat shock the cell viability of STHdh 111/111 treated with Compound 4, with 125% in comparison to pre-heat shock cell viability, was higher in comparison to untreated cells, with 25% in comparison to pre-heat shock cell viability **(Figure 28).**

*Physicochemical properties of Compound* 4 - Compound 4 is both soluble and it can cross the blood brain barrier. **Table 19** describes physicochemical properties of Compound 4 **(Figure 44).**

**Table 19:** Brain Exposure and Physicochemical Properties of Compound 4

| | Compound 4 |
|---|---|
| Thermodynamic Solubility | 5.1 mM |
| Kinetic solubility | 328 μM |
| Log D | 2.02 (pH 7,4) |
| Blood Brain Barrier Penetrability | $AUC_{brain}/AUC_{plasma}$ = 2.27 |

**Example 42** - **Phosphorylated tau reduction**

*Effect of Compound* 4 *on Phospho-Tau (Ser396) levels* - ST HDH Q7/7 (CH00097, Coriell Institute) striatal derived cell line from a knock in transgenic mouse containing HTT loci with a humanized Exon 1 containing 7 polyglutamine repeats. Immunocytochemistry was conducted as described in Example 33 with the exception that instead of 3B5H10 antibody for staining mHTT, phospho-Tau (Ser396) polyclonal antibody (ThermoFisher, Catalog 44-752G) was used to stain phospho-Tau (Ser396). Phospho-Tau (Ser396) was significantly reduced by 26.3% ($p = 0.01$, Welch's t-test) in STHdh 7/7 cells treated with 10 μM Compound 4 in comparison to untreated STHdh 7/7 cells. In STHdh 7/7 cells exposed to 10 mM autophagy inhibitor ammonium chloride (Sigma Aldrich) Compound 4 treatment with 5 μM could not reduce phospho-Tau (Ser396) in comparison to non-treated STHdh 7/7. But 10 μM Compound 4 treatment could reduce in STHdh 7/7 exposed to ammonium chloride 15,2% phospho-Tau (Ser396) in comparison to non-treated STHdh 7/7 **(Figure 31).**

*Effect of Compound* 4 *on Phospho-Tau (Ser396) levels* - ST HDH Q7/7 (CH00097, Coriell Institute) striatal derived cell line from a knock in transgenic mouse containing HTT loci with a humanized Exon 1 containing 7 polyglutamine

repeats. Diffuse mHTT quantified with immunocytochemistry as described in Example 33 with the exception that instead of 3B5H10 antibody for staining mHTT, phospho-Tau (Ser404) polyclonal antibody (ThermoFisher, Catalog 44-758G) was used to stain phospho-Tau (Ser396) and no heat shock was used. Phospho-Tau (Ser404) was significantly reduced by 40% ($p = 0.003$, Welch's t-test) in STHdh 7/7 cells treated with 10 μM Compound 4 in comparison to untreated STHdh 7/7 cells. In STHdh 7/7 cells exposed to 10 mM autophagy inhibitor ammonium chloride (Sigma Aldrich) Compound 4 treatment could not reduce phospho-Tau (Ser404) in Compound 4 STHdh 7/7 neurons **(Figure 32).**

**Example 43** - **Striatal cells expressing mutated huntingtin morphology improvements**

STHdh 111/111 are striatal cells expressing mutated huntingtin (Q111). STHdh 111/111 which were not treatment showed impaired morphology and high rate of cell death. STHdh 111/111 treated with Compound 4 depicted improved cell viability, dendrite outgrowth and increased cell size **(Figure 33).**

**Example 44** - **Motility Zebrafish Model**

Exposure to the neurotoxin 1-methyl-4-phenylpyridinium (MPP+) has been shown to induce dopaminergic ablation (1) and locomotor perturbations symptomatic of Parkinson's disease (2) in zebrafish larvae. MPP+ selectively targets dopaminergic neurons; the putative mechanism is induction of K+ efflux, cell membrane hyperpolarization and inhibition of neuronal firing. This ultimately leads to an inhibition of complex I, a decrease in ATP production and promotion of the generation of reactive oxygen species upon accumulation in the mitochondria (3). The observed phenotype following exposure to 500 μM from 24 hours post fertilization (hpf) to 120 hpf is characterized by a decrease in movement during lights-on phases of a photomotor assay. In unpublished data below, representative locomotor perturbations of the distance moved and movement frequency is shown.

To study the potential for neuroprotection, zebrafish embryos were treated with Compound 4 prior to MPP+ exposure at 0 hpf, and as co-incubation with MPP+ at 24 hpf onwards **(Figure 35).**

Six groups were included in each experiment. Immediately after spawning, 40 embryos per group were allocated to a 24-well plate and system water (solvent) was added to all wells. For assessment of neuroprotective potential, Compound 4 was added for a final concentration of 1, 10 and 20 μM (column 4-6). From the following day at 24 hpf, Compound 4 was co-incubated with 500 μM MPP+. MPP+ was prepared immediately prior to incubation and solutions were changed daily. At 120 hpf, drug treatment and MPP+ exposure was ceased and a 3X wash was performed with system water. Larvae were then relocated to 96-well plates for behavioral recording and placed in a separate recording room for 24-hour acclimation. Prior to behavioral recording, wells were fully replenished system water.

Naive group was exposed to solvent only. Vehicle group was exposed to solvent and vehicle corresponding to the vehicle of 20 μM drug solution (0.02% DMSO). MPP+ positive control was exposed to solvent and 500 μM MPP+. All compound groups were treated with the respective vehicle and respective concentration of compound 4.

| Compound | Concentration | Solvent | %DMSO |
|---|---|---|---|
| MPP+ | 500 μM | Water | 0 |
| Compound 4 | 100 mM | DMSO | 100 |
| Compound 4 | 10 mM (diluation of 100 mM) | Distilled Water | 10 |
| Compound 4 | 1 μM (dilution of 10 mM) | Water | 0.001 |
| Compound 4 | 10 μM (dilution of 10 mM) | Water | 0.01 |
| Compound 4 | 20 μM (dilution of 10 mM) | Water | 0.02 |
| Vehicle | 20 μM (DMSO content, no drug*) | Water | 0.02 |
| *0.02% DMSO vehicle was included for control for effect of DMSO to naïve larvae. | | | |

Behavioral data was recorded from 1 p.m. on 6 dpf until shortly after wakening on 7 dpf. The recording consisted of a photomotor assay in which lights switch off and on in 30-minute intervals between 1 p.m. and 6 p.m., producing a consistent photomotor response. Here, the larvae demonstrate a rapid increase in movement in response to lights-off followed by a gradual decrease. Upon lights being turned on again, the larvae cease movement followed by a return to baseline. This cycle is repeated five times. Following the last lights-off phase, constant illumination is presented until lights turn off at 10 p.m. for the night. Lights turn on again at 8 a.m. the following day.

Average movement frequency was measured during 30-minute lights-on phases of photomotor assay. Movement bouts were defined as initiated when velocity exceeded a threshold of 2 mm/s and ceased when velocity fell below 1 mm/s.

Distance moved (mm) was defined as the average distance moved during 30-minute lights-on phases of photomotor assay.

Data was obtained using EthoVision XT (Version 11.5.2016, Noldus) and exported to Microsoft Excel for data analysis. Statistical analysis was performed using GraphPad Prism Software (Version 5.01, GraphPad Software Inc.). All data are presented as mean ± standard error of the mean (sem). For analysis of differences between groups, a Kruskal-Wallis one-way ANOVA with Dunn's multiple comparison post hoc test was performed. $P < 0.05$ was considered statistically significant.

The neuroprotective potential of Compound 4 was assessed following co-incubation with MPP+. A statistically significant difference in distance moved between the different treatment groups, $_X2(5) = 29.20$, $p < 0.0001$, was observed. Larvae exposed to MPP+ alone moved significantly shorter distances than naïve larvae ($p < 0.001$), whereas no significant difference was observed for the drug treatment groups compared to MPP+.

Similarly, a statistically significant difference in movement frequency between the different treatment groups, $_X2(5) = 40.18$, $p < 0.0001$, was observed. Larvae exposed to MPP+ alone moved significantly less frequently with 13.3 movement bouts in 30 minutes than naïve larvae with 40.8 movement bouts in 30 minutes ($p < 0.001$), and larvae exposed to 1 μM Compound 4 moved significantly less frequently with 21.0 movement bouts in 30 minutes than naïve larvae ($p < 0.01$), whereas no significant difference was observed for the drug treatment groups compared to MPP+ **(Figure 36).**

The effect of Compound 4 on sleep parameters was assessed following co-incubation with MPP+. No statistically significant difference between the different groups for either sleep fragmentation, $_X2(5) = 6.573$, $p = 0.2544$; sleep ratio, $_X2(5) = 7.368$, $p = 0.1947$; velocity, $_X2(5) = 3.676$, $p = 0.5970$; wake bout duration, $_X2(5) = 5.015$, $p = 0.4141$; or sleep bout duration, $_X2(5) = 8.719$, $p = 0.1208$, was observed **(Figure 37 (A)-(D)).**

Despite no significant rescuing effects of Compound 4 in either of the performed experiments, the data indicates that concentrations ~10 μM and ~1 μM causes a subtle increase in overall movement during assessment of neuroprotection. This is supported by the plots depicting the movement during the entire recording sequence. In **Figure 39,** a separation between the movement of larvae treated with MPP+ alone and 1 μM Compound 4 with MPP+ during lights-on phases is visible. In **Figure 40,** a separation between the movement of larvae treated with MPP+ alone and 10 μM Compound 4 with MPP+ during lights-on phases is visible. But a separation between the movement of larvae treated with MPP+ alone and 20 μM Compound 4 with MPP+ during lights-on phases is not visible. The sleep phenotype of larvae treated with Compound 4, independent of concentration, appears similar to both naïve and MPP+-treated larvae. This suggests that Compound 4 does neither reduce nor increase sleep and consistencies in both velocity and sleep fragmentation indicates that spontaneous movement during sleep is retained.

## Example 45: Dynamic Light Scattering (DLS) experiments show induced aggregation of protein-oligomers by the compounds of the invention

In order to induce the formation of smaller oligomers of the α-Synuclein protein it was hypothised that using DMSO as a solvent induces oligomer formation Kostka et al., THE JOURNAL OF BIOLOGICAL CHEMISTRY VOL. 283, NO. 16, pp. 10992-11003, April 18, 2008). In order to assess the ability of certain compounds to induce formation of aggregates from protein-oligomers, the following experiments were conducted.

Proteins were ultracentrifuged with 50,000 rpm (rounds per minute) for one hour at 2 °C. The supernatant was used for DLS measurements. DLS measurements were conducted with Malvern Zetasizer Nano (Malvern Panalytical Ltd, United Kingdom).

Wild type (wt) α-Synuclein (catalog S-1001-2, rPeptide, Watkinsville, Georgia) was solved in 20 mM phosphate, pH 7.4 buffer.

Wild type (wt) α-Synuclein Sequence **(SEQ ID NO. 12)**

MDVFMKGLSKAKEGVVAAAEKTKQGVAEAAGKTKEGVLYVGSKTKEGVVHGVATVAEKTKEQVTNVGGAVVTGVTAVAQKTVEGA
GSIAAATGFVKKDQLGKNEEGAPQEGILEDMPVDPDNEAYEMPSEEGYQDYEPEA

**Table 20** Size Distribution by Volume of wt α-Synuclein

| | Size (nm) | % Volume | Standard Deviation |
|---|---|---|---|
| Peak 1 | 6.150 | 99.3 | 2.230 |

(continued)

| | Size (nm) | % Volume | Standard Deviation |
|---|---|---|---|
| Peak 2 | 102.3 | 0.2 | 33.91 |
| Peak 3 | 586.3 | 0.4 | 338.4 |

**Table 21** Size Distribution by Volume of wt α-Synuclein exposed to 70 μM equimolar Compound 4 solved in methanol

| | Size (nm) | % Volume | Standard Deviation |
|---|---|---|---|
| Peak 1 | 6.139 | 99.4 | 2.006 |
| Peak 2 | 80.87 | 0.3 | 30.86 |
| Peak 3 | 338.7 | 0.3 | 159.5 |

The volumes of wt α-Synuclein do not change when Compound 4 solved in methanol is added. Thus, wt α-Synuclein does not oligomerize when Compound 4 is added **(Figure 42 A-C, Table 20, Table 21).**

The volume of wt α-Synuclein changes when Compound 4 solved in DMSO is added. wt α-Synuclein forms aggregates when Compound 4 solved in DMSO is added. The average volume of wt α-Synuclein was 6.493 nm without Compound 4 solved in DMSO **(Figure 43A).** After adding equimolar Compound 4 wt α-Synuclein aggregates. The aggregates are large with an average size of 144.0 nm **(Figure 43B-C).**

**Table 22** Size Distribution by Volume of wt α-Synuclein

| | Size (nm) | % Volume | Standard Deviation |
|---|---|---|---|
| Peak 1 | 6.493 | 99.3 | 1.681 |
| Peak 2 | 33.27 | 0.2 | 9.251 |
| Peak 3 | 94.75 | 0.2 | 35.37 |

**Table 23** Size Distribution by Volume of wt α-Synuclein exposed to equimolar Compound 4 solved in DMSO

| | Size (nm) | % Volume | Standard Deviation |
|---|---|---|---|
| Peak 1 | 144.0 | 96.2 | 95.93 |
| Peak 2 | 4794 | 3.8 | 887.7 |
| Peak 3 | 0 | 0 | 0 |

As mentioned, it was described that 1% DMSO was used to induce α-Synuclein to form small oligomers (Kostka et al., THE JOURNAL OF BIOLOGICAL CHEMISTRY VOL. 283, NO. 16, pp. 10992-11003, April 18, 2008). 1% DMSO (Sigma) was used to trigger small oligomer formation of wt α-Synuclein. 1% DMSO was added to wt α-Synuclein to measure the effect of DMSO alone on wt α-Synuclein. With DLS there is no volume difference detectable between wt α-Synuclein with and without 1% DMSO **(Figure 44).** This indicates that aggregate formation induced by Compound 4 as described in **Paragraph 483** is caused by Compound 4 and not DMSO. 1% DMSO induces the formation of small oligomers, which interact with Compound 4 and form larger aggregates of the oligomers.

A30P mutant α-Synuclein (catalog S-1005-2, rPeptide, Watkinsville, Georgia) was solved in 20 mM phosphate, pH 7.4 buffer.

A30P mutant α-Synuclein Sequence **(SEQ ID NO. 13)**

MDVFMKGLSKAKEGVVAAAEKTKQGVAEAPGKTKEGVLYVGSKTKEGVVHGVATVAEKTKQVTNVGGAVVTGVTAVAQKTVEGAG
SIAAATGFVKKDQLGKNEEGAPQEGILEDMPVDPDNEAYEMPSEEGYQDYEPEA

**Table 24** Size Distribution by Volume of A30P mutant α-Synuclein

| | Size (nm) | % Volume | Standard Deviation |
|---|---|---|---|
| Peak 1 | 38.17 | 24.3 | 10.98 |
| Peak 2 | 95.10 | 19.8 | 27.39 |
| Peak 3 | 377.7 | 39.2 | 202.4 |

**Table 25** Size Distribution by Volume of A30P mutant α-Synuclein exposed to equimolar Compound 4

| | Size (nm) | % Volume | Standard Deviation |
|---|---|---|---|
| Peak 1 | 107.0 | 36.1 | 33.10 |
| Peak 2 | 313.6 | 36.3 | 141.1 |
| Peak 3 | 3063 | 27.6 | 1128 |

**Table 26** Size Distribution by Number of A30P mutant α-Synuclein

| | Size (nm) | % Number | Standard Deviation |
|---|---|---|---|
| Peak 1 | 33.83 | 100.0 | 17.70 |
| Peak 2 | 0 | 0 | 0 |
| Peak 3 | 0 | 0 | 0 |

**Table 27** Size Distribution by Number of A30P mutant α-Synuclein exposed to equimolar Compound 4

| | Size (nm) | % Number | Standard Deviation |
|---|---|---|---|
| Peak 1 | 86,77 | 100,0 | 40,69 |
| Peak 2 | 0 | 0 | 0 |
| Peak 3 | 0 | 0 | 0 |

The sizes of the oligomers of A30P shifts after Compound 4 is added. As seen in DLS measurements by size distribution by volume **(Figure 45A-C, Table 24, Table** 25) and size distribution by number **(Figure 46A-C, Table 26, Table 27**). Compound 4 induces the formation of aggregates of oligomers of A30P.

Futher experiments were made with Htt Exon-1 Q16 and Q46.

**Table 28** Size Distribution (Volume and Number) of Q16 under various conditions

| Size Distribution by Volume of Q16 | | | |
|---|---|---|---|
| | Size (nm) | % Volume | Standard Deviation |
| Peak 1 | 6.360 | 100 | 2.386 |
| Peak 2 | 0 | 0 | 0 |
| Peak 3 | 0 | 0 | 0 |
| Size Distribution by Volume of Q16 exposed to equimolar Compound 4 | | | |
| Peak 1 | 93.12 | 0.2 | 47.82 |
| Peak 2 | 6.949 | 99.8 | 1.851 |
| Peak 3 | 0 | 0 | 0 |

(continued)

| Size Distribution by Number of Q16 | | | |
|---|---|---|---|
| | Size (nm) | % Number | Standard Deviation |
| Peak 1 | 4.972 | 100 | 1.394 |
| Peak 2 | 0 | 0 | 0 |
| Peak 3 | 0 | 0 | 0 |
| Size Distribution by Number of Q16 exposed to equimolar Compound 4 | | | |
| Peak 1 | 6.063 | 100 | 1.370 |
| Peak 2 | 0 | 0 | 0 |
| Peak 3 | 0 | 0 | 0 |

Q16 without exposure to Compound 4 is monomeric **(Figure 47A, Table 28)**. Q16 exposed to Compound 4 is mainly monomeric. Only 0.2% of the total volume of Q16 exposed to Compound 4 is assembles to aggregates as measured by DLS **(Figure 47B-C, Table 28)**. The size increases for Q16 without Compound 4 exposure from 5.0 nm to 6.0 nm average size for Q16 which is exposed to Compound 4 **(Figure 48A-C, Table 28)**.

**Table 29** Size Distribution by Intensity of Q46

| | Size (nm) | % Intensity | Standard Deviation |
|---|---|---|---|
| Peak 1 | 10.03 | 81.2 | 3.669 |
| Peak 2 | 235.4 | 18.8 | 107.2 |
| Peak 3 | 0 | 0 | 0 |

**Table 30** Size Distribution by Intensity of Q46 exposed to equimolar Compound 4

| | Size (nm) | % Intensity | Standard Deviation |
|---|---|---|---|
| Peak 1 | 178.7 | 83.1 | 114.6 |
| Peak 2 | 9.275 | 12.9 | 2.490 |
| Peak 3 | 3917 | 4,1 | 1117 |

Q46 without exposure to Compound 4 is mainly monomeric **(Figure 49A, Table 29)**. Q46 with exposure to Compound 4 then assembles as mostly aggregates and larger aggregates and avoids oligomeric forms of Q46 **(Figure 49B, Table 30)**

Amyloid-β 1-40 (Ab40) (catalog A-1153-2, rPeptide, Watkinsville, Georgia) Sequence

DAEFRHDSGYEVHHQKLVFFAEDVGSNKGAIIGLMVGGVV **(SEQ ID NO.14)**

The aliquots of lyophilized Amyloid-β 1-40 (Ab40) peptide were hydrated at room temperature in 50 mM phosphate buffer pH 7.4 and 100 mM NaCl (Buffer 1)

The aliquots of lyophilized Ab peptide were hydrated at room temperature in 75 mM MOPS pH 7.4 and 2 mM CaCl2 (Buffer 2)

The mean size of 100% of Ab40 in Buffer1 was 2.163 nm in DLS measurements as reported by number **(Figure 50A).** The mean size of 99.8% of Ab40 in Buffer 1 was 2.763 nm in DLS measurements as reported by volume **(Figure 50C, Table 31 top.** After adding of Compound 4 Ab40 (in Buffer 1) formed large aggregates with the mean size of 141.5 nm distribution by number considering 100% of Ab40 **(Figure 50B)** and to mean size of 208.5 nm distribution by volume considering 100% of Ab40 **(Figure 50D).**

**Table 31** Size distribution by volume of Ab40 in various conditions

| Size distribution by volume of Ab40 in Buffer1 | | | |
|---|---|---|---|
| | Size (nm) | % Volume | Standard Deviation |
| Peak 1 | 2.763 | 99.8 | 1.139 |
| Peak 2 | 60.82 | 0.2 | 67.80 |
| Size Distribution by Intensity of Ab40 in Buffer2 | | | |
| | Size (nm) | % Intensity | Standard Deviation |
| Peak 1 | 140.2 | 73.8 | 104.9 |
| Peak 2 | 0.7981 | 10.3 | 0.1798 |
| Peak 3 | 4.888 | 10.2 | 1.850 |
| Size Distribution by Intensity of Ab40 in Buffer2 after adding Compound 4 | | | |
| | Size (nm) | % Intensity | Standard Deviation |
| Peak 1 | 304.8 | 55.5 | 152.2 |
| Peak 2 | 77.06 | 33.5 | 27.38 |
| Peak 3 | 0.7710 | 6.1 | 0.1345 |

Buffer 2 contains $Ca^{2+}$. The presence of $Ca^{2+}$ accelerates the formation of Ab40 oligomers (Itkin et al., 2011 PLoS ONE 6(3): e18250). There are three peaks of sizes of Ab40 in Buffer 2. Peak 1 represents large aggregates of Ab40 with an average diameter of 140.2 nm. Peak 2 represents monomeric Ab40 with a mean diameter of 0.8 nm (Zhang-Haagen et al., 2016 PLoS ONE 11(2): e0150267). Peak 3 represents small oligomers of Ab40 with a mean diameter of 4.9 nm **(Table 31 middle; Figure 51A).** After adding Compound 4 the peak for small oligomers disappeared and was replaced by two distinct peaks representing various large aggregates: peak 1 with a mean aggregate size of 304.8 nm and peak 2 with a mean aggregate size of 77.06 nm **(Table 31 bottom; Figure 51B).** This is an indication that Compound 4 does not bind to monomeric Ab40 (peak 2 of Ab40 in Buffer 2 without Compound 4) but rather to small oligomers (peak 3 of Ab40 in Buffer 2 without Compound 4), and such binding of Coumpound 4 enhances formation of larger aggregates rather than smaller oligomeric structures.

**Example 46 - Toxicity of Compound 4 on *C. elegans* worms**

*Background:* The ideal dose of a lifespan-active drug will balance providing a high enough dose to be effective with avoiding doses high enough to be either toxic or aversive to the animals. Because worms are physically resistant to environmental chemicals, and worm physiology differs from humans, cultured cells and other animal models, a series of experiments was conducted to empirically determine an ideal dosage and delivery strategy for a treatment.

Solubility and test article delivery results: The body of C. *elegans* is encased in a selectively permeable cuticle that only permits some compounds to be absorbed efficiently through the skin, so the most reliable mechanism for delivering compounds to the worms is through ingestion. Water-soluble compounds permeate the media and food and are readily taken up by the worms. Less soluble compounds require a vehicle such as DMSO and work best when combined directly with food. The first step is to check the solubility of the test article and determine the best delivery method.

*Solubilization:* The data provided with the initial batch of Compound 4 indicated a limited solubility in water. The compound proved to only have limited solubility in DMSO. A second batch was delivered with an HCl modification. This version was extremely hygroscopic, but readily dissolved in water at 200 mM forming a dark brown, but clear solution.

*Delivery strategy:* The indicated compound dosage is based on the total volume of the plates with the assumption that the water-soluble compound diffuses throughout the agar. The compound is dissolved in a working solution and then combined directly with the food bacteria before seeding on agar plates. The food spots are dried slowly, allowing the compound to diffuse into the food bacteria and the agar for 24 hours.

*Growth and development assay results:* High-resolution imaging and automated detection were used to precisely measure the growth rate of animals from hatching to the first day of adulthood (total of 4 days). The C. *elegans* growth and development assay is highly sensitive and widely used in toxicology studies. Performing this test over a range of doses helps to identify a set of doses that have a physiological impact and exclude dose ranges that are likely too toxic to benefit lifespan.

*Results:* Compound Compound 4 had been reported to exert effects with an EC50 of 160 nM in cultured cells. The following range of doses was tested: 0, 32nM, 160nM, 800nM, 4(μM, 20μM, 100μM, 500μM (5-fold dilution series). 4 μM was the highest concentration at which no adverse effects were detected **(Figure 52 (A), Figure 53).** Hence, compound 4 has a beneficial toxcicity profile in C. *elegans*.

**Example 47** - **Lifespan experiments on C.** *elegans* **exposed to Compound 4**

*Background:* First, acute toxicity assays were performed to test the toxicity of the compounds in adult worms by simulating the actual conditions of the lifespan assay. This accomplished two things. First, in the early stages it determines doses that are acutely toxic to adult worms. Second, over time the worms are scored to rule out doses that despite low initial toxicity, will likely have a negative impact on lifespan. Worms are plated on the exact media and sealed plates that will be used in the lifespan assay and then incubated at 25°C to provide a pilot lifespan that helps catch any other dosing and delivery pitfalls early. Adult worms were monitored in the days immediately following exposure for lethality or any other obvious defects.

*Results:* Worms were treated with either vehicle control or Compound 4 at concentrations of 50nM, 500nM, 5μM, 50μM, and 500μM. The conditions were identical to those used in the lifespan plates to ensure that the chemical exposure/delivery and environmental conditions are comparable to what the worms will experience in the actual assay. The exception is that the worms are incubated at 25°C instead of 20°C to accelerate the aging process. Initially, the worms appeared healthy at all concentrations so no immediate toxicity was observed. Worms were then scored periodically for early deaths. Worms treated with 5 μM Compound 4 or higher concentrations began to decline faster than vehicle control-treated worms. As these doses would be unlikely to increase lifespan in an actual lifespan assay, this placed the upper boundary of effective concentrations between 500 nM and 5 μM **(Figure 52 (B)).** Given that the growth and acute toxicity assays provided an upper boundary of 4 μM and 5 μM respectively, a concentration of 2μM was selected as the maximum dose for the lifespan. For comparison with previous studies a condition with a dose of 200 nM would also be tested in the lifespan assay.

*Experimental Design:* The lifespan assay was initiated by expanding all replicate groups to more than 1000 worms, then synchronizing by bleaching and allowing larval worms to hatch and arrest. To eliminate the effect of bacterial metabolism and growth on lifespan, synchronized worms were only exposed to dead food bacteria. To suppress progeny, worms are transferred to media containing 5-Fluorodeoxyuridine (FUdR) within 54-60 hours post plating. Worms are inspected at 24 and48 hours after this transfer to confirm infertility. Finally, the worms are inspected for general health and morphology before transferring to scanner plates. The scanner plates are incubated for an additional 2 days and inspected again before loading on to scanners. To understand the physiological impact of either genetic variation or pharmacological treatment on aging, it is crucial to use quantitative models. The analysis of lifespan data is grounded in the study of two mathematical functions: the survival curve and the hazard function. The survival curve **(Figure 54)** describes the fraction of a tested population that remains alive over time. The hazard function **(Figure 55)** is related to the survival curve and provides an intuitive measure of the risk of death. This function describes the probability that atypical individual who is currently alive will soon die, providing a clear visualization of the way a treatment may change patterns in mortality.

To obtain high-resolution lifespan data and eliminate confounding factors such as worm handling and operator bias, lifespan data was collected using an Automated Lifespan Machine (ALM). Three biological replicates, derived from synchronizing three independently-maintained lines of N2 worms, were distributed across scanner instruments. Images of the worms were then collected for the next 35 days with no interruption or manipulations. Some plates were excluded after quality checks, but plate number and worm counts for all replicates exceeded the threshold for statistical significance **(Table 32,** row 1).Lifespan data are represented as the percent of worms surviving over time and the profile of this curve can be modeled by the Kaplan-Meier Estimate of Survival function **(Figure 54).** The survival curves can be compared by several statistics including mean, median, and maximum lifespan **(Table 32),** as well as the age of worms at a specific percent survival **(Table 34).** However, to determine whether one treatment group lived longer than another, survival curves are holistically compared using a log-rank test **(Table 33).** In this study, all pairwise comparisons between conditions were statistically significant. **Tables 32 to 34** detail the raw measurements, calculations, and statistical analysis related to the survival curve in **Figure 54.**

*Lifespan Results:* General statement regarding survival curves. Overall, a minimum of 314 and a maximum of 329 worm lifespans per condition were recorded **(Table 32)** surpassing the requirement 150 required to eliminate subsampling errors. The consistent numbers across groups also adds confidence to comparing survival curves. Out of 96 culture plates, all plates passed quality tests for worm detection. However, a Cox Proportional Hazards analysis to determine if factors other than the treatment could confound the data and found that one of the five scanners had a disproportionate effect on survival, was run. Although it is possible that this could be driven by the specific assortment of conditions represented on that scanner, those plates were excluded from the analysis. This reduced the total worm counts, but had a minor impact on survival curves and did not affect the curve comparisons. Qualitatively, the worms

appeared content and adhered to standard death protocols, suggesting that they were not impacted by confounding hazards such as contamination or toxicity. Worms were treated with two different doses of Compound 4, 200nM and 2 μM, that will be referred to as the "low dose" and the "high dose." Treatment of worms with Compound 4 produced a dose-dependent increase in lifespan, with the high dose having greater effect **(Figure 54 A)**.Although Compound 4 increased the median lifespan from 24.3 to 26.2 (low dose) and 27.6 (high dose) days, it produced a slightly lower maximum lifespan **(Table 32).** Under ideal conditions, factors that act upon the rate of aging typically produce a survival curve that remains parallel to the control but right- or left- shifted. This is known as "temporal scaling" of lifespan. The 200 nM dose largely follows this paradigm running mostly parallel but right-shifted relative to the control group. The survival of the high dose APG325 group remains high until after 25 days and then drops steeply. These inflections in the survival curve relative to control indicate a change in the factors affecting mortality over time.

The hazard rate function **(Figure 54 B)** is the instantaneous risk of death at a given time and can help indicate whether different survival curves are determined by similar or different risk factors. In this case, the roughly parallel hazard rate functions of the low dose and control indicate that both conditions are responding to similar aging hazards. However, the inflection in the hazard rate curve for the high dose of Compound 4 indicates that the elevated dose is introducing a new risk factor late in life. This suggests that the increased dosage introduces additional factors that could confound lifespan analysis. Survival curves are commonly compared statistically using a log-rank test, which tests the hypothesis that the two curves are sampled from the same population. Using a standard Mantel-Cox log-rank test the low dose and high dose of Compound 4 differed from the control with P values of 0.09 and 0.01, respectively **(Table 33).** The marginal significance was surprising given the obvious visual differences in the survival curves. This test, however, compares the two curves evenly over the course of the lifespan and the re-convergence of the curves could be neutralizing some differences in the test. A Wilcoxon-Breslow-Gehan test weights each death by the number of worms at risk, reducing the contribution of the later deaths after the curves have reconverged. This test produces P values of .008 for the low dose and $5.0 \times 10^{-8}$ for the high dose.

Overall, the low dose (200 nM) of Compound 4 produced an increase in the median lifespan that would by itself be a compelling result comparable to other reported lifespan extending compounds. The higher dose produced an extraordinary increase in median lifespan. Taken with the different shape of the survival curve, this prompts the question of whether it introduced another factor, such as starvation, that contributed to the effect. Neither dose increased maximum lifespan. Many compounds that do extend maximum lifespan in C. *elegans* do so only by prolonging the period of late frailty that precedes death. Compound 4 by contrast, is notable in the increase in early survivorship.

**Table 32:** Lifespan assay summary

| | **Vehicle control** | **Compound 4 200nM** | **APG 345 2μM** |
|---|---|---|---|
| Number of worms | 327 | 314 | 329 |
| Median lifespan (days) | 24.3 | 26.2 | 27.6 |
| Median 95% C.I. | 23.6-25.1 | 25.3-26.9 | 27.1-27.8 |
| Mean lifespan | 24.7 | 25.7 | 26.6 |
| Mean 95% C.I. | 24.1 - 25.2 | 25.1 - 26.2 | 26.1 -27.0 |
| Maximum lifespan (days | 34.9 | 33.7 | 32.9 |

Lifespan is counted with day 0 set at day 1 of adulthood. No death times are recorded until worms are placed on the scanner on day 8 so earlier deaths are excluded from calculations of mean and median. Median lifespan is equal to the time at which 50% of the subjects have died. Mean lifespan is calculated from the area under the survival curve. Maximum lifespan is equal to 95th percentile of lifespans in each group. C.I.: Confidence Interval. See **Figure 55** and **Table 33** for statistical analysis

**Table 33:** Pairwise statistical analysis of survival curves.

| | Mantel-Cox Log Rank | | Wilcoxon-Breslow-Gehan | |
|---|---|---|---|---|
| Curve comparison | Text statistic ($X^2$) | Log-rank test P-value | Text statistic ($X^2$) | Log-rank test P-value |
| Compound 4 200 nM vs. control | 2.82 | 0.09 | 8.32 | 0.0078 * |
| Compound 4 2 μM vs. control | 6.3 | 0.01* | 31.08 | 5.0E-8 **** |

(continued)

| | Mantel-Cox Log Rank | | Wilcoxon-Breslow-Gehan | |
|---|---|---|---|---|
| Compound 4 2 μM vs. Compound 4 200 nM | 0.26 | 0.61 | 5.59 | 0.0361 * |

The Mantel-Cox log-rank test is a non-parametric test that compares two survival functions across the duration of the lifespan. The Wilcoxon-Breslow-Gehan test weights each death time by the total number of subjects at risk, thus assigning more weight to earlier death times. P value is corrected for multiple comparisons (Bonferroni correction). Numbers and asterisks represent P-value and significance, respectively.

**Table 34:** Age in days at percent mortality

| Treatment | 25% mortality | 50% | 75% | 90% | 100% |
|---|---|---|---|---|---|
| Vehicle control | 20.31 | 24.3 | 28.46 | 31.69 | 39.46 |
| Compound 4 200 nM | 21.42 | 26.16 | 29.57 | 31.75 | 39.46 |
| Compound 4 2 μM | 23.9 | 27.54 | 29.41 | 31.23 | 39.42 |

Age at percent mortality is the age in days at which the given percentage of worms are dead. Age at 50% mortality is equal to the median. These analyses are useful for examining early or late life-specific effects or when the survival curves are not parallel. Fisher's Exact Tests for differences at 25, 50, 75, and 90%mortality are shown **in Table 35.**

**Table 35:** Fishers's Exact Test for survival differences at key time points.

| treatment | P-value at 25% mortality | P-value at 50% mortality | P-value at 75% mortality | P-value at 90% mortality |
|---|---|---|---|---|
| Compound 4 200 nM vs. control | 0.0556 | 0.0027 | 0.0226 | 0.8957 |
| Compound 4 2 μM vs. control | 8.90E-08 | 2.50E-11 | 0.0709 | 0.2419 |
| Compound 4 2 μM vs. Compound 4 200 nM | 0.0019 | 0.0012 | 0.4117 | 0.0868 |

Age at percent mortality is the age in days at which the given percentage of worms are dead. Age at 50% mortality is equal to the median. These analyses are useful for examining early or late life-specific effects or when the survival curves are not parallel. Fisher's exact tests at these time points are included with the data supplement.

**Example 48 - Healthspan and movement analysis of C. *elegans* exposed to Compound 4**

*Background:* To measure healthspan, active worms were identified using the ALM scanner images from the lifespan assay. The worms' spatial location on the plate was quantified throughout their lifespan to assess healthspan. Worm Activity serves as a proxy for animal health. Changes in spatial distribution of the worms between time points is used to derive aggregate movement for the population over time. In this context, "aggregate movement" and "aggregate motility" do not refer to the movement or motility of aggregates, but to worm activity.

Worm Activity is described by two complementary measurements of aggregate movement: 1) Centroid Distance calculates a geometric center for each individual worm, and then measures the minimum collective distance that a group of worms moved between time points. It achieves measurement of the changes in spatial distribution betweentime-points. 2). Spatial Distribution uses changes in worm contours between time points to measure the changes in the spatial distribution of the worms' bodies. The normalized distribution of these distances in the population provides a measure of how much the group of worms altered their positions and posture on the plate between two time-points. For both of these activity metrics, a plot with higher numbers indicates good health, and all measures are normalized to fall between 0 and 1. All measurements are based on worms that are still alive and moving at the time of quantification. All measures start when worms were placed on the scanner at day 5 of age (120 hours of age, day 3 of adulthood).

*Healthspan Results:* Aggregate Movement. Both the Centroid Distance and the Spatial Distribution are concordant with the results and relative ranking of strains in the lifespan analysis. The low dose of Compound 4 showed a consistently higher aggregate movement than control over the entire course of the lifespan. **(Figure 55 and Figure 56).** The high-dose Compound 4 group starts off more slowly but then shows even more elevated movement for the rest of the lifespan. The high dose activity remained higher late in life than control despite having a slightly shorter maximum lifespan. This indicates that the Compound 4 treated worms remained mobile late into their lifespan whereas the controls experienced a longer period of immobility up until the time of death.

**Example 49: Gene expression analysis of C. *elegans* that was exposed to Compound 4**

*Differential gene expression:* To identify potential mechanisms of action through which Compound 4 could affect aging, global gene expression was analyzed by mRNA sequencing (RNA-Seq). Both young (adult day 3) and aged (adult day 10) worms were collected from the same population of worms tested in lifespan assay.

*Technical Background and Data Visualization Guide:* Differential gene expression was performed with EdgeR using false likelihood ratio tests based on fitting linear models. The likelihood ratios were used to determine the p-values which were subsequently corrected for using the BH false discovery rate (fdr) method. Eventually, differential expressed genes (DEG) were defined as genes with fdr-corrected p-value of 0.05 or lower, as well as a change in expression of at least 2-fold in a given between-group comparison. In this study, the following comparisons groups were used:

**Table 36:** Comparison groups for differential gene expression

| Group | Experiment | Control |
|---|---|---|
| 1 | Compound 4 200 nM day 3 | Vehicle day 3 |
| 2 | Compound 4 2 μM day 3 | Vehicle day 3 |
| 3 | Compound 4 200 nM day 10 | Vehicle day 10 |
| 4 | Compound 4 2 μM day 10 | Vehicle day 10 |

*Reading Gene Ontology Enrichment (Pathway) Analysis Tables:* Functional characterisation of gene lists using Gene Ontology (GO) enrichment analysis is a common approach in transcriptomic analysis. Once the table of differentially-expressed genes has been created, the annotation of those genes by biological process (BP), molecular function (MF), or cellular compartment (CC), is catalogued and a comparison is made between the likelihood of seeing genes in that category (ontology) being enriched in the list of differentially-expressed genes when compared to a random selection of genes. This allows patterns due to the interactions of multiple genes to emerge.

To identify genes that are differentially expressed upon treatment with Compound 4, the gene counts for each of the Compound 4-treated samples were compared against control **(Table 36)**. The list of significant differentially expressed genes (DEG) was relatively short and consistent between the all four experimental comparisons **(Table 37).** In general, the higher dose of Compound 4 induced a larger number of DEGs and the change tended to be of greater magnitude. A core of seven genes (cyp-35B3, cyp-35B2, cyp-35A1, pgp-13, irg-5, cyp-35B1, irg-4) was upregulated for both doses and both timepoints. In *C. elegans,* the cyp genes encode members of the Cytochrome P450 family, which are enzymes that break down drugs and other xenobiotic substances. Genes irg-4 and irg-5 are among several up and down-regulated genes involved with innate immune response in *C. elegans.* Worms treated with the higher dose upregulated additional genes involved with metabolizing xenobiotic substances, including gst-5 (Glutathione S-transferase) and cyp-35C1, consistent with a more intense response to a foreign substance.

**Table 37:** Compound 4 vs. Vehicle Control: Top differentially upregulated genes above threshold

| | **Day 3** | | **Day 10** | | | |
|---|---|---|---|---|---|---|
| **gene** | **200 nM** | **2 μM** | **200 nM** | **2 μM** | **Description/ protein Product/function** | **Human ortholog** |
| cyp-35B3 | 12.96 | 15.81 | 11.65 | 13.40 | Cytochrome P450 | CYP21A2, CYP2 |
| cyp-35B2 | 9.18 | 11.26 | 5.71 | 6.61 | Cytochrome P450 | CYP21A2, CYP2 |
| cyp-35A1 | 3.04 | 8.58 | 4.25 | 6.81 | Cytochrome P450 | CYP21A2, CYP2 |

(continued)

| | Day 3 | | Day 10 | | | |
|---|---|---|---|---|---|---|
| **gene** | **200 nM** | **2 μM** | **200 nM** | **2 μM** | **Description/ protein Product/function** | **Human ortholog** |
| pgp-13 | 3.55 | 5.44 | 4.36 | 6.36 | ATP binding activity and ATPase activity, coupled to transmembrane movement of substances | ABCB9, XXbac-BPG246D1 5.9 |
| irg-5 | 2.09 | 5.88 | 2.12 | 4.26 | Defense response to Gram-positive bacterium | not found |
| cyp-35B1 | 3.41 | 5.05 | 1.62 | 2.06 | Cytochrome P450 | CYP21A2, CYP2 |
| irg-4 | 1.37 | 3.88 | 1.22 | 2.95 | Defense response to Gram-negative bacterium | not found |
| F55G11.2 | | 2.80 | | 1.70 | Innate immune response | not found |
| gst-5 | | 2.71 | | 2.42 | Glutathione S-transferase | GSTA4 |
| cyp-35C1 | | 2.17 | | 1.60 | Cytochrome P450 | CYP21A2, CYP2 |
| cpr-4 | | 1.35 | | 1.80 | Cathepsin B, cysteine-type endopeptidase activity | CTSB |
| T19B10.2 | | 1.79 | | | Expressed in the hypodermis. | not found |
| far-3 | | 1.66 | | | Predicted lipid binding activity. Exp. in hypodermis, intestine, males, and vulva. | not found |
| F15B9.6 | | 1.57 | | | unknown | not found |
| ugt-44 | | 1.45 | | | UDP glycosyltransferase family 3 | UGT3A1, UGT3A2 |
| K08D8.6 | | | | 1.45 | Localizes to the membrane raft. | not found |
| cyp-33A1 | | 1.27 | | | Cytochrome P450 family 2 | CYP2J2, CYP2R1, CYP2U1 |
| C29F3.7 | | 1.25 | | | Innate immune response, membrane raft | not found |
| D1086.3 | | 1.19 | | | unknown | not found |
| C18H9.6 | | 1.16 | | | unknown | not found |

Top DEG upregulated by Compound 4 treatment. Log2(fold change) is shown in columns under day and Compound 4 dose. All DEG shown have P values under the cutoff of 0.05; in this case, most P values are <$10^{-8}$ and all P values <$10^{-4}$. Gene function annotations collected from WormBase and human orthologs are predicted by DRSC Integrative Ortholog Prediction Tool (DIOPT, www.flyrna.org). DEG downregulated by Compound 4 treatment. Log2(fold change) is shown in columns under day and Compound 4 dose. All DEG shown have P values under the cutoff of 0.05; in this case, most P values are <$10^{-8}$ and all P values <$10^{-3}$. Gene function annotations collected from WormBase and human orthologs are predicted by DRSC Integrative Ortholog Prediction Tool (DIOPT, www.flyrna.org).

*Gene Ontology (GO) enrichment:* In all of the Compound 4 treated groups the DEG overrepresentation of genes linked to exogenous drug catabolic process, oxidoreductase activity, steroid hydroxylase activity, cellular response toxenobiotic stimulus, response toxenobiotic stimulus, xenobiotic metabolic process, monooxygenase activity **(Table 38)**. It is likely that the same cohort of 4-6 genes are linked to each of these processes. Top underrepresented GO terms were mostly linked to innate immune response. Although these GO terms were significantly underrepresented, they represent only 3 genes out of more than 80 in all of these GO terms.

**Table 38:** Compound 4 vs. Vehicle Control: Top overexpressed GO terms common to all Compound 4 treatments

| GO ID | Term | Ont. | N | Up | Down | P value |
|---|---|---|---|---|---|---|
| GO:0042738 | exogenous drug catabolic process | BP | 6 | >4 | 0 | <0.0001 |
| GO:0016712 | oxidoreductase activity | MF | 6 | >4 | 0 | <0.0001 |
| GO:0008395 | steroid hydroxylase activity | MF | 6 | >4 | 0 | <0.0001 |
| GO:0071466 | cellular response to xenobiotic stimulus | BP | 7 | >4 | 0 | <0.0001 |
| GO:0009410 | response to xenobiotic stimulus | BP | 7 | >4 | 0 | <0.0001 |
| GO:0006805 | xenobiotic metabolic process | BP | 7 | >4 | 0 | <0.0001 |
| GO:0004497 | monooxygenase activity | MF | 9 | >4 | 0 | <0.0001 |

**Table 39:** Top under-represented GO terms common to all Compound 4 treatments

| GO ID | Term | Ont | N | Up | Down | P value |
|---|---|---|---|---|---|---|
| GO:0006952 | defense response | BP | 86 | 3 | 2-15 | <0.0001 |
| GO:0098542 | defense response to other organism | BP | 86 | 3 | 2-15 | <0.0001 |
| GO:0044419 | interspecies interaction between organisms | BP | 87 | 3 | 2-15 | <0.0001 |
| GO: 0009607 | response to biotic stimulus | BP | 87 | 3 | 2-15 | <0.0001 |
| GO:0043207 | response to external biotic stimulus | BP | 87 | 3 | 2-15 | <0.0001 |
| GO:0051707 | response to other organism | BP | 87 | 3 | 2-15 | <0.0001 |
| GO terms over-represented in all Compound 4 treatments. GO ID: Unique GO ID# cataloged at geneontology.org. Full GO term analysis can be found in the data supplement.<br>Ont: Ontology class biological process (BP), molecular function (MF), cellular compartment (CC)<br>N: Total number of Genes classified in that GO term.<br>Up/Down: The number of genes in that GO term (out of N) that are up or down-regulated.<br>P-value: Significance of gene enrichment (up) or depletion (down) in the set of differentially expressed genes vs. the null set | | | | | | |

REFERENCES

The references are:


Abel O, Powell JF, Andersen PM and Al-Chalabi A (2012) ALSod: a user- friendly online bioinformatics tool for amyotrophic lateral sclerosis genetics. Human Mutation 33, 1345-1351.

Acharya, M. M., Patel, N. H., Craver, B. M.,Tran, K. K., Giedzinski, E., Tseng, B. P., et al. (2015). Consequences of low dose ionizing radiation exposure on the hippocampal microenvironment. PLoS One 10:e0128316. doi: 10.1371/journal. pone.0128316

Acharya MM, Baulch JE, Klein PM, Baddour AAD, Apodaca LA, Kramar EA, Alikhani L, Garcia C Jr, Angulo MC, Batra RS, Fallgren CM, BorakTB, Stark CEL, Wood MA, Britten RA, Soltesz I, Limoli CL. New Concerns for Neurocognitive Function during Deep Space Exposures to Chronic, Low Dose- Rate, Neutron Radiation. eNeuro. 2019 Aug 22;6(4): ENEURO.0094-19.2019.

Bartels, T., Choi, J. G. & Selkoe, D. J. Alpha-synuclein occurs physiologically as a helically folded tetramer that resists aggregation. Nature 477, 107-110 (2011).

Bruijn LI, Houseweart MK, Kato S, Anderson KL, Anderson SD, Ohama E, et al. Aggregation and motor neuron toxicity of an ALS-linked SOD1 mutant independent from wild-type SOD1. Science. 1998;281:1851-4.

Burre, J., Sharma, M. & Sudhof, T. C. Alpha-synuclein assembles into higher-order multimers upon membrane binding to promote SNARE complex formation. Proc. Natl Acad. Sci. USA 111, E4274-4283 (2014).

Carnio S, LoVerso F, Baraibar MA, Longa E, Khan MM, Maffei M, Reischl M, Canepari M, Loefler S, Kern H, Blaauw B, Friguet B, Bottinelli R, Rudolf R, Sandri M. Autophagy impairment in muscle induces neuromuscular junction degeneration and precocious aging. Cell Rep. 2014 Sep 11;8(5):1509-21.

Chan, H.Y.E., Warrick, J.M., Gray-Board, G.L., Paulson, H.L., and Bonini, N.M. (2000). Mechanisms of chaperone suppression of poly- glutamine disease: selectivity, synergy and modulation of protein solubility in Drosophila. Hum. Mol. Genet. 9, 2811-2820.

Chhatwal JP, Schultz AP, Dang Y, Ostaszewski B, Liu L, Yang HS, Johnson KA, Sperling RA, Selkoe DJ. Plasma N-terminal tau fragment levels predict future cognitive decline and neurodegeneration in healthy elderly individuals. Nat Commun. 2020 Nov 27;11(1):6024.

Chauhan A, Sharma U, Jagannathan NR, Reeta KH, Gupta YK. Rapamycin protects against middle cerebral artery occlusion induced focal cerebral ischemia in rats. Behav Brain Res. 2011 Dec 1;225(2):603-9.

Chen, Z. et al. Learnings about the complexity of extracellular tau aid development of a blood-based screen for Alzheimer's disease. Alzheimers Dement. 15, 487-496 (2019).

Chien HM, He RY, Lee CC, Huang YA, et al. Nanoscopic investigation of C9orf72 poly-GA oligomers on nuclear membrane disruption by a photoinducible platform. COMMUNICATIONS CHEMISTRY 2021. 4:111

Cuervo, A. M. & Dice, J. F. Age-related decline in chaperone-mediated autophagy. J. Biol. Chem. 275, 31505-31513 (2000).

Davies SW, Turmaine M, Cozens BA, DiFiglia M, Sharp AH, Ross CA, Scherzinger E, Wanker EE, Mangiarini L, Bates GP. Formation of Neuronal Intranuclear Inclusions Underlies the Neurological Dysfunction in Mice Transgenic for the HD Mutation Cell. 1997 Aug 8;90(3):537-48. doi: 10.1016/s0092-8674(00)80513-9

Deng H, Gao K, Jankovic J. The role of FUS gene variants in neurodegenerative diseases. Nat Rev Neurol. 2014;10:337-48.

Dugger BN, Dickson DW. Pathology of Neurodegenerative Diseases, Cold Spring Harb Perspect Biol. 2017; 9(7): a028035

Esparza TJ, Zhao H, Cirrito JR, Cairns NJ, Bateman RJ, Holtzman DM, Brody DL (2013) Amyloid-beta oligomerization in Alzheimer dementia versus high- pathology controls. Ann Neurol 73: 104 - 119

Fusco, G. et al. Structural basis of membrane disruption and cellular toxicity byalpha-synuclein oligomers. Science 358, 1440-1443 (2017).

Garai K, Sureka R, Maiti S. Detecting amyloid-beta aggregation with fiber-based fluorescence correlation spectroscopy. Biophys J. 2007; 92: L55-7.

Gassen NC, Papies J, Bajaj T, et al. SARS-CoV-2-mediated dysregulation of metabolism and autophagy uncovers host-targeting antivirals. Nature Communications volume 12, Article number: 3818 (2021)

Gitler AD, Dhillon P, Shorter J. Neurodegenerative disease: models, mechanisms, and a new hope. Dis Model Mech. 2017; 10(5): 499-502

Guo L, Salt TE, Luong V, Wood N, Cheung W, Maass A, Ferrari G, Russo-Marie F, Sillito AM, Cheetham ME, Moss SE, Fitzke FW, Cordeiro MF. Targeting amyloid-beta in glaucoma treatment. Proc Natl Acad Sci USA. 2007 Aug 14;104(33):13444-9.

Haataja, L.; Gurlo, T.; Huang, C.J.; Butler, P.C. Islet amyloid in type 2 diabetes, and the toxic oligomer hypothesis. Endocr. Rev. 2008, 29, 303-316.

Hartl, F. U. (1996) Nature (London) 381, 571-579.

Hei, C., Liu, P., Yang, X., Niu, J., and Li, P. A. (2017). Inhibition of mTOR signaling confers protection against cerebral ischemic injury in acute hyperglycemic rats. Int. J. Biol. Sci. 13, 878-887.

Ihse, E., Suhr, O. B., Hellman, U. & Westermark, P. Variation in amount of wild-type transthyretin in different fibril and tissue types in ATTR amyloidosis. J. Mol. Med. (Berl.) 89, 171-180 (2011).

Jayaraman S, Gantz DL, Haupt C, Gursky O. Serum amyloid A forms stable oligomers that disrupt vesicles at lysosomal pH and contribute to the pathogenesis of reactive amyloidosis. Proc Natl Acad Sci U S A. 2017 Aug 8;114(32):E6507-E6515.

Jin M, Shepardson N, Yang T, Chen G, Walsh D, Selkoe DJ (2011) Soluble amyloid beta-protein dimers isolated from Alzheimer cortex directly induce Tau hyperphosphorylation and neuritic degeneration. Proc Natl Acad Sci USA 108: 5819 - 5824

Johnson, J. L. & Craig, E. A. (1997) Cell 90, 201-204. ACS Nano. 2018 Nov 27;12(11):10855-10866.

Kitamura A, Kubota H. Amyloid oligomers: dynamics and toxicity in the cytosol and nucleus. FEBS J. 2010 Mar;277(6): 1369-79.

Konarkowska B, Aitken JF, Kistler J, Zhang S, Cooper GJ 2006 The aggregation potential of human amylin determines its cytotoxicity towards islet β-cells. FEBS J 273:3614-3624

Ladiwala AR, Lin JC, Bale SS, Marcelino-Cruz AM, Bhattacharya M, Dordick JS, Tessier PM. Resveratrol Selectively Remodels Soluble Oligomers and Fibrils of Amyloid Aβ into Off-pathway Conformers. J Biol Chem. 2010 Jul 30;285(31):24228-3

Liu C, Gao Y, Barrett J, Hu B. Autophagy and protein aggregation after brain ischemia. J Neurochem. 2010;115:68-78.

Maeda S, Takashima A. Tau Oligomers. Adv Exp Med Biol. 2019; 1184:373-380. doi: 10.1007/978-981-32-9358-8_27.

McKinnon SJ, Lehman DM, Kerrigan-Baumrind LA, Merges CA, Pease ME, Kerrigan DF, Ransom NL, Tahzib NG, Reitsamer HA, Levkovitch-Verbin H, Quigley HA, Zack DJ. Caspase Activation and Amyloid Precursor Protein Cleavage in Rat Ocular Hypertension. Investigative Ophthalmology & Visual Science April 2002, Vol.43, 1077-1087.

Miao et al., 2021, Developmental Cell 56, 427-442

Millucci L, Paccagnini E, Ghezzi L, Bernardini G, Braconi D, Laschi M, Consumi M, Spreafico A, Tanganelli P, Lupetti P, Magnani A, Santucci A. Different Factors Affecting Human ANP Amyloid Aggregation and Their Implications in Congestive Heart Failure. PLoS One. 2011;6(7):e21870.

Miyazaki Y, Kaikita K, Endo M, Horio E, Miura M, Tsujita K, Hokimoto S, Yamamuro M, Iwawaki T, Gotoh T, Ogawa H, Oike Y. C/EBP homologous protein deficiency attenuates myocardial reperfusion injury by inhibiting myocardial apoptosis and inflammation. Arterioscler Thromb Vasc Biol. 2011 May; 31(5):1124-32.

Mizushima, N. & Levine, B. Autophagy in mammalian development and differentiation. Nat. Cell Biol. 9, 823-830 (2010).

Muchowski, P.J., Schaffar, G., Sittler, A., Wanker, E.E., Hayer-Hartl, M.K. and Hartl, F.U. (2000) Hsp70 and Hsp40 chaperones can inhibit self-assembly of polyglutamine proteins into amyloid-like fibrils. Proc. Natl Acad. Sci. USA, 97, 7841-7846.

Nacharaju P, Lewis J, Easson C, Yen S, Hackett J, Hutton M, Yen S-H. Accelerated filament formation from tau protein with specific FTDP-17 missense mutations. FEBS Lett. 1999;447 (2-3):195-9.

Olshina MA, Angley LM, Ramdzan YM, Tang J, Bailey MF, Hill AF, et al. Tracking mutant huntingtin aggregation kinetics in cells reveals three major populations that include an invariant oligomer pool. J Biol Chem. 2010; 285: 21807-16. pii. doi: 10.1074/jbc.M109.084434

Opherk C, Duering M, Peters N, Karpinska A, Rosner S, Schneider E, Bader B, Giese A, Dichgans M. CADASIL mutations enhance spontaneous multimerization of NOTCH3. Hum Mol Genet. 2009 Aug 1;18(15):2761-7.

Paglin S, Lee N-Y, Nakar C, Fitzgerald M, Plotkin J, Deuel B, et al. Rapamycin- sensitive pathway regulates mitochondrial membrane potential, autophagy, and survival in irradiated MCF-7 cells. Cancer Res 2005;65:11061-70

Palsdottir A, Snorradottir AO, Thorsteinsson L. Hereditary cystatin C amyloid angiopathy: genetic, clinical, and pathological aspects. Brain Pathol. 2006 Jan;16(1):55-9.

Plaza-Zabala A, Sierra-Torre V, Sierra A (2017) Autophagy and microglia: Novel partners in neurodegeneration and ageing. Int J Mol Sci 18: 598.

Sangwan S, Zhao A, Adams KL, Jayson CK, Sawaya MR, Guenther EL, Pan AC, Ngo J, Moore DM, Soriaga AB, Do TD, Goldschmidt L, Nelson R, Bowers MT, Koehler CM, Shaw DE, Novitch BG, Eisenberg DS. Atomic structure of a toxic, oligomeric segment of SOD1 linked to amyotrophic lateral sclerosis (ALS). Proc Natl Acad Sci U S A. 2017 Aug 15;114(33):8770-8775.

Shibu M. Poulose, Donna F. Bielinski, Kirsty Carrihill-Knoll, Bernard M. Rabin and Barbara Shukitt-Hale. Exposure to 16O-Particle Radiation Causes Aging-Like Decrements in Rats through Increased Oxidative Stress, Inflammation and Loss of Autophagy Radiation Research, 176(6):761-769. 2011.

Svetoni F, Frisone P, Paronetto MP. Role of FET proteins in neurodegenerative disorders. RNA Biol. 2016 Nov;13(11):1089-1102.

Talbot K, Wang HY, Kazi H, Han LY, Bakshi KP, Stucky A, et al. Demonstrated brain insulin resistance in Alzheimer's disease patients is associated with IGF-1 resistance, IRS-1 dysregulation, and cognitive decline. J Clin Invest 2012; 122: 1316-38.

Pyo JO et al. Overexpression of Atg5 in mice activates autophagy and extends lifespan. Nat. Commun 4,2300 (2013).

Rodriguez JA, Shaw BF, Durazo A, Sohn SH, Doucette PA, Nersissian AM, et al. Destabilization of apoprotein is insufficient to explain Cu, Zn-superoxide dismutase-linked ALS pathogenesis. Proc Natl Acad Sci USA. 2005;102: 10516-21.

Rodriguez-Rodriguez P, Sandebring-Matton A, Merino-Serrais P, Parrado-Fernandez C, Rabano A, Winblad B, Ávila J, Ferrer I, Cedazo-Minguez A. Tau hyperphosphorylation induces oligomeric insulin accumulation and insulin resistance in neurons. Brain. 2017 Dec 1;140(12):3269-3285.

Roher, A. E., Chaney, M. O., Kuo, Y. M., Webster, S. D., Stine, W. B., Haverkamp, L. J., Woods, A. S., Cotter, R. J., Tuohy, J. M., Krafft, G. A., Bonnell, B. S., and Emmerling, M. R. (1996) Morphology and toxicity of Abeta-(1-42) dimer derived from neuritic and vascular amyloid deposits of Alzheimer's disease. J. Biol. Chem. 271, 20631-20635

Sengupta U, Montalbano M, McAllen S, Minuesa G, Kharas M, Kayed R. Formation of Toxic Oligomeric Assemblies of RNA-binding Protein: Musashi in Alzheimer's disease. Acta Neuropathol Commun. 2018 Oct 26;6(1):113.

Shankar GM, Li S, Mehta TH, Garcia-Munoz A, Shepardson NE, Smith I, Brett FM, Farrell MA, Rowan MJ, Lemere CA et al (2008) Amyloid-beta protein dimers isolated directly from Alzheimer's brains impair synaptic plasticity and memory. Nat Med 14: 837 - 842

Simoneau S, Rezaei H, Sale's N, Kaiser-Schulz G, Lefebvre-Roque M, et al. (2007) In vitro and in vivo neurotoxicity of prion protein oligomers. PLoS Pathog 3(8): e125.

Soeda Y, Yoshikawa M, Almeida OFX, Sumioka A, Maeda S, Osada H, et al. Toxic tau oligomer formation blocked by capping of cysteine residues with 1,2-dihy- droxybenzene groups. Nat Commun [Internet]. 2015;6:1-12
Tam, S. et al. The chaperonin TRiC blocks a huntingtin sequence element that promotes the conformational switch to aggregation. Nat Struct Mol Biol 16, 1279-1285 (2009)
Tokuda E, Anzai I, Nomura T, Toichi K, Watanabe M, Ohara S, Watanabe S, Yamanaka K, Morisaki Y, Misawa H, Furukawa Y. Immunochemical characterization on pathological oligomers of mutant Cu/Zn- superoxide dismutase in amyotrophic lateral sclerosis. Mol Neurodegener. 2017 Jan 5;12(1):2. doi: 10.1186/s13024-016-0145-9
Viret C, Rozières A, Faure M. Autophagy during early virus-host cell interactions. J Mol Biol. 2018 Jun 8;430(12):1696-1713.
Wang W, Kang J, Li H, Su J, Wu J, Xu Y, Yu H, Xiang X, Yi H, Lu Y, Sun L. Regulation of endoplasmic reticulum stress in rat cortex by p62/ZIP through the Keap1-Nrf2-ARE signalling pathway after transient focal cerebral ischaemia. Brain Inj. 2013;27:924-933
Westermark P, Engstrom U, Johnson KH, Westermark GT, Betsholtz C 1990 Islet amyloid polypeptide: pinpointing amino acid residues linked to amyloid fibril formation. Proc Natl Acad Sci USA 87:5036-5040
Wittmann CW, Wszolek MF, Shulman JM, Salvaterra PM, Lewis J, Hutton M, et al. Tauopathy in Drosophila: neurodegeneration without neurofibril- lary tangles. Science. 2001;293(5530):711-4.
Xiao S, Sanelli T, Chiang H, Sun Y, Chakrabartty A, Keith J, Rogaeva E, Zinman L, Robertson J. Low molecular weight species of TDP-43 generated by abnormal splicing form inclusions in amyotrophic lateral sclerosis and result in motor neuron death. Acta Neuropathol (2015) 130:49-61.
Yellon DM, Hausenloy DJ. Myocardial reperfusion injury. N Engl J Med. 2007 Sep 13; 357(11):1121-35.
Younger S, Jang H, Davies HA, Niemiec MJ, Garcia JGN, Nussinov R, Migrino RQ, Madine J, Arce FT. Medin Oligomer Membrane Pore Formation: A Potential Mechanism of Vascular Dysfunction. Biophys J. 2020 Jun 2;118 (11):2769-2782

## Claims

1. **A protein-oligomer aggregation-agent for use in the treatment and/or prevention of a condition or disease which is a pathological aberration caused by the presence of a neurotoxic protein-oligomer,** wherein the protein-oligomer aggregation-agent is selected from a small molecule, an antibody, a nanoparticle, a nucleic acid, a peptide and a therapeutic protein, wherein the treatment and/or prevention comprises a reduction or inhibition of the neurotoxic protein-oligomers by binding of the protein-oligomer aggregation-agent to the neurotoxic protein-oligomers and thereby triggering aggregation of the neurotoxic protein-oligomers.

2. The protein-oligomer aggregation-agent for use of claim 1, wherein the neurotoxic protein-oligomers comprise mutated, misfolded and/or denatured proteins.

3. The protein oligomer aggregation-agent for use of claim 1 or 2, wherein the neurotoxic protein-oligomers are soluble.

4. The protein-oligomer aggregation-agent for use of any one of claims 1 to 3, wherein the neurotoxic protein-oligomers are composed of 2 to 200 monomers, preferably 2 to 80 monomers, most preferably 2 to 50 monomers of the proteins,.

5. The protein-oligomer aggregation-agent for use of any one of claims 1 to 4, wherein the small molecule, the antibody, the nanoparticle, the nucleic acid, the peptide and/or the therapeutic protein binds to exposed hydrophobic domains of the neurotoxic protein-oligomers.

6. The protein-oligomer aggregation-agent for use of any one of claims 1 to 5, wherein the neurotoxic protein-oligomers are derived from proteins selected from one or more of the group of $\alpha$ synuclein, amyloid $\beta$, mutated huntingtin, tau protein, prion proteins, misfolded superoxide dismutase 1 (SOD1) polymorphisms, isled amyloid polypeptide (IAPP), Musashi protein, TAR DNA-binding protein 43 (TDP-43), misfolded transthyretin protein (TTR), $NOTCH_3$ receptor, mutated cystatin C, serum amyloid A (SAA), mutated gelsolin, misfolded rhodopsin, medin, dipeptide repeat protein, atrial natriuretic peptide, fused-in-sarcoma, insulin oligomers, or fragments truncated, and/or alternatively spliced variants and/or fusion proteins thereof, preferably wherein the neurotoxic protein-oligomers are derived from proteins are selected from one or more of the group $\alpha$ synuclein, amyloid $\beta$, prion proteins, misfolded superoxide dismutase 1 (SOD1) polymorphisms, isled amyloid polypeptide (IAPP), Musashi protein, TAR DNA-binding protein 43 (TDP-43), misfolded transthyretin protein (TTR), $NOTCH_3$ receptor, mutated cystatin C, serum amyloid A (SAA), mutated gelsolin, misfolded rhodopsin, medin, glycine-alanine dipeptide repeats, atrial natriuretic peptide, fused-in-sarcoma, insulin oligomers, or fragments, truncated, and/or alternatively spliced variants and/or fusion proteins thereof.

**7.** The protein-oligomer aggregation-agent for use of any one of claims 1 to 6, wherein the protein-oligomer aggregation-agent is a small molecule which comprises the formula (I):

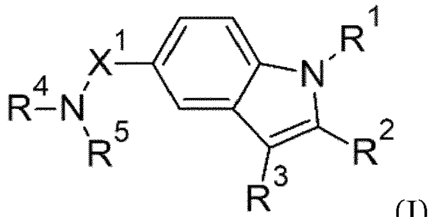

(I),

wherein

(i)

$R^1$ is independently -H, benzyl, —$C_1$-$C_6$alkyl, —$C_2$-$C_6$alkenyl, or —$C_2$-$C_6$alkynyl, wherein each alkyl, alkenyl and alkynyl is optionally substituted with one or more substituents independently selected from the group consisting of-Br and -F, or benzyl;
$R^2$ is -H, cyano, or —$C_1$-$C_6$alkyl, —$C_2$-$C_6$alkenyl, or —$C_2$-$C_6$alkynyl, wherein each alkyl, alkenyl and alkynyl is optionally substituted with one or more substituents independently selected from the group consisting of-Br and -F;
and $R^3$ is independently -H, cyano, —$C_1$-$C_6$alkyl, —$C_2$-$C_6$alkenyl, or —$C_2$-$C_6$alkynyl, wherein each alkyl, alkenyl and alkynyl is optionally substituted with one or more substituents independently selected from the group consisting of-Br and -F;
or $R^1$ and $R^2$ can optionally combine, together with the atoms to which they are attached, to form a -$C_4$-$C_8$heterocycle, wherein the heterocycle is optionally substituted with one or more substituents independently selected from the group consisting of -Br, -F, —$C_1$-$C_6$alkyl, —$C_2$-$C_6$alkenyl, and —$C_2$-$C_6$alkynyl, and wherein each alkyl, alkenyl, and alkynyl is optionally substituted with one or more -Br or -F;
or $R^2$ and $R^3$ can optionally combine, together with the atoms to which they are attached, to form a -$C_4$-$C_8$carbocycle or a -$C_4$-$C_8$heterocycle, wherein the carbocycle or heterocycle is optionally substituted with one or more substituents independently selected from the group consisting of -Br, -F, —$C_1$-$C_6$alkyl, —$C_2$-$C_6$alkenyl, and —$C_2$-$C_6$alkynyl, and wherein each alkyl, alkenyl, and alkynyl is optionally substituted with one or more -Br or -F;

(ii) $X^1$ is -(C=O)- or -($CH_2$)- or -(SO2)-;
(iii) $R^4$ is -H or -$CH_3$;
(iv) and $R^5$ is

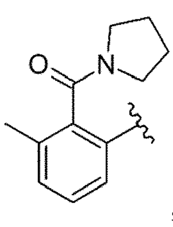

,

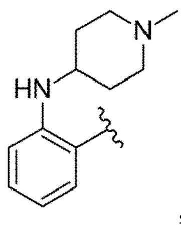

,

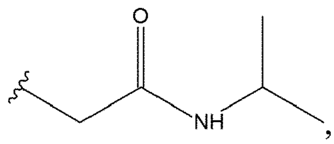

,

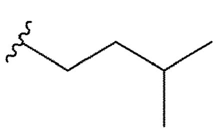

or

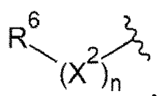

,

(v) wherein

n is 1 to 4;

$X^2$ is $-(CH_2)-$, $-(C=O)-$, $-(CH(-O-Met))-$, $-(C(CH_3)_2)-$, $-(CH(CH(CH_3)_2))-$, a - $C_6$cyclyl and/or non-substituted or mono-substituted piperidin-4-yl;

and $R^6$ is 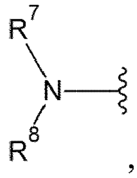, or 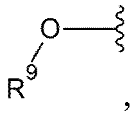,

wherein

$R^7$ and $R^8$ are each independently selected from -H, cyano linear or branched —$(C_1\text{-}C_6)$ alkyl, —$C_2\text{-}C_6$alkenyl, or —$C_2\text{-}C_6$alkynyl, -O-$(CH_3)$, -$C_3\text{-}C_5$cyclyl, and/or 2-methylpropionyl, wherein each alkyl, alkenyl and alkynyl is optionally substituted with one or more substituents independently selected from the group consisting of-Br and -F;

or wherein $R^7$ and $R^8$ can optionally combine, together with the nitrogen atom to which they are attached, to form a $C_3\text{-}C_8$heterocycle wherein the heterocycle is optionally substituted with one or more substituents independently selected from the group consisting of-Br, -F, —$C_1\text{-}C_6$alkyl, —$C_2\text{-}C_6$alkenyl, and —$C_2\text{-}C_6$alkynyl, and wherein each alkyl, alkenyl and alkynyl is optionally substituted with one or more -Br or -F.

and $R^9$ is H, $-CH_3$ or cyclopropylmethyl;

(vi) or wherein $R^4$ and $R^5$ form together with the bridging N atom a 6-7 membered heterocyclyl containing 2 heteroatoms that is optionally mono substituted, preferably 4-(propan-2-yl)piperazin-1-yl or 4-(propan-2-yl)-1,4,diazepan-1-yl.

or a pharmaceutically acceptable salt, hydrate, solvate, prodrug, stereoisomer, or tautomer thereof.

**8.** The protein-oligomer aggregation agent for use of claim 7, wherein

(i)

$R^1$ is -H, $-CH_3$ or benzyl;
$R^2$ is -H or $-CH_3$;
and $R^3$ is -H, $-CH_3$ or ethyl;

(ii) $X^1$ is -(C=O)- or $-(CH_2)-$ or $-(SO_2)-$;
(iii) $R^4$ is -H or $-CH_3$;
(iv) and $R^5$ is

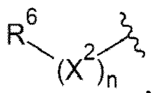,

wherein n is 1 to 4;

$X^2$ is $-(CH_2)-$, $-(C=O)-$, $-(CH(-O-Met))-$, $-(C(CH_3)_2)-$, $-(CH(CH(CH_3)_2))-$, and/or non-substituted or mono-substituted piperidin-4-yl;
and $R^6$ is

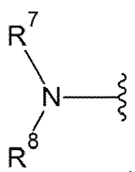, 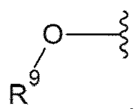,

wherein $R^7$ and $R^8$ are each independently selected from -H, linear or branched - ($C_1$-$C_4$) alkyl, -O-($CH_3$), cyclopropyl, and/or 2-methylpropionyl, or wherein $R^7$ and $R^8$ form together with the bridging N a 5- or 6-membered heterocyclyl that can be non-substituted, mono-substituted or bisubstituted.
and $R^9$ is H, -$CH_3$ or cyclopropylmethyl;

(v) or wherein $R^4$ and $R^5$ form together with the bridging N atom a 6-7. membered heterocyclyl containing 2 heteroatoms that is optionally mono substituted, preferably 4-(propan-2-yl)piperazin-1-yl or 4-(propan-2-yl)-1,4,diazepam-1-yl.

**9.** The protein-oligomer aggregation agent for use of any one of claims 1 to 7, wherein the protein-oligomer aggregation-agent is a small molecule, and selected from the group consisting of:

| | | | |
|---|---|---|---|
| 1 | 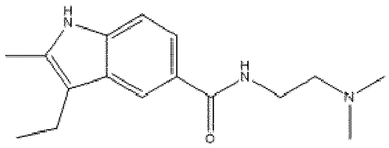 | 2 | 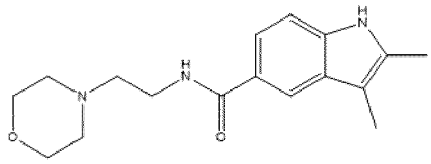 |
| 3 | 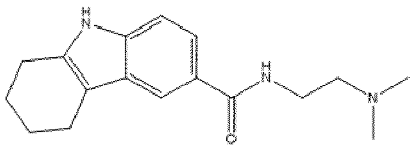 | 4 | 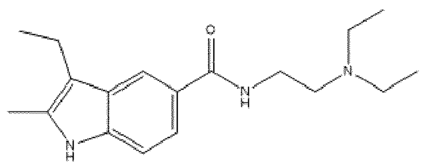 |
| 5 | 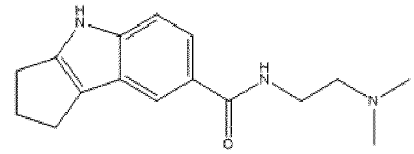 | 6 | 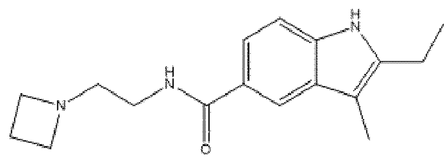 |
| 7 | 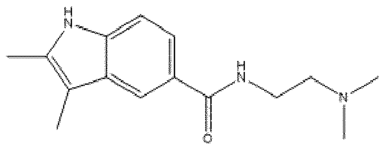 | 8 | 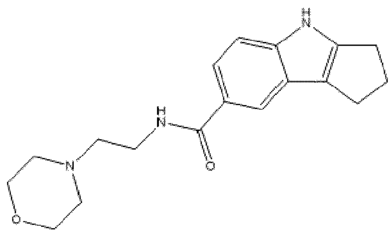 |
| 9 | 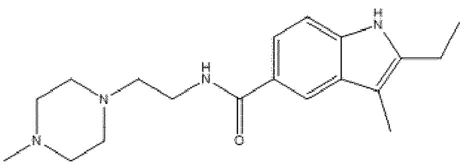 | 10 | 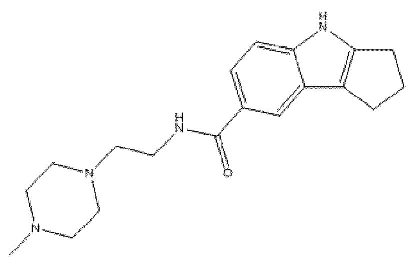 |
| 11 | 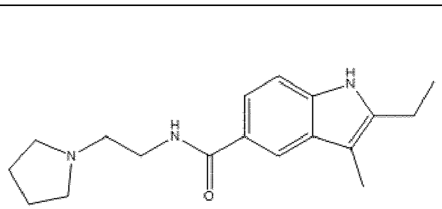 | 12 | 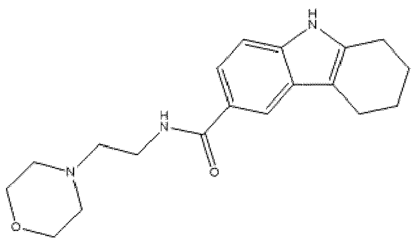 |

(continued)

| | | | |
|---|---|---|---|
| 13 | 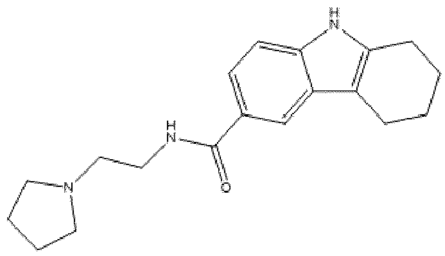 | 14 | 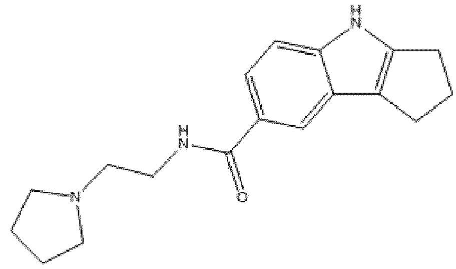 |
| 15 | 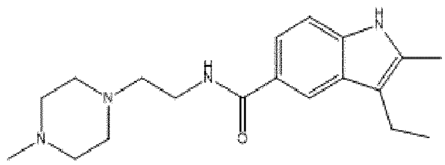 | 16 | 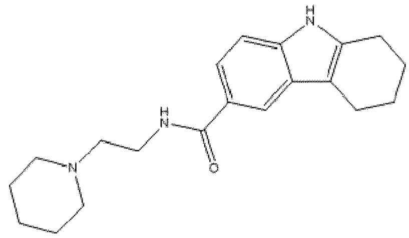 |
| 17 | 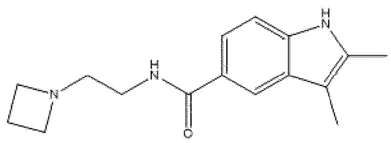 | 18 | 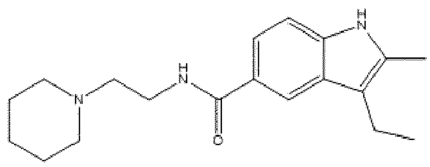 |
| 19 | 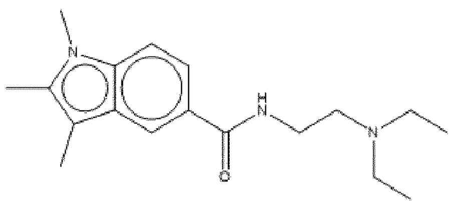 | 20 | 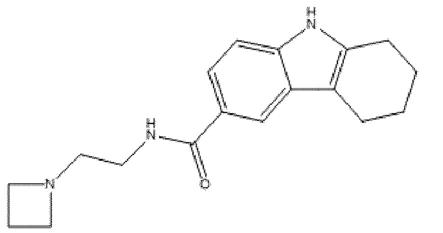 |
| 21 | 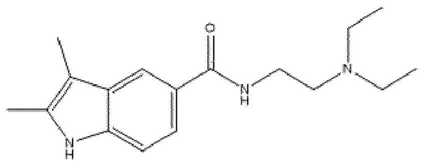 | 22 | 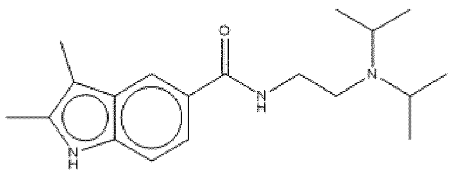 |
| 23 | 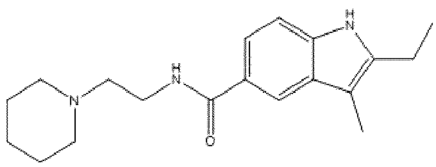 | 24 | 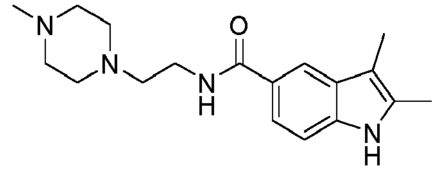 |
| 25 | 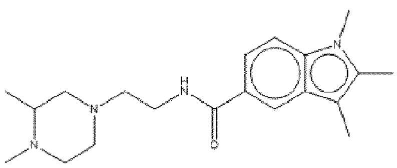 | 26 | 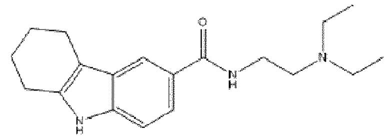 |

(continued)

| | | | |
|---|---|---|---|
| 27 | 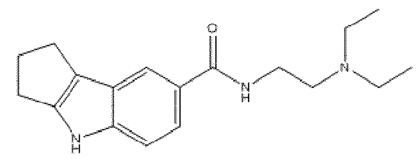 | 28 | 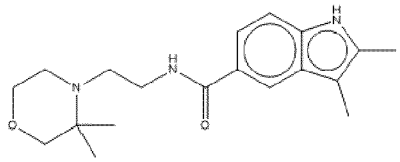 |
| 29 | 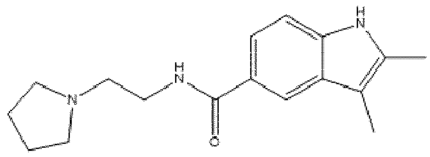 | 30 | 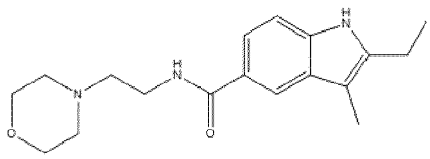 |
| 31 | 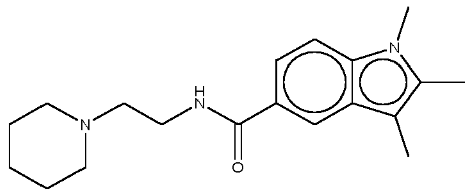 | 32 | 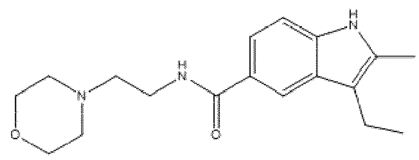 |
| 33 | 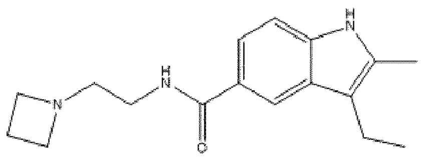 | 34 | 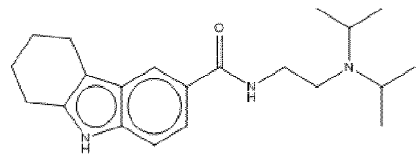 |
| 35 | 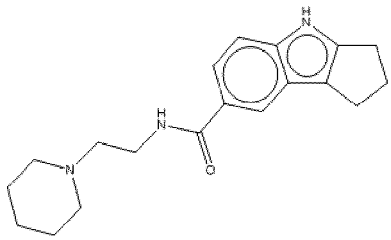 | 36 | 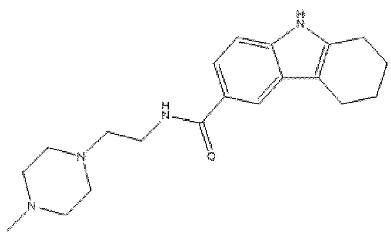 |
| 37 | 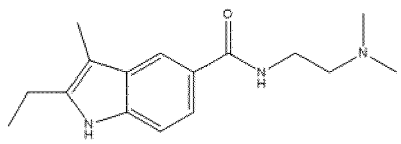 | 38 | 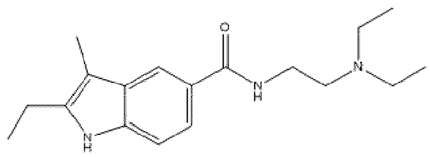 |
| 39 | 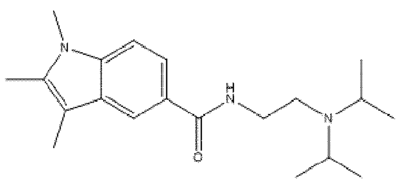 | 40 | 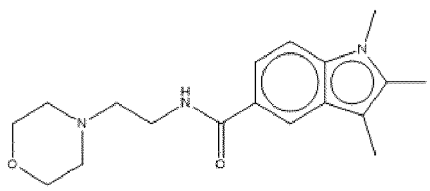 |
| 41 | 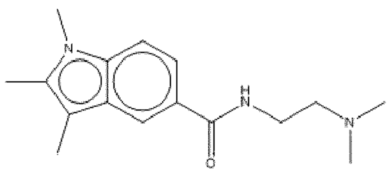 | 42 | 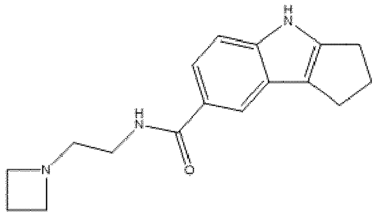 |

(continued)

| | | | |
|---|---|---|---|
| 43 | 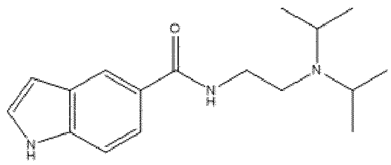 | 44 | 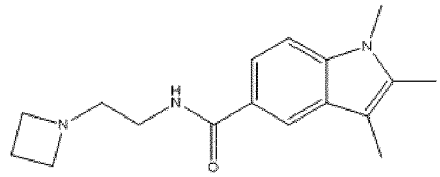 |
| 45 | 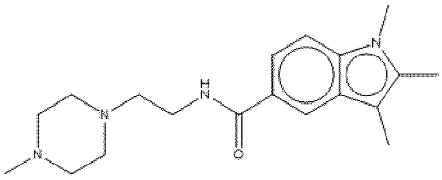 | 46 | 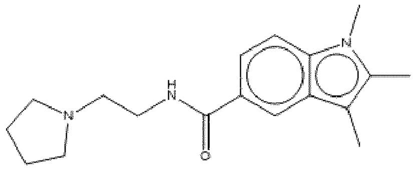 |
| 47 | 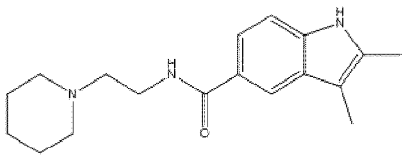 | 48 | 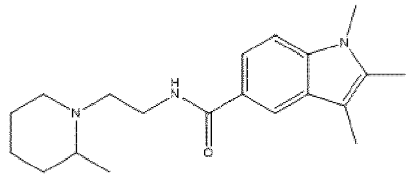 |
| 50 | 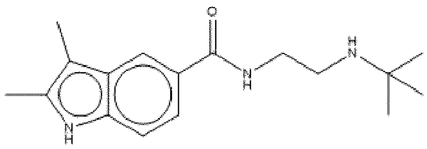 | 52 | 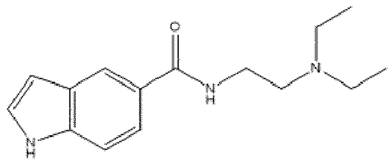 |
| 53 | 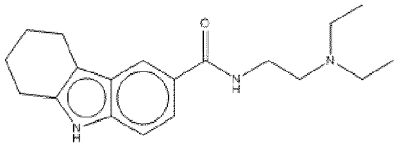 | 54 | 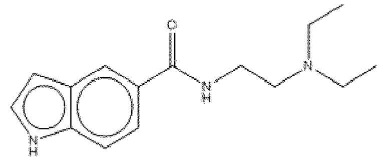 |
| 55 | 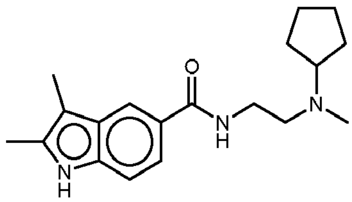 | 56 | 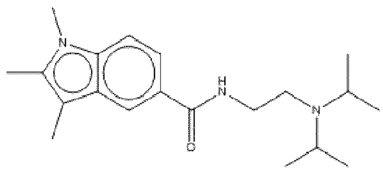 |
| 57 | 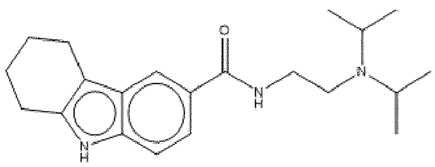 | 58 | 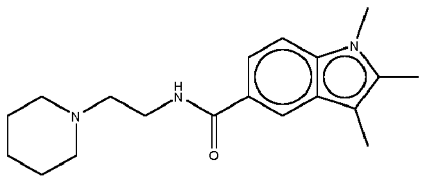 |
| 59 | 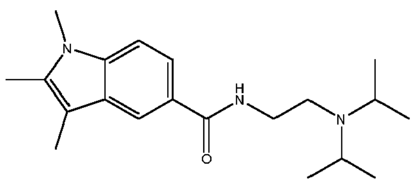 | 72 | 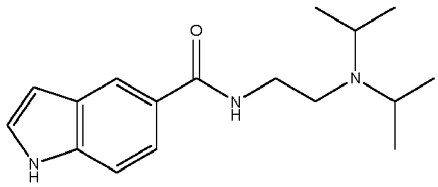 |

(continued)

| | | | |
|---|---|---|---|
| 101 | 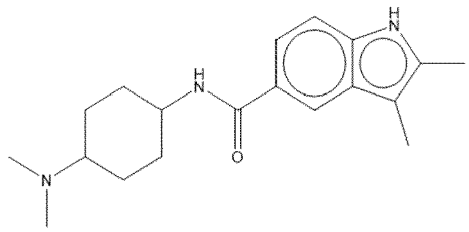 | 102 | 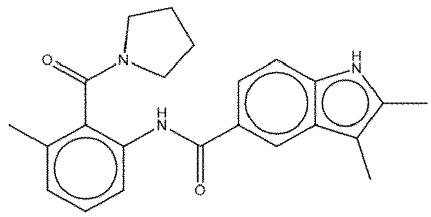 |
| 103 | 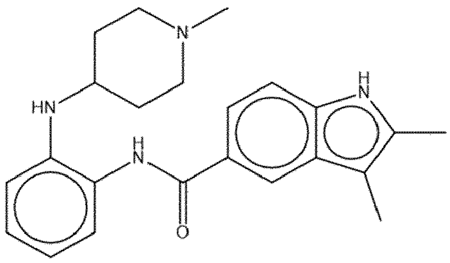 | 105 | 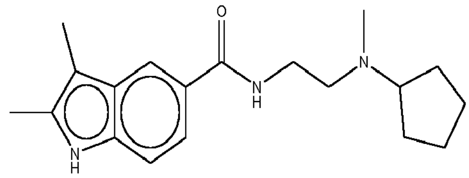 |
| 106 | 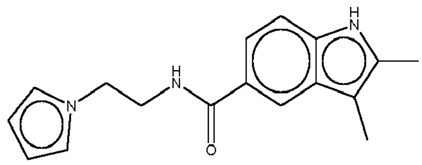 | 112 | 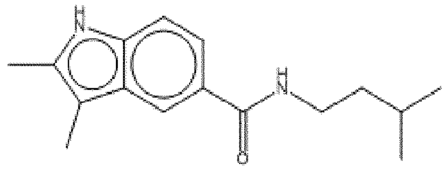 |
| 113 | 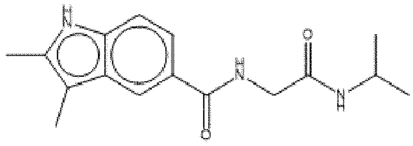 | 114 | 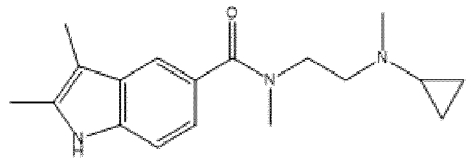 |
| 115 | 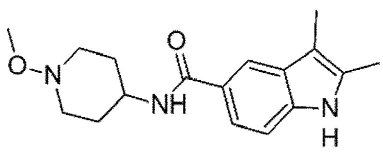 | 116 | 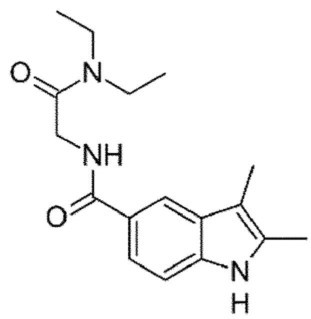 |
| 117 | 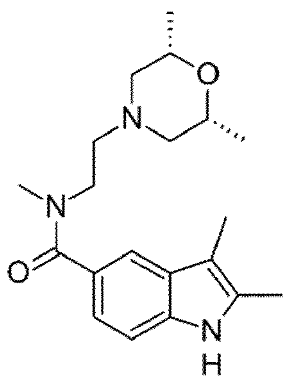 | 118 | 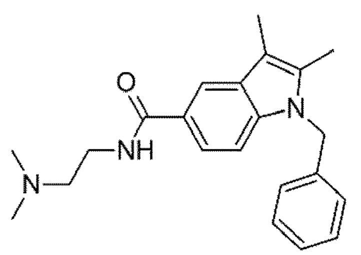 |

(continued)

| | | | |
|---|---|---|---|
| 119 | 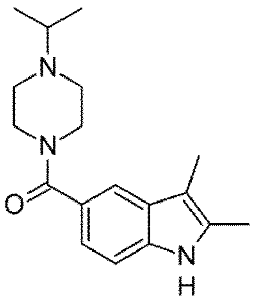 | 120 | 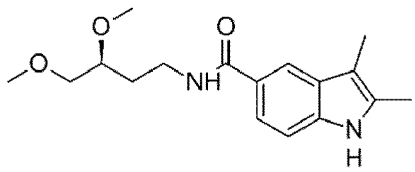 |
| 121 | 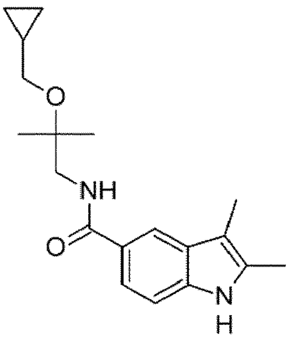 | 122 | 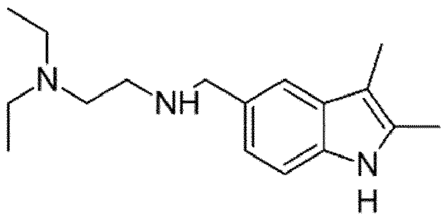 |
| 123 | 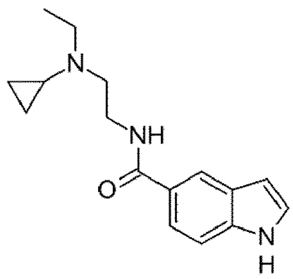 | 124 | 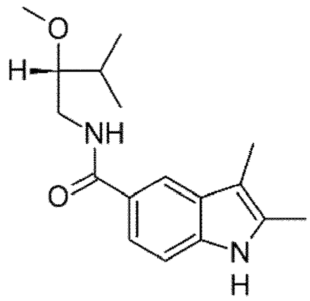 |
| 125 | 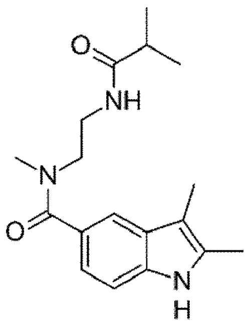 | 126 | 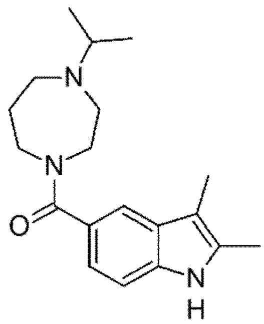 |
| 127 | 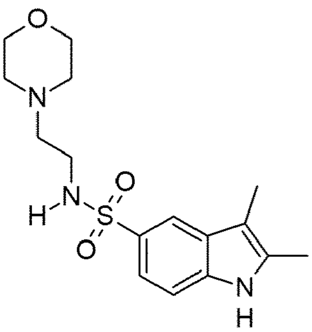 | 128 | 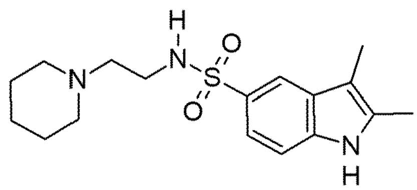 |

or a pharmaceutically acceptable salt, hydrate, solvate, prodrug, stereoisomer, or tautomer thereof.

**10.** The protein-oligomer aggregation-agent for use of any one of claims 1 to 6, wherein the protein-oligomer aggregation-agent is a small molecule and comprises a compound shown below

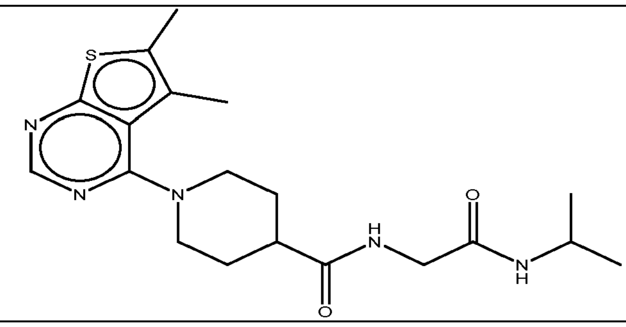

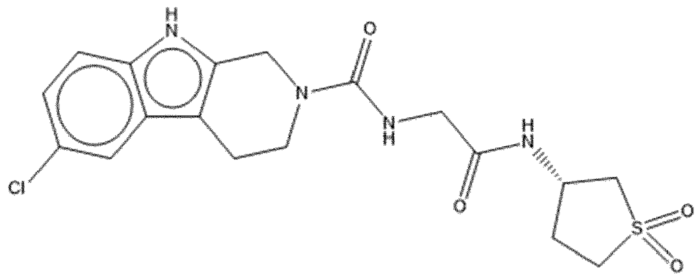

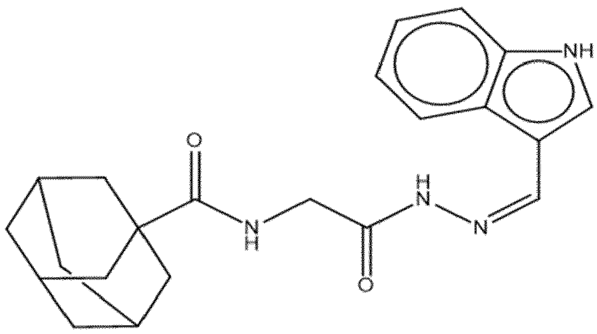

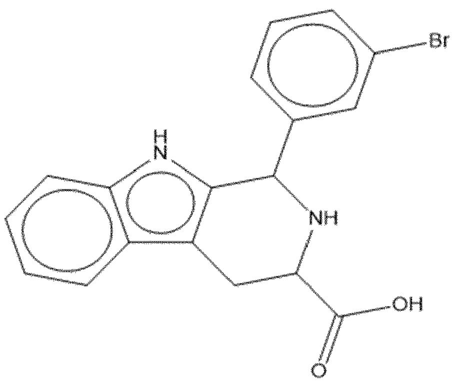

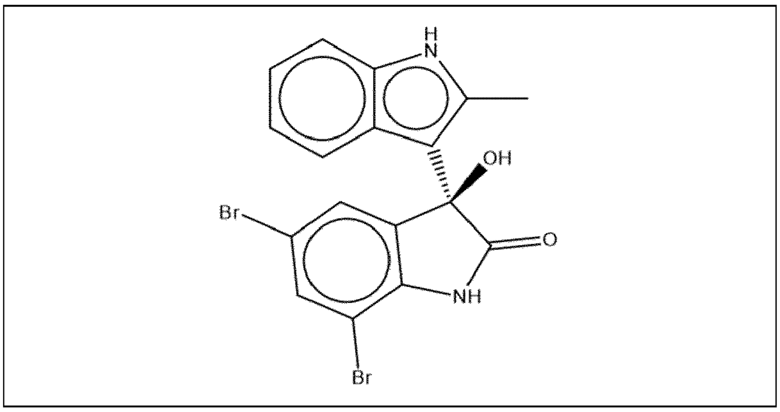

or a pharmaceutically acceptable salt, hydrate, solvate, prodrug, stereoisomer, or tautomer thereof.

11. The protein-oligomer aggregation-agent for use of any one of claims 1 to 10, wherein the condition or disease is Huntington's disease, spinocerebellar ataxia, Kennedy's disease, dentatorubral-pallidoluysian atrophy, multiple system atrophy, frontotemporal dementia, frontotemporal lobar degeneration (FTLD), Alzheimer's disease, Parkinson's disease, cerebral amyloid angiopathy (CAA), retinal disease glaucoma, progressive supranuclear palsy (PSP), corticobasal degeneration (CBD), Pick's disease, Argyrophilic grain disease, Parkinsonism linked to chromosome 17, familial amyloid polyneuropathy, leptomeningeal amyloidosis, CADASIL (Cerebral Autosomal Dominant Arteriopathy with Subcortical Infarcts and Leukoencephalopathy) syndrome, hereditary cystatin C amyloid angiopathy, chronic inflammation AA (amyloid A) amyloidosis, gelsolin amyloidosis, lattice corneal dystrophy type 2 (LCD2), retinal degradation, aortic aneurysm, aortic dissection, vascular dementia, isolated atrial amyloidosis (IAA), congestive heart failure, FUS-associated amyotrophic lateral sclerosis, FUS-associated dementia, Creutzfeldt-Jakob disease (CJD), Fatal Familial Insomnia (FFI), Gerstmann-Straussler-Scheinker disease (GSS), Huntington disease-like type 1 (HDL1), Kuru and Spongiform encephalopathy, an ischemic stroke, a ischemic heart disease, a viral infection, radiation caused brain function impairment amyotrophic lateral sclerosis (ALS), Lewy Body Dementia, Down's syndrome, type-2 diabetes mellitus and/or a prion disease, preferably wherein the disease is Lewy Body Dementia, Down's syndrome, type-2 diabetes mellitus, cerebral amyloid angiopathy (CAA), retinal disease glaucoma, progressive supranuclear palsy (PSP), corticobasal degeneration (CBD), Pick's disease, Argyrophilic grain disease, Parkinsonism linked to chromosome 17, familial amyloid polyneuropathy, leptomeningeal amyloidosis, CADASIL syndrome, hereditary cystatin C amyloid angiopathy, chronic inflammation AA (amyloid A) amyloidosis, gelsolin amyloidosis, lattice corneal dystrophy type 2 (LCD2), retinal degradation, aortic aneurysm, aortic dissection, vascular dementia, isolated atrial amyloidosis (IAA), congestive heart failure, FUS-associated amyotrophic lateral sclerosis, FUS-associated dementia, Creutzfeldt-Jakob disease (CJD), Fatal Familial Insomnia (FFI), Gerstmann-Straussler-Scheinker disease (GSS), Huntington disease-like type 1 (HDL1), Kuru and Spongiform encephalopathy, an ischemic stroke, an ischemic heart disease, a viral infection, radiation caused brain function impairment and/or a prion disease.

12. **A diagnostic method for detecting** a disease associated with an accumulation of neurotoxic protein-oligomers in a tissue, cell, intracellular- or extracellular fluid of a subject, wherein the method comprises:

(i) Providing a sample of the tissue, cell, intracellular- or extracellular fluid of the subject, optionally wherein the sample is a biopsy of a tissue or bodyfluid associated with the disease in the subject;
(ii) Bringing into contact the sample and the protein-oligomer aggregation-agent according to any one of claims 1 to 11;
(iii) Detecting the presence or absence of aggregated protein-oligomer in the sample, wherein the presence or absence of aggregated protein-oligomer in the sample is indicative of a disease associated with an accumulation of neurotoxic protein-oligomers in a tissue cell, intracellular or extracellular fluid.

13. **A diagnostic method for detecting** a disease associated with an accumulation of neurotoxic protein-oligomers in a tissue, in a cell, or in an intracellular- or extracellular fluid, wherein the method comprises:

(i) Providing a sample, optionally wherein the sample is a biopsy sample of a subject;
(ii) Bringing into contact the sample and the protein-oligomer aggregation-agent according to any one of claims 1 to 11;

(iii) Detecting a binding of the protein-oligomer aggregation-agent to the neurotoxic protein-oligomer, wherein the presence or absence of binding is indicative of a disease associated with an accumulation of neurotoxic protein-oligomers in a tissue cell, intracellular or extracellular fluid.

14. **A method for the identification of a compound** useful in the treatment of a condition associated with the presence of neurotoxic protein-oligomers, the method comprising the steps of:

(i) Contacting a candidate compound with at least two of the neurotoxic protein-oligomers associated with the condition;
(ii) Determining the formation of an oligomer-aggregate composed of a plurality of neurotoxic protein-oligomers of the at least two neurotoxic protein-oligomers associated with the condition in the presence of the candidate compound compared to the formation of an oligomer-aggregate composed of a plurality of neurotoxic protein-oligomers of the at least two neurotoxic protein-oligomers associated with the condition in the absence of the candidate compound;

Wherein an increased formation of a protein-oligomer aggregate in the presence of the compound compared to the absence of the compound indicates that the compound is useful in the treatment of a condition associated with the presence of the neurotoxic protein-oligomer.

15. A protein-oligomer aggregation-agent, wherein the protein-oligomer aggregation-agent comprises a small molecule selected from the group of

| | | |
|---|---|---|
| 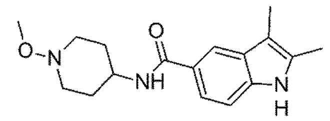 | 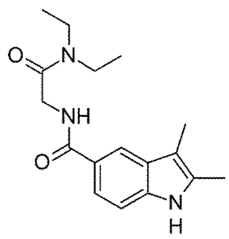 | 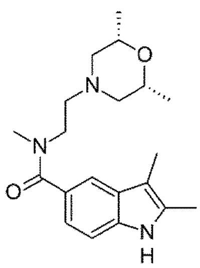 |
| 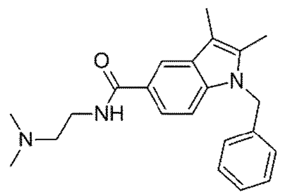 | 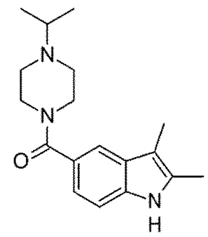 | 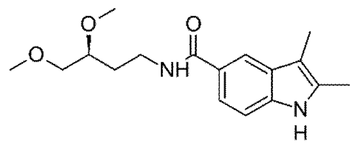 |
| 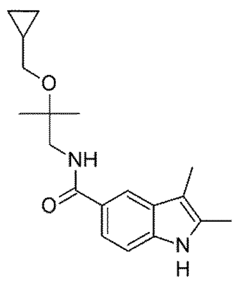 | 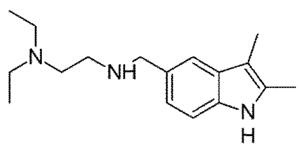 | 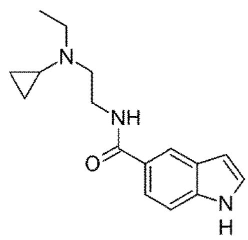 |

| | | |
|---|---|---|
| 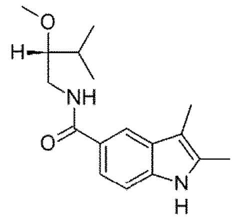 | 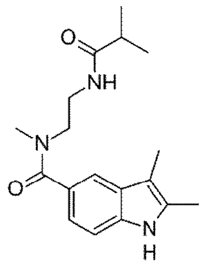 | 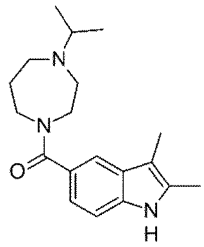 |
| 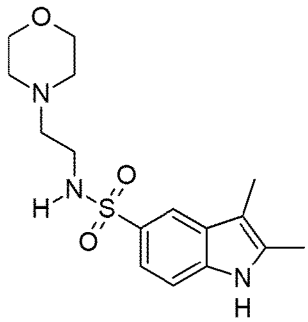 | 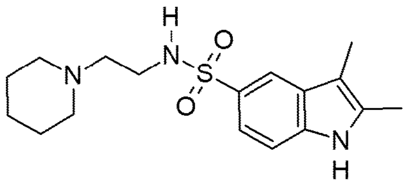 | |

or a pharmaceutically acceptable salt of such compound, hydrate, solvate, prodrug, stereoisomer, or tautomer thereof.

FIGURE 1

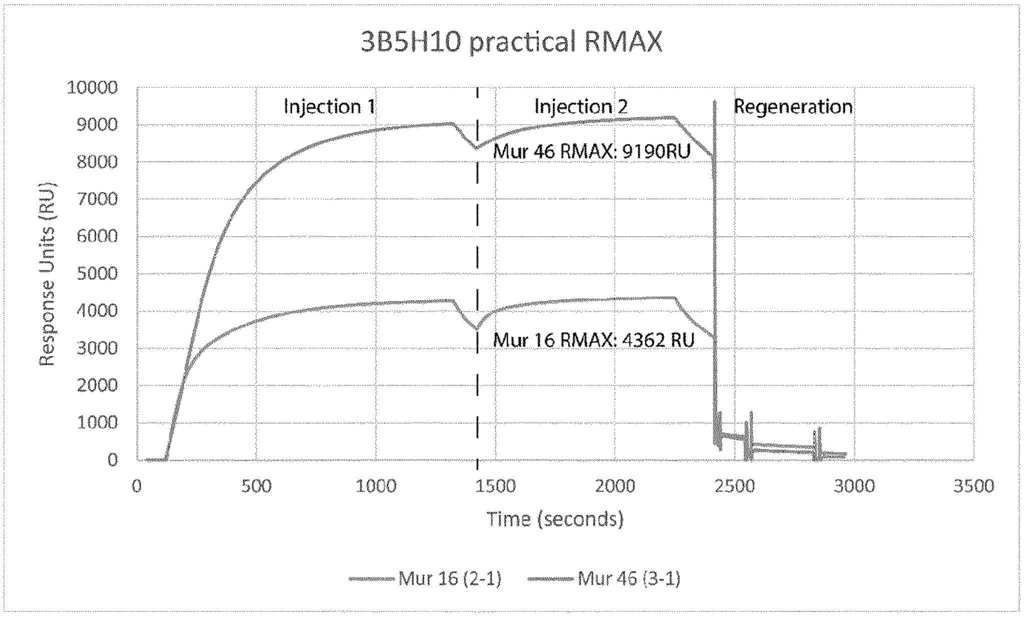


FIGURE 2

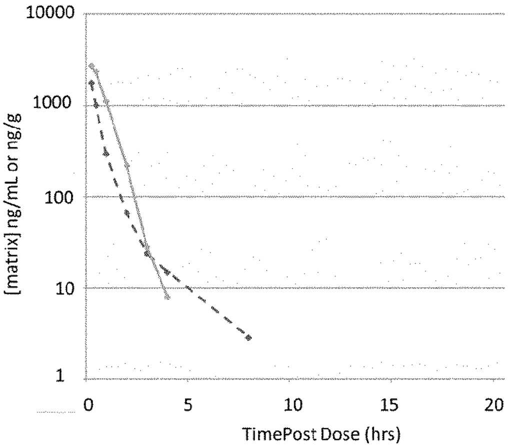


FIGURE 3

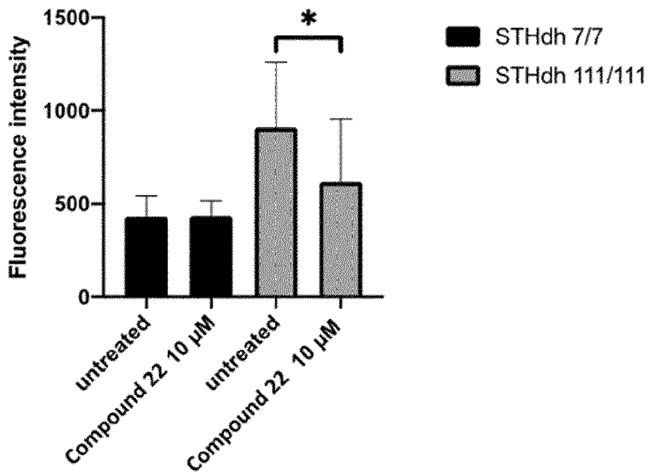

FIGURE 4

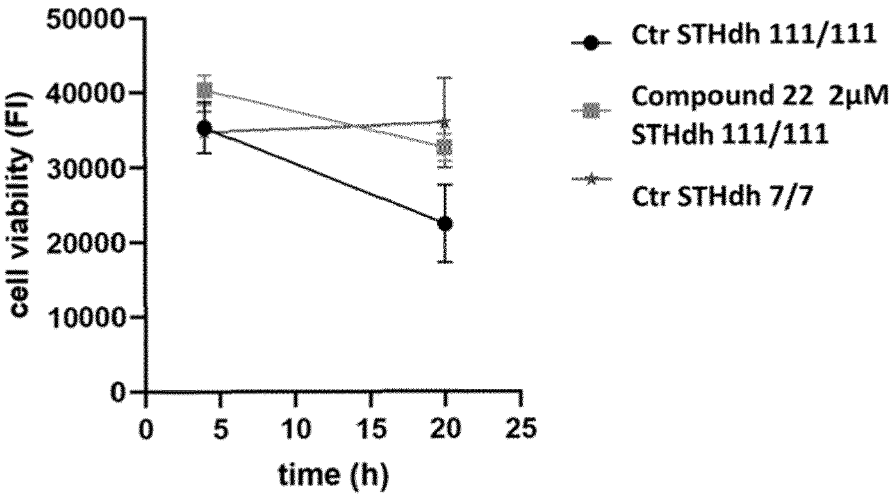


FIGURE 5

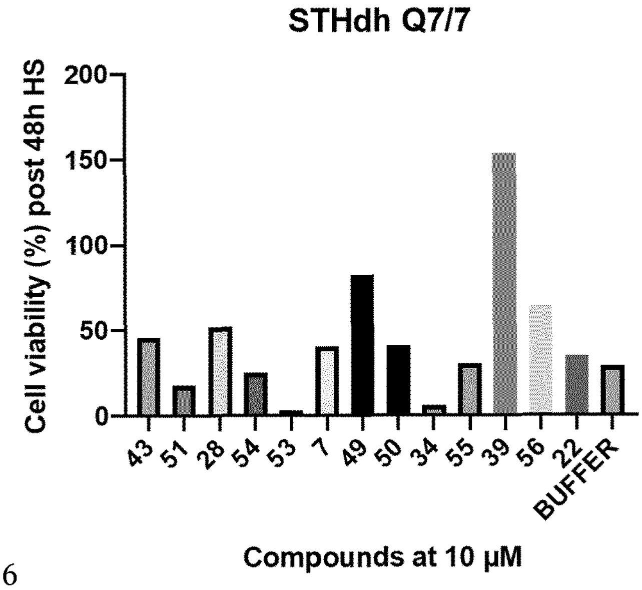


FIGURE 6

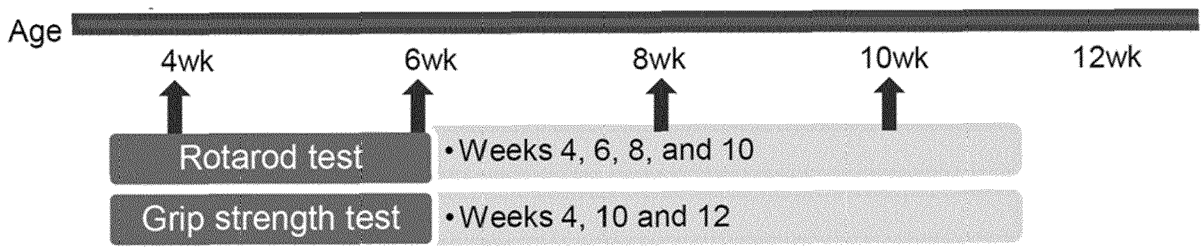

FIGURE 7

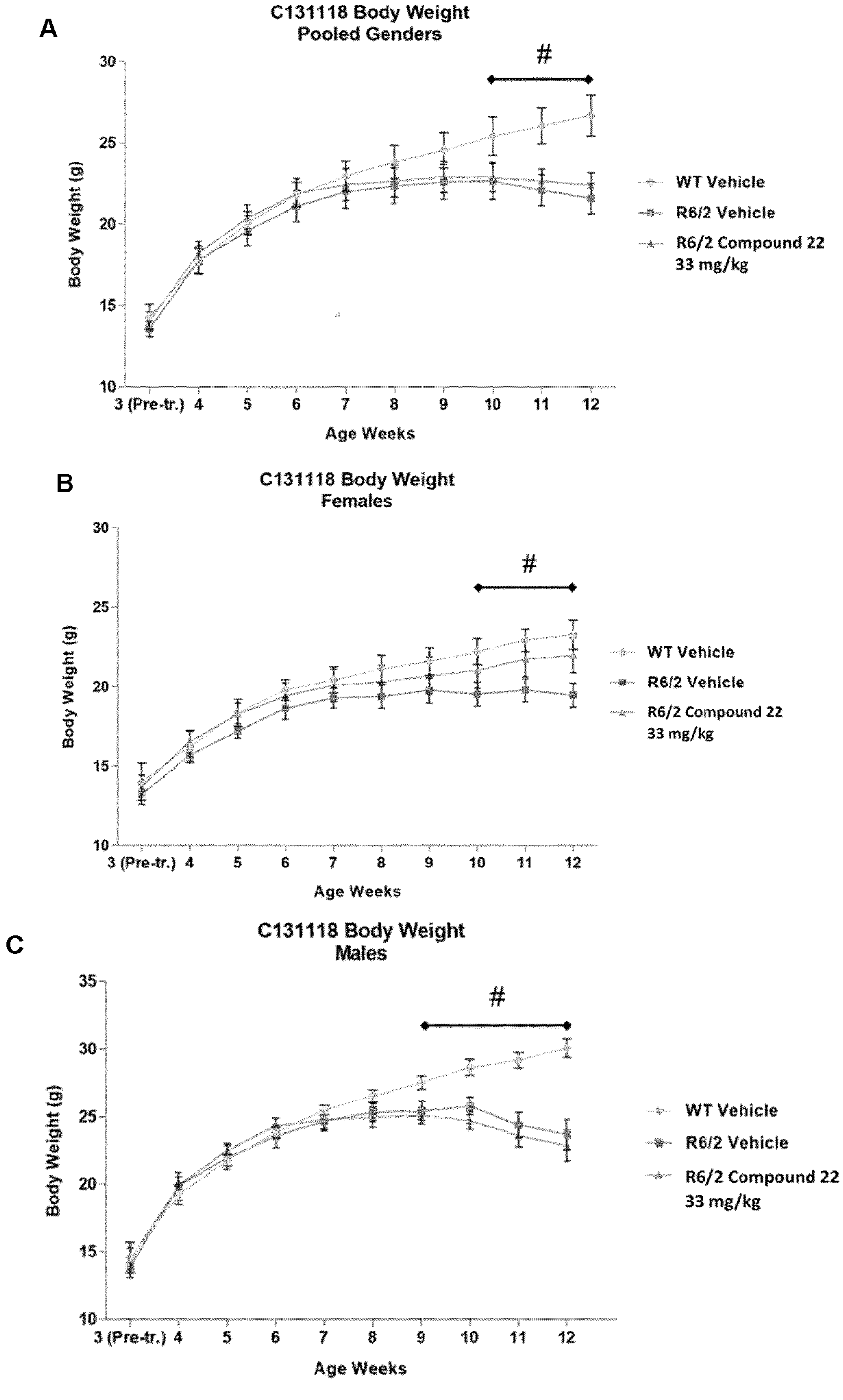
A
C131118 Body Weight
Pooled Genders
#
Body Weight (g)
Age Weeks
3 (Pre-tr.) 4 5 6 7 8 9 10 11 12
WT Vehicle
R6/2 Vehicle
R6/2 Compound 22
33 mg/kg
B
C131118 Body Weight
Females
#
Body Weight (g)
Age Weeks
WT Vehicle
R6/2 Vehicle
R6/2 Compound 22
33 mg/kg
C
C131118 Body Weight
Males
#
Body Weight (g)
Age Weeks
WT Vehicle
R6/2 Vehicle
R6/2 Compound 22
33 mg/kg

FIGURE 8

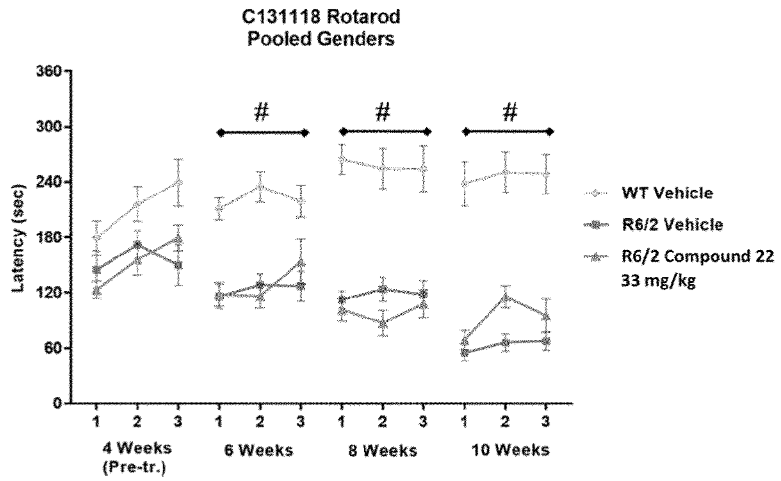


FIGURE 9

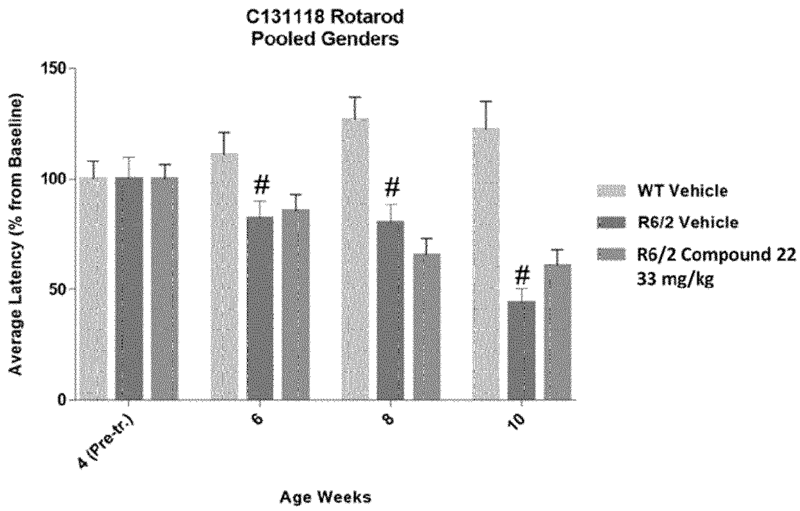


FIGURE 10

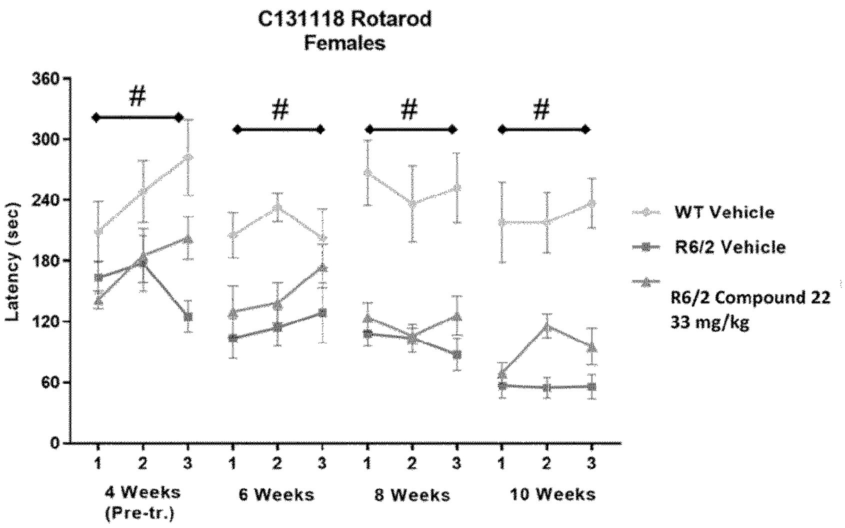

FIGURE 11

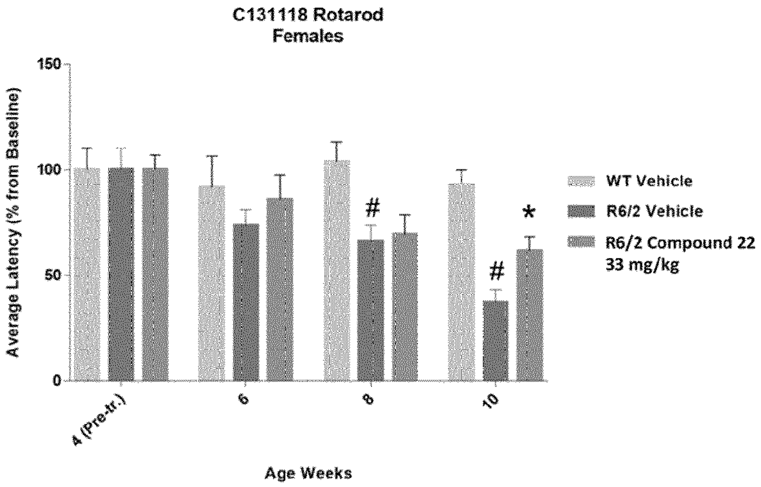


FIGURE 12

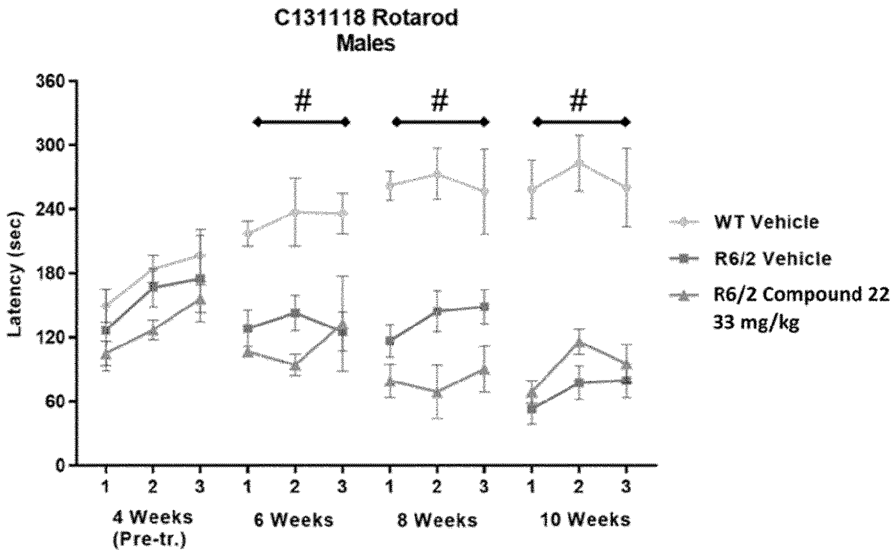


FIGURE 13

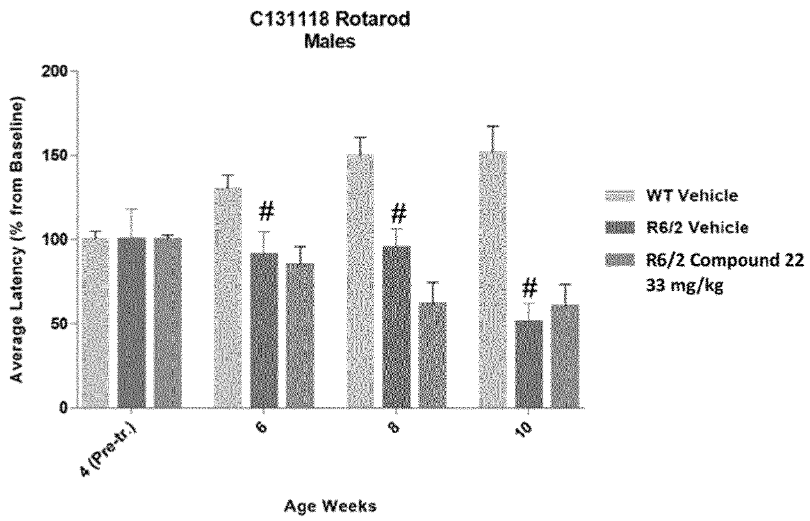

FIGURE 14

A

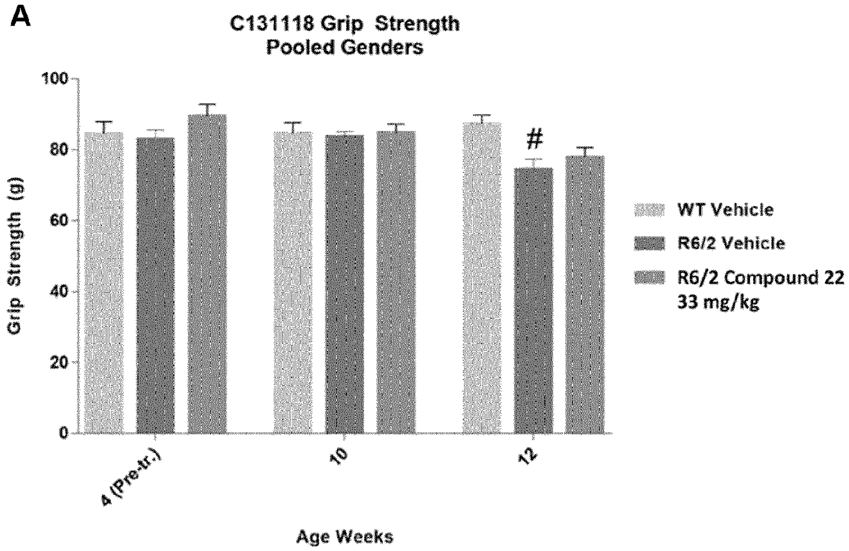


B

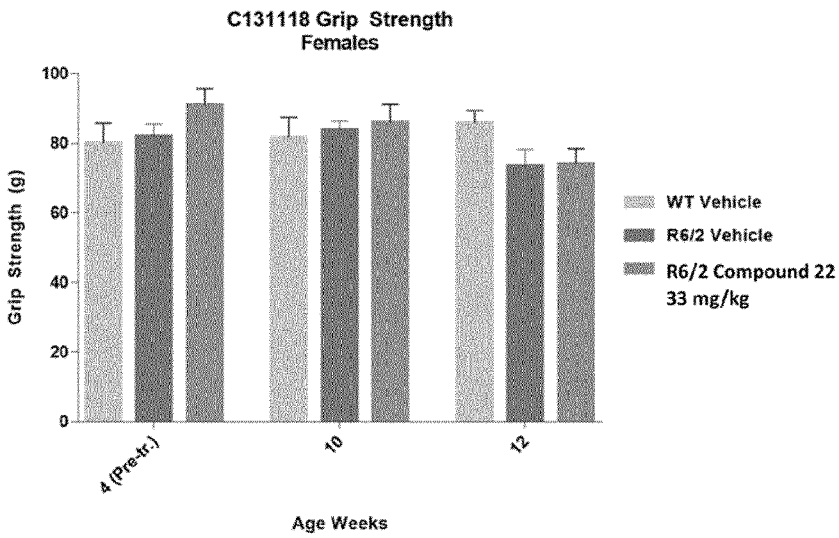


C

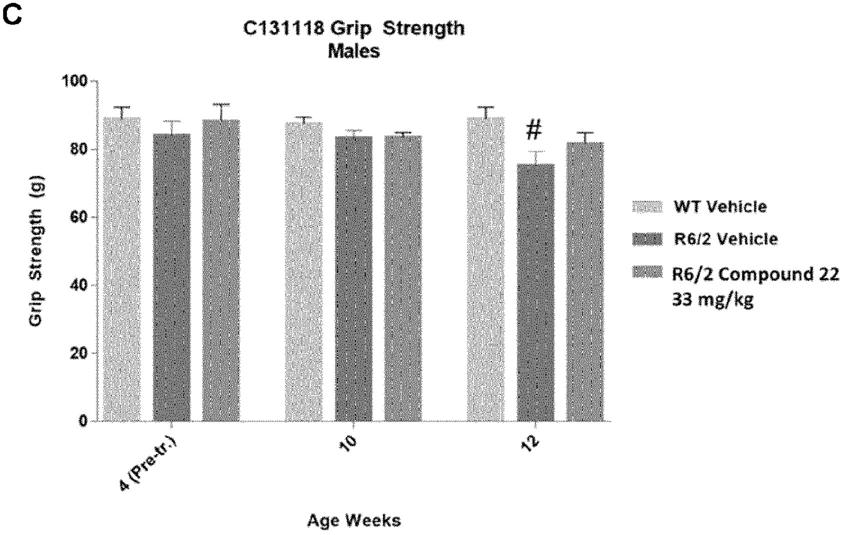

FIGURE 15

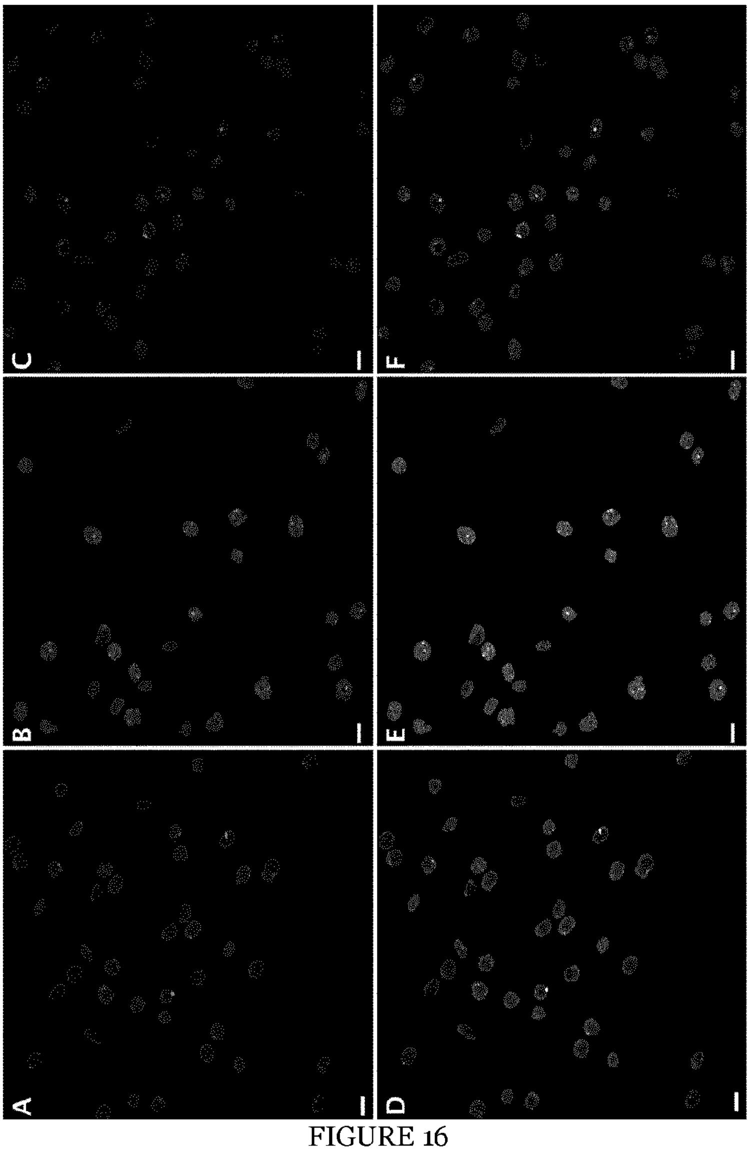


FIGURE 16

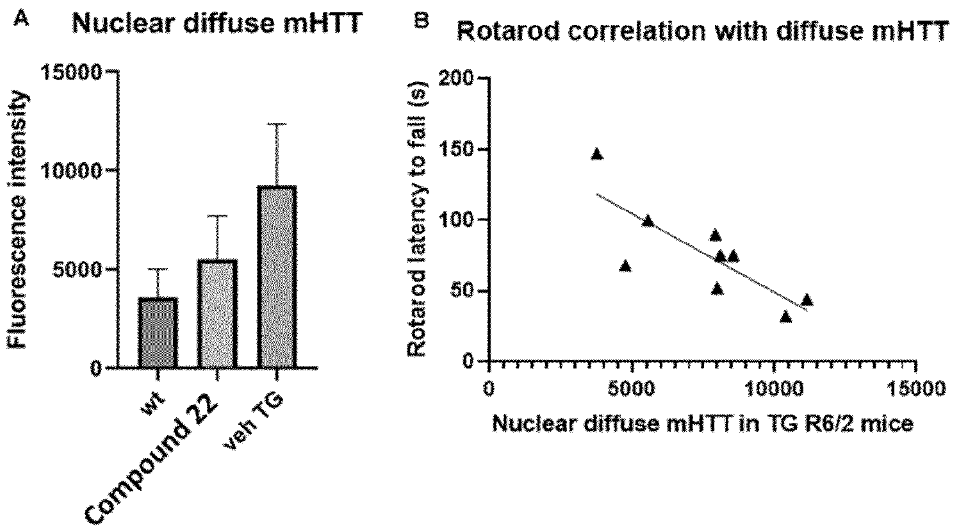

FIGURE 17

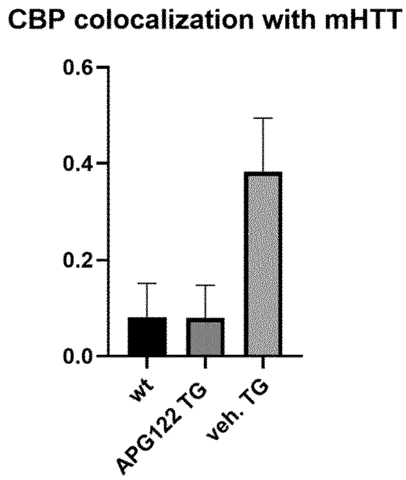


FIGURE 18

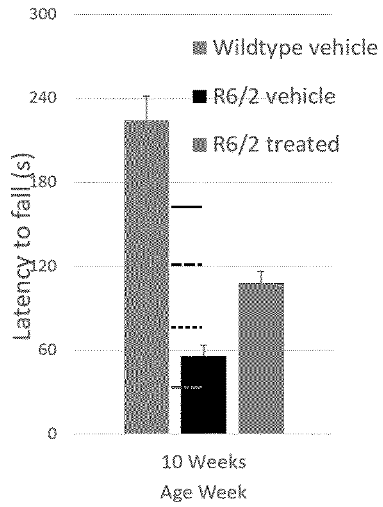


FIGURE 19

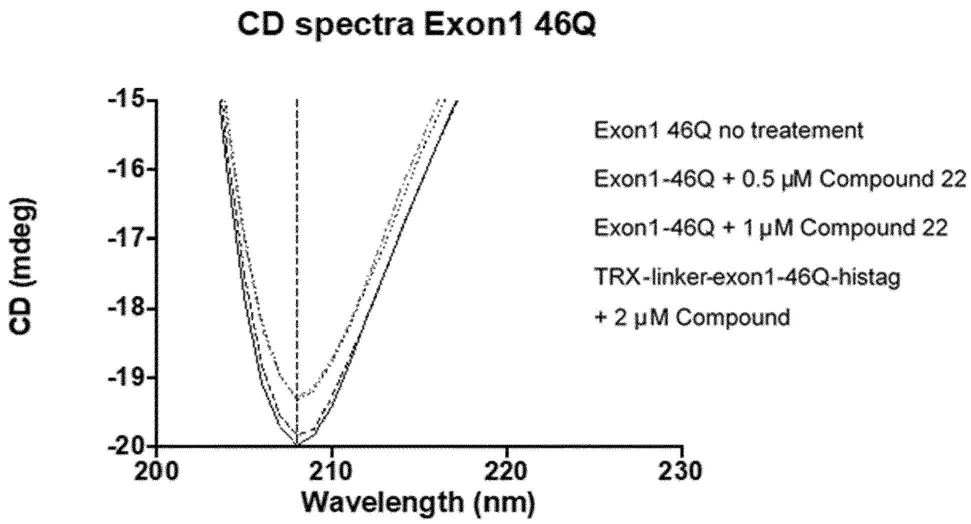

FIGURE 20

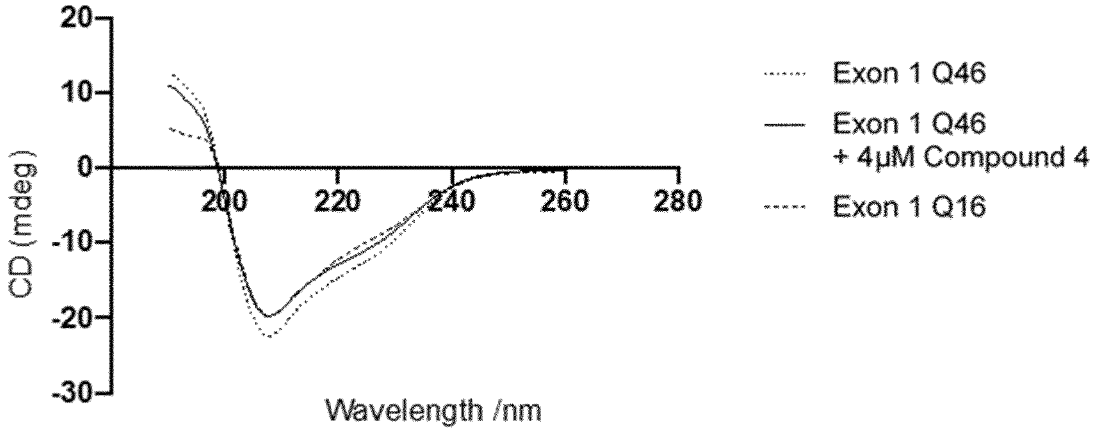


FIGURE 21

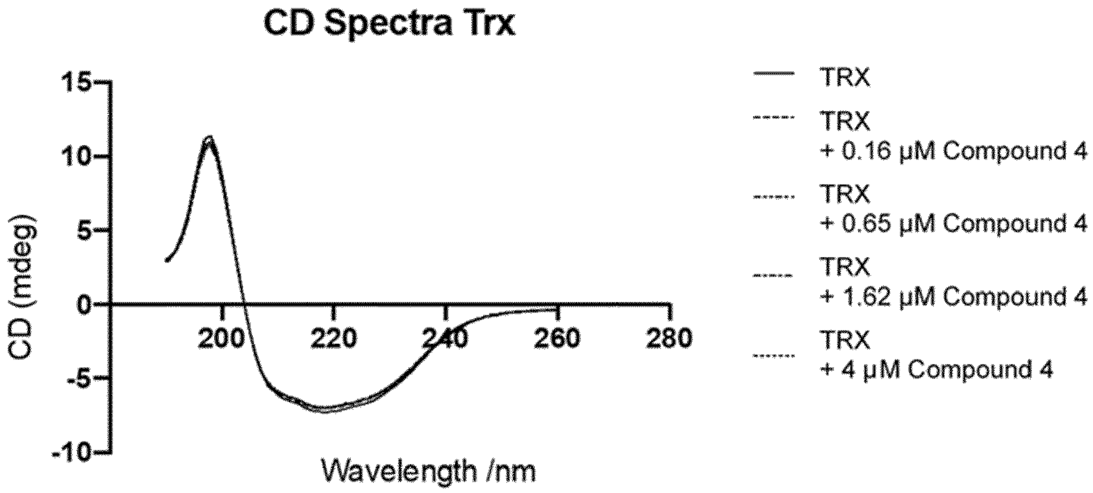


FIGURE 22

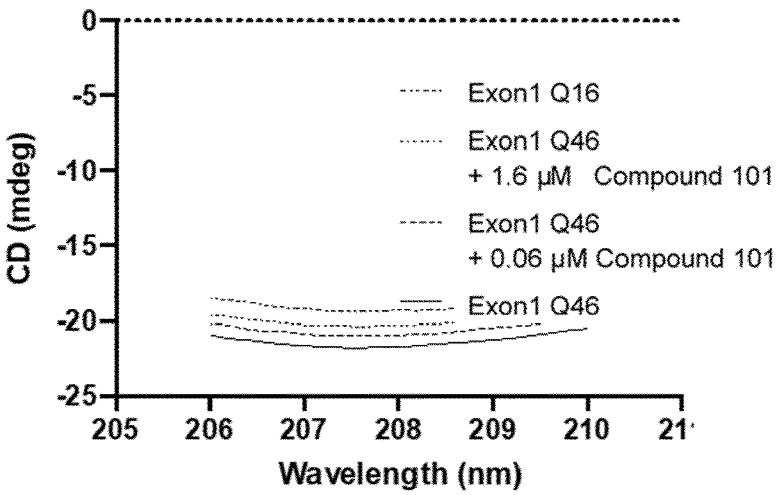

FIGURE 23

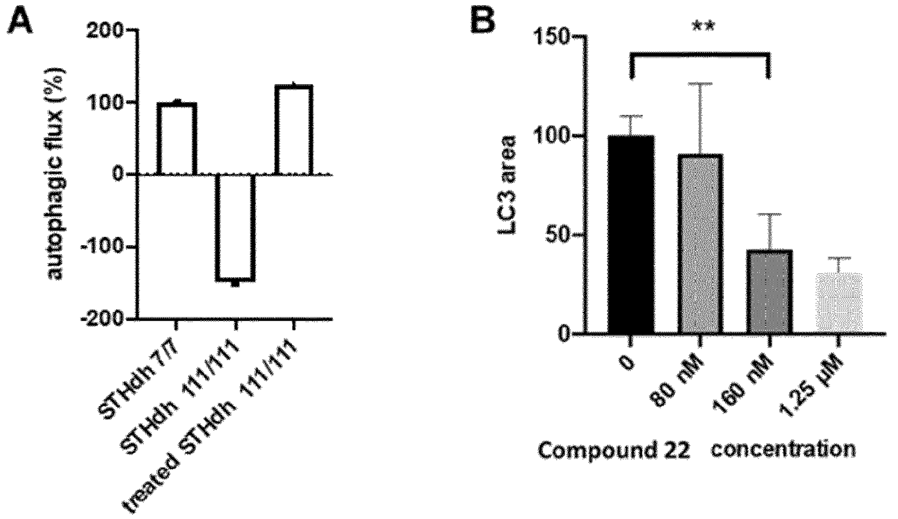


FIGURE 24

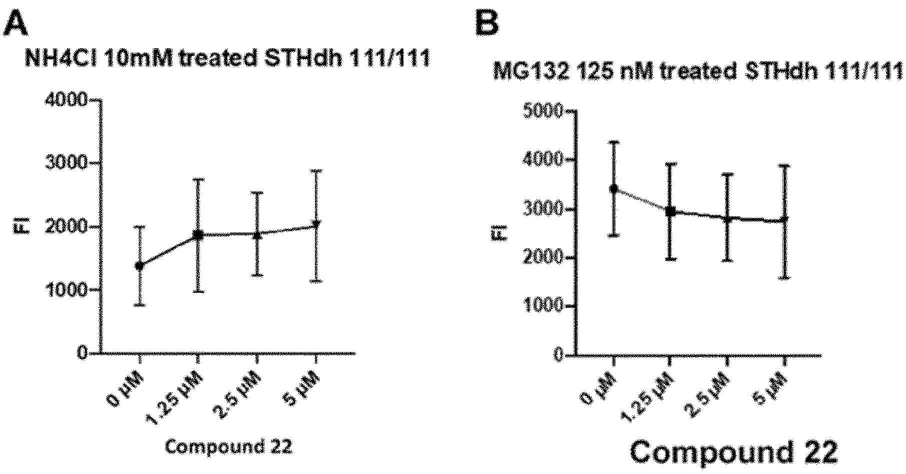


FIGURE 25 A and B

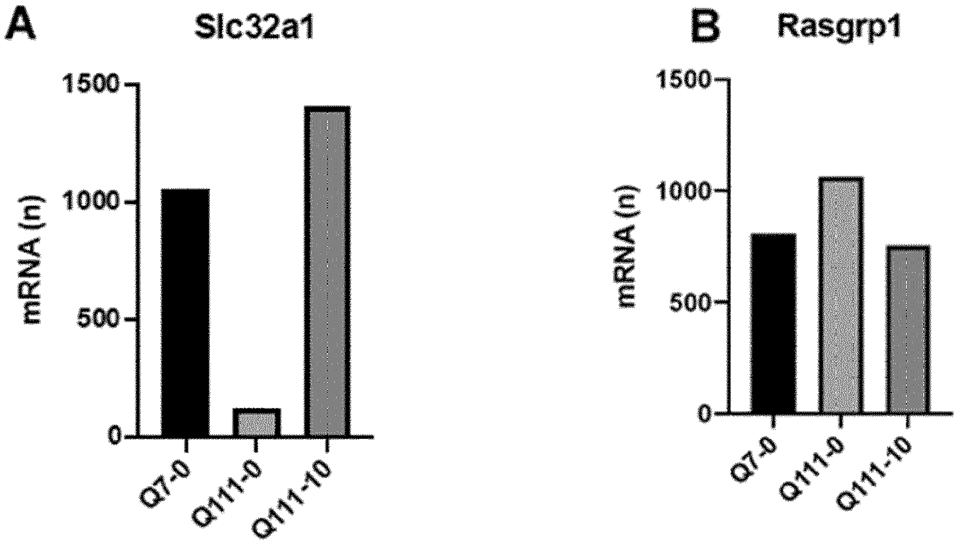

FIGURE 25 continued

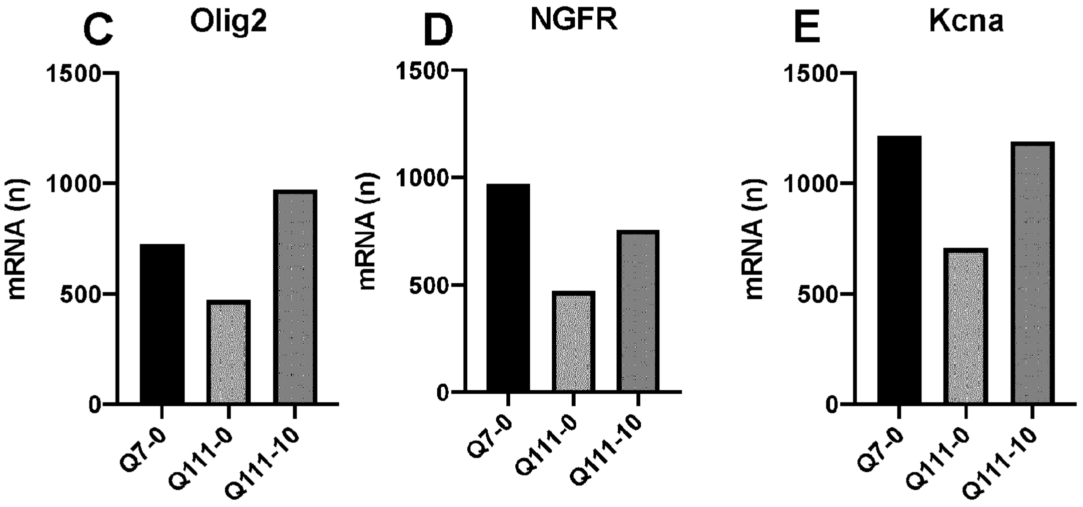


FIGURE 26

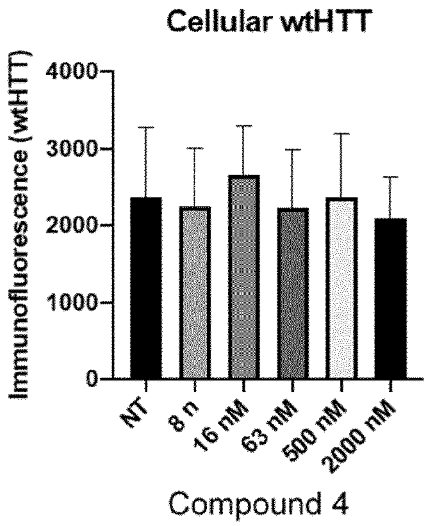


FIGURE 27

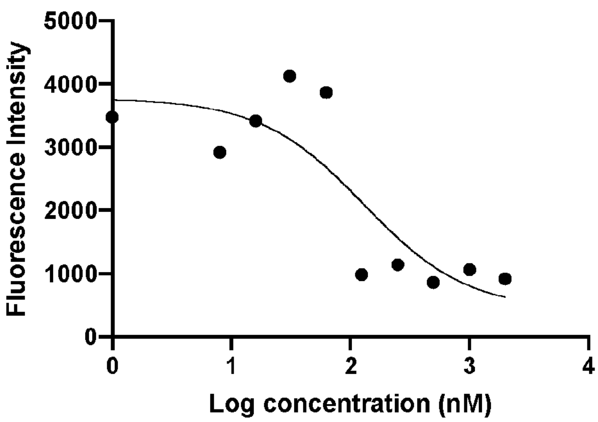

FIGURE 28

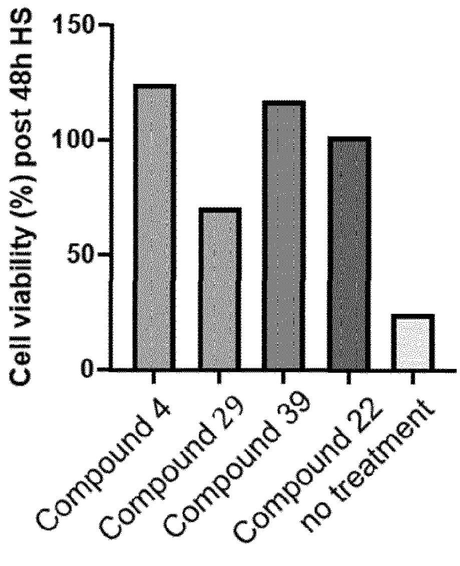


FIGURE 29

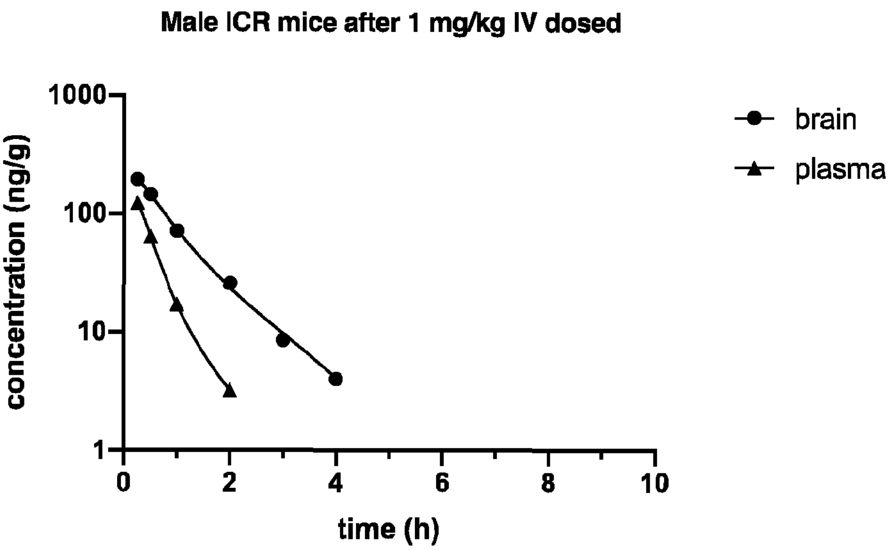


FIGURE 30

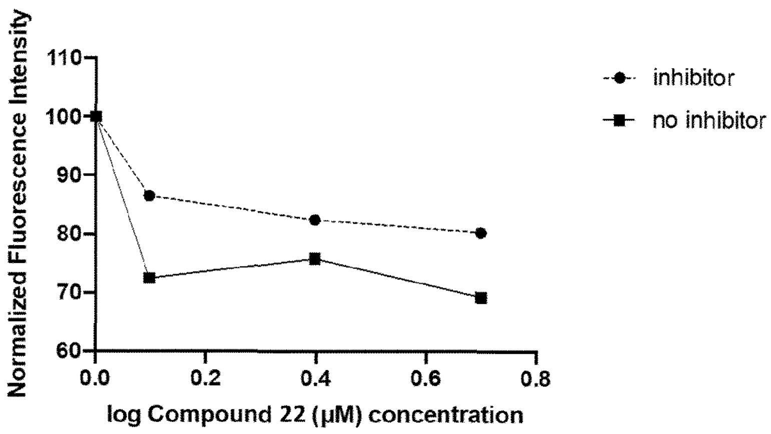

FIGURE 31

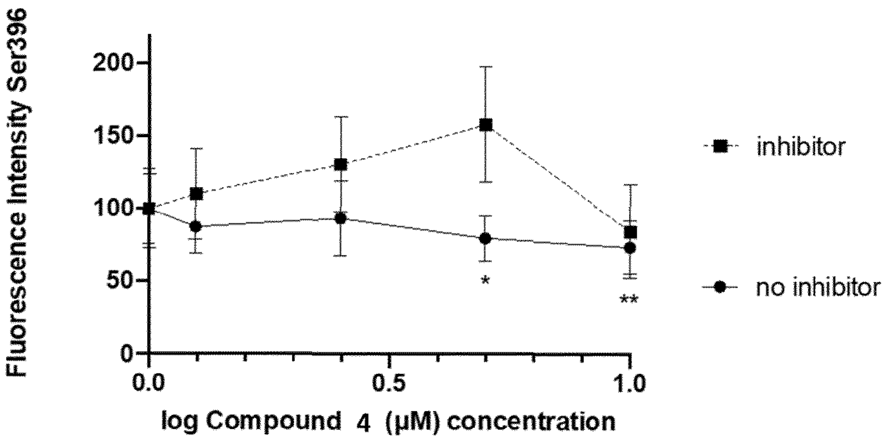


FIGURE 32

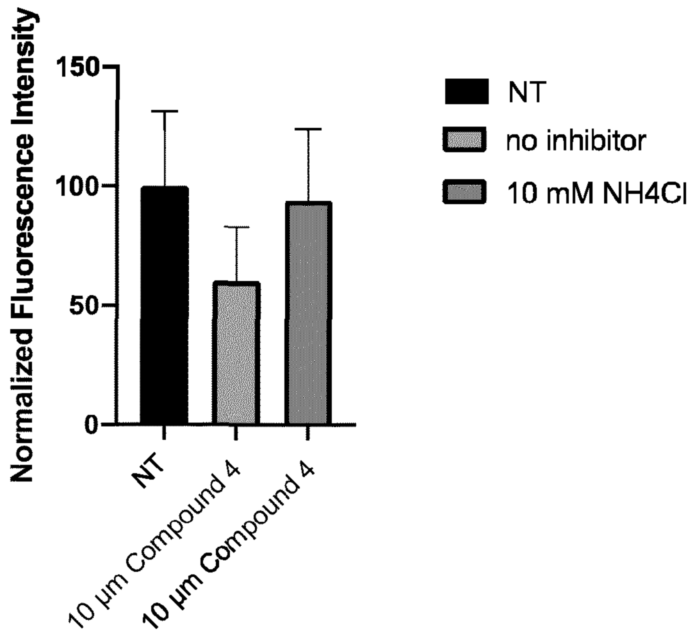


FIGURE 33

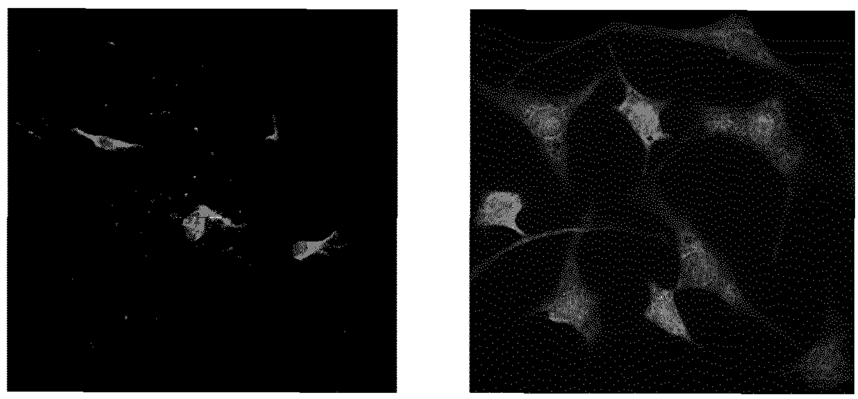

FIGURE 34

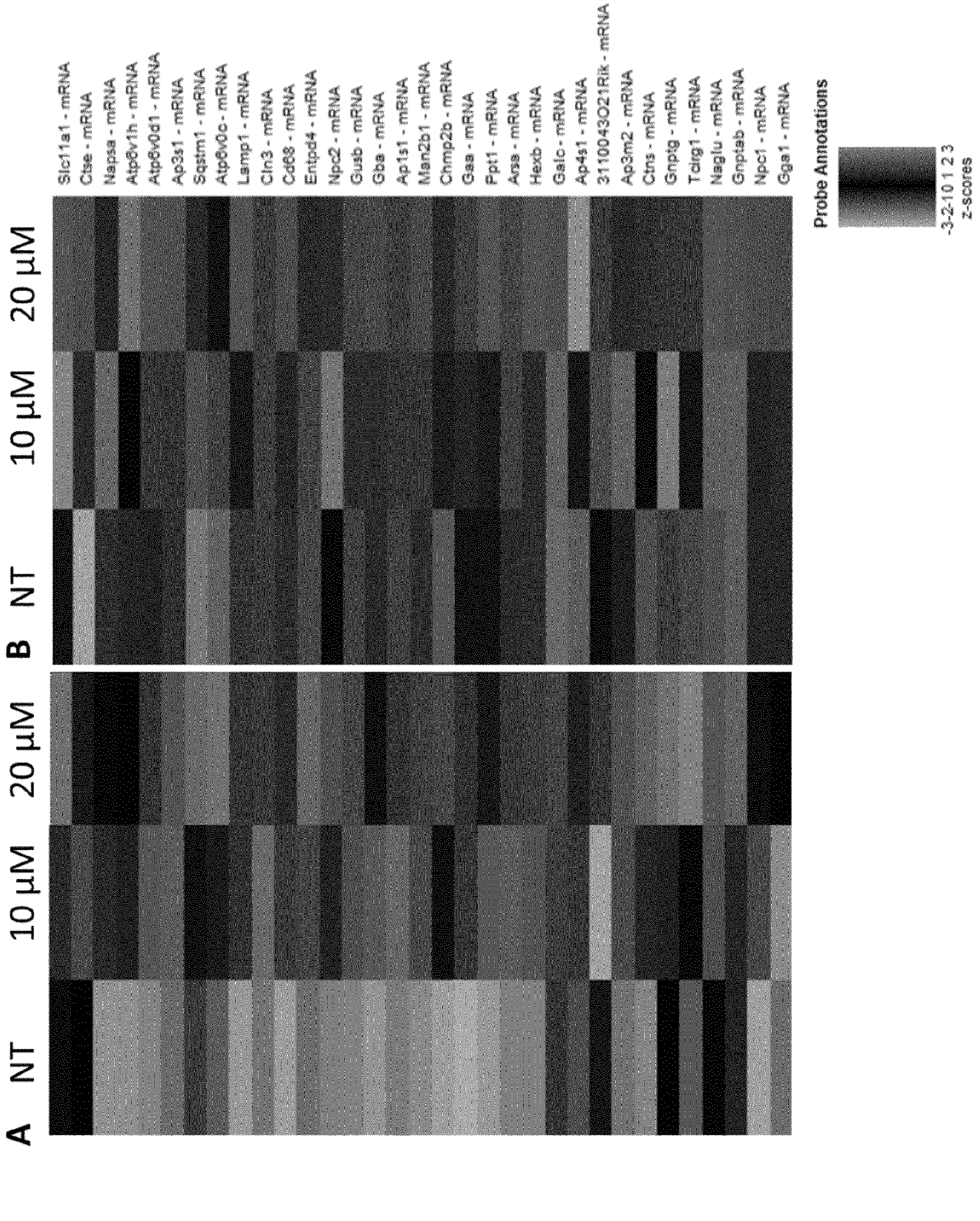
A
NT
10 µM
20 µM
B
NT
10 µM
20 µM
Slc11a1 - mRNA
Ctse - mRNA
Napsa - mRNA
Atp6v1h - mRNA
Atp6v0d1 - mRNA
Ap3s1 - mRNA
Sqstm1 - mRNA
Atp6v0c - mRNA
Lamp1 - mRNA
Cln3 - mRNA
Cd68 - mRNA
Entpd4 - mRNA
Npc2 - mRNA
Gusb - mRNA
Gba - mRNA
Ap1s1 - mRNA
Man2b1 - mRNA
Chmp2b - mRNA
Gaa - mRNA
Ppt1 - mRNA
Arsa - mRNA
Hexb - mRNA
Galc - mRNA
Ap4s1 - mRNA
3110043O21Rik - mRNA
Ap3m2 - mRNA
Ctns - mRNA
Gnptg - mRNA
Tcirg1 - mRNA
Naglu - mRNA
Gnptab - mRNA
Npc1 - mRNA
Gga1 - mRNA
Probe Annotations
-3-2-1 0 1 2 3
z-scores

FIGURE 35

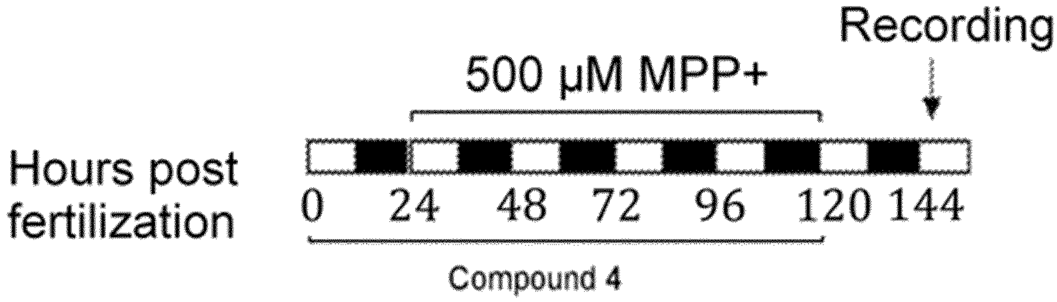


FIGURE 36

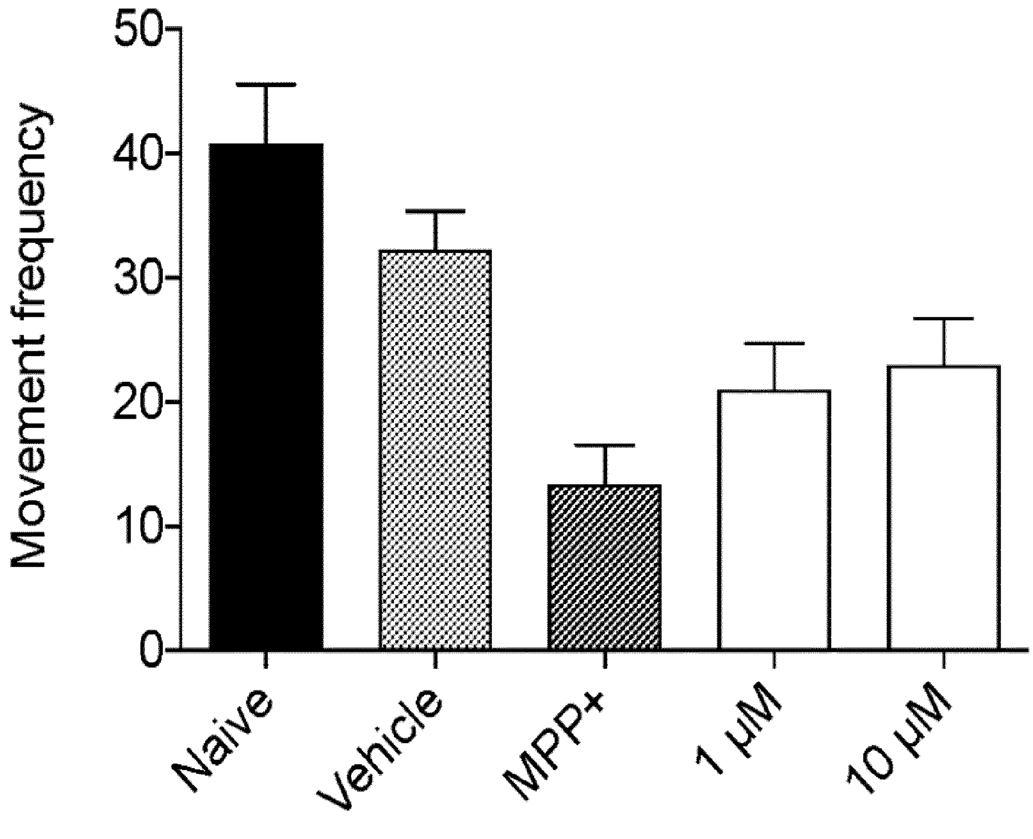

FIGURE 37

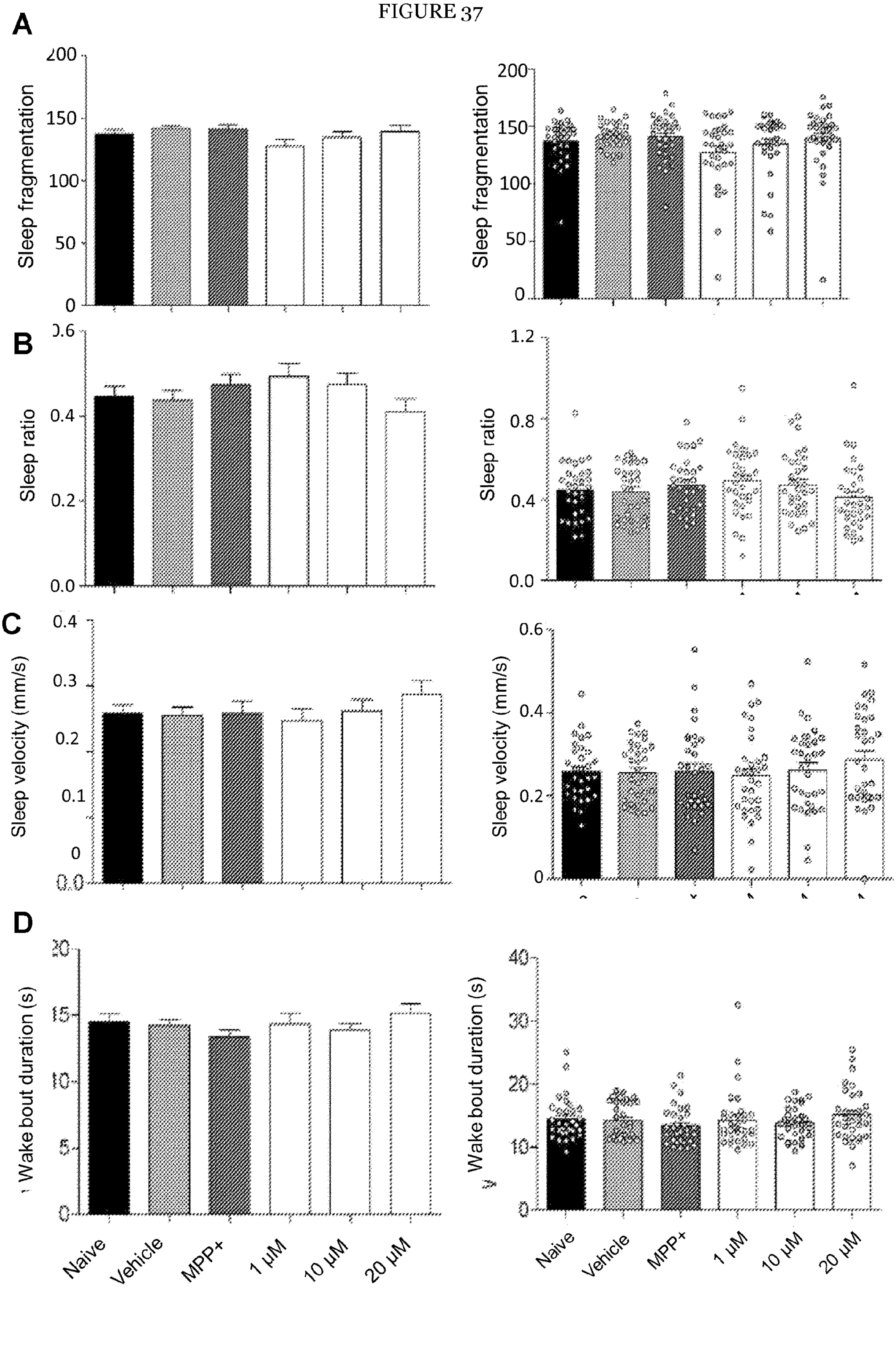
A
Sleep fragmentation
200
150
100
50
0
B
Sleep ratio
0.6
0.4
0.2
0.0
1.2
0.8
C
Sleep velocity (mm/s)
0.4
0.3
0.2
0.1
0.6
D
Wake bout duration (s)
20
15
10
5
40
30
Naive
Vehicle
MPP+
1 µM
10 µM
20 µM

FIGURE 38

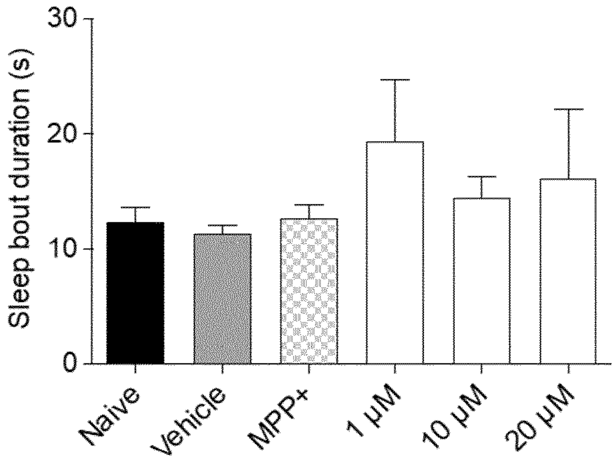


FIGURE 39

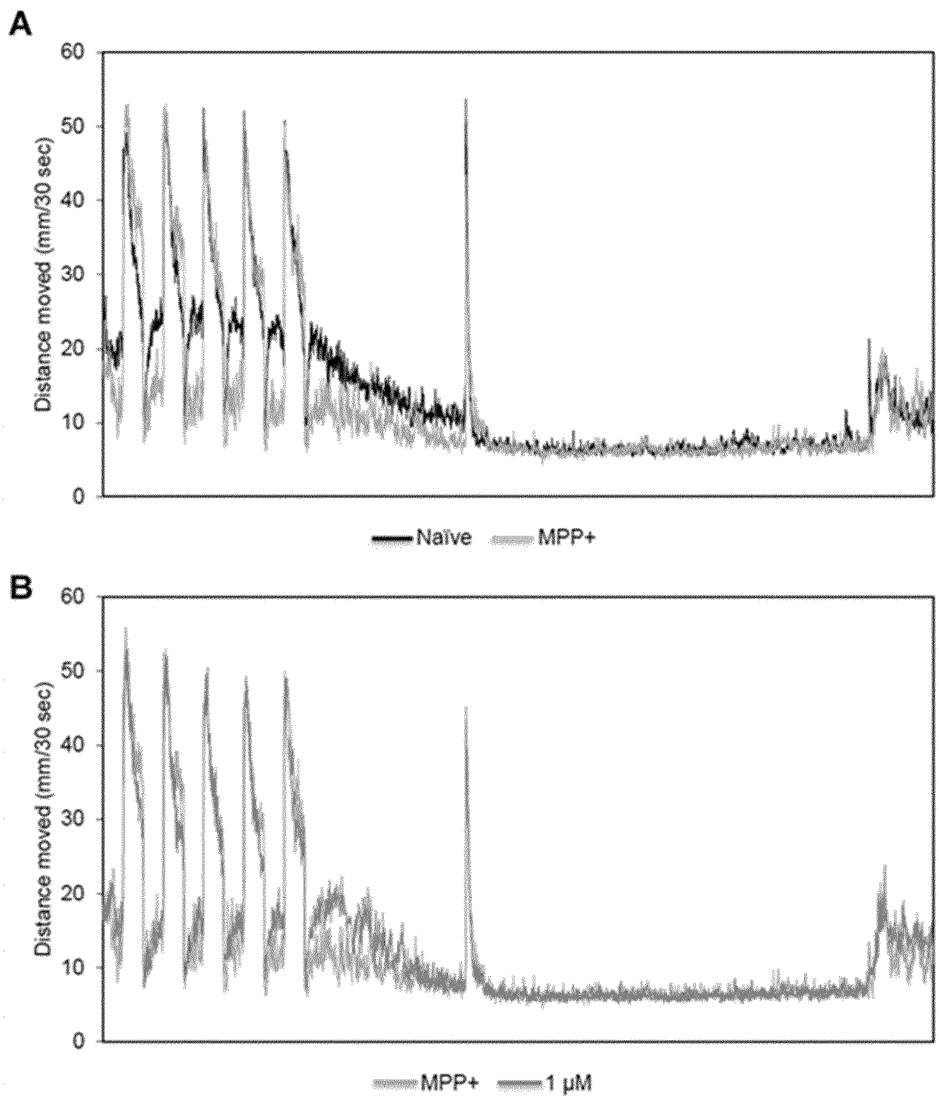

FIGURE 40

A

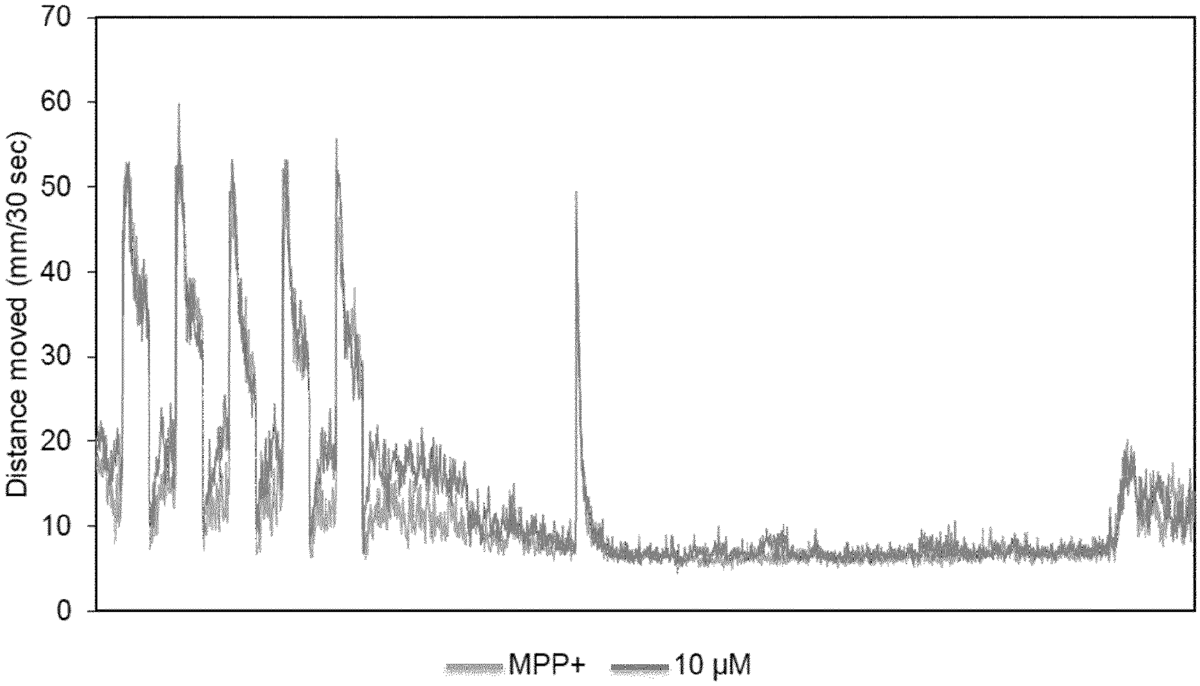
70
60
50
40
30
20
10
0
Distance moved (mm/30 sec)
MPP+
10 µM

B

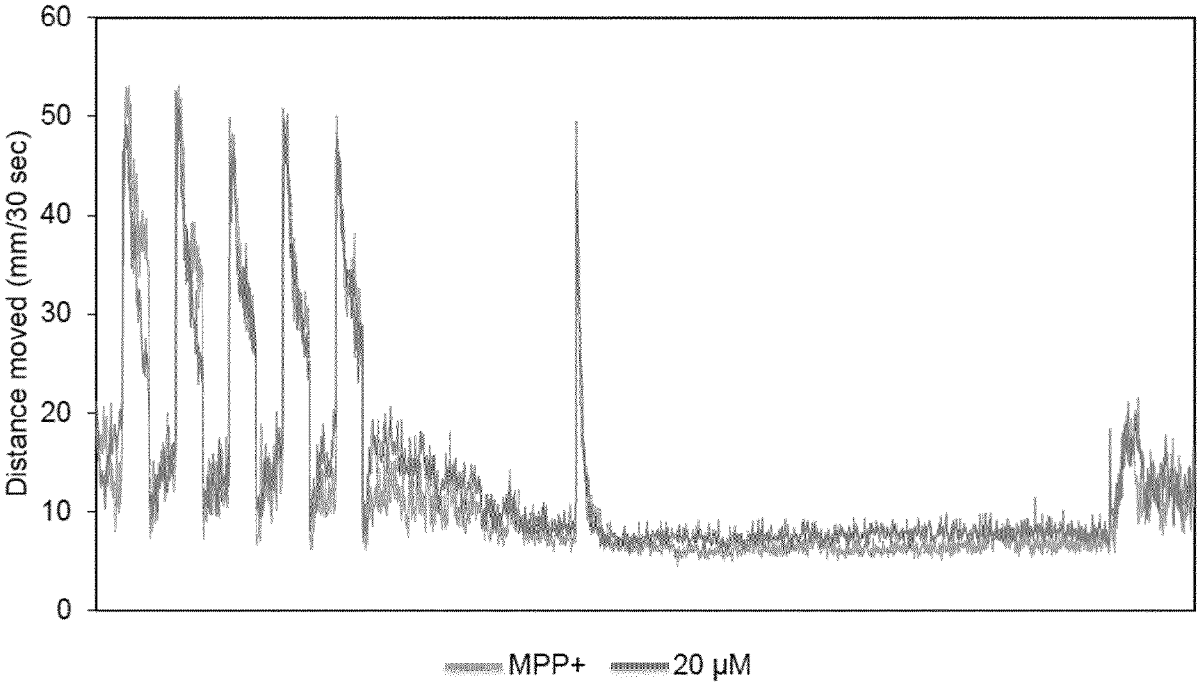
60
50
40
30
20
10
0
Distance moved (mm/30 sec)
MPP+
20 µM

FIGURE 41

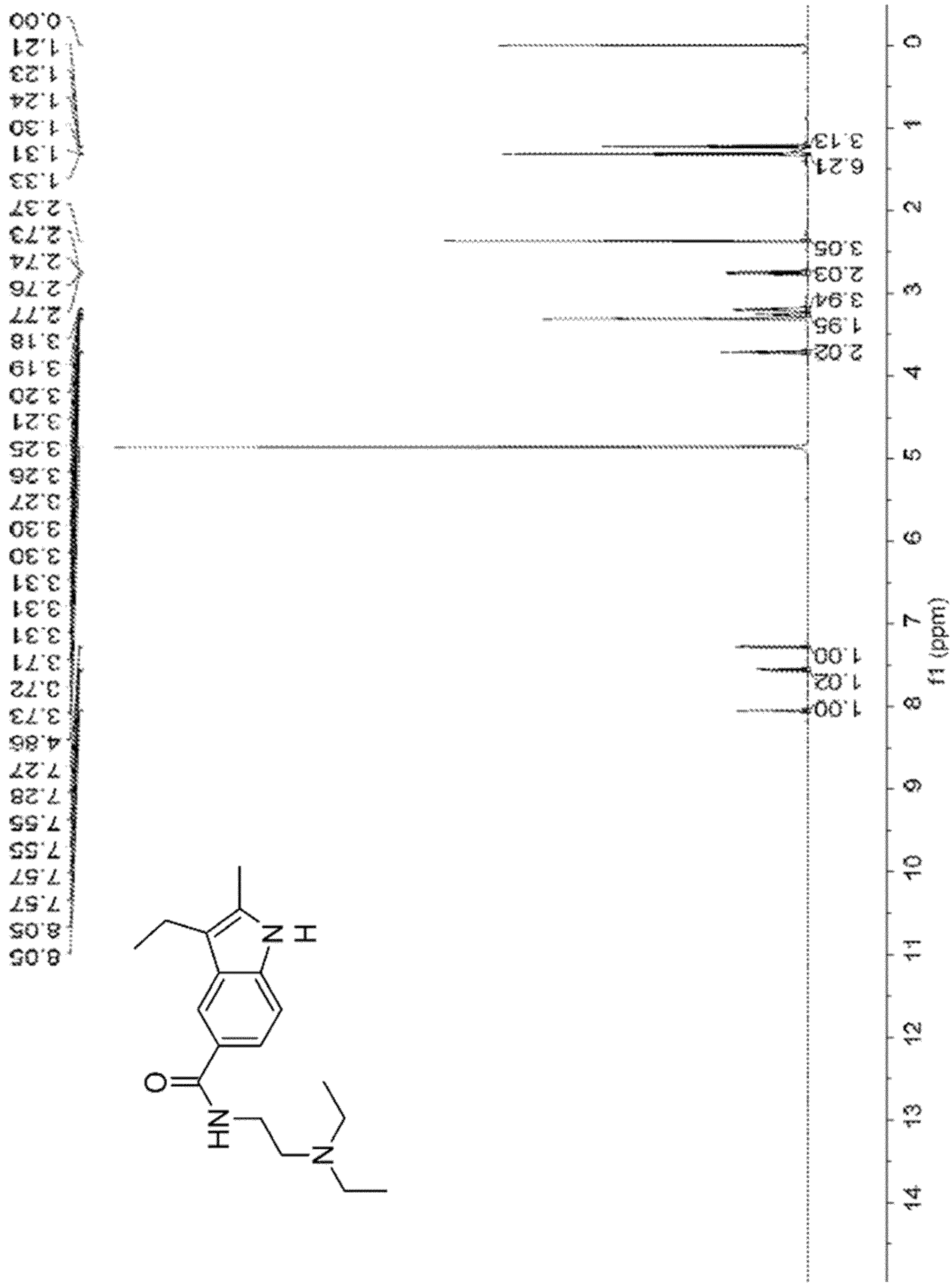
0.00
1.21
1.23
1.24
1.30
1.31
1.33
2.37
2.73
2.74
2.76
2.77
3.18
3.19
3.20
3.21
3.25
3.26
3.27
3.30
3.30
3.31
3.31
3.31
3.71
3.72
3.73
4.86
7.27
7.28
7.55
7.55
7.57
7.57
8.05
8.05
3.13
6.21
3.05
2.03
3.94
1.95
2.02
1.00
1.02
1.00
0
1
2
3
4
5
6
7
8
9
10
11
12
13
14
f1 (ppm)

FIGURE 42

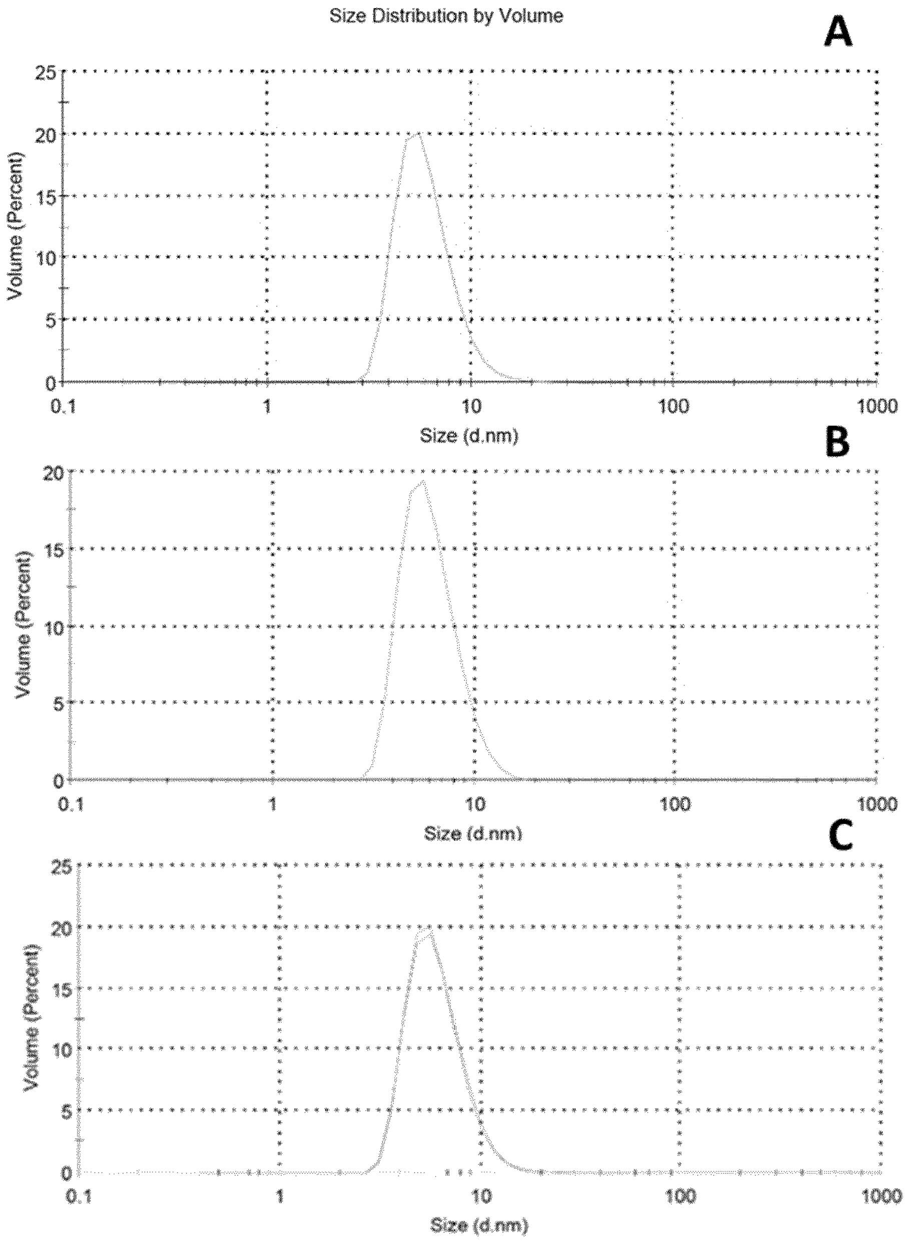
Size Distribution by Volume
A
Volume (Percent)
25
20
15
10
5
0
0.1
1
10
100
1000
Size (d.nm)
B
Volume (Percent)
20
15
10
5
0
0.1
1
10
100
1000
Size (d.nm)
C
Volume (Percent)
25
20
15
10
5
0
0.1
1
10
100
1000
Size (d.nm)

FIGURE 43

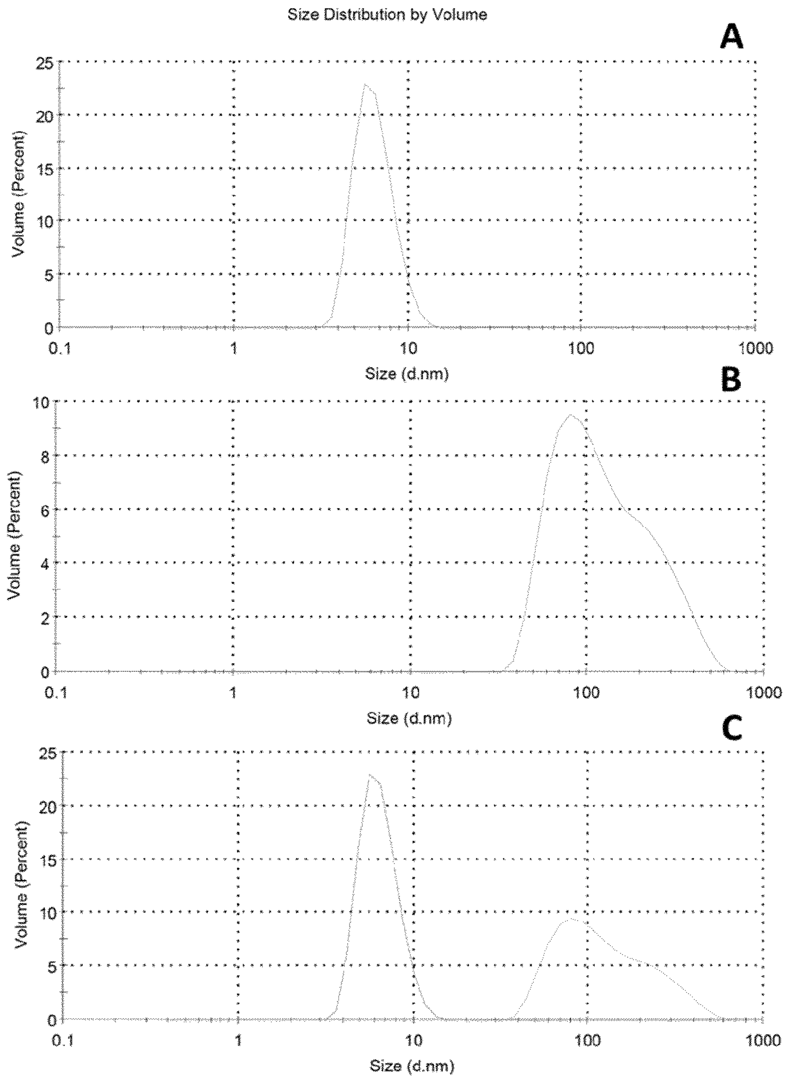


FIGURE 44

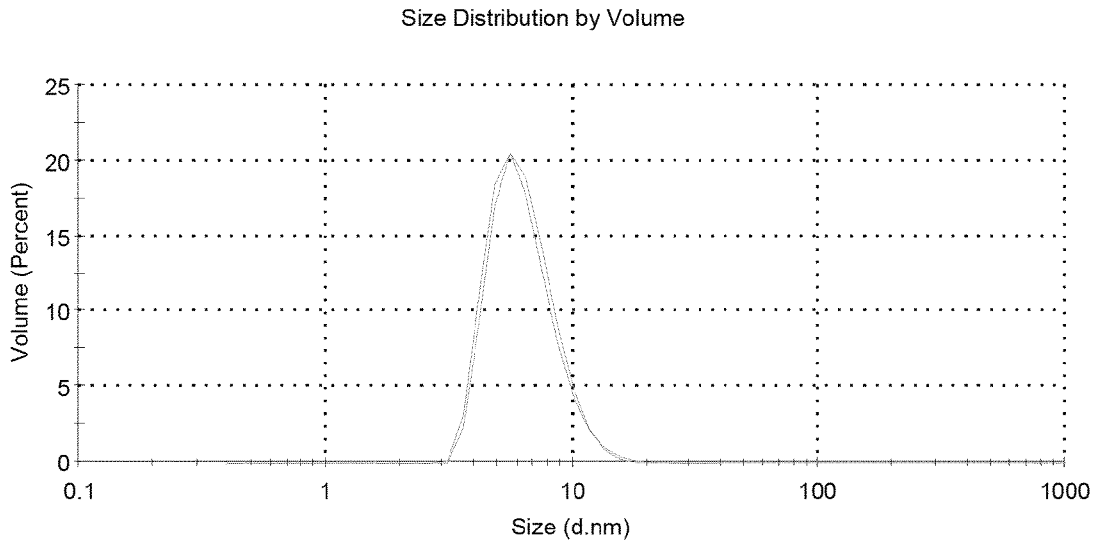

FIGURE 45

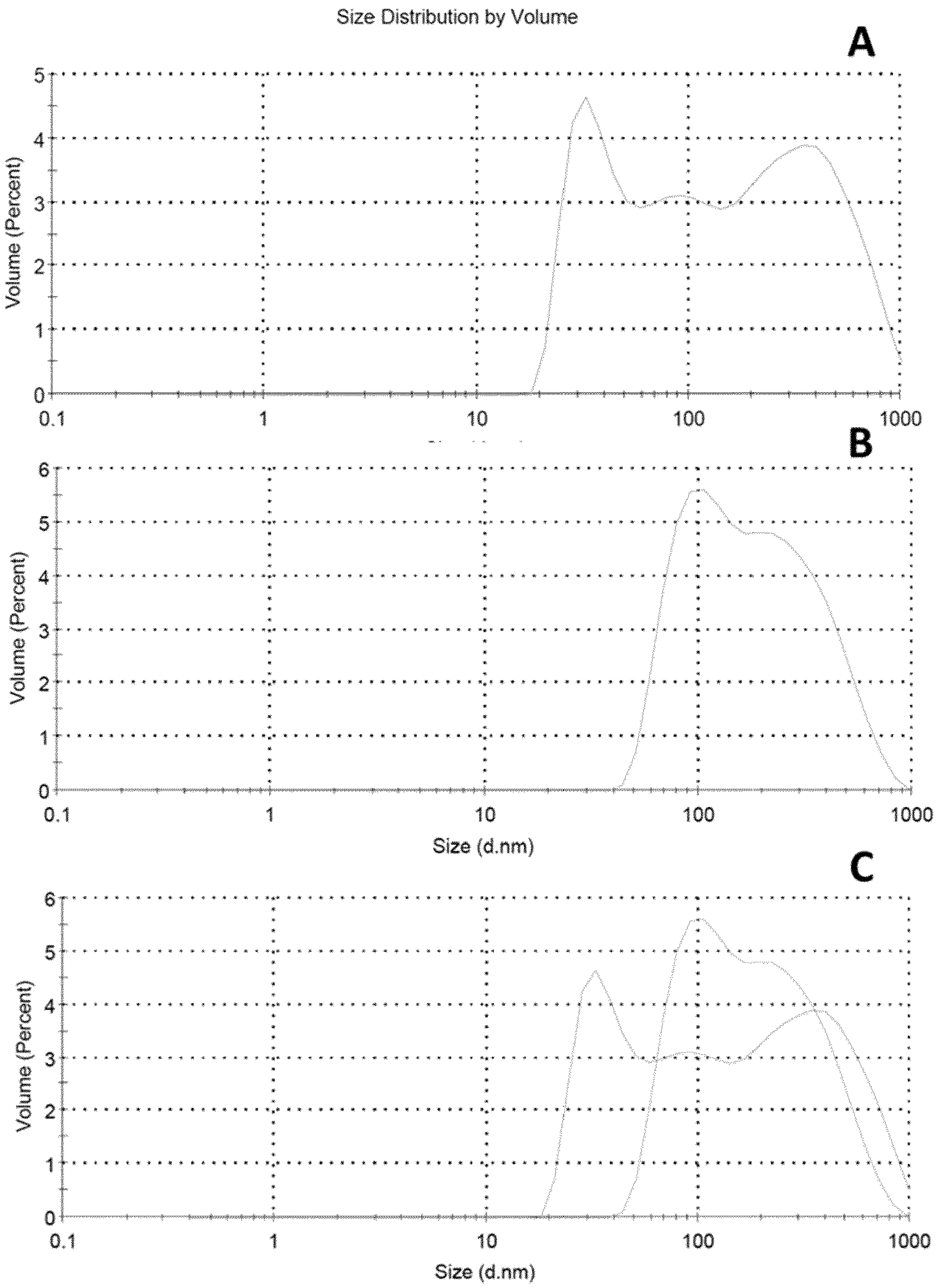
Size Distribution by Volume
A
Volume (Percent)
5
4
3
2
1
0
0.1
1
10
100
1000
B
Volume (Percent)
6
5
4
3
2
1
0
0.1
1
10
100
1000
Size (d.nm)
C
Volume (Percent)
6
5
4
3
2
1
0
0.1
1
10
100
1000
Size (d.nm)

FIGURE 46

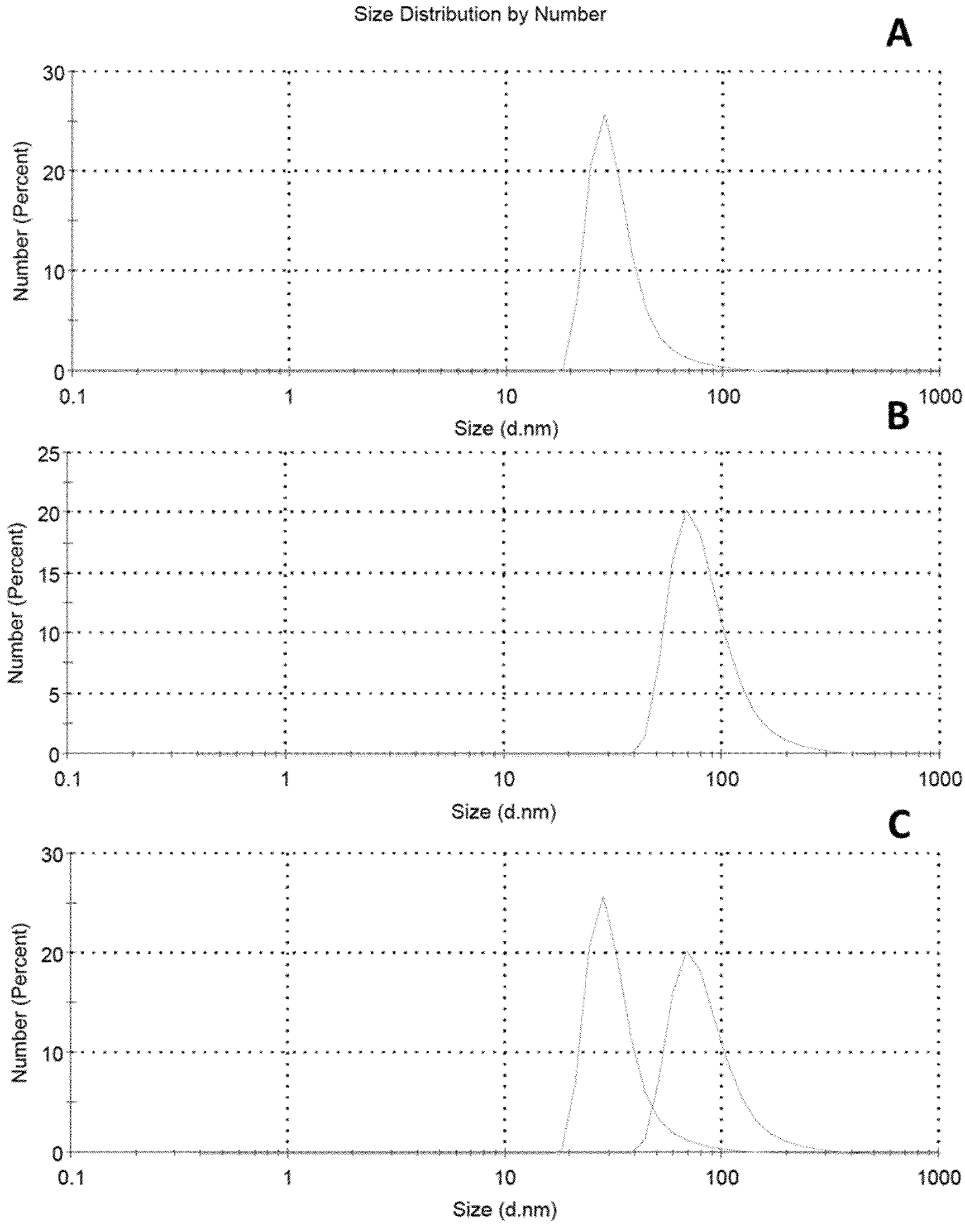
Size Distribution by Number
A
Number (Percent)
0
10
20
30
0.1
1
10
100
1000
Size (d.nm)
B
Number (Percent)
0
5
10
15
20
25
0.1
1
10
100
1000
Size (d.nm)
C
Number (Percent)
0
10
20
30
0.1
1
10
100
1000
Size (d.nm)

FIGURE 47

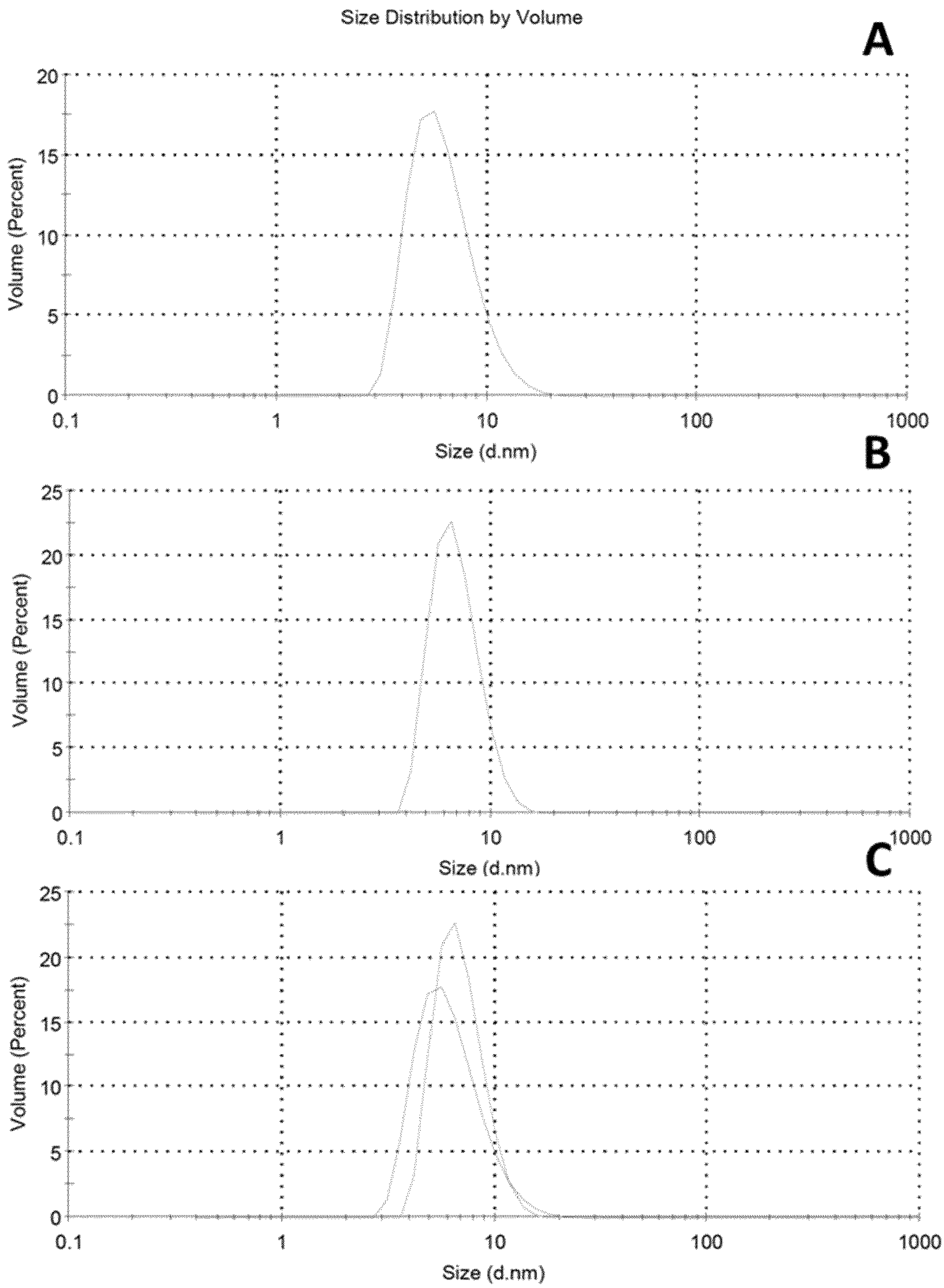
Size Distribution by Volume
A
Volume (Percent)
20
15
10
5
0
0.1
1
10
100
1000
Size (d.nm)
B
25
20
15
10
5
0
Volume (Percent)
0.1
1
10
100
1000
Size (d.nm)
C
25
20
15
10
5
0
Volume (Percent)
0.1
1
10
100
1000
Size (d.nm)

FIGURE 48

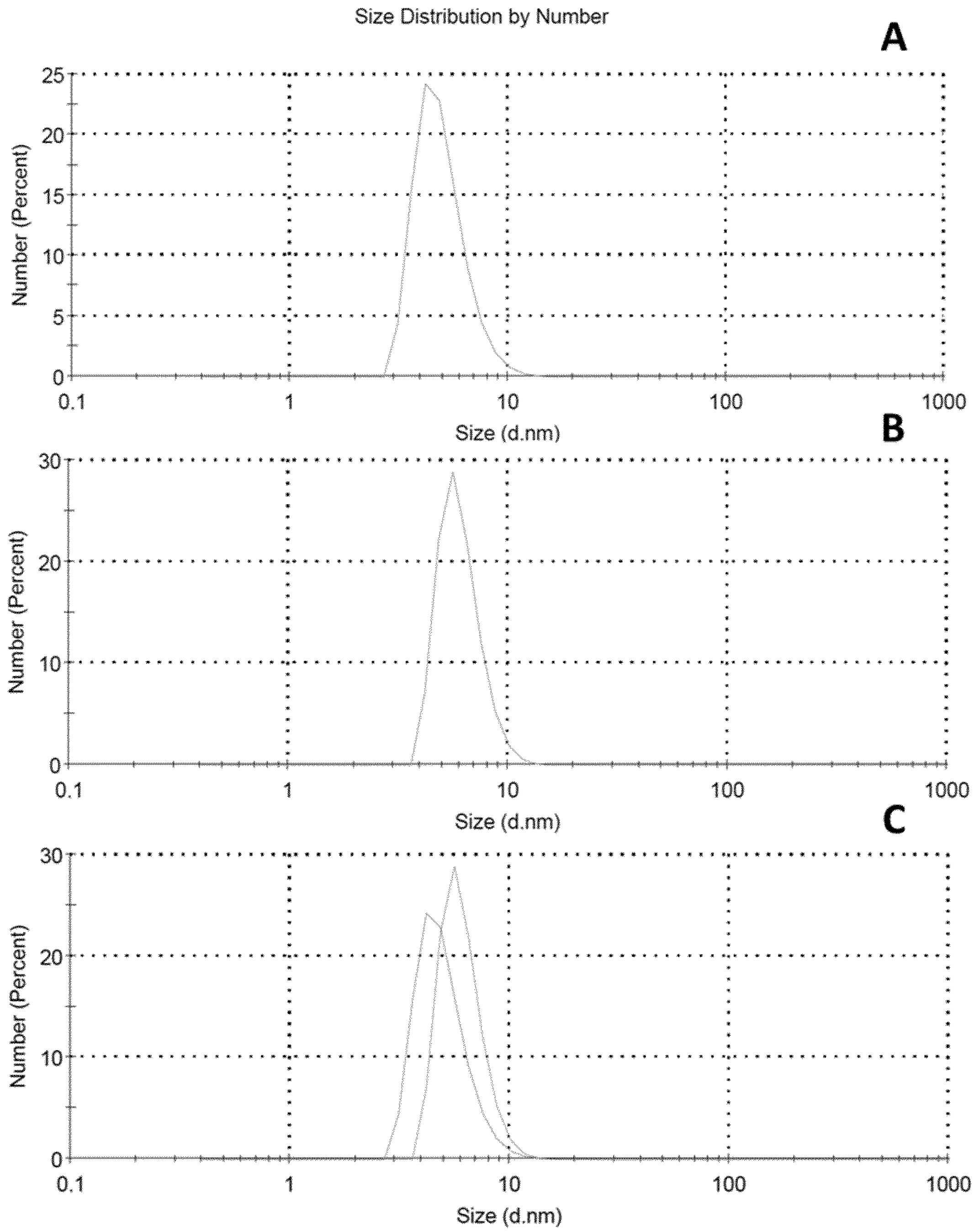
Size Distribution by Number
A
Number (Percent)
Size (d.nm)
B
Number (Percent)
Size (d.nm)
C
Number (Percent)
Size (d.nm)

FIGURE 49

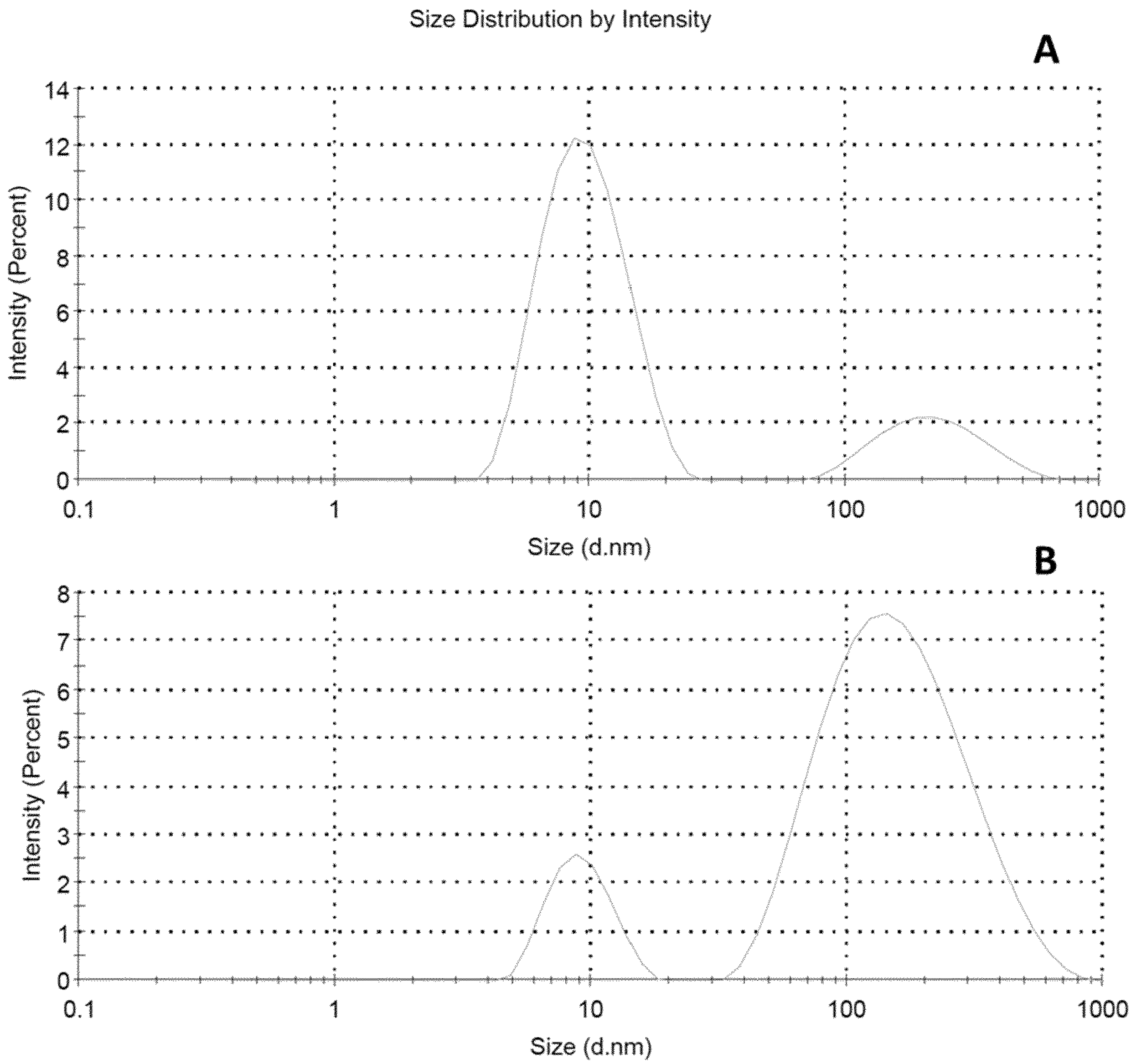
Size Distribution by Intensity
A
14
12
10
8
6
4
2
0
Intensity (Percent)
0.1
1
10
100
1000
Size (d.nm)
B
8
7
6
5
4
3
2
1
0
Intensity (Percent)
0.1
1
10
100
1000
Size (d.nm)

FIGURE 50

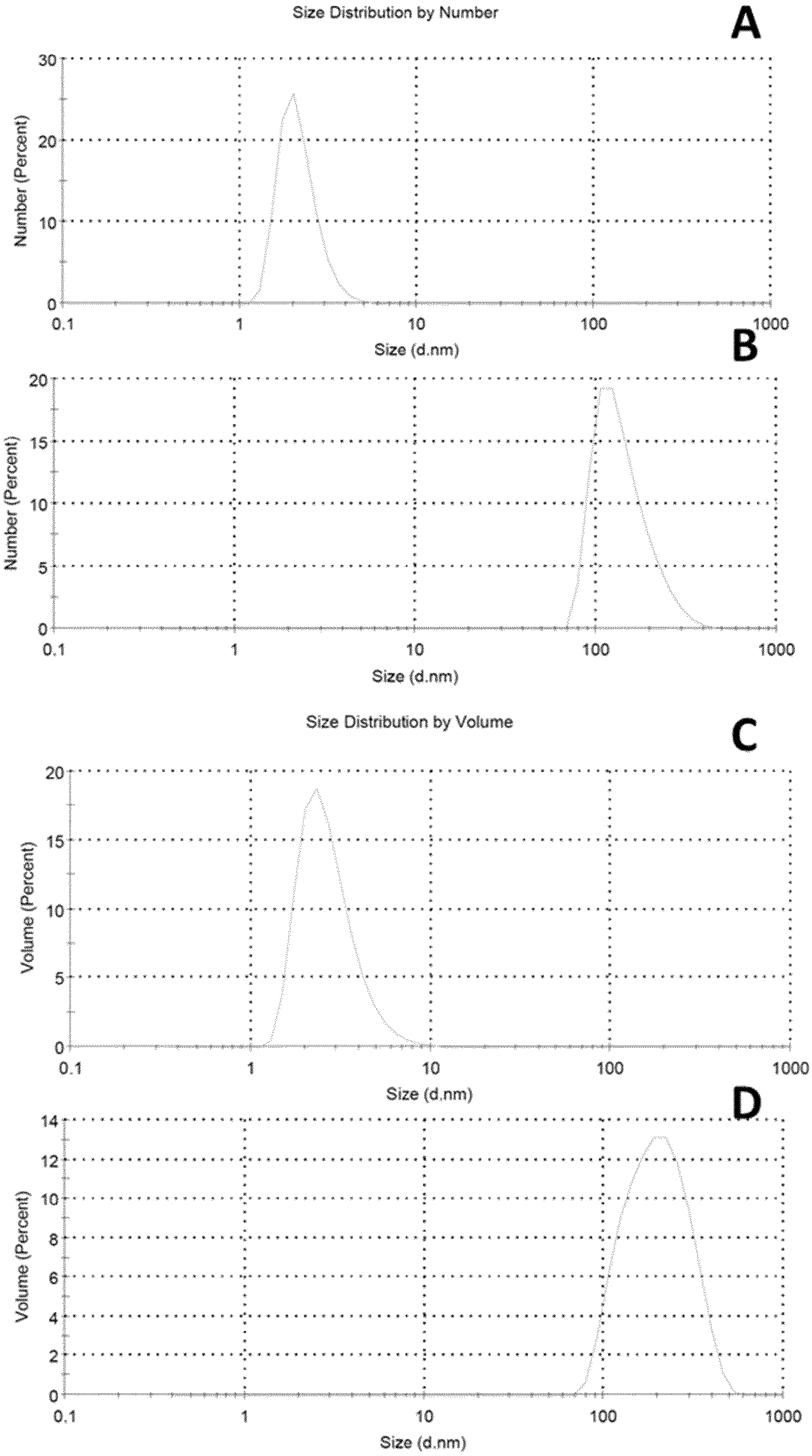
Size Distribution by Number
A
Number (Percent)
30
20
10
0
0.1
1
10
100
1000
Size (d.nm)
B
Number (Percent)
20
15
10
5
0
0.1
1
10
100
1000
Size (d.nm)
Size Distribution by Volume
C
Volume (Percent)
20
15
10
5
0
0.1
1
10
100
1000
Size (d.nm)
D
Volume (Percent)
14
12
10
8
6
4
2
0
0.1
1
10
100
1000
Size (d.nm)

FIGURE 51

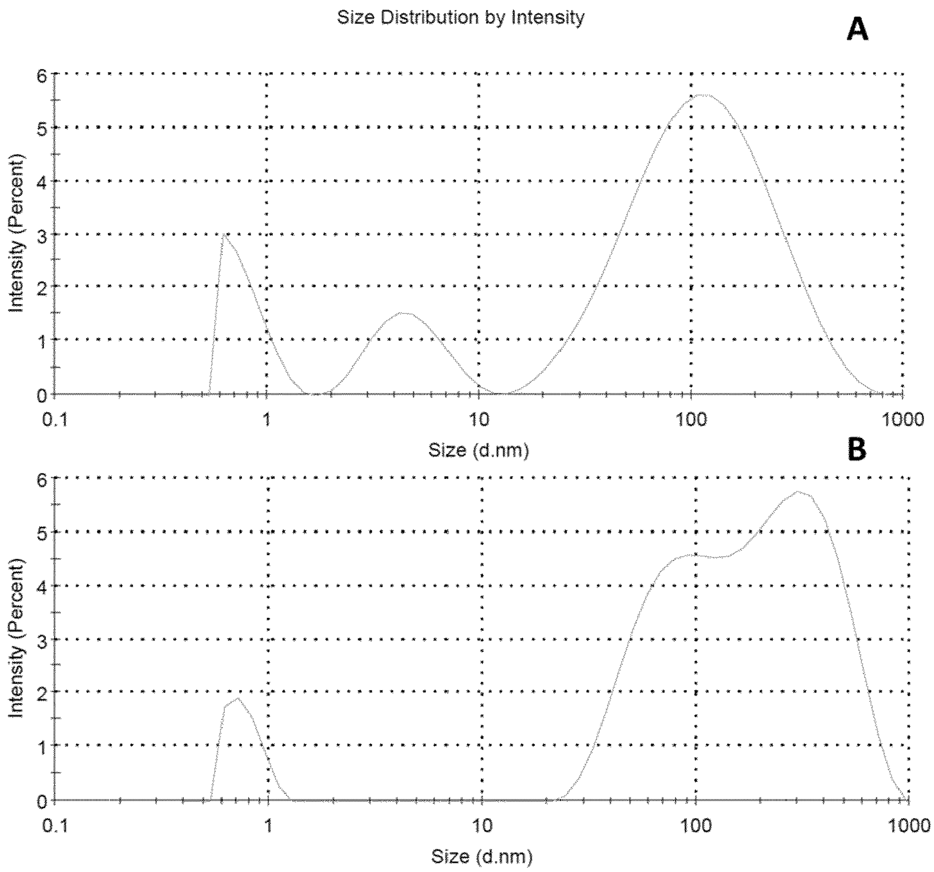


FIGURE 52 A

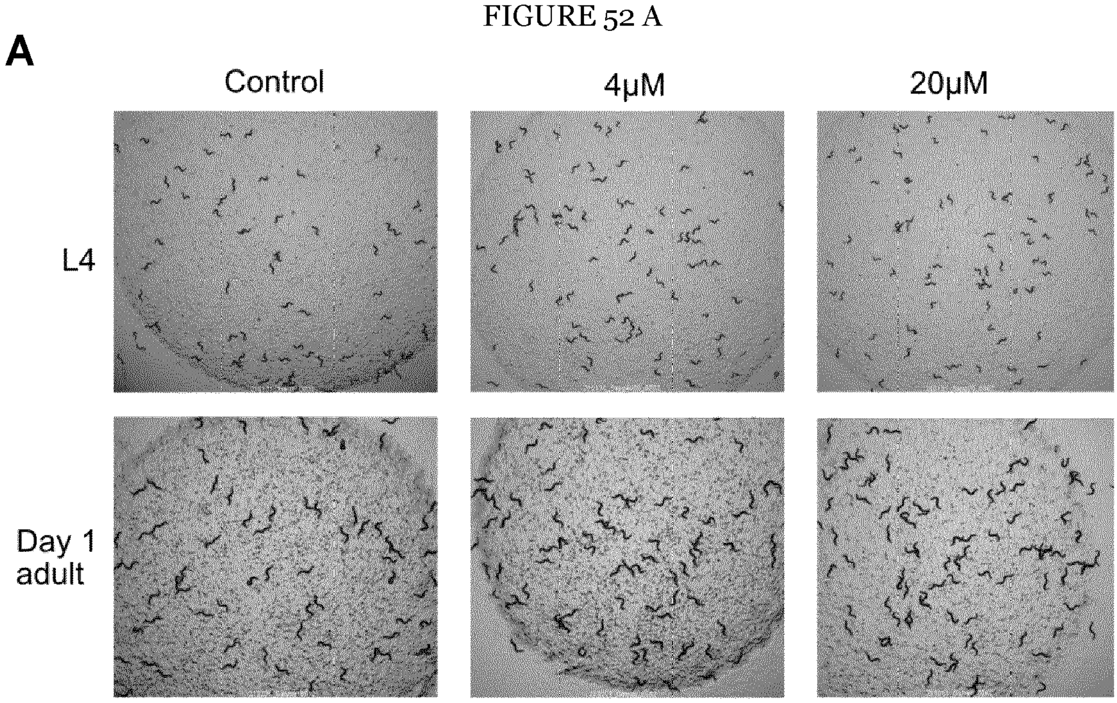

FIGURE 52 continued

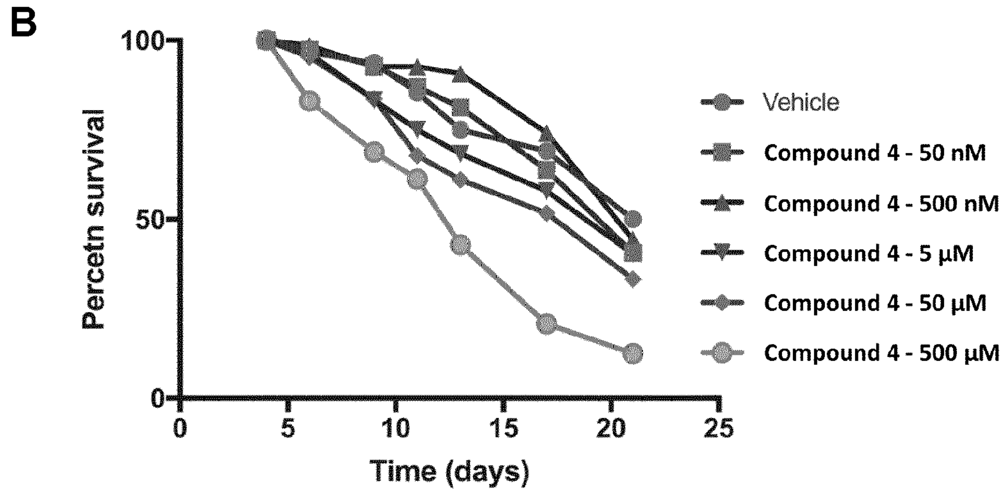


FIGURE 53

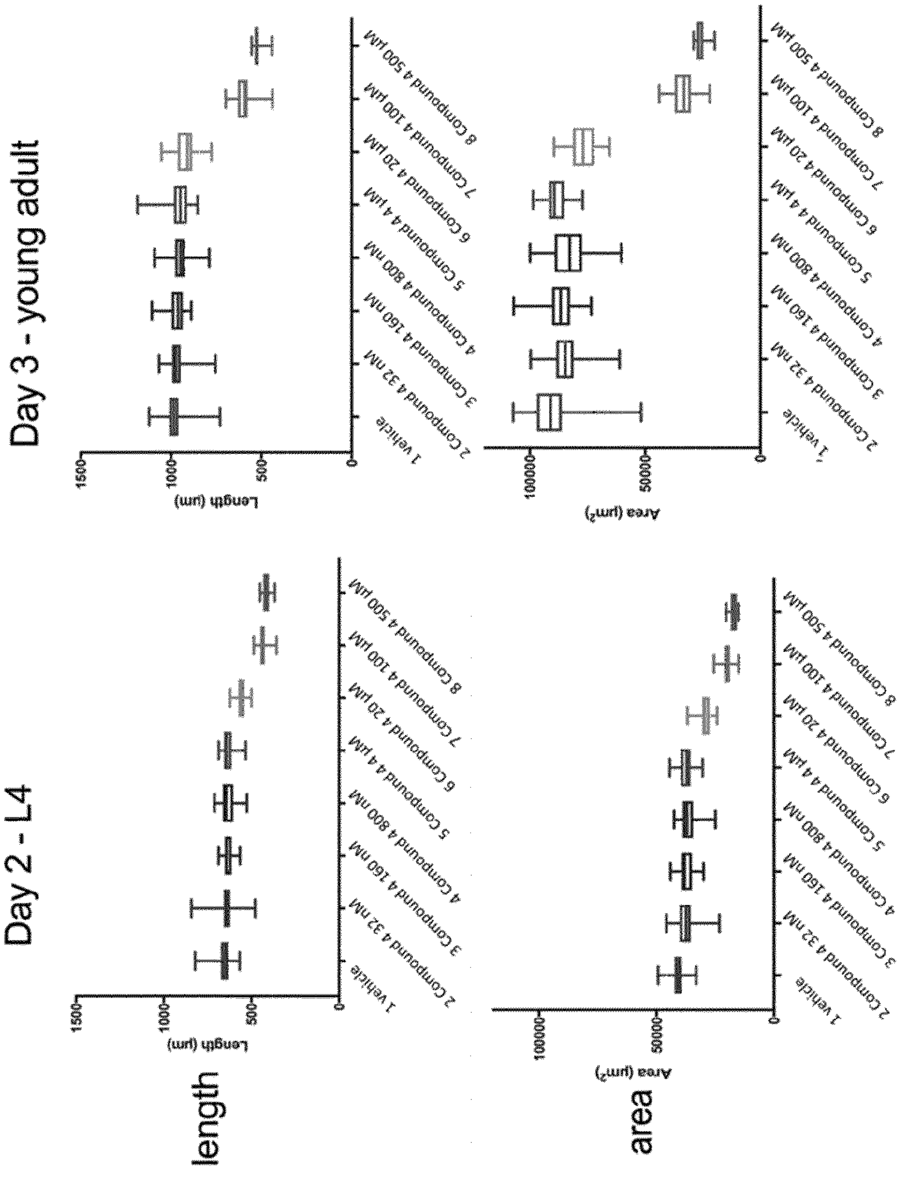

FIGURE 54

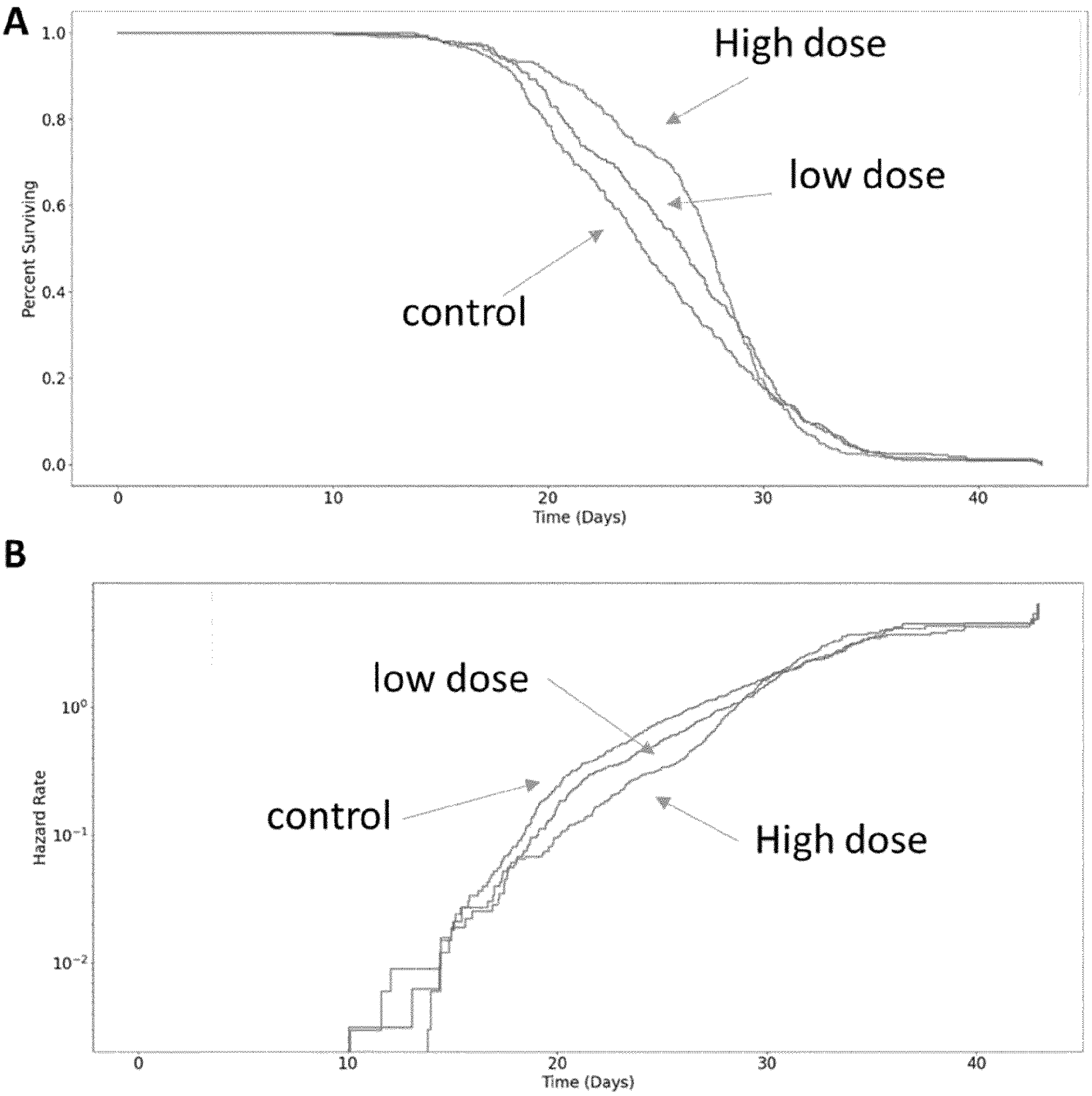
A
High dose
low dose
control
Percent Surviving
1.0
0.8
0.6
0.4
0.2
0.0
0
10
20
30
40
Time (Days)
B
low dose
control
High dose
Hazard Rate
$10^0$
$10^{-1}$
$10^{-2}$
0
10
20
30
40
Time (Days)

FIGURE 55

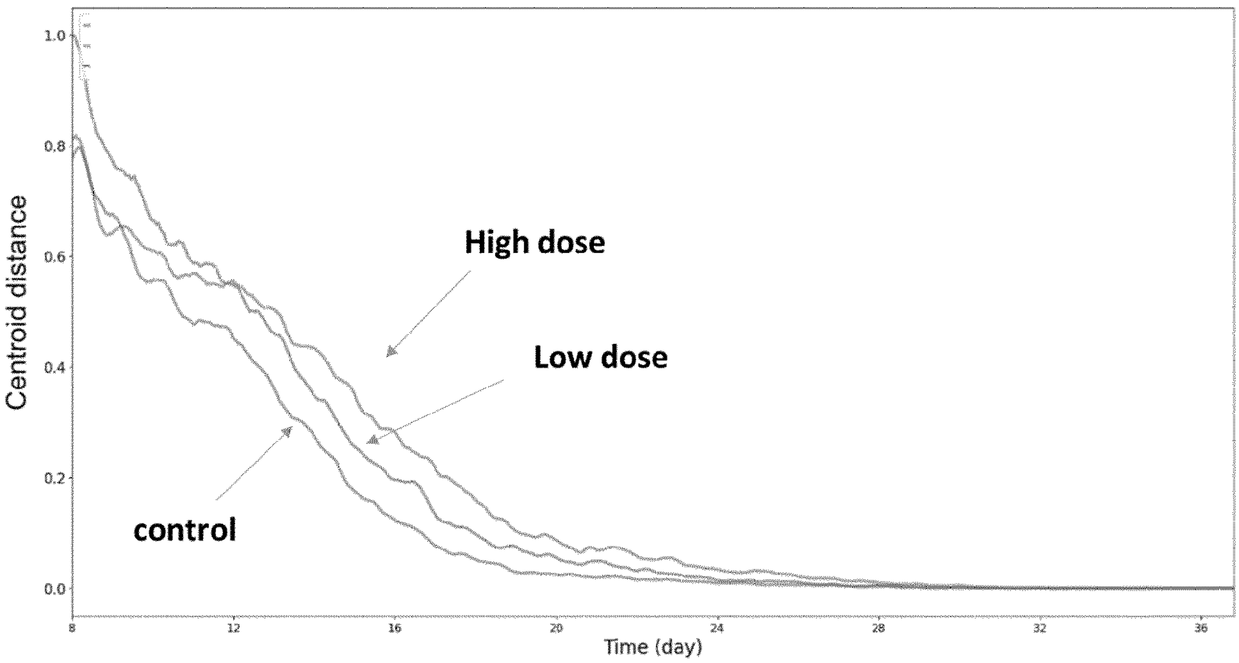


FIGURE 56

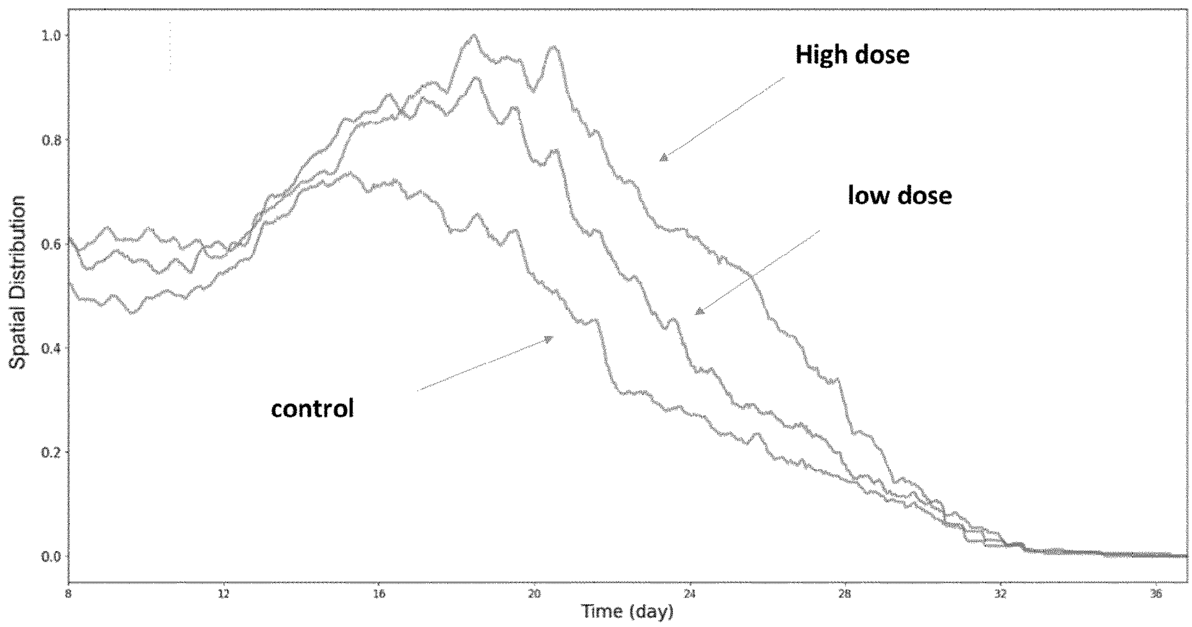

Europäisches Patentamt
European Patent Office
Office européen des brevets

# EUROPEAN SEARCH REPORT

Application Number
EP 21 19 3388

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/024819 A1 (ASTRAZENECA AB [SE]; ASTRAZENECA UK LTD [GB] ET AL.) 26 February 2009 (2009-02-26) * Formula (I); claim 21; examples 10-12, 15-16 * ----- | 1-15 | INV. A61K31/404 A61K31/4045 A61K31/437 A61K31/454 A61K31/496 A61P25/28 A61K31/519 A61K31/5377 C07D209/42 C07D295/13 C07D401/12 C07D403/06 C07D413/12 |
| A | WO 2021/007378 A1 (PTC THERAPEUTICS INC [US]) 14 January 2021 (2021-01-14) * the whole document * ----- | 1-15 | |
| E | WO 2021/173593 A1 (GALYAN BIO INC [US]) 2 September 2021 (2021-09-02) * the whole document * ----- | 1-14 | |
| X | DATABASE REGISTRY [Online] CHEMICAL ABSTRACTS SERVICE, COLUMBUS, OHIO, US; 19 November 2018 (2018-11-19), Anonymous: "Extract from Zreg", XP055884693, Database accession no. 2249586-46-5 * the whole document * ----- | 15 | TECHNICAL FIELDS SEARCHED (IPC) A61P A61K C07D |

2

The present search report has been drawn up for all claims

| Place of search | Date of completion of the search | Examiner |
|---|---|---|
| Munich | 27 January 2022 | Kirsch, Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 21 19 3388

This annex lists the patent family members relating to the patent documents cited in the above-mentioned European search report. The members are as contained in the European Patent Office EDP file on
The European Patent Office is in no way liable for these particulars which are merely given for the purpose of information.

27-01-2022

| Patent document cited in search report | Publication date | Patent family member(s) | Publication date |
|---|---|---|---|
| WO 2009024819 A1 | 26-02-2009 | AR 067954 A1 | 28-10-2009 |
| | | AU 2008290325 A1 | 26-02-2009 |
| | | BR PI0815493 A2 | 10-02-2015 |
| | | CA 2696697 A1 | 26-02-2009 |
| | | CL 2008002431 A1 | 05-06-2009 |
| | | CN 101827838 A | 08-09-2010 |
| | | EP 2190838 A1 | 02-06-2010 |
| | | JP 2010536737 A | 02-12-2010 |
| | | KR 20100061491 A | 07-06-2010 |
| | | PE 20090598 A1 | 10-06-2009 |
| | | RU 2010102992 A | 27-09-2011 |
| | | TW 200908963 A | 01-03-2009 |
| | | US 2009062251 A1 | 05-03-2009 |
| | | US 2011160180 A1 | 30-06-2011 |
| | | UY 31294 A1 | 31-03-2009 |
| | | WO 2009024819 A1 | 26-02-2009 |
| WO 2021007378 A1 | 14-01-2021 | NONE | |
| WO 2021173593 A1 | 02-09-2021 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5262564 A


### Non-patent literature cited in the description


- **ZURLO E et al.** *PLOS ONE,* 2021, vol. 16 (1), e0245548
- **FODALE V et al.** *PLoS One,* 2014, vol. 9 (12), e112262
- **CUI X et al.** *Sci Rep,* 2014, vol. 4, 5601
- **BENNETT, M.J. et al.** *Proc. Natl. Acad. Sci. USA,* 2002, vol. 99, 11634-11639
- **DI, L. et al.** *Eur. J. Med. Chem.,* March 2003, vol. 38 (3), 223-232
- **RUEDEN, C.T. et al.** *BMC Bioinformatics,* 29 November 2017, vol. 18 (1), 529
- **SCHINDELIN, J et al.** *Nature methods,* 28 June 2012, vol. 9 (7), 676-82
- **BEAUMONT, V. et al.** *Neuron,* 21 December 2016, vol. 92 (6), 1220-1237
- **JIANG, H. et al.** *Neurobiol Dis,* September 2006, vol. 23 (3), 543-551
- **LA SPADA, A.R. et al.** *Nat Rev Genet,* April 2011, vol. 11 (4), 247-2589
- **CONG, S.Y. et al.** *Mol Cell Neurosci,* December 2005, vol. 30 (4), 560-571
- **LEE, J et al.** *Acta Neuropathol,* November 2017, vol. 13 (5), 729-748
- **STEFFAN, J.S. et al.** *Nature,* 18 October 2001, vol. 413 (6857), 739-743
- **NUCIFORA, F.C. et al.** *Science,* 23 March 2001, vol. 291 (5512), 2423-2428
- **BRAVO-ARREDONDO, J.M. et al.** *J Biol Chem.,* 21 December 2018, vol. 293 (51), 19613-19623
- **FASS, E. et al.** *J Biol Chem,* 24 November 2006, vol. 281 (47), 36303-16
- **KLIONSKY, D.J. et al.** *Autophagy,* November 2012, vol. 7 (11), 1273-94
- **TANIDA, I. et al.** *Autophagy,* June 2005, vol. 273 (11), 2553-62
- **WALTER, C. et al.** *Neuropharmacology,* September 2016, vol. 108, 24-38
- **REINUS, R.** *Front Behav Neurosci,* 27 March 2015, vol. 9, 71
- **SHAHANI, N. et al.** *Sci Signal,* 15 November 2016, vol. 9 (454), ra111
- **OSIPOVITCH, M. et al.** *Cell Stem Cell,* 03 January 2019, vol. 24 (1), 107-122
- **LI, S.H. et al.** *Mol Cell Biol,* March 2002, vol. 22 (5), 1277-87
- **KOSTKA et al.** *THE JOURNAL OF BIOLOGICAL CHEMISTRY,* 18 April 2008, vol. 283 (16), 10992-11003
- **ITKIN et al.** *PLoS ONE,* 2011, vol. 6 (3), e18250
- **ZHANG-HAAGEN et al.** *PLoS ONE,* 2016, vol. 11 (2), e0150267
- **ABEL O ; POWELL JF ; ANDERSEN PM ; AL-CHALABI A.** ALSod: a user- friendly online bioinformatics tool for amyotrophic lateral sclerosis genetics. *Human Mutation,* 2012, vol. 33, 1345-1351
- **ACHARYA, M. M. ; PATEL, N. H. ; CRAVER, B. M. ; TRAN, K. K. ; GIEDZINSKI, E. ; TSENG, B. P. et al.** Consequences of low dose ionizing radiation exposure on the hippocampal microenvironment. *PLoS One,* 2015, vol. 10, e0128316
- **ACHARYA MM ; BAULCH JE ; KLEIN PM ; BADDOUR AAD ; APODACA LA ; KRAMAR EA ; ALIKHANI L ; GARCIA C JR ; ANGULO MC ; BATRA RS.** New Concerns for Neurocognitive Function during Deep Space Exposures to Chronic, Low Dose-Rate, Neutron Radiation. *eNeuro,* 22 August 2019, vol. 6 (4), ENEURO.0094-19
- **BARTELS, T. ; CHOI, J. G. ; SELKOE, D. J.** Alpha-synuclein occurs physiologically as a helically folded tetramer that resists aggregation. *Nature,* 2011, vol. 477, 107-110
- **BRUIJN LI ; HOUSEWEART MK ; KATO S ; ANDERSON KL ; ANDERSON SD ; OHAMA E et al.** Aggregation and motor neuron toxicity of an ALS-linked SOD1 mutant independent from wild-type SOD1. *Science,* 1998, vol. 281, 1851-4
- **BURRE, J. ; SHARMA, M. ; SUDHOF, T. C.** Alpha-synuclein assembles into higher-order multimers upon membrane binding to promote SNARE complex formation. *Proc. Natl Acad. Sci. USA,* 2014, vol. 111, E4274-4283

- **CARNIO S ; LOVERSO F ; BARAIBAR MA ; LONGA E ; KHAN MM ; MAFFEI M ; REISCHL M ; CANEPARI M ; LOEFLER S ; KERN H.** Autophagy impairment in muscle induces neuromuscular junction degeneration and precocious aging. *Cell Rep,* 11 September 2014, vol. 8 (5), 1509-21
- **CHAN, H.Y.E. ; WARRICK, J.M. ; GRAY-BOARD, G.L. ; PAULSON, H.L. ; BONINI, N.M.** Mechanisms of chaperone suppression of poly- glutamine disease: selectivity, synergy and modulation of protein solubility in Drosophila. *Hum. Mol. Genet.,* 2000, vol. 9, 2811-2820
- **CHHATWAL JP ; SCHULTZ AP ; DANG Y ; OSTASZEWSKI B ; LIU L ; YANG HS ; JOHNSON KA ; SPERLING RA ; SELKOE DJ.** Plasma N-terminal tau fragment levels predict future cognitive decline and neurodegeneration in healthy elderly individuals. *Nat Commun,* 27 November 2020, vol. 11 (1), 6024
- **CHAUHAN A ; SHARMA U ; JAGANNATHAN NR ; REETA KH ; GUPTA YK.** Rapamycin protects against middle cerebral artery occlusion induced focal cerebral ischemia in rats. *Behav Brain Res,* 01 December 2011, vol. 225 (2), 603-9
- **CHEN, Z. et al.** Learnings about the complexity of extracellular tau aid development of a blood-based screen for Alzheimer's disease. *Alzheimers Dement,* 2019, vol. 15, 487-496
- **CHIEN HM ; HE RY ; LEE CC ; HUANG YA et al.** Nanoscopic investigation of C9orf72 poly-GA oligomers on nuclear membrane disruption by a photoinducible platform. *COMMUNICATIONS CHEMISTRY,* 2021, vol. 4, 111
- **CUERVO, A. M. ; DICE, J. F.** Age-related decline in chaperone-mediated autophagy. *J. Biol. Chem.,* 2000, vol. 275, 31505-31513
- **DAVIES SW ; TURMAINE M ; COZENS BA ; DIFIGLIA M ; SHARP AH ; ROSS CA ; SCHERZINGER E ; WANKER EE ; MANGIARINI L ; BATES GP.** Formation of Neuronal Intranuclear Inclusions Underlies the Neurological Dysfunction. *Mice Transgenic for the HD Mutation Cell,* 08 August 1997, vol. 90 (3), 537-48
- **DENG H ; GAO K ; JANKOVIC J.** The role of FUS gene variants in neurodegenerative diseases. *Nat Rev Neurol,* 2014, vol. 10, 337-48
- **DUGGER BN ; DICKSON DW.** Pathology of Neurodegenerative Diseases. *Cold Spring Harb Perspect Biol,* 2017, vol. 9 (7), a028035
- **ESPARZA TJ ; ZHAO H ; CIRRITO JR ; CAIRNS NJ ; BATEMAN RJ ; HOLTZMAN DM ; BRODY DL.** Amyloid-beta oligomerization in Alzheimer dementia versus high- pathology controls. *Ann Neurol,* 2013, vol. 73, 104-119
- **FUSCO, G. et al.** Structural basis of membrane disruption and cellular toxicity byalpha-synuclein oligomers. *Science,* 2017, vol. 358, 1440-1443
- **GARAI K ; SUREKA R ; MAITI S.** Detecting amyloid-beta aggregation with fiber-based fluorescence correlation spectroscopy. *Biophys J,* 2007, vol. 92, L55-7
- **GASSEN NC ; PAPIES J ; BAJAJ T et al.** SARS-CoV-2-mediated dysregulation of metabolism and autophagy uncovers host-targeting antivirals. *Nature Communications,* 2021, vol. 12
- **GITLER AD ; DHILLON P ; SHORTER J.** Neurodegenerative disease: models, mechanisms, and a new hope. *Dis Model Mech,* 2017, vol. 10 (5), 499-502
- **GUO L ; SALT TE ; LUONG V ; WOOD N ; CHEUNG W ; MAASS A ; FERRARI G ; RUSSO-MARIE F ; SILLITO AM ; CHEETHAM ME.** Targeting amyloid-beta in glaucoma treatment. *Proc Natl Acad Sci USA,* 14 August 2007, vol. 104 (33), 13444-9
- **HAATAJA, L. ; GURLO, T. ; HUANG, C.J. ; BUTLER, P.C.** Islet amyloid in type 2 diabetes, and the toxic oligomer hypothesis. *Endocr. Rev.,* 2008, vol. 29, 303-316
- **HARTL, F. U.** *Nature (London),* 1996, vol. 381, 571-579
- **HEI, C. ; LIU, P. ; YANG, X. ; NIU, J. ; LI, P. A.** Inhibition of mTOR signaling confers protection against cerebral ischemic injury in acute hyperglycemic rats. *Int. J. Biol. Sci.,* 2017, vol. 13, 878-887
- **IHSE, E. ; SUHR, O. B. ; HELLMAN, U. ; WESTERMARK, P.** Variation in amount of wild-type transthyretin in different fibril and tissue types in ATTR amyloidosis. *J. Mol. Med. (Berl.),* 2011, vol. 89, 171-180
- **JAYARAMAN S ; GANTZ DL ; HAUPT C ; GURSKY O.** Serum amyloid A forms stable oligomers that disrupt vesicles at lysosomal pH and contribute to the pathogenesis of reactive amyloidosis. *Proc Natl Acad Sci U S A.,* 08 August 2017, vol. 114 (32), E6507-E6515
- **JIN M ; SHEPARDSON N ; YANG T ; CHEN G ; WALSH D ; SELKOE DJ.** Soluble amyloid beta-protein dimers isolated from Alzheimer cortex directly induce Tau hyperphosphorylation and neuritic degeneration. *Proc Natl Acad Sci USA,* 2011, vol. 108, 5819-5824
- **JOHNSON, J. L. ; CRAIG, E. A.** *Cell,* 1997, vol. 90, 201-204
- *ACS Nano.,* 27 November 2018, vol. 12 (11), 10855-10866
- **KITAMURA A ; KUBOTA H.** Amyloid oligomers: dynamics and toxicity in the cytosol and nucleus. *FEBS J,* March 2010, vol. 277 (6), 1369-79
- **KONARKOWSKA B ; AITKEN JF ; KISTLER J ; ZHANG S ; COOPER GJ.** The aggregation potential of human amylin determines its cytotoxicity towards islet β-cells. *FEBS J,* 2006, vol. 273, 3614-3624

- **LADIWALA AR ; LIN JC ; BALE SS ; MARCELINO-CRUZ AM ; BHATTACHARYA M ; DORDICK JS ; TESSIER PM.** Resveratrol Selectively Remodels Soluble Oligomers and Fibrils of Amyloid Aβ into Off-pathway Conformers. *J Biol Chem,* 30 July 2010, vol. 285 (31), 24228-3
- **LIU C ; GAO Y ; BARRETT J ; HU B.** Autophagy and protein aggregation after brain ischemia. *J Neurochem,* 2010, vol. 115, 68-78
- **MAEDA S ; TAKASHIMA A.** Tau Oligomers. *Adv Exp Med Biol,* 2019, vol. 1184, 373-380
- **MCKINNON SJ ; LEHMAN DM ; KERRIGAN-BAUMRIND LA ; MERGES CA ; PEASE ME ; KERRIGAN DF ; RANSOM NL ; TAHZIB NG ; REITSAMER HA ; LEVKOVITCH-VERBIN H.** Caspase Activation and Amyloid Precursor Protein Cleavage in Rat Ocular Hypertension. *Investigative Ophthalmology & Visual Science,* April 2002, vol. 43, 1077-1087
- **MIAO et al.** *Developmental Cell,* 2021, vol. 56, 427-442
- **MILLUCCI L ; PACCAGNINI E ; GHEZZI L ; BERNARDINI G ; BRACONI D ; LASCHI M ; CONSUMI M ; SPREAFICO A ; TANGANELLI P ; LUPETTI P.** Different Factors Affecting Human ANP Amyloid Aggregation and Their Implications in Congestive Heart Failure. *PLoS One,* 2011, vol. 6 (7), e21870
- **MIYAZAKI Y ; KAIKITA K ; ENDO M ; HORIO E ; MIURA M ; TSUJITA K ; HOKIMOTO S ; YAMAMURO M ; IWAWAKI T ; GOTOH T.** C/EBP homologous protein deficiency attenuates myocardial reperfusion injury by inhibiting myocardial apoptosis and inflammation. *Arterioscler Thromb Vasc Biol,* May 2011, vol. 31 (5), 1124-32
- **MIZUSHIMA, N. ; LEVINE, B.** Autophagy in mammalian development and differentiation. *Nat. Cell Biol.,* 2010, vol. 9, 823-830
- **MUCHOWSKI, P.J. ; SCHAFFAR, G. ; SITTLER, A. ; WANKER, E.E. ; HAYER-HARTL, M.K. ; HARTL, F.U.** Hsp70 and Hsp40 chaperones can inhibit self-assembly of polyglutamine proteins into amyloid-like fibrils. *Proc. Natl Acad. Sci. USA,* 2000, vol. 97, 7841-7846
- **NACHARAJU P ; LEWIS J ; EASSON C ; YEN S ; HACKETT J ; HUTTON M ; YEN S-H.** Accelerated filament formation from tau protein with specific FTDP-17 missense mutations. *FEBS Lett,* 1999, vol. 447 (2-3), 195-9
- **OLSHINA MA ; ANGLEY LM ; RAMDZAN YM ; TANG J ; BAILEY MF ; HILL AF et al.** Tracking mutant huntingtin aggregation kinetics in cells reveals three major populations that include an invariant oligomer pool. *J Biol Chem.,* 2010, vol. 285, 21807-16
- **OPHERK C ; DUERING M ; PETERS N ; KARPINSKA A ; ROSNER S ; SCHNEIDER E ; BADER B ; GIESE A ; DICHGANS M.** CADASIL mutations enhance spontaneous multimerization of NOTCH3. *Hum Mol Genet,* 01 August 2009, vol. 18 (15), 2761-7
- **PAGLIN S ; LEE N-Y ; NAKAR C ; FITZGERALD M ; PLOTKIN J ; DEUEL B et al.** Rapamycin- sensitive pathway regulates mitochondrial membrane potential, autophagy, and survival in irradiated MCF-7 cells. *Cancer Res,* 2005, vol. 65, 11061-70
- **PALSDOTTIR A ; SNORRADOTTIR AO ; THORSTEINSSON L.** Hereditary cystatin C amyloid angiopathy: genetic, clinical, and pathological aspects. *Brain Pathol,* January 2006, vol. 16 (1), 55-9
- **PLAZA-ZABALA A ; SIERRA-TORRE V ; SIERRA A.** Autophagy and microglia: Novel partners in neurodegeneration and ageing. *Int J Mol Sci,* 2017, vol. 18, 598
- **SANGWAN S ; ZHAO A ; ADAMS KL ; JAYSON CK ; SAWAYA MR ; GUENTHER EL ; PAN AC ; NGO J ; MOORE DM ; SORIAGA AB.** Atomic structure of a toxic, oligomeric segment of SOD1 linked to amyotrophic lateral sclerosis (ALS). *Proc Natl Acad Sci U S A,* 15 August 2017, vol. 114 (33), 8770-8775
- **SHIBU M. POULOSE ; DONNA F. BIELINSKI ; KIRSTY CARRIHILL-KNOLL ; BERNARD M. RABIN ; BARBARA SHUKITT-HALE.** Exposure to 16O-Particle Radiation Causes Aging-Like Decrements in Rats through Increased Oxidative Stress. *Inflammation and Loss of Autophagy Radiation Research,* 2011, vol. 176 (6), 761-769
- **SVETONI F ; FRISONE P ; PARONETTO MP.** Role of FET proteins in neurodegenerative disorders. *RNA Biol,* November 2016, vol. 13 (11), 1089-1102
- **TALBOT K ; WANG HY ; KAZI H ; HAN LY ; BAKSHI KP ; STUCKY A et al.** Demonstrated brain insulin resistance in Alzheimer's disease patients is associated with IGF-1 resistance, IRS-1 dysregulation, and cognitive decline. *J Clin Invest,* 2012, vol. 122, 1316-38
- **PYO JO et al.** Overexpression of Atg5 in mice activates autophagy and extends lifespan. *Nat. Commun,* 2013, vol. 4, 2300
- **RODRIGUEZ JA ; SHAW BF ; DURAZO A ; SOHN SH ; DOUCETTE PA ; NERSISSIAN AM et al.** Destabilization of apoprotein is insufficient to explain Cu, Zn-superoxide dismutase-linked ALS pathogenesis. *Proc Natl Acad Sci USA.,* 2005, vol. 102, 10516-21

- **RODRIGUEZ-RODRIGUEZ P ; SANDE-BRING-MATTON A ; MERINO-SERRAIS P ; PAR-RADO-FERNANDEZ C ; RABANO A ; WINBLAD B ; ÁVILA J ; FERRER I ; CEDAZO-MINGUEZ A.** Tau hyperphosphorylation induces oligomeric insulin accumulation and insulin resistance in neurons. *Brain,* 01 December 2017, vol. 140 (12), 3269-3285
- **ROHER, A. E. ; CHANEY, M. O. ; KUO, Y. M. ; WEB-STER, S. D. ; STINE, W. B. ; HAVERKAMP, L. J. ; WOODS, A. S. ; COTTER, R. J. ; TUOHY, J. M. ; KRAFFT, G. A.** Morphology and toxicity of Abeta-(1-42) dimer derived from neuritic and vascular amyloid deposits of Alzheimer's disease. *J. Biol. Chem.,* 1996, vol. 271, 20631-20635
- **SENGUPTA U ; MONTALBANO M ; MCALLEN S ; MINUESA G ; KHARAS M ; KAYED R.** Formation of Toxic Oligomeric Assemblies of RNA-binding Protein: Musashi in Alzheimer's disease. *Acta Neuropathol Commun.,* 26 October 2018, vol. 6 (1), 113
- **SHANKAR GM ; LI S ; MEHTA TH ; GARCIA-MUNOZ A ; SHEPARDSON NE ; SMITH I ; BRETT FM ; FARRELL MA ; ROWAN MJ ; LEMERE CA et al.** Amyloid-beta protein dimers isolated directly from Alzheimer's brains impair synaptic plasticity and memory. *Nat Med,* 2008, vol. 14, 837-842
- **SIMONEAU S ; REZAEI H ; SALE'S N ; KAI-SER-SCHULZ G ; LEFEBVRE-ROQUE M et al.** In vitro and in vivo neurotoxicity of prion protein oligomers. *PLoS Pathog,* 2007, vol. 3 (8), e125
- **SOEDA Y ; YOSHIKAWA M ; ALMEIDA OFX ; SUMIOKA A ; MAEDA S ; OSADA H et al.** Toxic tau oligomer formation blocked by capping of cysteine residues with 1,2-dihy- droxybenzene groups. *Nat Commun,* 2015, vol. 6, 1-12
- **TAM, S. et al.** The chaperonin TRiC blocks a huntingtin sequence element that promotes the conformational switch to aggregation. *Nat Struct Mol Biol,* 2009, vol. 16, 1279-1285
- **TOKUDA E ; ANZAI I ; NOMURA T ; TOICHI K ; WATANABE M ; OHARA S ; WATANABE S ; YA-MANAKA K ; MORISAKI Y ; MISAWA H.** Immunochemical characterization on pathological oligomers of mutant Cu/Zn- superoxide dismutase in amyotrophic lateral sclerosis. *Mol Neurodegener,* 05 January 2017, vol. 12 (1), 2
- **VIRET C ; ROZIÈRES A ; FAURE M.** Autophagy during early virus-host cell interactions. *J Mol Biol,* 08 June 2018, vol. 430 (12), 1696-1713
- **WANG W ; KANG J ; LI H ; SU J ; WU J ; XU Y ; YU H ; XIANG X ; YI H ; LU Y.** Regulation of endoplasmic reticulum stress in rat cortex by p62/ZIP through the Keap1-Nrf2-ARE signalling pathway after transient focal cerebral ischaemia. *Brain Inj,* 2013, vol. 27, 924-933
- **WESTERMARK P ; ENGSTROM U ; JOHNSON KH ; WESTERMARK GT ; BETSHOLTZ C.** Islet amyloid polypeptide: pinpointing amino acid residues linked to amyloid fibril formation. *Proc Natl Acad Sci USA,* 1990, vol. 87, 5036-5040
- **WITTMANN CW ; WSZOLEK MF ; SHULMAN JM ; SALVATERRA PM ; LEWIS J ; HUTTON M et al.** Tauopathy in Drosophila: neurodegeneration without neurofibril- lary tangles. *Science,* 2001, vol. 293 (5530), 711-4
- **XIAO S ; SANELLI T ; CHIANG H ; SUN Y ; CHAKRABARTTY A ; KEITH J ; ROGAEVA E ; ZINMAN L ; ROBERTSON J.** Low molecular weight species of TDP-43 generated by abnormal splicing form inclusions in amyotrophic lateral sclerosis and result in motor neuron death. *Acta Neuropathol,* 2015, vol. 130, 49-61
- **YELLON DM ; HAUSENLOY DJ.** Myocardial reperfusion injury. *N Engl J Med,* 13 September 2007, vol. 357 (11), 1121-35
- **YOUNGER S ; JANG H ; DAVIES HA ; NIEMIEC MJ ; GARCIA JGN ; NUSSINOV R ; MIGRINO RQ ; MADINE J ; ARCE FT.** Medin Oligomer Membrane Pore Formation: A Potential Mechanism of Vascular Dysfunction. *Biophys J,* 02 June 2020, vol. 118 (11), 2769-2782